(19) Europäisches Patentamt / European Patent Office / Office européen des brevets

(11) EP 4 781 984 A1

(12) **EUROPEAN PATENT APPLICATION**
published in accordance with Art. 153(4) EPC

(43) Date of publication:
**29.07.2026 Bulletin 2026/31**

(21) Application number: **24878960.4**

(22) Date of filing: **14.10.2024**

(51) International Patent Classification (IPC):
***A61K 31/416*** (2006.01) ***C07D 413/14*** (2006.01)
***C07D 401/06*** (2006.01) ***C07D 401/12*** (2006.01)
***C07D 401/14*** (2006.01) ***C07D 403/12*** (2006.01)
***C07D 403/14*** (2006.01) ***C07D 487/04*** (2006.01)
***C07D 487/06*** (2006.01) ***A61K 9/08*** (2006.01)
***A61K 47/34*** (2017.01) ***A61K 47/40*** (2006.01)
***A61K 47/00*** (2006.01) ***A61P 27/02*** (2006.01)
***A61P 27/06*** (2006.01) ***A61P 35/00*** (2006.01)

(52) Cooperative Patent Classification (CPC):
**A61K 9/08; A61K 31/416; A61K 47/00; A61K 47/34; A61K 47/40; A61P 27/02; A61P 27/06; A61P 35/00; C07D 401/06; C07D 401/12; C07D 401/14; C07D 403/12; C07D 403/14; C07D 413/14; C07D 487/04;** (Cont.)

(86) International application number:
**PCT/CN2024/124727**

(87) International publication number:
**WO 2025/082316 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.10.2023 CN 202311343653**

(71) Applicant: **Beyang Therapeutics Co., Ltd.**
**Suzhou, Jiangsu 215127 (CN)**

(72) Inventors:
- **MIAO, Zhenyu**
  **Suzhou, Jiangsu 215127 (CN)**
- **HU, Qiyue**
  **Suzhou, Jiangsu 215127 (CN)**
- **GUKASYAN, Hovhannes John**
  **Suzhou, Jiangsu 215127 (CN)**

(74) Representative: **HGF**
**HGF Limited**
**4th Floor, 1 City Square**
**Leeds LS1 2ES (GB)**

(54) **OPHTHALMIC FORMATION, AND PREPARATION METHOD THEREFOR AND USE THEREOF**

(57) The present invention discloses an ophthalmic formulation, and a preparation method and use thereof. The ophthalmic formulation comprises a protein tyrosine kinase inhibitor compound. The protein tyrosine kinase inhibitor is used for treating protein tyrosine kinase-mediated proliferative diseases or conditions, such as ocular diseases (e.g., those accompanied by pathological neovascularization, retinal ischemia, retinal edema, and diabetic retinopathy) and malignant tumors. The formulation can improve the solubility of the compound, achieving a clear or near-clear solution state. The formulation has the potential to prolong the residence time of the compound on the ocular surface, increase its exposure to fundus tissues, and enhance bioavailability, thereby demonstrating excellent application prospects.

EP 4 781 984 A1

(52) Cooperative Patent Classification (CPC): (Cont.)
**C07D 487/06**

**Description**

TECHNICAL FIELD

The present invention relates to the field of pharmaceuticals, and specifically relates to a protein tyrosine kinase inhibitor, use thereof in treating a protein tyrosine kinase-mediated proliferative disease or condition, and an ophthalmic formulation comprising the protein tyrosine kinase inhibitor as well as a preparation method and use thereof.

BACKGROUND ART

Age-related macular degeneration (AMD) and diabetic retinopathy (DR) are the most common ocular diseases and are the leading causes of blindness in the elderly and working-age populations. These pathologies are associated with neovascularization and exudation in the posterior segment of the eye. In particular, a hallmark of wet AMD is the formation of new vessels from the choroidal microvascular bed invading the subretinal space, while DR preferentially presents alterations of exudation and neovascularization at the retinal level. Both AMD and DR are characterized by endothelial cell (EC) proliferation and migration, increased vascular permeability, and inflammation, in which vascular endothelial growth factor-A (VEGF-A) and its corresponding receptors (VEGFR) play a key role. In addition, tumor growth and metastasis rely on the tumor vascular network to provide adequate oxygen and nutrients. Tumor angiogenesis depends on a highly complex program involving growth factor signaling, endothelial cell proliferation, extracellular matrix (ECM) remodeling, and stromal cell interactions. Among the most important pro-angiogenic drivers is vascular endothelial growth factor (VEGF). It has been shown that many proliferative disorders, such as ophthalmic diseases, tumors, and cancers, involve overexpression or upregulation of receptor tyrosine kinase (RTK) activity. Receptor tyrosine kinases are kinase enzymes that modify proteins by chemically adding phosphate groups (phosphorylation). Phosphorylation often leads to functional changes in the target protein by altering enzyme activity, cellular localization, or association with other proteins. Kinases are known to regulate most cellular pathways, especially those involving signal transduction. To date, one approach to inhibiting the VEGF pathway is to inhibit receptor tyrosine kinase (RTK) activity. In treating ocular diseases such as age-related macular degeneration (AMD) and diabetic retinopathy (DR), the goal of protein tyrosine kinase inhibitor therapy is to counteract pathological neovascularization and disease progression, thereby preventing visual impairment. Meanwhile, the importance of VEGFR as a pro-angiogenic inducer in tumor growth, invasion, and exudation makes it an excellent therapeutic target for various cancers. To date, the U.S. FDA has not approved small molecule tyrosine kinase inhibitors for the treatment of neovascular age-related macular degeneration and diabetic retinopathy.

SUMMARY OF THE INVENTION

The present invention provides a compound for treating protein tyrosine kinase-mediated proliferative diseases or conditions, with the target being, for example, ocular diseases such as those accompanied by pathological neovascularization, retinal ischemia, retinal edema, and diabetic retinopathy, as well as malignant tumors. The clinical success of VEGF antibody drugs (pegaptanib, ranibizumab, aflibercept, and brolucizumab) has validated this concept. For example, the U.S. Food and Drug Administration (FDA) approved aflibercept (VEGF Trap-Eye) for the treatment of neovascular age-related macular degeneration. The same molecular structure, ziv-aflibercept, was also approved by the FDA for systemic use in combination therapy for patients with metastatic colorectal cancer. However, these VEGF antibody drugs require multiple intravitreal injections administered by retinal specialists. For clinicians and patients, frequent intravitreal injections are inconvenient and carry rare but serious injection-related risks (endophthalmitis, retinal detachment, cataract, intraocular inflammation, etc.), leading to many patients not receiving injections on schedule, resulting in suboptimal efficacy. The favorable clinical outcomes of VEGF antibody biologics, coupled with the challenges associated with intravitreal injections, have led to efforts to develop small molecule anti-angiogenic agents as therapies for neovascular age-related macular degeneration, particularly VEGFR tyrosine kinase inhibitors, for ocular administration. Compared to recombinant proteins, small molecule VEGFR tyrosine kinase inhibitors offer many advantages. They can be formulated as eye drops to avoid intravitreal injections, can cross cell membranes and directly interact with the cytoplasmic domain of receptor tyrosine kinases (RTKs), and small molecule eye drops are economically cost-effective. However, to date, no small molecule tyrosine kinase inhibitor for the treatment of neovascular age-related macular degeneration and diabetic retinopathy has been approved for marketing by the U.S. FDA.

Vascular endothelial growth factor (VEGF) and its receptors (VEGFRs) are known as the most potent vascular permeabilizing agents and endothelial cell-specific mitogens, playing a key role in the proliferation, migration, and angiogenesis of endothelial cells. Angiogenesis is an important mechanism in many physiological and pathological processes, involving the proliferation, migration, and survival of endothelial cells, leading to further tubule formation and ultimately promoting blood vessel formation. Vascular endothelial growth factor (VEGF) and its receptors (VEGFRs) play a significant role in angiogenesis associated with pathologies such as tumor development and ocular neovascular diseases.

For example, VEGF expression levels show a significant positive correlation with the degree of tumor tissue vascularization. VEGF acts on the VEGFR2 receptor, activating the phosphorylation of VEGFR receptor tyrosine kinases and leading to abnormal cellular signal transduction, thereby promoting endothelial cell proliferation and new blood vessel formation. It is a major player in many different cancers and ocular diseases accompanied by pathological neovascularization. The compound of the present invention (e.g., prepared as in Example 1) possesses inhibitory activity against anti-angiogenic tyrosine kinases, effectively antagonizing all VEGFR receptor (VEGFR1, VEGFR2, VEGFR3) tyrosine kinase activity while exhibiting high selectivity against EGFR inhibition (Example 2).

VEGFR2 is the primary receptor for VEGF-induced endothelial cell signaling. During development and/or after tissue injury, upon ligand VEGF binding, VEGFR2 undergoes autophosphorylation and activation, inducing blood vessel formation and bypassing blocked vessels. The compound of the present invention exhibits significant inhibitory activity against VEGF-induced VEGFR2 autophosphorylation (pVEGFR2) in human endothelial cells, blocking abnormal cellular signal transduction and thereby inhibiting new blood vessel formation (Example 3). A major function derived from VEGFR receptor signaling is to promote endothelial cell proliferation and new blood vessel formation. The compound of the present invention demonstrates inhibitory activity against VEGF-induced human endothelial cell proliferation at nanomolar concentration levels. In summary, the compound of the present invention is a novel tyrosine kinase inhibitor. This novel tyrosine kinase inhibitor can be applied not only to the treatment of neovascular age-related macular degeneration and diabetic retinopathy but also to tumor indication therapies. By blocking tumor neovascularization, it cuts off the blood and nutrient supply required for tumor growth, leading to tumor cell death.

One of the goals of medicinal chemistry is to improve the bioavailability and stability of compounds, thereby enhancing efficacy. Bioavailability represents the rate and extent to which a therapeutic agent is absorbed from its dosage form and becomes available at the site of action. Existing VEGF tyrosine kinase inhibitors suffer from problems such as low solubility and/or insufficient kinase inhibitory activity, which greatly affect the bioavailability of such compounds, thereby reducing efficacy. Formulating compounds with low solubility as eye drops is particularly challenging. For example, although Axitinib has good inhibitory activity against VEGF tyrosine kinases (VEGFR1, VEGFR2, and VEGFR3), its solubility is low (<2 $\mu$g/mL). The present invention provides a compound that can possess significant solubility, improved advantages, and/or excellent kinase inhibitory activity (Example 4). For example, the thermodynamic solubility is 10 $\mu$g/mL or above, or the thermodynamic solubility is 100 $\mu$g/mL or above, or the thermodynamic solubility is 1000 $\mu$g/mL or above. For instance, the $IC_{50}$ for VEGFR2 is 100 nM or below, or the $IC_{50}$ for VEGFR2 is 10 nM or below, or the $IC_{50}$ for VEGFR2 is 1 nM or below.

Ocular melanin-containing cells are located in the retinal pigment epithelium and choroid in the posterior part of the eye, and in the ciliary body and iris in the anterior part of the eye. Binding of compounds to melanin may affect ocular pharmacokinetics after local administration. The present invention provides an eye drop formulation containing a compound intended for the treatment of age-related macular degeneration and diabetic retinopathy. These are diseases in the posterior segment of the eye. The eye drop formulation containing the compound aims for effective delivery to the posterior ocular tissues. In these cases, many drugs may bind tightly to melanin tissues in the posterior part of the eye (retinal pigment epithelium, choroid) or the anterior part of the eye (ciliary body, iris). Many clinical drugs bind to melanin, thereby affecting their ocular pharmacokinetics. The binding rate of a compound to melanin is an important factor in ocular pharmacokinetics and pharmacodynamics, primarily related to the tissue distribution of the compound within the eyeball. This must be considered in drug discovery and development. The present invention provides information on the effect of melanin on the compound (Example 5). One of the key challenges in developing small molecule tyrosine kinase inhibitors for clinical use in age-related macular degeneration and diabetic retinopathy is overcoming the risk of "on-target" toxicity. Inhibiting VEGFR in the healthy vasculature has the potential to cause serious adverse events, such as hypertension, hemorrhage, and thrombosis. Despite many clinical successes in tumor indications, the safety profile of oral VEGFR-2 inhibitors may be a major reason limiting their clinical use and/or development in patients with age-related macular degeneration and diabetic retinopathy. Theoretically, topical eye drops could provide an effective therapy that limits systemic exposure and avoids on-target toxicity issues.

Although topical ocular administration has proven to be a successful strategy for treating diseases associated with the anterior part of the eye (e.g., glaucoma), there are currently no FDA-approved topical therapies for ocular diseases associated with posterior ocular tissues (e.g., retina and choroid), such as neovascular AMD and diabetic retinopathy. This is largely due to the anatomical and physiological barriers of the human eye, which have evolved to protect the eye from exogenous substances. The tear film is one of the first barriers to overcome. Compounds in the anterior part of the eye can be rapidly washed away by the tear film, leading to nasolacrimal drainage. Therefore, compounds may need to be absorbed quickly after topical instillation. However, absorption/permeation into ocular tissues can also be challenging. One absorption pathway involves permeation through the cornea. The cornea consists of an epithelium containing tight junctions and alternating lipophilic and hydrophilic layers. Another absorption pathway is permeation into the conjunctiva, followed by diffusion into the sclera. The sclera is a relatively more permeable ocular tissue. However, drugs entering the conjunctiva tend to be "lost" to the systemic circulation due to the highly vascularized nature of this tissue. Compounds exposed in the sclera have the potential to diffuse into the choroid. The choroid is a primary target tissue for neovascular

AMD. Diffusion from the choroid to the retina (a secondary target tissue for neovascular AMD) is further attenuated by the blood-retinal barrier (BRB). The BRB functions similarly to the blood-brain barrier and can be a formidable barrier to compound diffusion. Due to these anatomical and physiological barriers, it is estimated that less than 5% of a topically administered dose reaches the posterior ocular tissues. Despite these challenges associated with topical administration, the present invention focuses on developing structure-activity relationships (SAR) related to ocular and blood exposure. Through the ophthalmic formulation containing the compound, effective delivery to the posterior ocular tissues (choroid and retina) can be achieved (Example 6). Due to the observed rapid degradation of the compound in plasma, it is beneficial for avoiding systemic target-related toxicity issues. In contrast, the exposure of Axitinib in the posterior ocular tissues is low, being below the lower limit of quantification in the retina. The present invention provides novel ophthalmic formulations containing the compound, which achieve maintaining sufficient drug concentrations in the posterior ocular segment (e.g., choroid and retina) to bind relevant receptors targeting the eye, increase posterior ocular segment bioavailability, and improve issues encountered in the ocular delivery of existing topical therapeutic agents.

A major limitation of ocular drops may be the need for high concentrations of the compound in the ophthalmic formulation to achieve therapeutically effective drug levels in the posterior ocular tissues, due to the limited permeability of many ocular drops across the corneal and conjunctival barriers. Depending on the compound (the molecule itself or its high concentration), the ophthalmic formulation may have side effects on the anterior ocular tissues (including the conjunctiva, cornea, and/or lens), leading to various ocular surface injuries, such as corneal epithelial defects and erosions. In particular, ocular side effects such as epithelial degeneration and defects, ulcers, corneal epithelial thinning, erosions, and/or corneal edema, keratitis have been clinically observed following treatment with EGFR antibody drugs. EGFR is a major factor in human corneal epithelial cell wound healing. Therefore, it is essential to select compounds for topical ophthalmic formulations that avoid inhibiting EGFR activity. In addition to effectively antagonizing VEGFR1, VEGFR2, and VEGFR3 tyrosine kinase activity, the compounds of the present invention also demonstrate high selectivity for EGFR tyrosine kinase inhibition (Example 2).

Age-related macular degeneration (AMD) is an ocular disease that causes vision loss due to macular pathology, the macula being an area of the retina that enables sharp and detailed vision. In wet AMD, choroidal vessels (choroidal neovascularization, CNV) sprout and expand into the sub-macular space, exuding fluid and blood. This leads to retinal edema, scar tissue formation, and irreversible damage to the macula. Diabetic retinopathy is a common complication of diabetes, with many structural and functional abnormalities of the retina associated with the development of diabetes. Almost all patients with diabetes for more than 15 years suffer from diabetic retinopathy. Diabetic retinopathy is divided into two stages based on its duration of development: the non-proliferative stage, also known as the early stage, characterized by vascular leakage; and the proliferative or advanced stage, where various growth factors induce retinal neovascularization. Based on the property of the compounds of the present invention as potent VEGF receptor inhibitors, the present invention uses several animal models of choroidal and retinal neovascular diseases to evaluate the in vivo efficacy of the compounds of the present invention. Upregulation of VEGF leads to abnormal growth and leakage of retinal blood vessels, thereby causing visual impairment. Intravitreal injection of VEGF in Dutch Belted rabbits causes transient retinal leakage, similar to human wet AMD and diabetic retinopathy. The compounds of the present invention can demonstrate efficacy in inhibiting retinal leakage in this model. The oxygen-induced retinopathy (OIR) mouse model has reproducible and quantifiable proliferative retinal neovascularization, and human vascular pathology (vitreal neovascularization) is very similar to the clusters formed in mouse OIR. The compounds of the present invention are used in the OIR model to evaluate in vivo efficacy against retinal vascular lesions. The laser-induced choroidal neovascularization (CNV) rat model applies photocoagulative green laser pulses to rat eyes to cause localized rupture of Bruch's membrane (Bruch's membrane is an extracellular matrix between the retina and choroid). Rupture of Bruch's membrane induces the production of local inflammatory factors and VEGF, leading to choroidal neovascularization (CNV). This model has confirmed CNV formation following laser injury in humans, monkeys, pigs, and rodents, and is a VEGF-dependent pathology. Streptozotocin (STZ)-induced diabetic rats are considered a useful preclinical animal model for studying the pathogenesis and treatment of human diabetic retinopathy. Significantly increased retinal vascular permeability, such as intraretinal hemorrhage and fluorescein leakage, was detected in all diabetic rat groups, representing pre-proliferative changes of diabetic retinopathy. However, a portion of them showed vitreal neovascularization attached to the retina and retinal folds, a prominent manifestation of severe and proliferative diabetic retinopathy, representing the proliferative stage of diabetic retinopathy. During the progression of diabetic retinopathy, the expression of the angiogenesis-related growth factor VEGF and its receptors VEGFR1 and VEGFR2 increases in the retinas of streptozotocin (STZ)-induced diabetic mice. The ophthalmic formulations containing the compound of the present invention are used in the STZ-induced diabetic mouse retinopathy rat model to evaluate in vivo efficacy against diabetic retinopathy, particularly in inhibiting vascular leakage and angiogenesis involved in the development of diabetic mouse retinopathy.

Based on the property of the compounds of the present invention as potent VEGF receptor inhibitors, the present invention relates to compounds useful for treating pathological conditions caused or exacerbated by ocular angiogenesis, neovascularization, and/or vascular leakage, for example in age-related macular degeneration (AMD), including neovascular (wet/exudative) AMD, dry AMD, and geographic atrophy; diabetic retinopathy (including proliferative diabetic

retinopathy, non-proliferative diabetic retinopathy, and diabetic macular edema); pathological choroidal neovascularization (CNV) and vascular leakage due to any mechanism, such as sickle cell disease, high myopia, trauma; traumatic choroidal rupture, ocular histoplasmosis, optic nerve drusen, angioid streaks, or some retinal dystrophies; pathological retinal neovascularization and vascular leakage due to any mechanism, such as sickle cell retinopathy, carotid cavernous fistula, Eales disease, ocular ischemic syndrome, hyperviscosity syndrome, familial exudative vitreoretinopathy, idiopathic obliterative arteriolitis, retinal vasculitis, birdshot retinochoroidopathy, sarcoidosis, or toxoplasmosis; uveitis; retinal vein occlusion (central or branch); ocular trauma; surgically induced neovascularization; surgically induced edema; ocular ischemia; cystoid macular edema; retinopathy of prematurity; sickle cell retinopathy; Coats' disease; and/or neovascular glaucoma.

Furthermore, the occurrence, development, and metastasis of many tumors, as well as the formation of tumor neovascularization, are closely related to the abnormal expression of tyrosine kinases. In particular, some tyrosine kinase receptors are abnormally expressed in solid tumor cells, among which the vascular endothelial growth factor receptor (VEGFR) is highly expressed in many tumor cells and tumor vascular endothelial cells. The vascular endothelial growth factor receptor (VEGFR) family is directly related to tumor occurrence, development, and the formation of tumor angiogenesis. In addition to ocular diseases accompanied by pathological neovascularization, the clinical success of VEGF antibody drugs in treating malignant tumor indications validates this concept. The present invention relates to compounds useful for treating solid tumors and leukemias, such as breast cancer, lung cancer (particularly non-small cell lung cancer), adenocarcinoma, colorectal cancer, kidney cancer, liver cancer, pancreatic cancer, ovarian cancer, prostate cancer, glioma, glioblastoma, myeloma, acute myeloid leukemia, agnogenic myeloid metaplasia, mesothelioma, myelodysplastic syndrome.The present invention relates to compounds for preventing metastatic spread of tumors and growth of micrometastases. According to an embodiment of the present invention, the compound is an anti-angiogenic kinase inhibitor. Examples of protein tyrosine kinase inhibitors that can be used to produce beneficial therapeutic results include receptor tyrosine kinase inhibitors, such as but not limited to VEGFR, Tie-2, and FGFR.

In one aspect, the present invention provides a compound represented by formula (I),

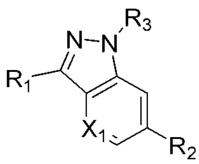
Formula (I),

wherein $R_1$ comprises a substituted cyclic structure;
$R_2$ comprises an optionally substituted substituent;
$R_3$ comprises an optionally substituted substituent;
$X_1$ is an optionally substituted atom;
or a pharmaceutically acceptable prodrug, a pharmaceutically active metabolite, and/or a pharmaceutically acceptable salt thereof.

In one aspect, the present invention provides the compound as shown in the present invention, or a pharmaceutically acceptable prodrug, a pharmaceutically active metabolite, and/or a pharmaceutically acceptable salt thereof.

In one aspect, the present invention provides a pharmaceutical composition comprising a compound of the present invention, or a pharmaceutically acceptable prodrug, a pharmaceutically active metabolite, and/or a pharmaceutically acceptable salt thereof, and optionally a carrier.

In one aspect, the present invention provides a use of the compound of the present invention, or a pharmaceutically acceptable prodrug, a pharmaceutically active metabolite, and/or a pharmaceutically acceptable salt thereof, and/or the pharmaceutical composition of the present invention, in the manufacture of a medicament for treating a protein tyrosine kinase-mediated disease.

In one aspect, the present invention provides the compound of the present invention, or a pharmaceutically acceptable prodrug, a pharmaceutically active metabolite, and/or a pharmaceutically acceptable salt thereof, and/or the pharmaceutical composition of the present invention, for use in treating a protein tyrosine kinase-mediated disease.

In one aspect, the present invention provides a method for treating a disease, comprising administering the compound of the present invention, or a pharmaceutically acceptable prodrug, a pharmaceutically active metabolite, and/or a pharmaceutically acceptable salt thereof, and/or the pharmaceutical composition of the present invention. In one aspect, the present invention provides a method for modulating kinase receptor activity, comprising administering the compound of the present invention, or a pharmaceutically acceptable prodrug, a pharmaceutically active metabolite, and/or a pharmaceutically acceptable salt thereof, and/or the pharmaceutical composition of the present invention.

In one aspect, the present invention provides an ophthalmic formulation I, comprising the compound of the

present invention or a pharmaceutically acceptable salt thereof, a co-solvent, and pharmaceutically acceptable excipients. In one aspect, the present invention provides a method for preparing an ophthalmic formulation I, comprising the steps of: (1) adding said compound or a pharmaceutically acceptable salt thereof to a co-solvent and dispersing; (2) adding the mixture obtained in step (1) to said pharmaceutically acceptable excipients and mixing.

In one aspect, the present invention provides an ophthalmic formulation II, the raw materials for preparing the ophthalmic formulation II comprise the compound of the present invention, pharmaceutically acceptable excipients, an acidic pH adjuster, and a basic pH adjuster.

In one aspect, the present invention provides a method for preparing an ophthalmic formulation II, comprising the steps of: (1) adding said compound to a solution containing a portion of pharmaceutically acceptable excipients and dispersing; (2) adding an acidic pH adjuster and mixing; (3) adding the remaining pharmaceutically acceptable excipients and mixing; (4) adding a basic pH adjuster.

In one aspect, the present invention provides a use of ophthalmic formulation I or II in the preparation of a medicament for preventing and/or treating an ocular disease.

Other aspects and advantages of the present invention will be readily apparent to those skilled in the art from the detailed description below. Only exemplary embodiments of the present invention are shown and described in the detailed description below. As those skilled in the art will recognize, the content of the present invention enables those skilled in the art to make modifications to the disclosed specific embodiments without departing from the spirit and scope of the invention involved. Accordingly, the descriptions in the specification of the present invention are merely exemplary and not restrictive.

## DETAILED DESCRIPTION OF THE PREFERRED EMBODIMENTS

The following specific examples illustrate the embodiments of the present invention. Those skilled in the art can easily understand other advantages and effects of the present invention from the content disclosed in this specification.

### Definition of Terms

In the present invention, the term "basic nitrogen atom" generally refers to a nitrogen atom having basicity. For example, a basic nitrogen atom may have the ability to provide an unshared electron pair. For example, a nitrogen atom having basicity is known in the art, for example, the conjugate acid of said basic nitrogen atom has a pKa higher than 3, or higher than 5. For example, a nitrogen atom not having basicity may be an N atom directly bonded to O, an N atom directly bonded to an aromatic ring, or an N atom directly bonded to a carbonyl group.

In the present invention, the term "methyl" generally refers to a residue derived by removal of a hydrogen atom from a group of one carbon atom. Methyl may be substituted or unsubstituted. The term "alkyl" generally refers to a saturated straight or branched aliphatic hydrocarbon group, which is a residue derived by removal of hydrogen atoms from the same or different carbon atoms of the parent alkane, and may be a straight or branched chain group containing 1 to 20 carbon atoms, for example, containing 1 to 12 carbon atoms, for example, containing 1 to 6 carbon atoms. Non-limiting examples of alkyl include, but are not limited to, methyl ($-CH_2-$), 1,1-ethyl ($-CH(CH_3)-$), 1,2-ethyl ($-CH_2CH_2-$), 1,1-propyl ($-CH(CH_2CH_3)-$), 1,2-propyl ($-CH_2CH(CH_3)-$), 1,3-propyl ($-CH_2CH_2CH_2-$), 1,4-butyl ($-CH_2CH_2CH_2CH_2-$) and 1,5-butyl ($-CH_2CH_2CH_2CH_2CH_2-$) and the like. Alkyl may be substituted or unsubstituted. For example, when substituted, the substituent may be substituted at any available point of attachment, said substituent preferably being independently optionally selected from one or more substituents selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, and oxo, for example, may be hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O)H, $-N(H)SO_2H$, or a $C_{1-6}$ aliphatic group.

In the present invention, the term "aryl" generally refers to a residue derived by removal of hydrogen atoms from the same or different carbon atoms of an aromatic ring. The term "aromatic ring" may refer to a 6- to 14-membered all-carbon monocyclic or fused polycyclic ring (i.e., rings sharing adjacent pairs of carbon atoms) having a conjugated $\pi$-electron system, and may be 6- to 10-membered, for example, benzene and naphthalene. The aromatic ring may be fused to a heteroaryl, heterocyclyl, or cycloalkyl ring, wherein the ring attached to the parent structure is an aryl ring. Aryl may be substituted or unsubstituted. When substituted, the substituent is preferably one or more groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, and heterocycloalkylthio.

In the present invention, the term "heteroaryl" generally refers to a residue derived by removal of hydrogen atoms from the same or different carbon atoms of a heteroaromatic ring. The term "heteroaromatic ring" refers to a heteroaromatic system containing 1 to 4 heteroatoms and 5 to 14 ring atoms, wherein the heteroatoms may be selected from the group consisting of: oxygen, sulfur, and nitrogen. Heteroaryl may be 5- to 10-membered, may be 5-membered or 6-membered, for example, furanyl, thienyl, pyridyl, pyrrolyl, N-alkylpyrrolyl, pyrimidinyl, pyrazinyl, imidazolyl, tetrazolyl, and the like. The heteroaryl ring may be fused to an aryl, heterocyclyl, or cycloalkyl ring, wherein the ring attached to the parent structure is a heteroaryl ring. Heteroarylene may be optionally substituted or unsubstituted. When substituted, the substituent is preferably one or more groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, and heterocycloalkylthio.

In the present invention, the term "aliphatic heterocyclyl" generally refers to a stable non-aromatic 3- to 7-membered monocyclic structure, a fused 7- to 10-membered bicyclic heterocyclic structure, or a bridged 6- to 10-membered bicyclic heterocyclic structure. These cyclic structures may be saturated or partially saturated. In addition to carbon atoms, these cyclic structures also contain one or more heteroatoms, wherein the heteroatoms may be selected from the group consisting of: oxygen, sulfur, and nitrogen. For example, it contains 1-4 heteroatoms as defined above. When used to denote an atom on a heterocyclic ring structure, the term "nitrogen" may include nitrogen that has undergone a substitution reaction. Heterocyclylene may be substituted or unsubstituted.

In the present invention, the term "alicyclic group" generally refers to a residue derived by removal of hydrogen atoms from the same or different carbon atoms of a carbocyclic ring. The term "alicyclic ring" generally refers to a saturated or partially unsaturated monocyclic or polycyclic cyclic hydrocarbon. The carbocycle contains 3 to 20 carbon atoms, may contain 3 to 12 carbon atoms, may contain 3 to 10 carbon atoms, and may contain 3 to 8 carbon atoms. Non-limiting examples of monocyclic carbocycles include cyclopropane, cyclobutane, cyclopentane, cyclopentene, cyclohexane, cyclohexene, cyclohexadiene, cycloheptane, cycloheptatriene, cyclooctane, and the like; polycyclic carbocycles may include spirocyclic, fused, and bridged carbocycles. Carbocyclylene may be substituted or unsubstituted.

In the present invention, the term "partially unsaturated" generally means that the cyclic structure contains at least one double or triple bond between the ring molecules. The term "partially unsaturated" encompasses cyclic structures with multiple unsaturations but is not intended to include aromatic or heteroaromatic rings as defined in the present invention. The term "unsaturated" indicates that a portion has one or more degrees of unsaturation.

In the present invention, the term "halogen" generally refers to fluorine, chlorine, bromine, and iodine, for example, it may be fluorine or chlorine.

In the present invention, the term "aliphatic group" generally refers to a straight-chain, branched-chain, or cyclic hydrocarbon having 1-12 carbon atoms, which is either fully saturated or contains one or more unsaturated units, provided the unsaturated units are not aromatic groups. For example, suitable aliphatic groups may include substituted or unsubstituted straight-chain, branched, or cyclic alkyl, alkenyl, alkynyl, and mixtures thereof; such as (cycloalkyl)alkyl, (cycloalkenyl)alkyl, or (cycloalkyl)alkenyl. For example, the aliphatic group has 1-12, 1-8, 1-6, 1-4, or 1-3 carbon atoms.

In the present invention, the term "optional" or "optionally" generally means that the subsequently described event or circumstance may but need not occur, and the description includes instances where said event or circumstance occurs and instances where it does not. For example, "a heterocyclic group optionally substituted by alkyl" means that the alkyl may but need not be present, and the description includes instances where the heterocyclic group is substituted by alkyl and instances where it is not substituted by alkyl.

In the present invention, the term "substituted" generally means that one or more hydrogen atoms on a group, for example up to 5, for example 1 to 3 hydrogen atoms, are each independently substituted by a corresponding number of substituents. Substituents are only at their possible chemical positions, and a person skilled in the art can determine (experimentally or theoretically) possible or impossible substitutions without undue effort. For example, an amino or hydroxyl group having a free hydrogen may be unstable when bonded to a carbon atom having an unsaturated (e.g., olefinic) bond.

One or more hydrogen atoms on a group, for example up to 5, for example 1 to 3 hydrogen atoms, are each independently substituted by a corresponding number of substituents. Substituents are only at their possible chemical positions, and a person skilled in the art can determine (experimentally or theoretically) possible or impossible substitutions without undue effort. For example, an amino or hydroxyl group having a free hydrogen may be unstable when bonded to a carbon atom having an unsaturated (e.g., olefinic) bond.

In the present invention, the term "compound" generally refers to a substance composed of two or more different elements. For example, the compound of the present invention may be an organic compound. For example, the compound of the present invention may be a compound with a molecular weight below 500, may be a compound with a molecular weight below 1000, may also be a compound with a molecular weight above 1000, and may also be a compound with a molecular weight above 10,000 or above 100,000. In the present invention, the compound may also refer to a compound linked by chemical bonds. For example, it may be a compound where one or more molecules with a molecular weight below 1000 are linked via chemical bonds to a biomacromolecule, said biomacromolecule may be a polysaccharide, protein, nucleic acid, polypeptide, etc. For example, the compound of the present invention may include a compound comprising a protein linked to one or more molecules with a molecular weight below 1000, may include a compound comprising a protein linked to one or more molecules with a molecular weight below 10,000, and may include a compound comprising a protein linked to one or more molecules with a molecular weight below 100,000.

In some embodiments, the compound of the present invention may be in a "mixture form". The "mixture form" of the compound refers to a composition comprising one or more of the compound of the present invention, its tautomers, mesomers, racemates, enantiomers, and diastereomers.

Unless otherwise indicated, the structures described in the present invention may also include compounds that differ only in the presence or absence of one or more isotopically enriched atoms. For example, compounds that are otherwise consistent with the structures of the present invention, except for the substitution of hydrogen atoms with deuterium or tritium, or carbon atoms with carbon-13 or carbon-14, are within the scope of the present invention. In the present invention, the term "pharmaceutical composition" generally refers to a mixture containing one or more compounds of the present invention, or physiologically/pharmaceutically acceptable salts or prodrugs thereof, with other chemical components, and other components such as physiologically/pharmaceutically acceptable carriers and excipients. The pharmaceutical composition may facilitate administration to an organism, aid in the absorption of the active ingredient, and thereby exert biological activity. The preparation of conventional pharmaceutical compositions can be found in the Chinese Pharmacopoeia.

In the present invention, the term "pharmaceutically acceptable salt" or "medicinally acceptable salt" generally refers to a salt of the compound or ligand-drug conjugate of the present invention, or a salt of the compound described herein, which salt, when administered to a mammal, can possess safety and/or efficacy and can have the intended biological activity. The antibody-drug conjugate compound of the present invention can form salts with acids. Non-limiting examples of pharmaceutically acceptable salts include: hydrochloride, hydrobromide, hydroiodide, sulfate, bisulfate, citrate, acetate, succinate, ascorbate, oxalate, nitrate, pivalate, hydrogen phosphate, dihydrogen phosphate, salicylate, hydrogen citrate, tartrate, maleate, fumarate, formate, benzoate, methanesulfonate, ethanesulfonate, benzenesulfonate, p-toluenesulfonate.

In the present invention, the term "solvate" or "solvent compound" generally refers to a pharmaceutically acceptable solvate formed by the ligand-drug conjugate compound of the present invention with one or more solvent molecules. Non-limiting examples of solvent molecules include water, ethanol, acetonitrile, isopropanol, DMSO, and ethyl acetate. A "pharmaceutically acceptable prodrug" is a compound that can be converted under physiological conditions or by solvolysis into a specific compound or a pharmaceutically acceptable salt of such a compound. "Pharmaceutically active metabolite" is intended to mean a pharmacologically active product produced by metabolism in vivo of a specific compound or a salt thereof. Metabolites of compounds can be identified using conventional techniques known in the art, and their activity can be determined using tests as described herein. The pharmaceutical composition may be in the form of a sterile injectable aqueous or oily suspension for intramuscular and subcutaneous administration. Such suspensions can be formulated according to known techniques using suitable dispersing or wetting agents and suspending agents as mentioned above. Sterile injectable preparations may also be sterile injectable solutions or suspensions prepared in non-toxic parenterally acceptable diluents or solvents, for example, solutions prepared in 1,3-butanediol. Furthermore, sterile fixed oils can conveniently be employed as a solvent or suspending medium. For example, any bland fixed oil including synthetic mono- or diglycerides may be used. Fatty acids such as oleic acid can also be used in the preparation of injectables. For ocular administration, the compounds of the present invention are delivered in a pharmaceutically acceptable ophthalmic carrier such that the compound remains in contact with the ocular surface for a sufficient period to allow the compound to penetrate the cornea and internal areas of the eye, including, for example, the anterior chamber, posterior chamber, vitreous, aqueous humor, vitreous humor, cornea, iris/ciliary body, lens, choroid/retina, and sclera. The pharmaceutically acceptable ophthalmic carrier may be an ointment, vegetable oil, or encapsulating material. The compounds of the present invention may also be injected directly into the vitreous and aqueous humor. In the present invention, the term "comprising" generally means including the explicitly specified features but not excluding other elements. The terms "above" and "below" generally include the number itself.

In the present invention, the term "about" generally means a variation within a range of 0.5% to 10% above or below the specified value, for example, within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

Detailed Description of the Invention

In one aspect, the present invention provides a compound represented by formula (I),

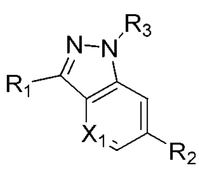
Formula (I),

wherein $R_1$ comprises a substituted cyclic structure;
$R_2$ comprises an optionally substituted substituent;
$R_3$ comprises an optionally substituted substituent;
$X_1$ is an optionally substituted atom;
or a pharmaceutically acceptable prodrug, a pharmaceutically active metabolite, and/or a pharmaceutically acceptable salt thereof.

For example, the cyclic structure of the $R_1$ is substituted with a substituent comprising a basic nitrogen atom. For example, the cyclic structure of the $R_1$ is substituted with a substituent selected from the group consisting of: optionally substituted amino, and optionally substituted nitrogen-containing aliphatic heterocyclic groups.
In one aspect, the present invention provides a compound represented by formula (I),

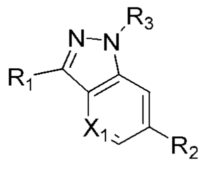 Formula (I),

wherein the $R_1$ comprises $(R_{1-0})_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein each of $R_{1-0}$, $R_{1}$-$_{1}$, $R_{1-2}$, $R_{1-3}$, $R_{1-4}$, and $R_{1-5}$ independently comprises an optionally substituted substituent, wherein nr0, nr1, nr2, nr3, nr4 and nr5 are each independently selected from 0 or a positive integer; wherein the cyclic structure of $R_1$ is substituted with a substituent comprising a basic nitrogen atom;
$R_2$ comprises an optionally substituted substituent;
$R_3$ comprises an optionally substituted substituent;
$X_1$ is an optionally substituted atom;
or a pharmaceutically acceptable prodrug, a pharmaceutically active metabolite, and/or a pharmaceutically acceptable salt thereof.

In one aspect, the present invention provides a compound represented by formula (I),

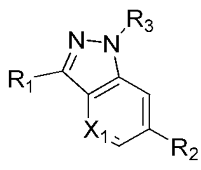 Formula (I),

wherein the $R_1$ comprises $(C=C)_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein each of $R_{1-1}$, $R_{1-2}$, $R_{1-3}$, $R_{1-4}$, and $R_{1-5}$ independently comprises an optionally substituted substituent, wherein nr0, nrl, nr2, nr3, nr4 and nr5 are each independently selected from 0 or a positive integer; wherein the cyclic structure of $R_1$ is substituted with a substituent comprising a basic nitrogen atom;
$R_2$ comprises an optionally substituted substituent;
$R_3$ comprises an optionally substituted substituent;
$X_1$ is an optionally substituted atom;
or a pharmaceutically acceptable prodrug, a pharmaceutically active metabolite, and/or a pharmaceutically acceptable salt thereof.

In one aspect, the present invention provides a compound represented by formula (I),

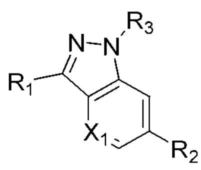 Formula (I),

wherein the $R_1$ comprises $(C=C)_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein nr0, nr1, nr2, nr3, nr4, and

nr5 are each independently selected from 0 or a positive integer;

wherein each $R_{1-1}$ is selected from the group consisting of: optionally substituted aryl, and optionally substituted heteroaryl groups; for example, each $R_{1-1}$ is selected from the group consisting of: optionally substituted aryl, optionally substituted 5-membered heteroaryl groups, and optionally substituted 6-membered heteroaryl groups; for example, each $R_{1-1}$ is selected from the group consisting of: optionally substituted aryl, optionally substituted 5-membered heteroaryl groups containing 1 N atom, 5-membered heteroaryl groups containing 2 N atoms, 6-membered heteroaryl groups containing 1 N atom, and optionally substituted 6-membered heteroaryl groups containing 2 N atoms; for example, each $R_{1-1}$ is selected from the group consisting of: optionally substituted aryl, optionally substituted pyridyl, and optionally substituted pyrazolyl;

wherein each $R_{1-2}$ is selected from the group consisting of: a chemical bond and hydroxyl; for example, each $R_{1-2}$ can be - or -O-;

wherein each $R_{1-3}$ is selected from optionally substituted alkyl; for example, each $R_{1-3}$ can be optionally substituted methyl, optionally substituted ethyl, and optionally substituted propyl;

wherein each $R_{1-4}$ is selected from optionally substituted aliphatic heterocyclic groups; for example, each $R_{1-4}$ can be optionally substituted 5-membered aliphatic heterocyclic groups, and optionally substituted 6-membered aliphatic heterocyclic groups; for example, each $R_{1-4}$ is selected from the group consisting of: optionally substituted 5-membered aliphatic heterocyclic groups containing 1 N atom, 5-membered aliphatic heterocyclic groups containing 2 N atoms, 6-membered aliphatic heterocyclic groups containing 1 N atom, and optionally substituted 6-membered aliphatic heterocyclic groups containing 2 N atoms; for example, each $R_{1-4}$ is selected from the group consisting of: optionally substituted pyrrolyl, optionally substituted piperidinyl, and optionally substituted piperazinyl;

wherein each $R_{1-5}$ is selected from optionally substituted alkyl; for example, each $R_{1-5}$ can be optionally substituted methyl; for example, it may comprise two $R_{1-5}$;

$R_2$ comprises an optionally substituted substituent;

$R_3$ comprises an optionally substituted substituent;

$X_1$ is an optionally substituted atom;

or a pharmaceutically acceptable prodrug, a pharmaceutically active metabolite, and/or a pharmaceutically acceptable salt thereof.

In one aspect, the present invention provides a compound represented by formula (1),

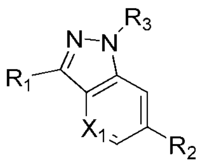
Formula (1),

wherein the $R_1$ comprises $(C{=}C)_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein nr0, nr1, nr2, nr3, nr4, and nr5 are each independently selected from 0 or a positive integer;

wherein each $R_{1-1}$ is selected from the group consisting of: optionally substituted aryl, and optionally substituted heteroaryl groups; for example, each $R_{1-1}$ is selected from the group consisting of: optionally substituted aryl, optionally substituted 5-membered heteroaryl groups, and optionally substituted 6-membered heteroaryl groups; for example, each $R_{1-1}$ is selected from the group consisting of: optionally substituted aryl, optionally substituted 5-membered heteroaryl groups containing 1 N atom, 5-membered heteroaryl groups containing 2 N atoms, 6-membered heteroaryl groups containing 1 N atom, and optionally substituted 6-membered heteroaryl groups containing 2 N atoms; for example, each $R_{1-1}$ is selected from the group consisting of: optionally substituted aryl, optionally substituted pyridyl, and optionally substituted pyrazolyl;

wherein each $R_{1-2}$ is selected from the group consisting of: a chemical bond and hydroxyl; for example, each $R_{1-2}$ can be - or -O-;

wherein each $R_{1-3}$ is selected from optionally substituted alkyl; for example, each $R_{1-3}$ can be optionally substituted methyl, optionally substituted ethyl, and optionally substituted propyl;

wherein each $R_{1-4}$ is selected from optionally substituted aliphatic heterocyclic groups; for example, each $R_{1-4}$ can be optionally substituted 5-membered aliphatic heterocyclic groups, and optionally substituted 6-membered aliphatic heterocyclic groups; for example, each $R_{1-4}$ is selected from the group consisting of: optionally substituted 5-membered aliphatic heterocyclic groups containing 1 N atom, 5-membered aliphatic heterocyclic groups containing 2 N atoms, 6-membered aliphatic heterocyclic groups containing 1 N atom, and optionally substituted 6-membered aliphatic heterocyclic groups containing 2 N atoms; for example, each $R_{1-4}$ is selected from the group consisting of: optionally substituted pyrrolyl, optionally substituted piperidinyl, and optionally substituted piperazinyl;

wherein each $R_{1-5}$ is selected from optionally substituted alkyl; for example, each $R_{1-5}$ can be optionally substituted methyl; for example, it may comprise two $R_{1-5}$;
$R_2$ comprises optionally substituted -S-optionally substituted aryl-optionally substituted amido-optionally substituted alkyl, or -S-optionally substituted aryl-optionally substituted amido-optionally substituted alicyclic group; for example, $R_2$ comprises optionally substituted -S-optionally halogen-substituted aryl-optionally substituted amido-optionally substituted methyl, or -S-optionally substituted aryl-optionally substituted amido-optionally substituted cyclopropyl;
$R_3$ comprises hydrogen or optionally an isotope of hydrogen;
$X_1$ is optionally substituted CH;
or a pharmaceutically acceptable prodrug, a pharmaceutically active metabolite, and/or a pharmaceutically acceptable salt thereof.

For example, the $R_1$ comprises $(R_{1-0})_{nr0}$-$(R_{1-1})_{nr1}$-$(R_{1-2})_{nr2}$-$(R_{1-3})_{nr3}$-$(R_{1-4})_{nr4}$-$(R_{1-5})_{nr5}$, wherein each of $R_{1-0}$, $R_{1-1}$, $R_{1-2}$, $R_{1-3}$, $R_{1-4}$, and $R_{1-5}$ independently comprises an optionally substituted substituent, wherein nr0, nr1, nr2, nr3, nr4, and nr5 are each independently selected from 0 or a larger number.

For example, the $R_{1-0}$ is a chemical bond or is selected from the group consisting of: optionally substituted alkenyl, optionally substituted alkynyl, and optionally substituted amino. For example, the $R_{1-0}$ is a chemical bond or is selected from the group consisting of: optionally substituted ethenyl, optionally substituted ethynyl, and optionally substituted amino. For example, the nr0 is 0 or 1. For example, nr0 is 0, 1, 2, or 3.

For example, the $R_{1-1}$ is a chemical bond or is selected from the group consisting of: optionally substituted amido, optionally substituted aryl groups, and optionally substituted heteroaryl groups. For example, the $R_{1-1}$ is a chemical bond or is selected from the group consisting of: optionally substituted amido, optionally substituted phenyl, and optionally substituted heteroaryl groups. For example, each $R_{1-1}$ is selected from the group consisting of: optionally substituted aryl, optionally substituted 5-membered heteroaryl groups, and optionally substituted 6-membered heteroaryl groups; for example, each $R_{1-1}$ is selected from the group consisting of: optionally substituted aryl, optionally substituted 5-membered heteroaryl groups containing 1 N atom, 5-membered heteroaryl groups containing 2 N atoms, 6-membered heteroaryl groups containing 1 N atom, and optionally substituted 6-membered heteroaryl groups containing 2 N atoms; for example, each $R_{1-1}$ is selected from the group consisting of: optionally substituted aryl, optionally substituted pyridyl, and optionally substituted pyrazolyl. For example, the $R_{1-1}$ is a chemical bond or is selected from the group consisting of: optionally substituted amido, optionally substituted phenyl, optionally substituted pyridyl, and optionally substituted pyrazolyl. For example, the nr1 is 0 or 1. For example, nr1 is 0, 1, 2, or 3.

For example, the $R_{1-2}$ is a chemical bond or is selected from the group consisting of: hydrogen, protium, deuterium, tritium, and optionally substituted hydroxyl. For example, each $R_{1-2}$ can be - or -O-. For example, the nr2 is 0 or 1. For example, nr2 is 0, 1, 2, or 3.

For example, the $R_{1-3}$ is a chemical bond or is selected from the group consisting of: hydrogen, protium, deuterium, tritium, and optionally substituted alkyl. For example, the $R_{1-3}$ is a chemical bond or is selected from the group consisting of: hydrogen, protium, deuterium, tritium, optionally substituted methyl, optionally substituted ethyl, and optionally substituted propyl. For example, each $R_{1-3}$ can be optionally substituted methyl, optionally substituted ethyl, and optionally substituted propyl. For example, the nr3 is 0 or 1. For example, nr3 is 0, 1, 2, or 3.

For example, the $R_{1-4}$ is a chemical bond or is selected from the group consisting of: hydrogen, protium, deuterium, tritium, optionally substituted amino, and optionally substituted aliphatic heterocyclic groups. For example, the $R_{1-4}$ is a chemical bond or is selected from the group consisting of: hydrogen, protium, deuterium, tritium, optionally substituted amino, optionally substituted pyrrolyl, optionally substituted piperidinyl, optionally substituted piperazinyl, and optionally substituted morpholinyl. For example, each $R_{1-4}$ can be optionally substituted 5-membered aliphatic heterocyclic groups, and optionally substituted 6-membered aliphatic heterocyclic groups; for example, each $R_{1-4}$ is selected from the group consisting of: optionally substituted 5-membered aliphatic heterocyclic groups containing 1 N atom, 5-membered aliphatic heterocyclic groups containing 2 N atoms, 6-membered aliphatic heterocyclic groups containing 1 N atom, and optionally substituted 6-membered aliphatic heterocyclic groups containing 2 N atoms; for example, each $R_{1-4}$ is selected from the group consisting of: optionally substituted pyrrolyl, optionally substituted piperidinyl, and optionally substituted piperazinyl. For example, the nr4 is 0 or 1. For example, nr4 is 0, 1, 2, or 3.

For example, the $R_{1-5}$ is a chemical bond or is selected from the group consisting of: hydrogen, protium, deuterium, tritium, and optionally substituted alkyl. For example, the $R_{1-5}$ is a chemical bond or is selected from the group consisting of: hydrogen, protium, deuterium, tritium, optionally substituted methyl, and optionally substituted ethyl. For example, each $R_{1-5}$ can be optionally substituted methyl; for example, it may comprise 2 $R_{1-5}$. For example, the nr5 is 0, 1, or 2. For example, nr5 is 0, 1, 2, or 3.

For example, the $R_2$ is optionally substituted mercapto. For example, the $R_2$ is substituted with one or more $R_{2-1}$, each the $R_{2-1}$ being independently optionally substituted substituents. For example, the $R_{2-1}$ is optionally substituted aryl. For example, the $R_{2-1}$ is optionally substituted phenyl.

For example, the $R_{2-1}$ is substituted with one or more $R_{2-2}$, each the $R_{2-2}$ being independently optionally

substituted substituents. For example, the $R_{2-2}$ is selected from the group consisting of: hydrogen, protium, deuterium, tritium, halogen, optionally substituted carbonyl, optionally substituted carboxyl, optionally substituted amido, and optionally substituted alkyl. For example, the $R_{2-2}$ is selected from the group consisting of: hydrogen, protium, deuterium, tritium, halogen, optionally substituted carbonyl, optionally substituted carboxyl, optionally substituted amido, and optionally substituted methyl.

For example, the $R_{2-2}$ is substituted with one or more $R_{2-3}$, each the $R_{2-3}$ being independently optionally substituted substituents. For example, the $R_{2-3}$ is selected from the group consisting of: hydrogen, protium, deuterium, tritium, halogen, optionally substituted alkyl, optionally substituted alicyclic groups, and optionally substituted amino. For example, the $R_{2-3}$ is selected from the group consisting of: hydrogen, protium, deuterium, tritium, halogen, optionally substituted methyl, optionally substituted ethyl, optionally substituted cyclopropyl, and optionally substituted amino.

For example, the $R_{2-3}$ is substituted with one or more $R_{2-4}$, each the $R_{2-4}$ being independently optionally substituted substituents. For example, the $R_{2-4}$ is selected from the group consisting of: hydrogen, protium, deuterium, tritium, halogen, optionally substituted alkyl, and optionally substituted alicyclic groups. For example, the $R_{2-4}$ is selected from the group consisting of: hydrogen, protium, deuterium, tritium, halogen, optionally substituted methyl, optionally substituted ethyl, and optionally substituted cyclopropyl.

For example, $R_2$ comprises optionally substituted -S-optionally substituted aryl-optionally substituted amido-optionally substituted alkyl, or -S-optionally substituted aryl-optionally substituted amido-optionally substituted alicyclic groups; for example, $R_2$ comprises optionally substituted -S-optionally halogen-substituted aryl-optionally substituted amido-optionally substituted methyl, or -S-optionally substituted aryl-optionally substituted amido-optionally substituted cyclopropyl; for example, $R_2$ comprises optionally substituted -S-F-substituted or unsubstituted aryl-amido-methyl, or -S-F-substituted or unsubstituted aryl-amido-cyclopropyl.

For example, the $R_3$ is selected from the group consisting of: hydrogen, protium, deuterium, and tritium.

For example, the $X_1$ is selected from the group consisting of: optionally substituted CH, and N. For example, $X_1$ is optionally substituted CH.

In some embodiments of the present invention, the compound has the following structure:

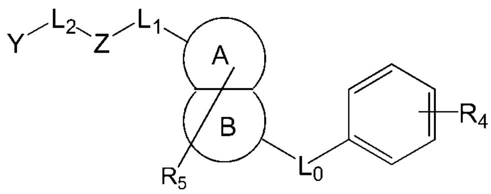

(II)

wherein,

Ring A is a 5- to 7-membered nitrogen-containing heterocycle;
Ring B is a 5- to 7-membered aromatic ring or heterocycle;
Z is -C(O)NH-, -NHC(O)-, or

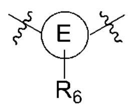

;

Ring E is an aromatic ring or heteroaromatic ring;

$L_0$ is selected from: O, S, $N(R_{La})$, $C_1$-$C_6$ alkylene, C(O); $R_{La}$ is selected from: H, $C_1$-$C_6$ alkyl;

$L_1$ is selected from: a single bond, $C_1$-$C_6$ alkylene, $C_2$-$C_6$ alkenylene, $C_2$-$C_6$ alkynylene, -($C_0$-$C_6$ alkylene)-$Q_1$-($C_0$-$C_6$ alkylene)-, $Q_1$ is selected from: -O-, -S-, -C(O)-, -C(O)O-, -OC(O)-, -C(O)N($R_{Lb}$)-, -N($R_{Lb}$)C(O)-, - N($R_{Lb}$)C(O)O-, -N($R_{Lb}$)C(O)N($R_{Lb}$)-, -N($R_{Lb}$)-, -S(O)$_2$-, -S(O)$_2$N($R_{Lb}$)-, -N($R_{Lb}$)S(O)$_2$-, -S(O)-, -S(O)N($R_{Lb}$)-, - N($R_{Lb}$)S(O)-; H atoms in the alkylene may optionally be substituted by groups selected from: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lb}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$L_2$ is selected from: a single bond, $C_1$-$C_6$ alkylene, -($C_0$-$C_6$ alkylene)-$Q_2$-($C_0$-$C_6$ alkylene)-, $Q_2$ is selected from: - O-, -S-, -C(O)-, -C(O)O-, -OC(O)-, -C(O)N($R_{Lc}$)-, -N($R_{Lc}$)C(O)-, -N($R_{Lc}$)C(O)O-, -N($R_{Lc}$)C(O)N($R_{La}$)-, -N($R_{Lc}$)-, -S(O)$_2$-, -S(O)$_2$N($R_{Lc}$)-, -N($R_{Lc}$)S(O)$_2$-, -S(O)-, -S(O)N($R_{Lc}$)-, -N($R_{Lc}$)S(O)-; H atoms in the alkylene may optionally be substituted by groups selected from: H, $C_1$-$C_{10}$ alkyl, $C_2$-$C_{10}$ alkenyl, $C_2$-$C_{10}$ alkynyl, halogen, cyano, nitro, azido,

$C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lc}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

Y is selected from: H, -$NR_7R_8$, nitrogen-containing heterocyclyl, wherein the nitrogen-containing heterocyclyl may optionally be substituted by one or more $R_9$ groups, and $R_9$ is selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_7$ and $R_8$ are each independently selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, 4- to 10-membered heterocyclyl, 4- to 10-membered heterocyclylalkyl; wherein the $C_1$-$C_{10}$ alkyl, $C_2$-$C_{10}$ alkenyl, $C_2$-$C_{10}$ alkynyl, $C_3$-$C_{10}$ cycloalkyl, 4- to 10-membered heterocyclyl may optionally be substituted by one or more groups selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$R_4$ is one or more independent substituents on the benzene ring, each $R_4$ being independently selected from: H, halogen, $C_1$-$C_{10}$ alkyl, cyano, $C_1$-$C_{10}$ haloalkyl, $C_1$-$C_{10}$ haloalkoxy, $C_1$-$C_{10}$ cyanoalkyl, -$OR_{401}$, -C(O)$R_{401}$, - C(O)$OR_{401}$, -$NR_{402}$C(O)$OR_{401}$, -OC(O)$R_{401}$, -$NR_{402}SO_2R_{401}$, -$SO_2NR_{401}R_{402}$, -$NR_{402}$C(O)$R_{401}$, -C(O)$NR_{401}R_{402}$, -$NR_{401}R_{402}$, -($C_0$-$C_6$ alkylene)-$NR_{401}R_{402}$, -$SR_{401}$, -S(O)$R_{401}$, -S(O)$_2R_{401}$, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); wherein the $C_0$-$C_6$ alkylene, $C_1$-$C_{10}$ alkyl, $C_2$-$C_{10}$ alkenyl, $C_2$-$C_{10}$ alkynyl, $C_3$-$C_{10}$ cycloalkyl, $C_6$-$C_{10}$ aryl, 4- to 10-membered heterocyclyl may optionally be substituted by one or more groups selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_{401}$ and $R_{402}$ are each independently selected from: H, $C_1$-$C_{10}$ alkyl, $C_3$-$C_{10}$ cycloalkyl, $C_3$-$C_{10}$ cycloalkylalkyl, $C_6$-$C_{10}$ aryl, $C_6$-$C_{10}$ aralkyl, 4- to 10-membered heterocyclyl, 4- to 10-membered heterocyclylalkyl;

$R_5$ is one or more independent substituents on the

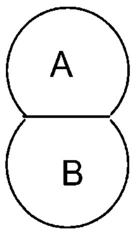

ring, each $R_5$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_6$ is one or more independent substituents on Ring E, each $R_6$ is independently selected from: **H,** halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl,

-($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl).

In some embodiments of the present invention, Ring A is a five-membered nitrogen-containing heteroaromatic ring, for example

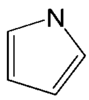 , 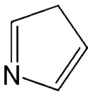 , 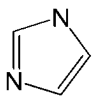 , 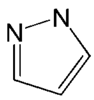 , 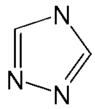 .

**In** some embodiments of the present invention, Ring B is a phenyl ring or a six-membered nitrogen-containing heterocyclic ring, for example

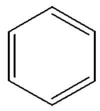 , 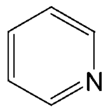 , 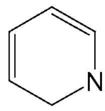 , 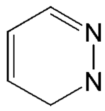 .

**In** some embodiments of the present invention, the

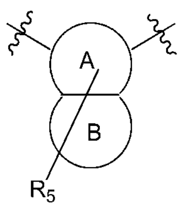

moiety is one of the following structures:

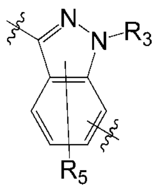 , 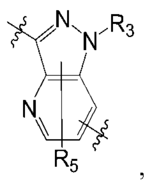 , 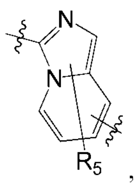 , 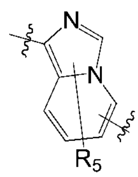 , 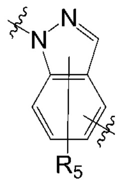 , 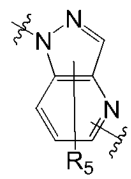 ,

particularly:

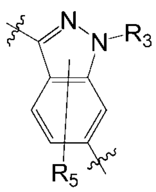 ,

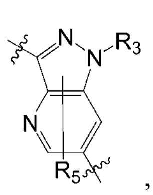 , 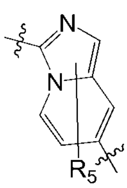 , 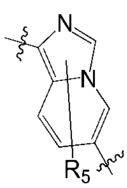 , 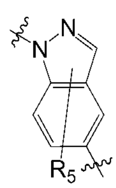 .

In some embodiments of the present invention, the

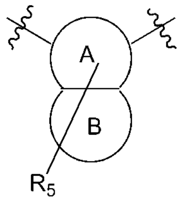

moiety is

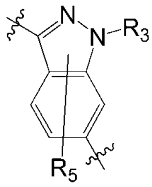

,

$L_2$ is $C_1$-$C_6$ alkylene or -($C_0$-$C_6$ alkylene)-$Q_2$-($C_0$-$C_6$ alkylene)-, Y is -$NR_7R_8$ or nitrogen-containing heterocyclyl, wherein the nitrogen-containing heterocyclyl may optionally be substituted with one or more $R_9$ groups.

**In** some embodiments of the present invention, the

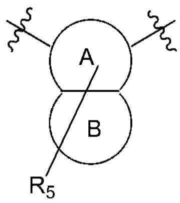

moiety is

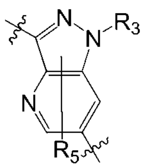

,

Y is selected from: H, -$NR_7R_8$, nitrogen-containing heterocyclyl, wherein the nitrogen-containing heterocyclyl may optionally be substituted with one or more $R_9$ groups.

**In** some embodiments of the present invention, the

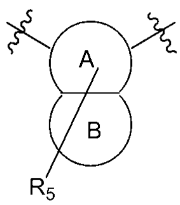

moiety is

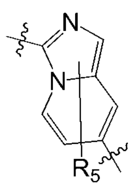

,

Y is selected from: H, -$NR_7R_8$, nitrogen-containing heterocyclyl, wherein the nitrogen-containing heterocyclyl may

optionally be substituted with one or more $R_9$ groups.

**In** some embodiments of the present invention, the

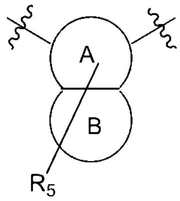

moiety is

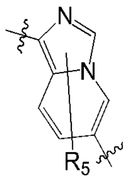

,

Y is selected from: H, $-NR_7R_8$, nitrogen-containing heterocyclyl, wherein the nitrogen-containing heterocyclyl may optionally be substituted with one or more $R_9$ groups.

In some embodiments of the present invention, the

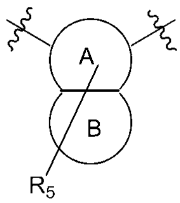

moiety is

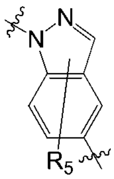

,

Y is selected from: H, $-NR_7R_8$, nitrogen-containing heterocyclyl, wherein the nitrogen-containing heterocyclyl may optionally be substituted with one or more $R_9$ groups.

In some embodiments of the present invention, $R_5$ is selected from: H, halogen, cyano, amino, hydroxy, $C_1$-$C_6$ alkyl, $C_1$-$C_6$ haloalkyl.

In some embodiments of the present invention, $R_5$ is H.

In some embodiments of the present invention, $R_3$ is selected from: H, $C_1$-$C_6$ alkyl.

In some embodiments of the present invention, $R_3$ is H.

In some embodiments of the present invention, $L_0$ is S.

In some embodiments of the present invention, $L_0$ is O.

In some embodiments of the present invention, $L_0$ is NH.

In some embodiments of the present invention, $L_0$ is methylene.

In some embodiments of the present invention, $L_0$ is NH, Z is -NHC(O)-, and the compound has the following structure:

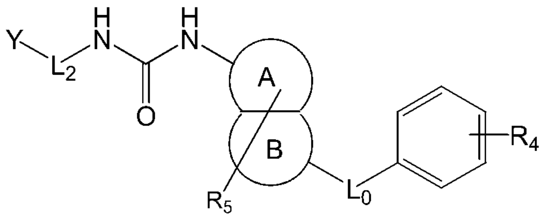

(III-1)

wherein, $L_2$ is selected from: $C_1$-$C_6$ alkylene, -($C_1$-$C_6$ alkylene)-$Q_2$-($C_1$-$C_6$ alkylene)-; Y is -$NR_7R_8$ or optionally substituted nitrogen-containing heterocyclyl, wherein the nitrogen-containing heterocyclyl may optionally be substituted with one or more $R_9$ groups.

**In** some embodiments of the present invention, Z is

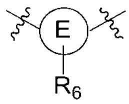

,

and the compound has the following structure:

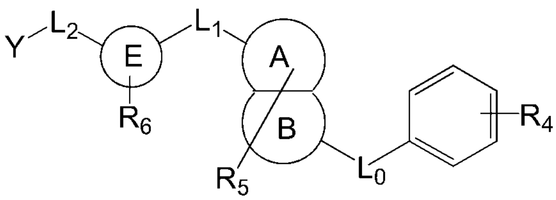

(III-2).

**In** some embodiments of the present invention, the compound has the following structure:

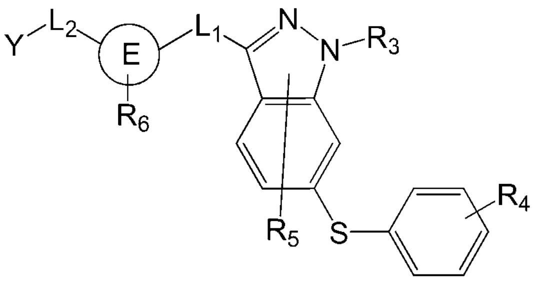

(IV).

In some embodiments of the present invention, $L_1$ is $C_2$-$C_6$ alkenylene, for example

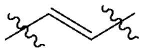

,

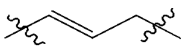

,

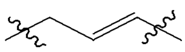

,

particularly

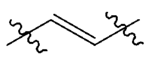

.

In some embodiments of the present invention, $L_1$ is $C_2$-$C_6$ alkynylene, for example

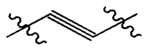,

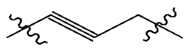, 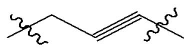,

particularly

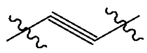.

In some embodiments of the present invention, $L_1$ is -$Q_1$-($C_0$-$C_6$ alkylene)-.
In some embodiments of the present invention, $L_1$ is -($C_0$-$C_6$ alkylene)-$Q_1$-.
In some embodiments of the present invention, $Q_1$ is -N($R_{Lb}$)-, $R_{Lb}$ is selected from: H, $C_1$-$C_3$ alkyl, particularly H.
In some embodiments of the present invention, $L_1$ is -NH-.
In some embodiments of the present invention, ring E is a benzene ring or a five- or six-membered nitrogen-containing heteroaryl ring, for example,

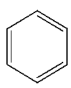, 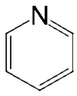, 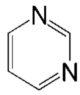, 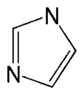, 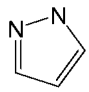.

Specifically, when ring E is a benzene ring, Y is -$NR_7R_8$ or optionally substituted nitrogen-containing heterocyclyl, wherein the nitrogen-containing heterocyclyl may optionally be substituted with one or more $R_9$ groups.
**In** some embodiments of the present invention, the

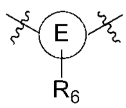

moiety has the following structures:

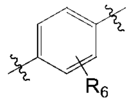,

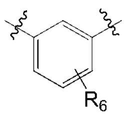, 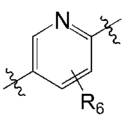, 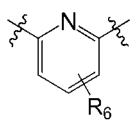, 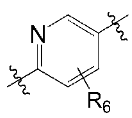, 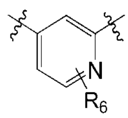, 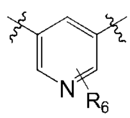, 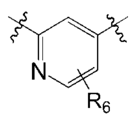, 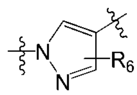,

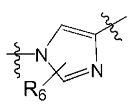, 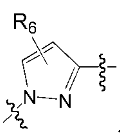, 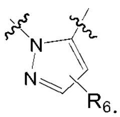.

In some embodiments of the present invention, $R_6$ is selected from: H, halogen, cyano, amino, hydroxy, $C_1$-$C_6$ alkyl, $C_1$-$C_6$ haloalkyl.
In some embodiments of the present invention, $R_6$ is H.
In some embodiments of the present invention, $L_2$ is a single bond.
In some embodiments of the present invention, $L_2$ is $C_1$-$C_6$ alkylene, for example,

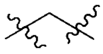, 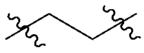,

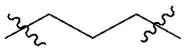.

In some embodiments of the present invention, $L_2$ is $-Q_2-(C_0-C_6$ alkylene)-.

In some embodiments of the present invention, $Q_2$ is selected from: -O-, -S-, $-N(R_{Lc})-$, $R_{Lc}$ is selected from: H, $C_1-C_3$ alkyl, particularly H.

In some embodiments of the present invention, $L_2$ is $-O-(C_0-C_6$ alkylene)-, for example,

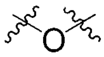, 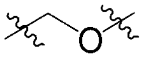,

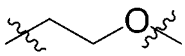, 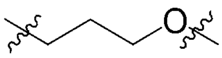.

In some embodiments of the present invention, ring E is a five- or six-membered nitrogen-containing heteroaryl ring, $L_2$ is a single bond, and Y is H.

In some embodiments of the present invention, Y is $-NR_7R_8$, $R_7$ and $R_8$ are independently selected from: H, $C_1-C_6$ alkyl, $-(C_0-C_6$ alkylene)-$(C_3-C_6$ cycloalkyl), for example,

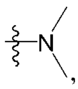, 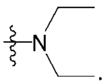.

In some embodiments of the present invention, Y is a nitrogen-containing heterocyclyl, which may optionally be substituted with one or more $R_9$ groups. Specifically, the nitrogen-containing heterocyclyl is a 4- to 8-membered saturated heterocycle, for example,

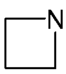, 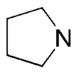, 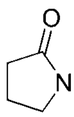, 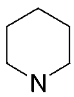, 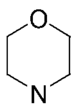, 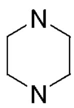, 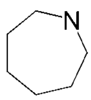, 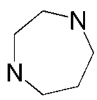.

In some embodiments of the present invention, Y is selected from:

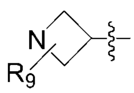, 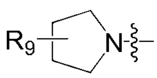, 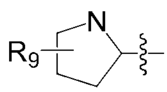,

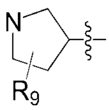, 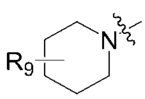, 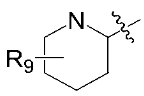, 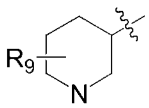, 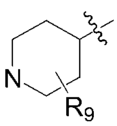, 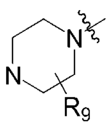, 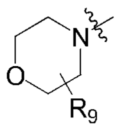, 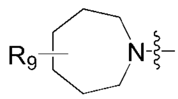,

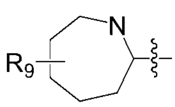, 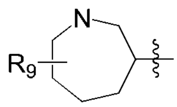, 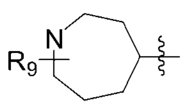, 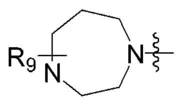

(wherein the substitution position of $R_9$ can be on any suitable carbon or nitrogen atom); more specifically, Y is selected from:

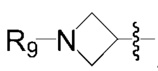, 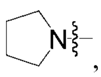,

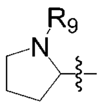, 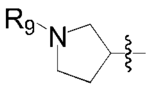, 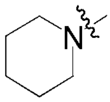, 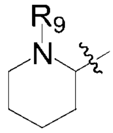, 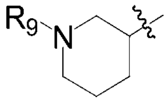, 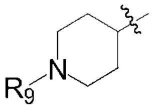, 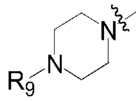, 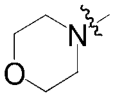,

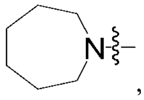, 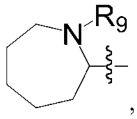, 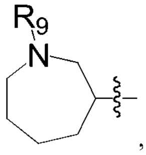, 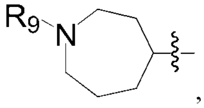, 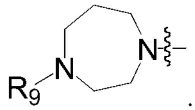.

In some embodiments of the present invention, $R_9$ is selected from: H, $C_1$-$C_6$ alkyl (e.g., $-CH_3$,

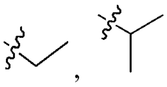

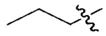),

$C_1$-$C_6$ hydroxyl-substituted alkyl (e.g.,

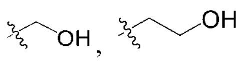),

$C_1$-$C_6$ amino-substituted alkyl (e.g.,

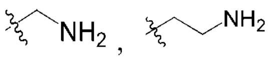),

$C_1$-$C_6$ alkoxy-substituted alkyl (e.g.,

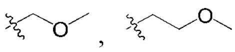),

$C_1$-$C_6$ alkylamino-substituted alkyl (e.g.,

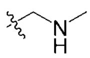, 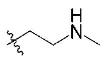, 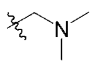, 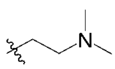),

-($C_0$-$C_6$ alkylene)-($C_3$-$C_6$ cycloalkyl) (e.g.,

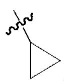, 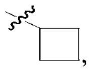, 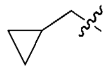, 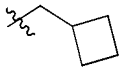).

**In** some embodiments of the present invention, Y is selected from:

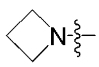, 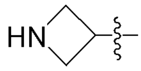, 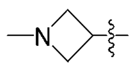,

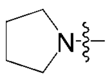, 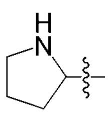, 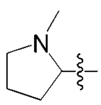, 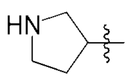, 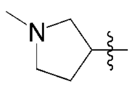, 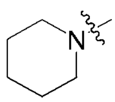, 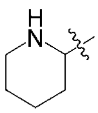, 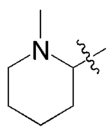, 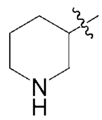,

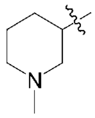, 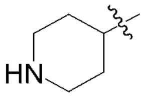, 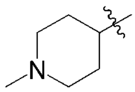, 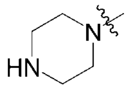, 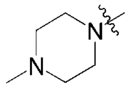, 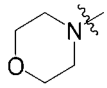, 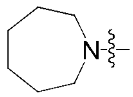, 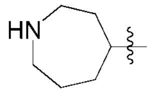,

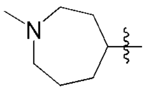, 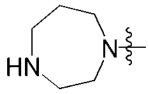, 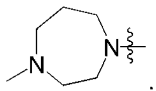.

In some embodiments of the present invention, the

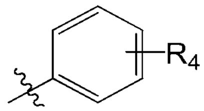

moiety is

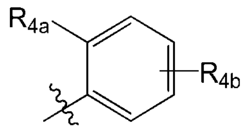,

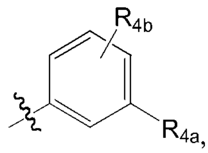, or 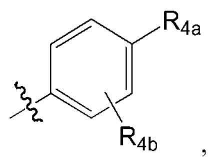,

wherein,

$R_{4a}$ is selected from: $-C(O)NR_{401}R_{402}$, $-(C_0-C_6$ alkylene)$-NR_{401}R_{402}$, $-C(O)R_{401}$, $-C(O)OR_{401}$, $R_{401}$ and $R_{402}$ are independently selected from: H, $C_1-C_6$ alkyl, $-(C_0-C_6$ alkylene)$-(C_3-C_6$ cycloalkyl); wherein the $C_0-C_6$ alkylene, $C_1-C_6$ alkyl, $C_3-C_6$ cycloalkyl may optionally be substituted with one or more groups selected from: halogen, cyano, amino, hydroxyl, $C_1-C_6$ alkyl, $C_1-C_6$ haloalkyl;

$R_{4b}$ is one or more independent substituents on the benzene ring, selected from: H, halogen, cyano, amino, hydroxyl, $C_1-C_6$ alkyl, $C_1-C_6$ haloalkyl.

In some embodiments of the present invention, $R_{4a}$ is selected from:

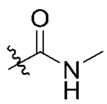, 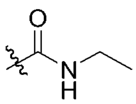, 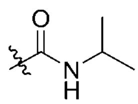,

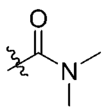, 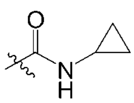, 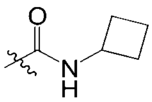, 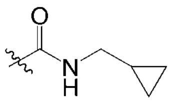, 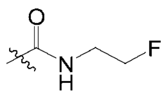, 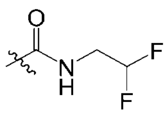, 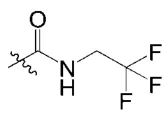,

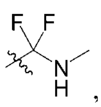, 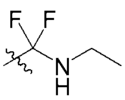, 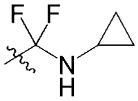, 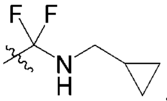, 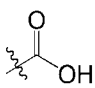, 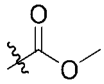, 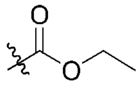, 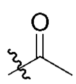, 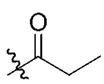,

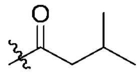, 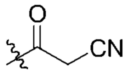, 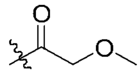, 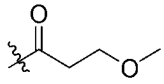, 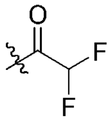, 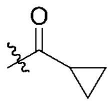, 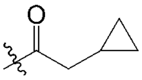.

In some embodiments of the present invention, $R_{4b}$ is H.

In some embodiments of the present invention, $R_{4b}$ is halogen, for example F.

In some embodiments of the present invention, the

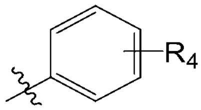

moiety is

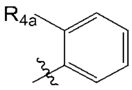, 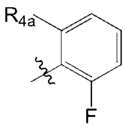,

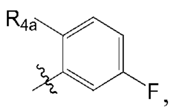, 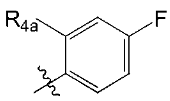, 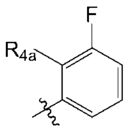, 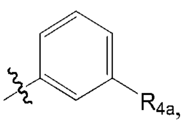 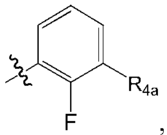, 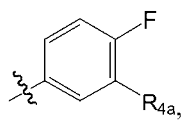

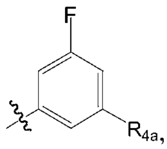 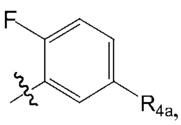 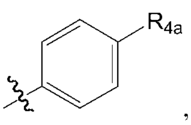, 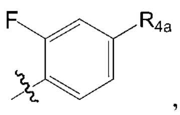, 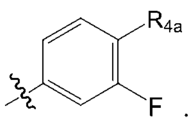.

In one aspect, the present invention provides a compound having a structure selected from the group consisting of:

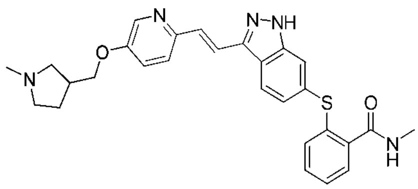, 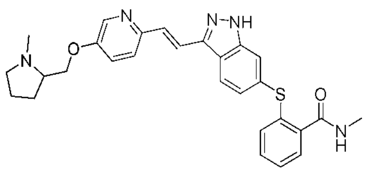, 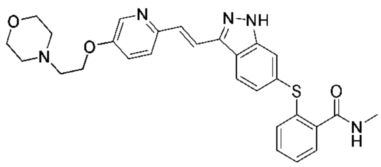,

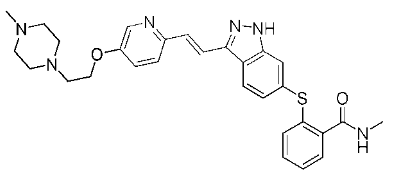, 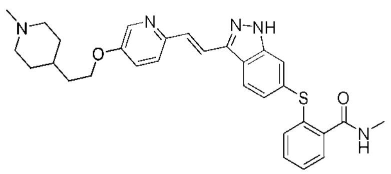, 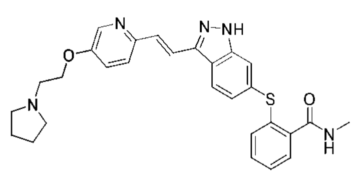,

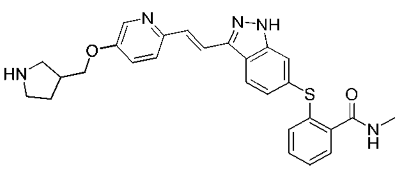, 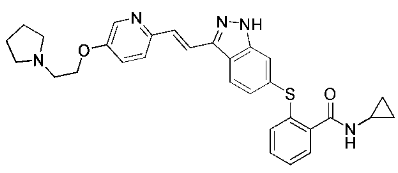, 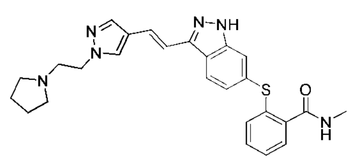,

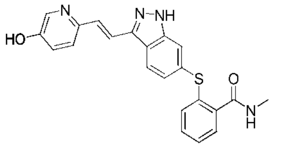,
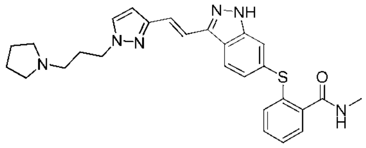,
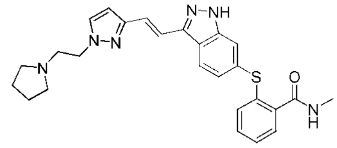,

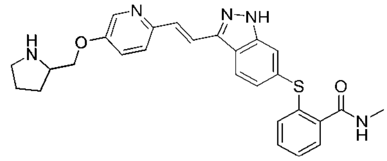,
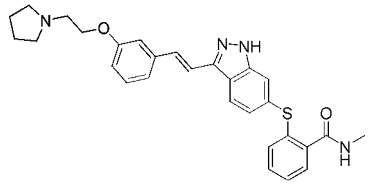,
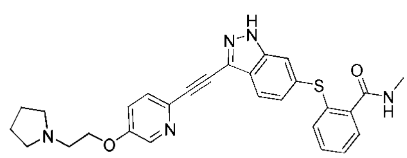,

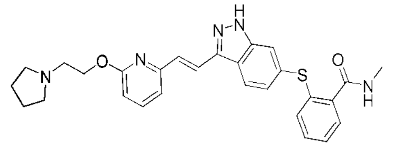,
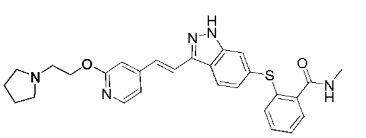,
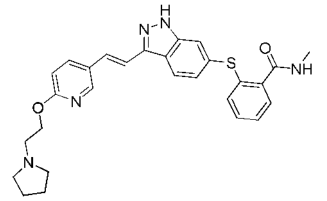,

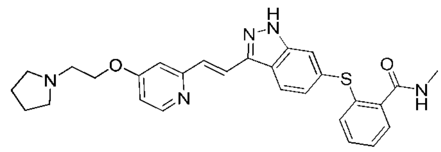,
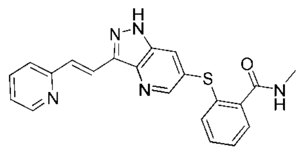,
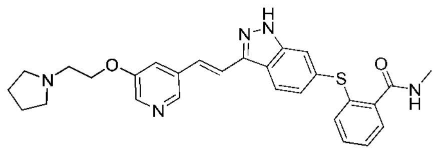,

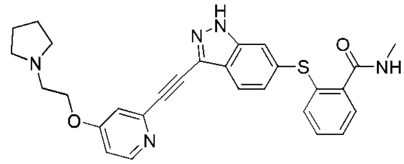,
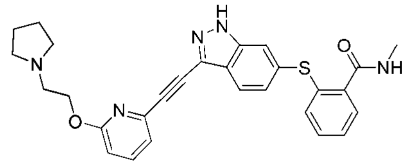,
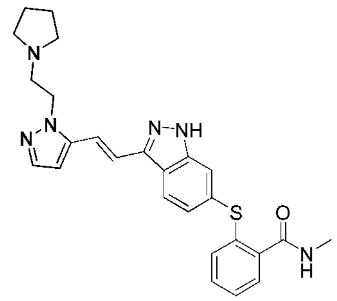,

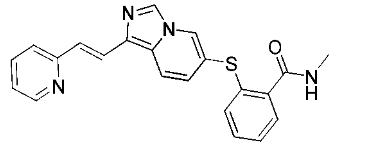,
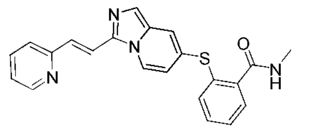,
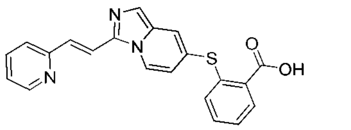,

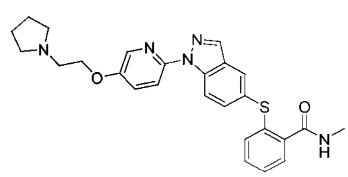,
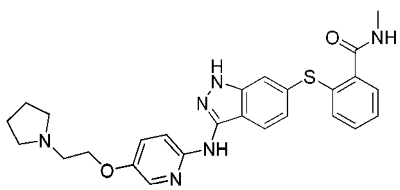,
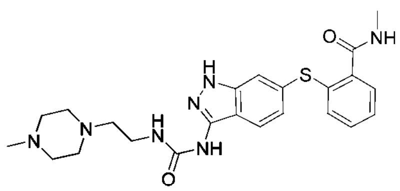,

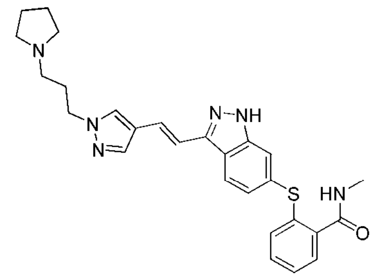

,

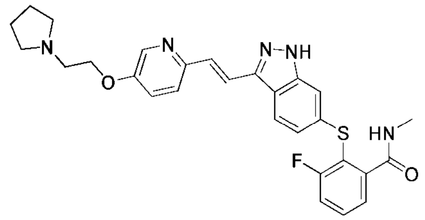

,

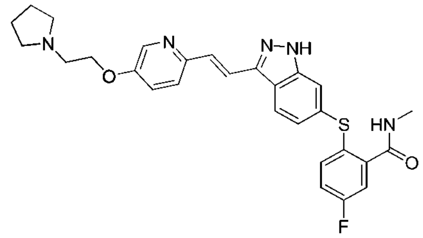

,

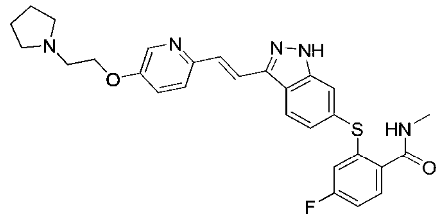

,

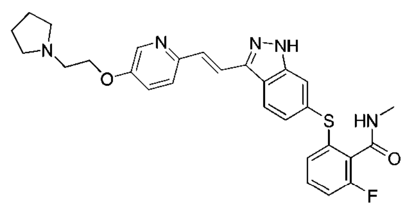

,

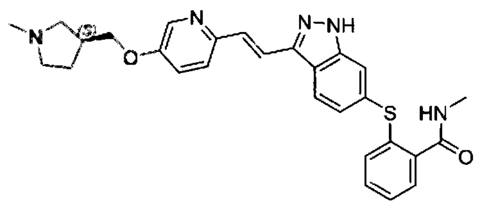

,

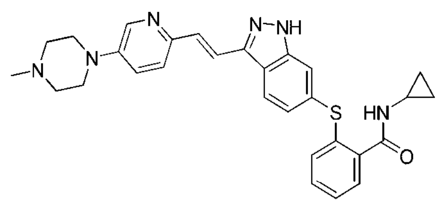

,

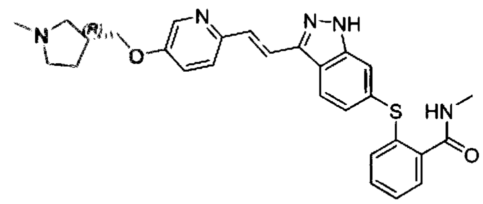

,

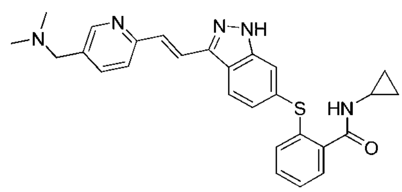

,

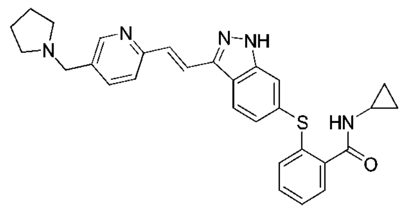

,

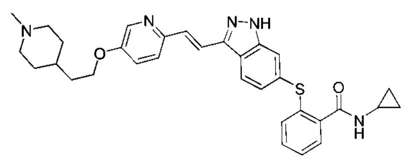

,

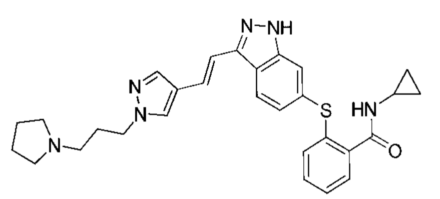

,

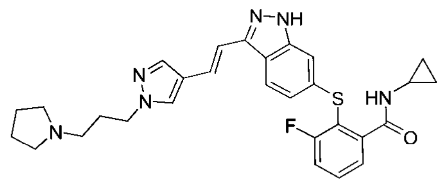,
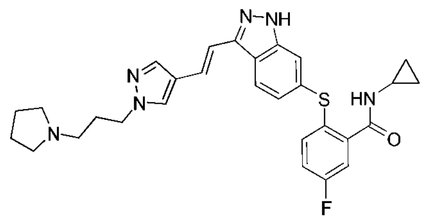,

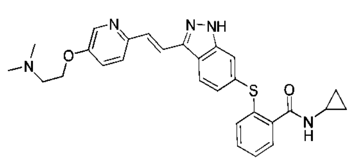,
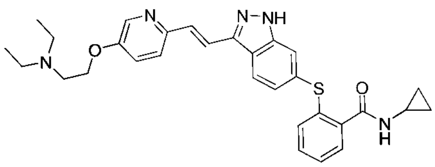,
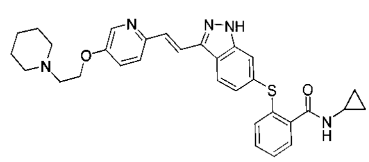,

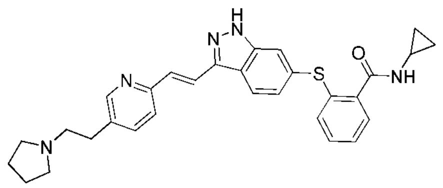,
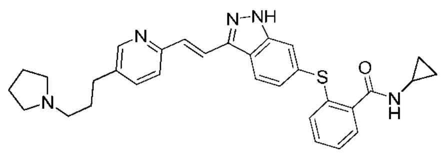,

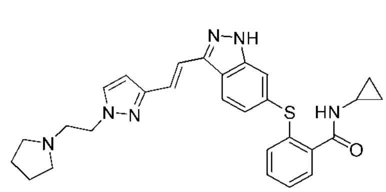,
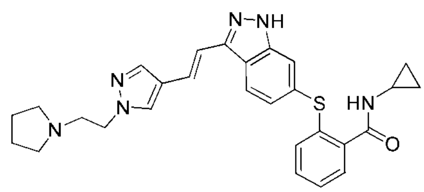,
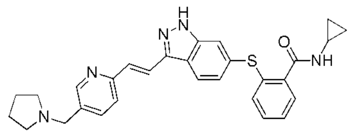,

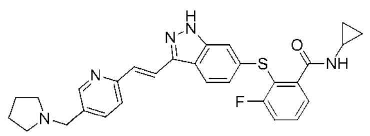,
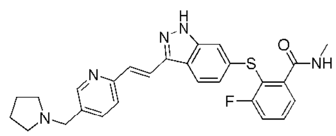,
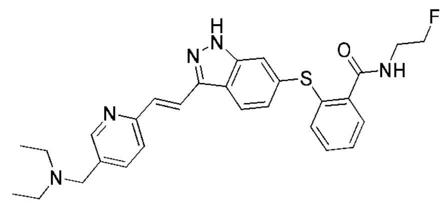,

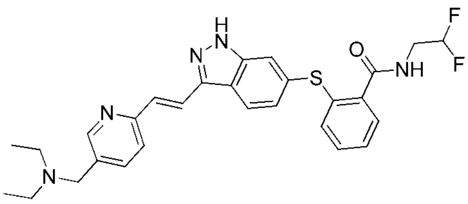, and
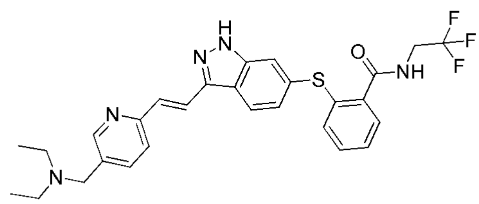,

or a pharmaceutically acceptable prodrug, a pharmaceutically active metabolite, and/or a pharmaceutically acceptable salt thereof.

In one aspect, the present invention provides a compound having a structure selected from the group consisting of:

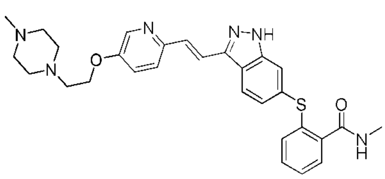,
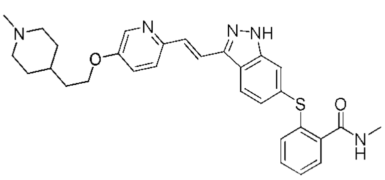,
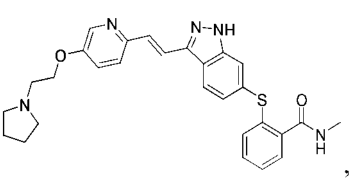,

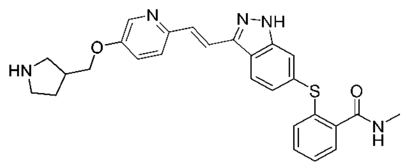 , 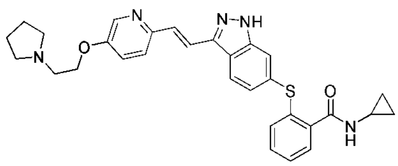 , 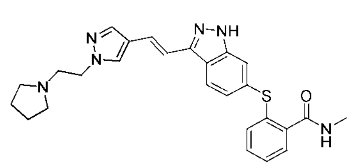 ,

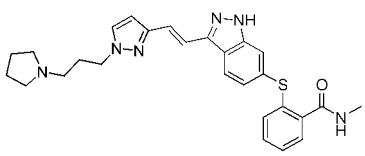 , 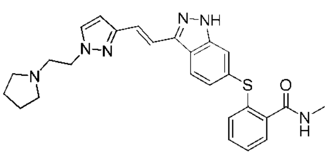 , 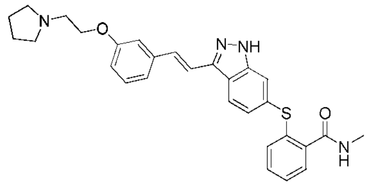 ,

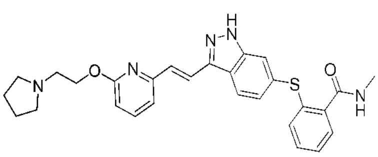 , 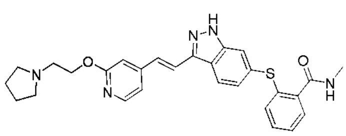 , 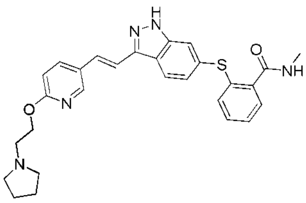 ,

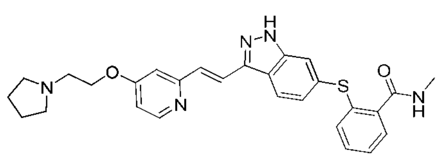 , 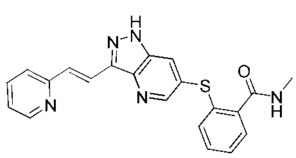 , 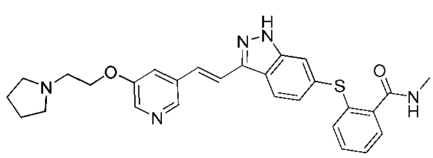 ,

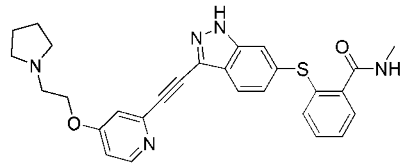 , 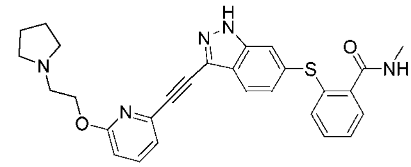 , 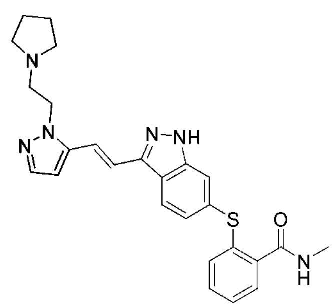 ,

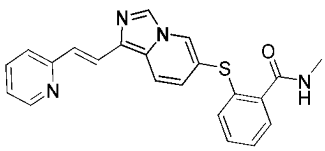 , 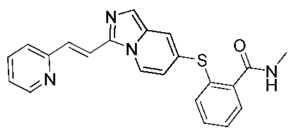 , 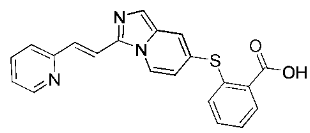 ,

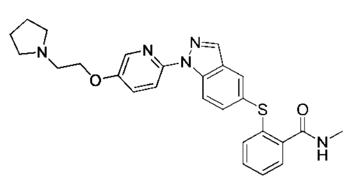 , 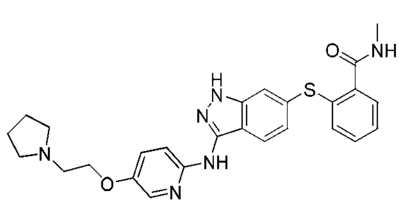 , 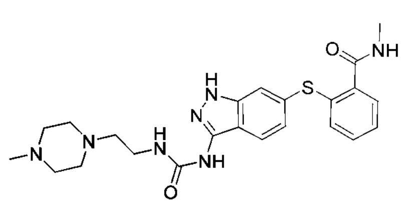 ,

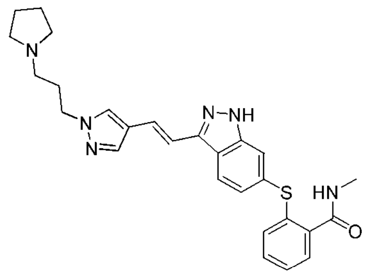, 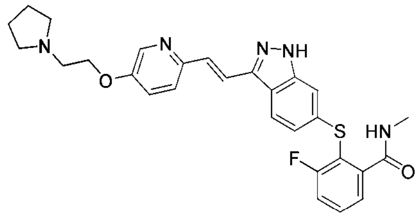,

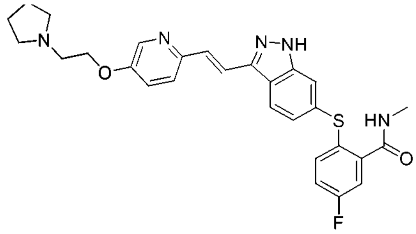, 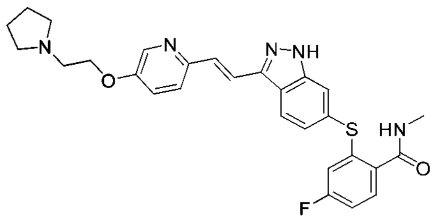,

and

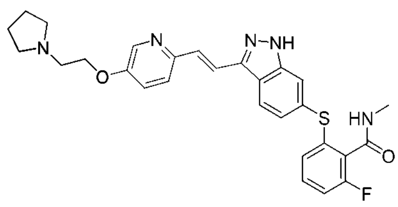,

or a pharmaceutically acceptable prodrug, a pharmaceutically active metabolite, and/or a pharmaceutically acceptable salt thereof.

In one aspect, the present invention provides a compound having a structure selected from the group consisting of:

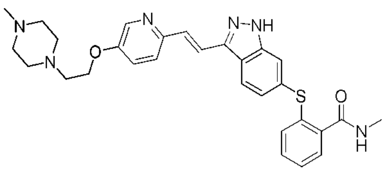, 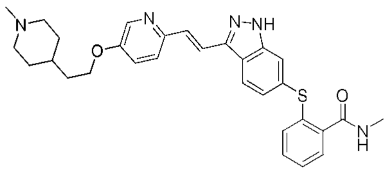, 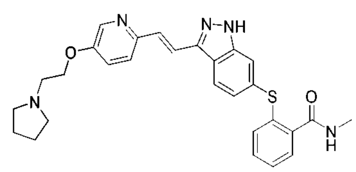,

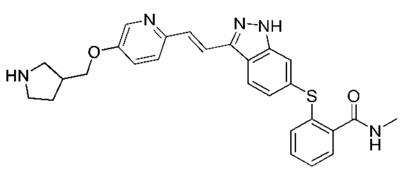, 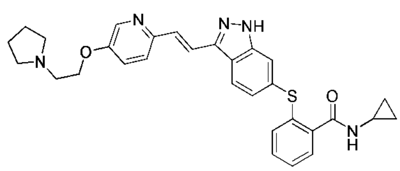, 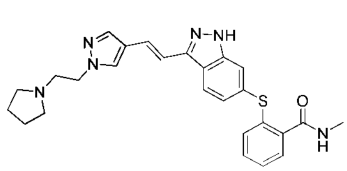,

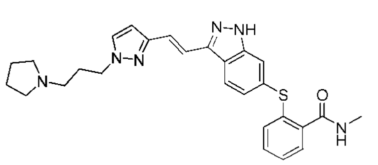, 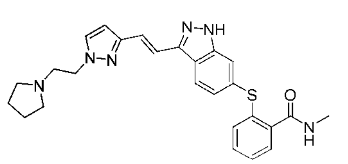, 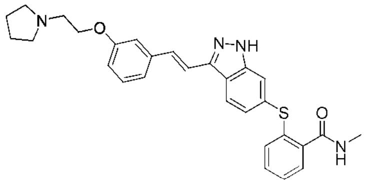,

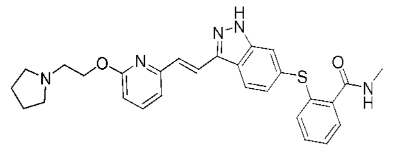, 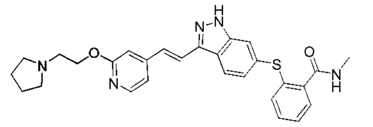, 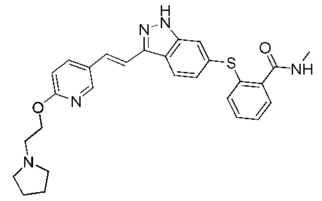,

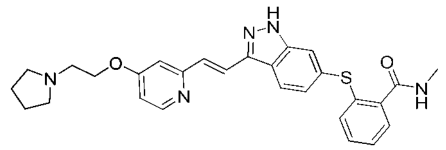, 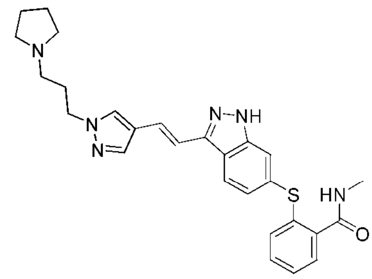,

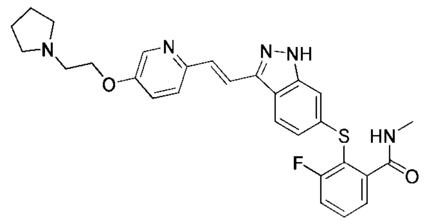, 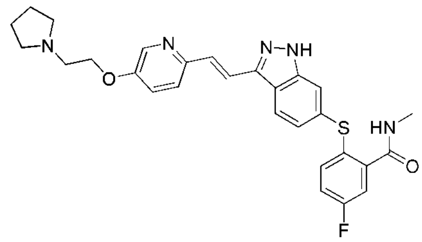,

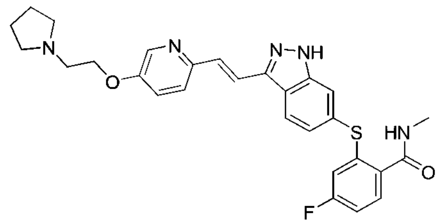, and 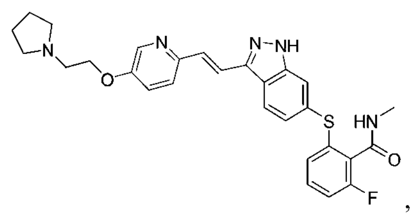,

or a pharmaceutically acceptable prodrug, a pharmaceutically active metabolite, and/or a pharmaceutically acceptable salt thereof.

In one aspect, the present invention provides a compound having a structure selected from the group consisting of:

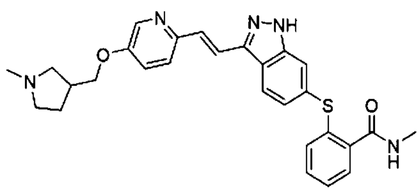, 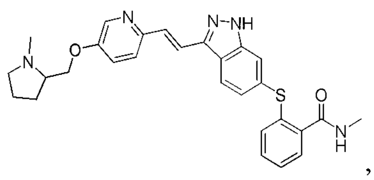, 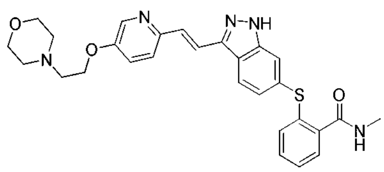,

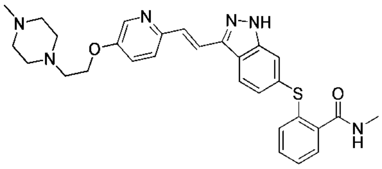, 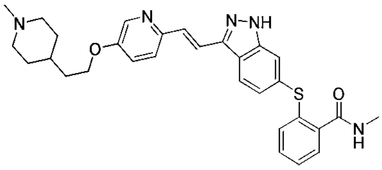, 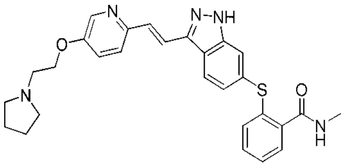,

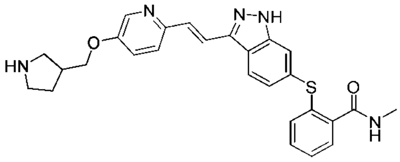, 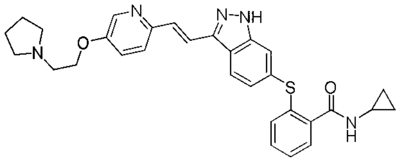, 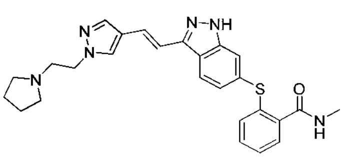,

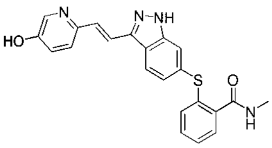, 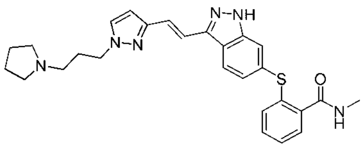, 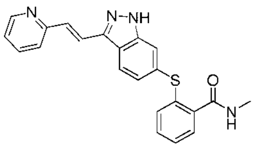,

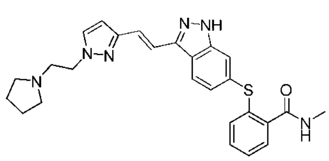, 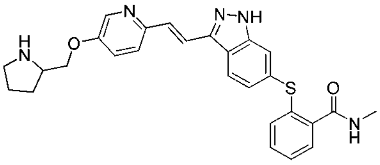, 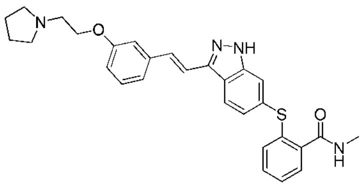,

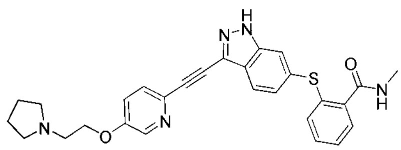, 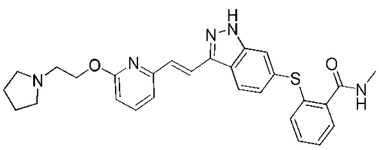, 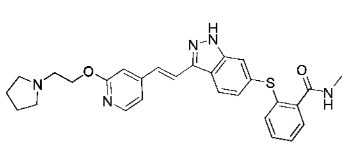,

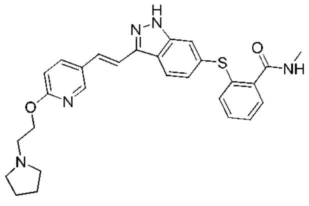, 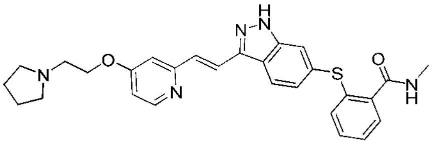, 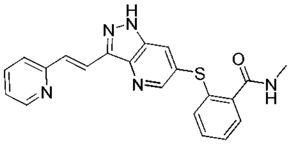,

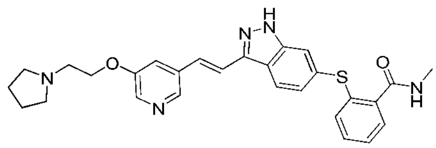, 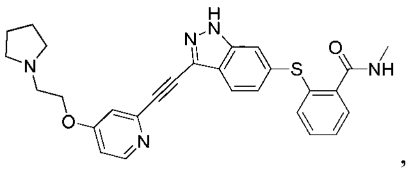,

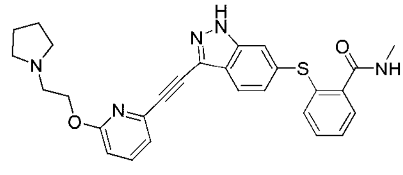, 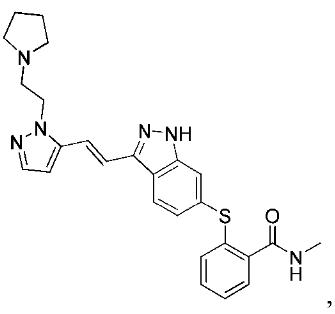, 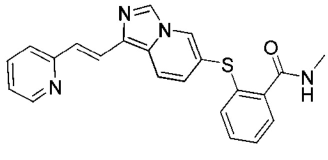,

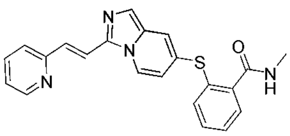,
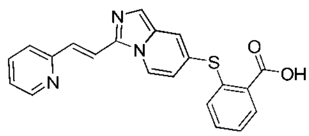,
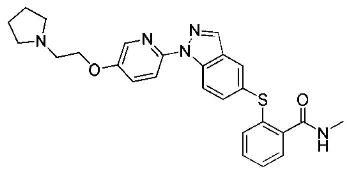,

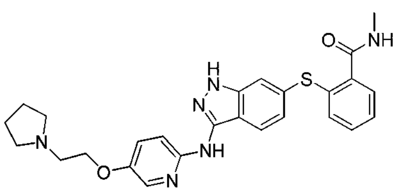,
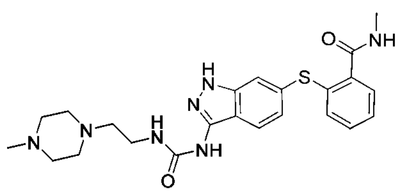,
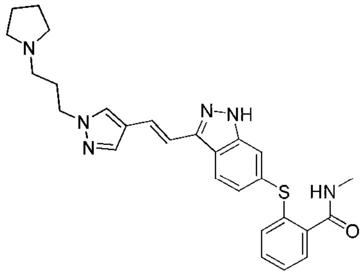,

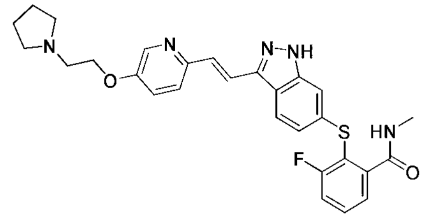,
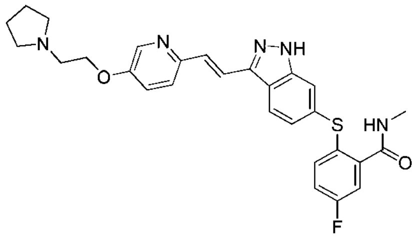,

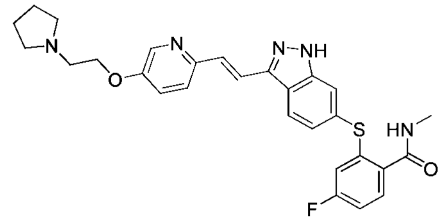,
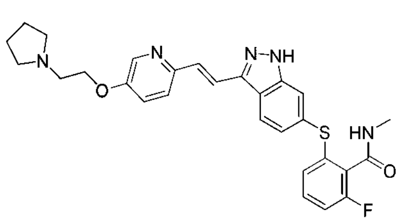,

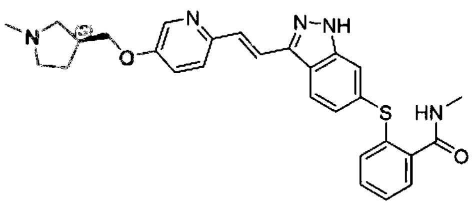,
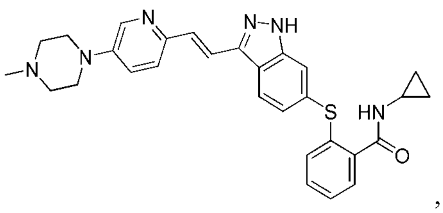,

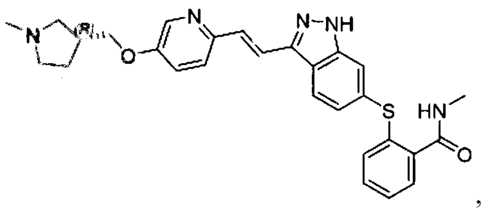,
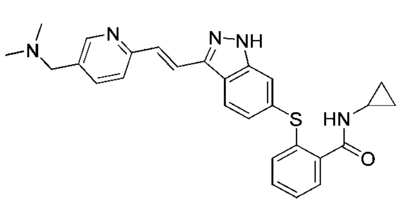,

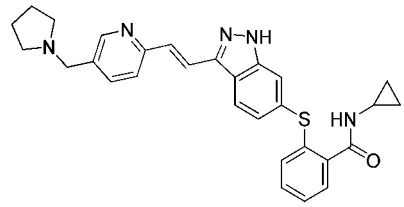,
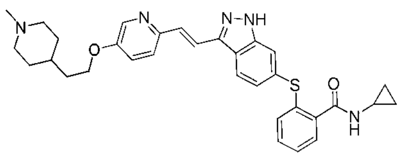,

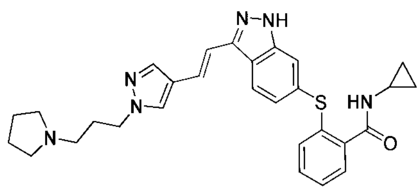,
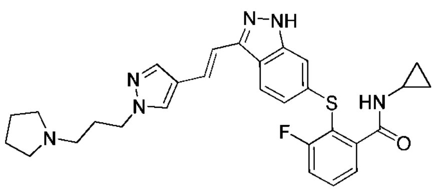,

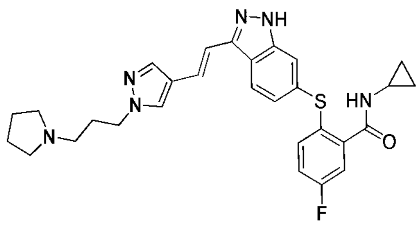,
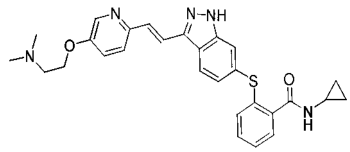,

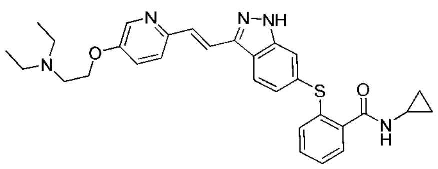,
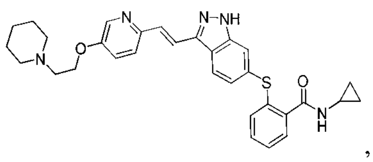,

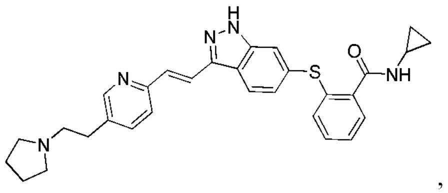,
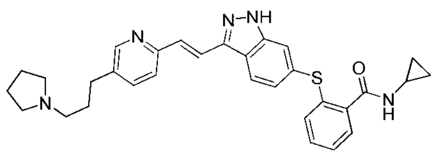,

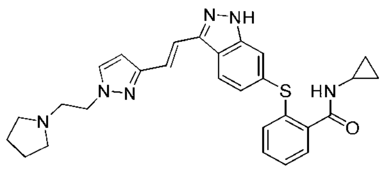,
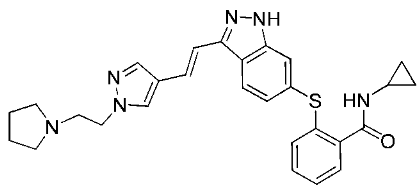,
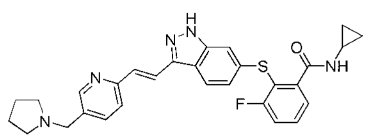,

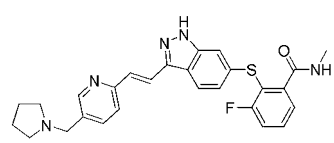,
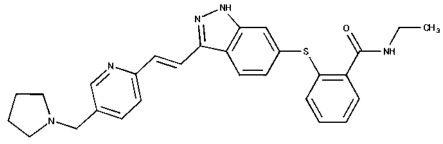,

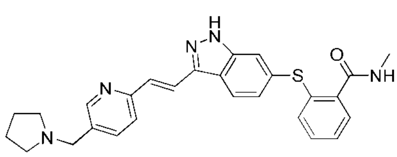,
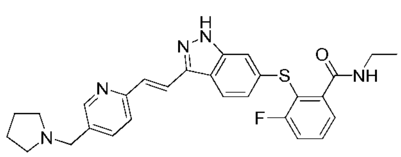,

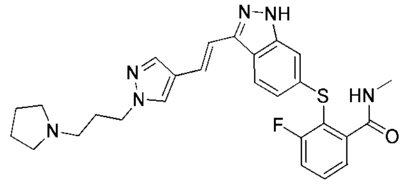,

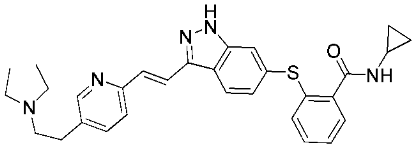,

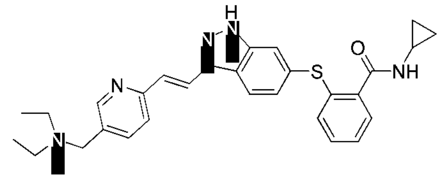,

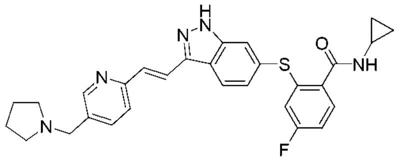,

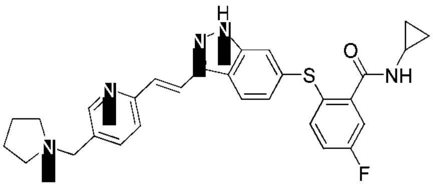,

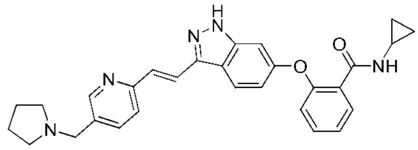,

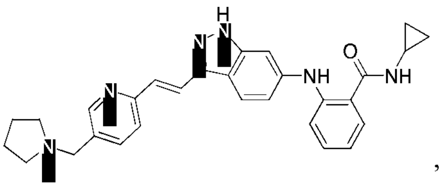,

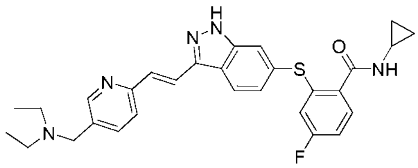,

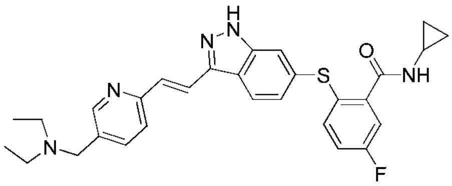,

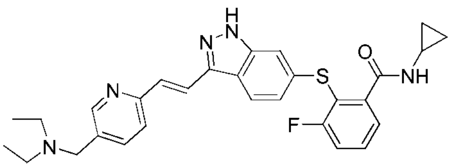,

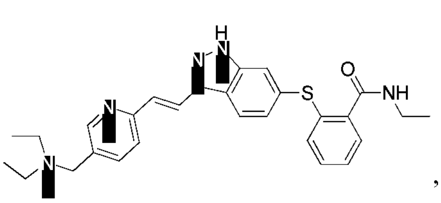,

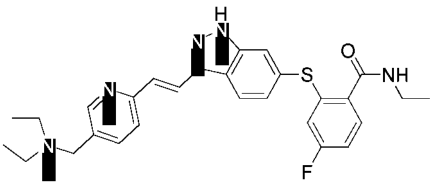,

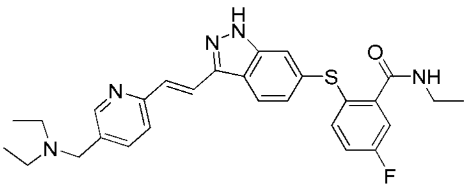,

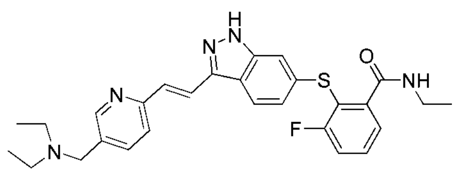,

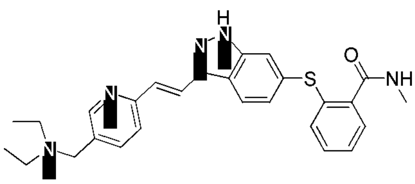,

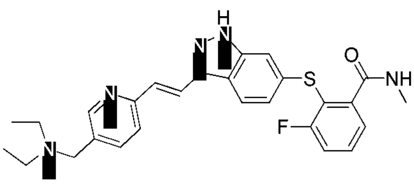,

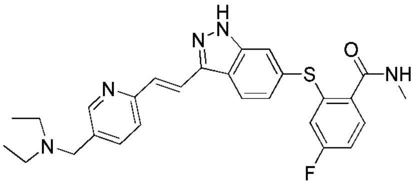, 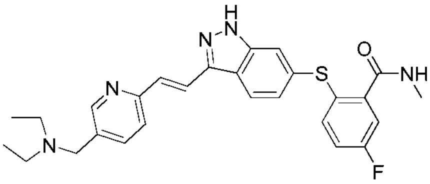,

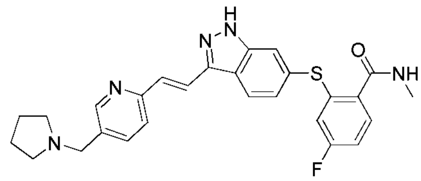, 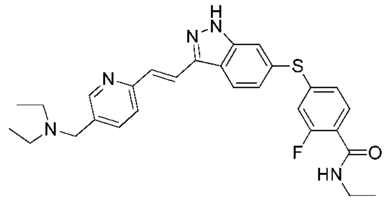,

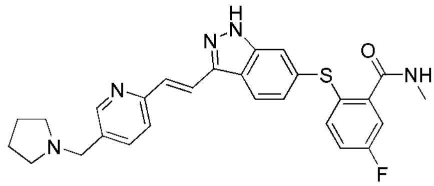, 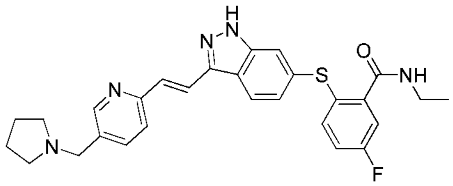,

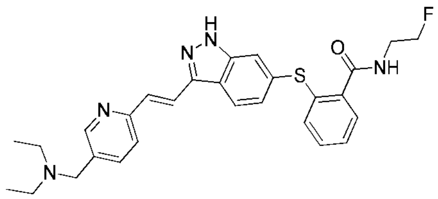, 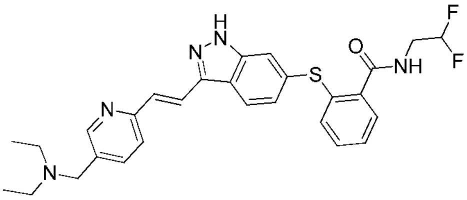,

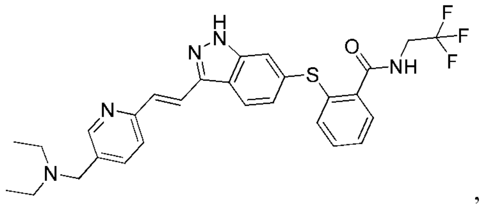, 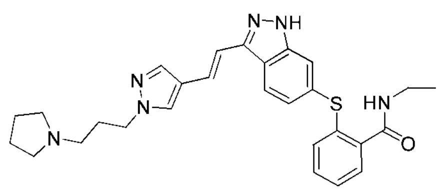,

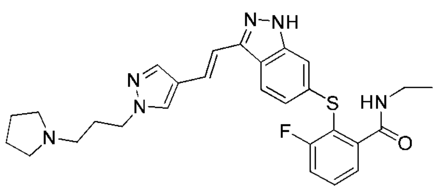, 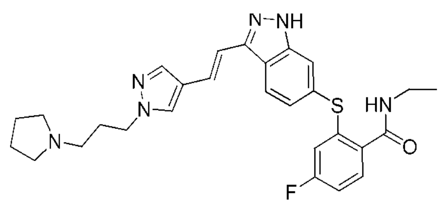,

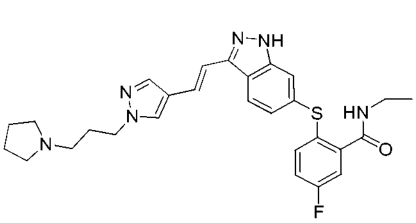, and 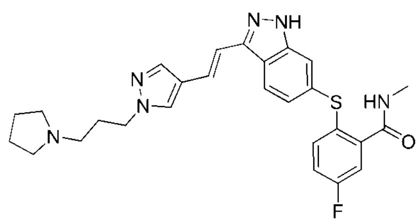,

or a pharmaceutically acceptable prodrug, a pharmaceutically active metabolite, and/or a pharmaceutically acceptable salt thereof.

In one aspect, the present invention provides a compound having a structure selected from the group consisting

of:

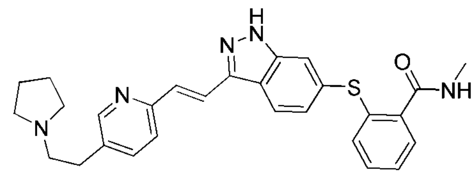,
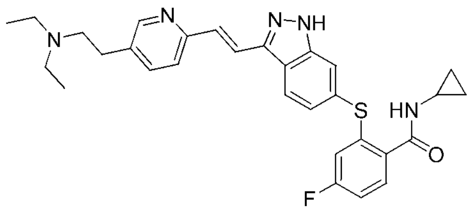,

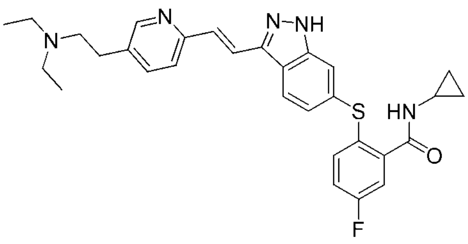,
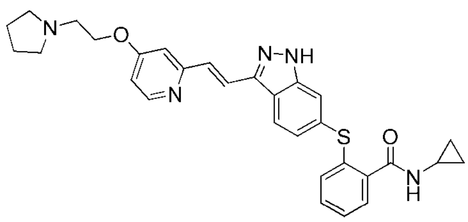,

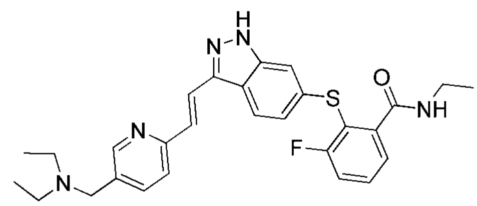,
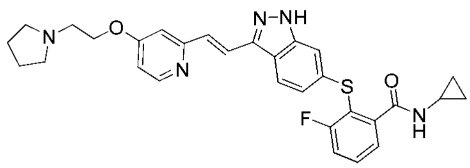,

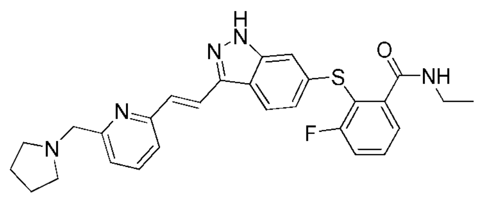,
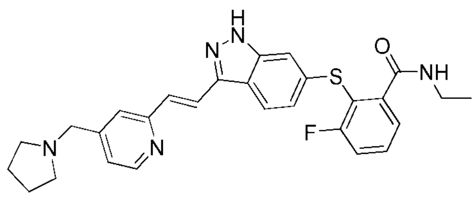,

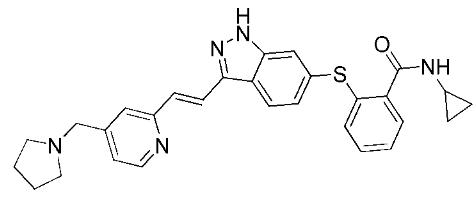,
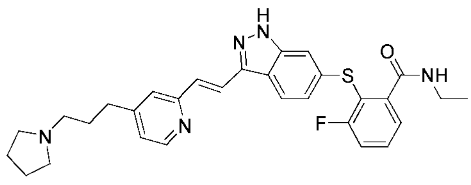,

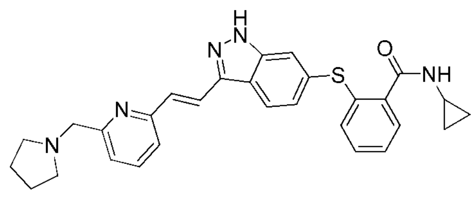,
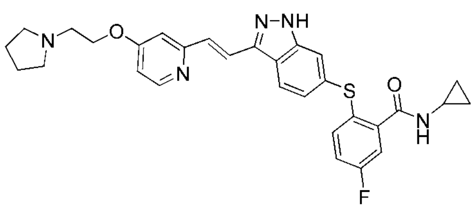,

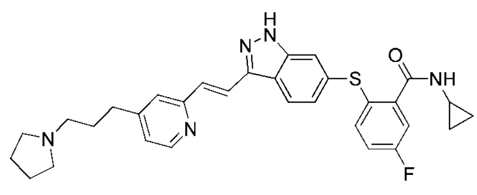,
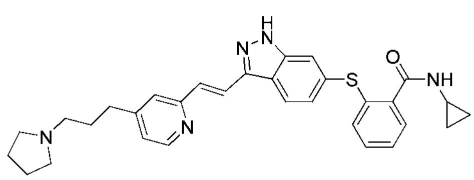,

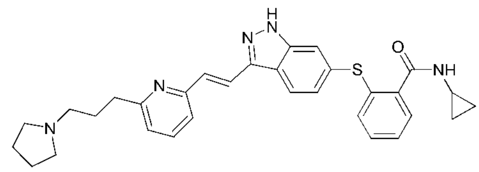, 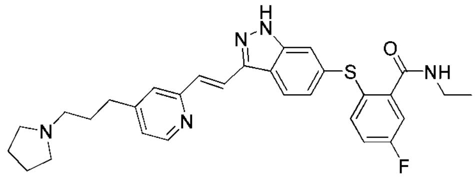,

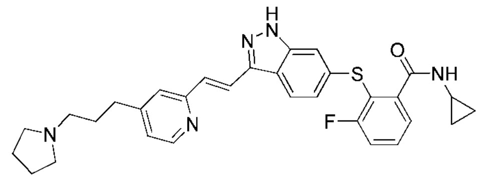, 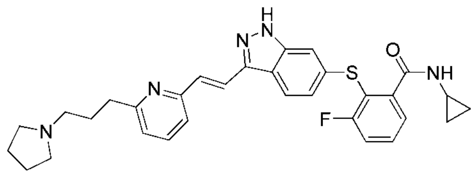,

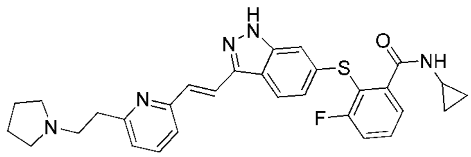, 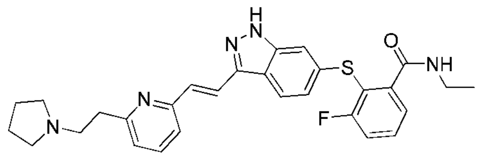,

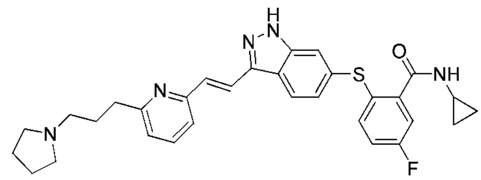, 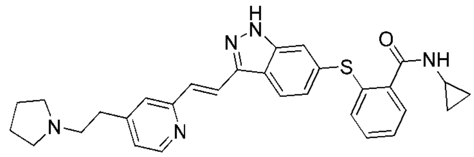,

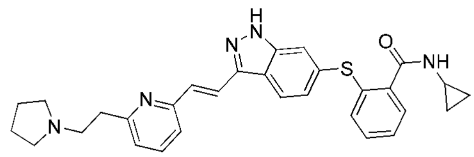, 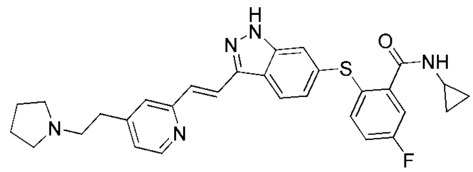,

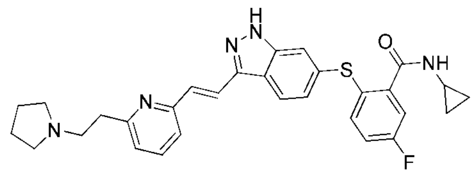, 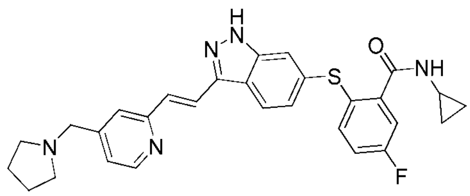,

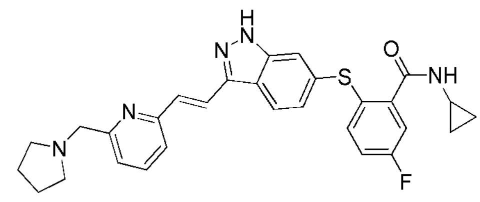, 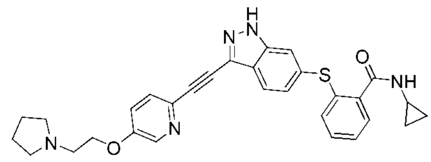

or a pharmaceutically acceptable prodrug, a pharmaceutically active metabolite, and/or a pharmaceutically acceptable salt thereof.

Pharmaceutical Compositions

In one aspect, the present invention provides a pharmaceutical composition comprising a compound of the present invention, or a pharmaceutically acceptable prodrug, a pharmaceutically active metabolite, and/or a pharmaceutically acceptable salt thereof, and optionally a carrier.

In addition to the active compound, the pharmaceutical composition of the present invention may contain one or more excipients, which may be selected from the group consisting of fillers (diluents), binders, wetting agents, disintegrants, etc. Depending on the method of administration, the composition may contain from 0.1 to 99% by weight of the active compound.

The pharmaceutical composition containing the active ingredient may be in a form suitable for ocular administration, such as aqueous or oily suspensions, dispersible powders or granules, emulsions, or pastes. The composition for ocular administration may be prepared by any method known in the art for preparing pharmaceutical compositions, and may contain binders, fillers, lubricants, disintegrants, or pharmaceutically acceptable wetting agents, etc. The composition may further contain one or more ingredients selected from the group consisting of sweeteners, flavoring agents, coloring agents, and preservatives.

Aqueous suspensions may contain the active substance and excipients suitable for preparing aqueous suspensions for mixing. Aqueous suspensions may also contain one or more preservatives, for example, one or more coloring agents, one or more flavoring agents, and one or more sweeteners. Oily suspensions may be formulated by suspending the active ingredient in a vegetable oil. Oily suspensions may contain thickening agents.

Specifically, the pharmaceutical composition may be administered via any suitable route, such as gastrointestinal administration (e.g., oral, sublingual, rectal administration) or parenteral administration (e.g., intravenous, intramuscular, intranasal, intraocular, intracerebral, intravaginal, intraperitoneal, transdermal, subcutaneous, intradermal, respiratory administration, etc.). In some embodiments of the present invention, the pharmaceutical composition is administered via an intraocular route (e.g., eye drop administration, eye ointment administration, subconjunctival injection administration, intraocular injection administration).

Specifically, the pharmaceutical composition may be in any suitable dosage form, for example, gastrointestinal dosage forms, including, but not limited to, tablets, pills, powders, granules, capsules, lozenges, syrups, liquids, emulsions, suspensions, etc.; parenteral dosage forms, for example, injectable dosage forms: such as injections (e.g., for subcutaneous injection, intravenous injection, intramuscular injection, intraperitoneal injection), respiratory dosage forms: such as sprays, aerosols, powder aerosols, etc., dermal dosage forms: such as topical solutions, lotions, ointments, plasters, pastes, patches, etc., mucosal dosage forms: such as eye drops, ophthalmic ointments, nasal drops, gargles, sublingual tablets, etc., cavity dosage forms: such as suppositories, aerosols, effervescent tablets, drops, dropping pills, etc., for rectal, vaginal, urethral, nasal, ear canal, etc.

In some embodiments of the present invention, the aforementioned pharmaceutical composition is an ophthalmic formulation, such as eye drops or ophthalmic ointment.

In one aspect, the present invention provides an ophthalmic formulation I, comprising:

a. the compound of the present invention or a pharmaceutically acceptable salt thereof;
b. a co-solvent;
c. a pharmaceutically acceptable excipient;

wherein the pharmaceutically acceptable excipient comprises one or more of a solubilizer, a stabilizer, a viscosity modifier, and a buffer.

Specifically, the pharmaceutically acceptable salts of the compound include, but are not limited to, aspartate, glutamate, malonate, salicylate, maleate, fumarate, succinate, benzoate, propionate, acetate, decanoate, stearate, oleate, hexanoate, octanoate, tartrate, citrate, malate, gluconate, glycolate, lactate, hydrobromide, hydrochloride, sulfate, nitrate, phosphate, methanesulfonate, benzenesulfonate, p-toluenesulfonate, ethanesulfonate, isethionate. Specifically, the concentration of the compound in ophthalmic formulation I is from 0.01% w/v (0.1 mg/mL) to 2.5% w/v (25 mg/mL) (e.g., 0.01%, 0.05%, 0.1%, 0.2%, 0.4%, 0.5%, 0.6%, 0.8%, 1%, 2%, 2.2%, 2.4%, 2.5% w/v), particularly from 0.1% w/v (1 mg/mL) to 1.5% w/v (15 mg/mL) (the compound concentration described herein refers to the concentration of the free form, even if it is added to the formulation composition in a salt form). Specifically, the co-solvent is selected from: polyethylene glycol (particularly liquid polyethylene glycol, e.g., polyethylene glycol 200, 300, 400), propylene glycol, glycerol; in some embodiments of the present invention, the co-solvent is polyethylene glycol 400.

Specifically, the concentration of the co-solvent in ophthalmic formulation I is from 0.1% v/v to 10% v/v (e.g., 0.1%, 0.2%, 0.4%, 0.5%, 0.6%, 0.8%, 1%, 2%, 4%, 5%, 6%, 8%, 10% v/v), particularly from 0.5% v/v to 5% v/v.

In one embodiment of the present invention, the solubilizer is a cyclodextrin, including a β-cyclodextrin derivative, a γ-cyclodextrin derivative, or a combination thereof. Literature indicates that cyclodextrins can increase the contact time of compounds with the ocular surface and have the potential to reduce ocular irritation, as exemplified in publications such as P. Jansook et al. Eur J Pharm Biopharm, 76, 208-214 (2010); T. J..arvinen et al. J Ocul Pharmacol Ther, 11, 95-106 (1995); P. Jarho et al. J Pharm Pharmacol, 48, 264-270 (1996); P. Suhonen et al. Pharmaceutical Research, 12, 529-533 (1995). A particularly preferred β-cyclodextrin derivative is a hydroxyalkyl-β-cyclodextrin, for example, hydroxypropyl-β-cyclodextrin (HP-β-CD). Another particularly preferred β-cyclodextrin derivative is a sulfoalkyl ether-β-cyclodextrin, for example,

sulfobutyl ether-β-cyclodextrin (SBE-β-CD). A particularly preferred γ-cyclodextrin derivative is a hydroxyalkyl-γ-cyclodextrin, for example, hydroxypropyl-γ-cyclodextrin (HP-γ-CD). Typically, one of the aforementioned three is employed. Sulfobutyl-β-cyclodextrin has compatibility issues with the viscosity modifier gellan gum, but can be used in formulations containing other viscosity modifiers besides gellan gum. In some embodiments of the present invention, the solubilizer is hydroxypropyl-β-cyclodextrin (HP-β-CD).

Specifically, the concentration of the solubilizer in ophthalmic formulation I is from 1% w/v to 30% w/v (e.g., 1%, 5%, 6%, 8%, 10%, 12%, 14%, 15%, 20%, 25%, 30% w/v), particularly from 5% w/v to 15% w/v.

Specifically, the stabilizer is a polymer, for example: poloxamer, polyethenyl alcohol, polyethenylpyrrolidone, or a combination thereof. Polymers at suitable concentrations can inhibit the precipitation of the compound from solution and increase the viscosity of the formulation composition. Studies have shown that amphiphilic polymers can also reduce surface tension and increase the solubility of the compound. In some embodiments of the present invention, the stabilizer is poloxamer 188.

Specifically, the concentration of the stabilizer in ophthalmic formulation I is from 0.1% w/v to 10% w/v (e.g., 0.1 %, 0.5%, 0.8%, 1%, 1.2%, 1.5%, 2%, 3%, 4%, 5%, 6%, 8%, 10% w/v), particularly from 0.5% w/v to 2% w/v.

Specifically, the viscosity modifier is selected from: cellulose derivatives (e.g., methylcellulose, hydroxypropyl cellulose, hydroxypropyl methylcellulose), biocompatible polysaccharides (e.g., gellan gum, xanthan gum, chitosan and its derivatives, sodium alginate, sodium hyaluronate), carbomer, or a combination thereof. A wide variety of viscosity modifiers are expected to be selectable. Depending on the type, the selection is primarily made from the aforementioned viscosity modifiers. Gellan gum is preferred as a viscosity modifier due to its excellent ion-sensitive gelling properties. In the presence of monovalent or divalent cations (such as sodium, potassium, calcium ions), it rapidly gels to form a film structure covering the surface, which is beneficial for prolonged drug retention and release.

In some embodiments of the present invention, the viscosity modifier is gellan gum, which is present in ophthalmic formulation I at a concentration ranging from 0.01% v/v to 1% v/v (e.g., 0.01%, 0.05%, 0.1%, 0.15%, 0.2%, 0.3%, 0.4%, 0.5%, 0.6%, 0.8%, 1% v/v), particularly from 0.1% v/v to 0.5% v/v.

In some embodiments of the present invention, the viscosity modifier is carbomer, which is present in ophthalmic formulation I at a concentration ranging from 0.01% v/v to 1% v/v (e.g., 0.01%, 0.05%, 0.1%, 0.15%, 0.2%, 0.3%, 0.4%, 0.5%, 0.6%, 0.8%, 1% v/v), particularly from 0.1% v/v to 0.6% v/v.

In some embodiments of the present invention, the viscosity modifier is hydroxypropyl methylcellulose, which is present in ophthalmic formulation I at a concentration ranging from 0.01% v/v to 1% v/v (e.g., 0.01%, 0.05%, 0.1%, 0.15%, 0.2%, 0.3%, 0.4%, 0.5%, 0.6%, 0.8%, 1% v/v).

Specifically, the buffer is selected from: borate, phosphate, bicarbonate, carbonate, citrate, tetraborate, hydrogen phosphate, tromethamine, 2-(2-hydroxyethyl)morpholine, tris(hydroxymethyl)aminomethane, L-carnosine. For formulation compositions containing ion-sensitive viscosity modifiers (e.g., gellan gum), buffers containing large amounts of monovalent or divalent cations, such as phosphates, should be avoided. In some embodiments of the present invention, the buffer is tris(hydroxymethyl)aminomethane-hydrochloric acid buffer.

Specifically, buffers have different buffering capacities, but the concentration is typically from 5 mM to 100 mM (e.g., 5, 10, 20, 30, 40, 50, 60, 70, 80, 90, 100 mM).

In one embodiment of the present invention, the pharmaceutically acceptable excipient further comprises a surfactant.

Specifically, the surfactant is selected from: polyoxyethylene castor oil, tyloxapol, polysorbate, polyoxyl hydrogenated castor oil, or a combination thereof. Polyoxyethylene castor oil EL has a more universal solubilizing effect. In some embodiments of the present invention, the surfactant is polyoxyethylene castor oil EL. Specifically, the concentration of the surfactant in ophthalmic formulation I is from 0.1% w/v to 10% w/v (e.g., 0.1%, 0.5%, 0.8%, 1%, 1.5%, 2%, 3%, 4%, 5%, 6%, 8%, 10% w/v), particularly from 0.5% w/v to 2% w/v.

In one embodiment of the present invention, the pharmaceutically acceptable excipient further comprises a pH adjuster for adjusting the pH of the ophthalmic formulation to a desired level. Specifically, the pH adjuster is selected from one or more of hydrochloric acid, sodium hydroxide, acetic acid or salts thereof, citric acid or salts thereof, fumaric acid, succinic acid, sorbic acid, phosphoric acid, sodium dihydrogen phosphate, disodium hydrogen phosphate, boric acid, borax, tartaric acid or salts thereof.

In one embodiment of the present invention, the pharmaceutically acceptable excipient further comprises an osmotic pressure adjuster for adjusting the osmotic pressure of the ophthalmic formulation to a desired level (close to or slightly above physiological osmotic pressure is preferred). Specifically, the osmotic pressure adjuster is selected from one or more of: glucose, sodium chloride, potassium chloride, mannitol, sorbitol, sodium citrate, potassium citrate, and glycerol. In formulation compositions containing gellan gum, the amount of osmotic pressure adjusters containing metal cations (e.g., sodium chloride) should be controlled or their use should be avoided.

In one embodiment of the present invention, the volume ratio of b. co-solvent to c. pharmaceutically acceptable excipient mixture is 1-10:90-99, for example, 5:95.

In one embodiment of the present invention, the pharmaceutically acceptable excipient further comprises a

preservative, including but not limited to polyhexamethylene biguanide, polymeric quaternary ammonium compounds, chlorine-containing preservatives, chlorite preservatives, or others.

In one embodiment of the present invention, the pharmaceutically acceptable excipient further comprises a solvent, particularly water, for example, deionized water.

In one embodiment of the present invention, ophthalmic formulation I comprises:

a the compound of the present invention or a pharmaceutically acceptable salt thereof;
b polyethylene glycol 400;
c hydroxypropyl-β-cyclodextrin, poloxamer 188, gellan gum, tris(hydroxymethyl)aminomethane-hydrochloric acid buffer, deionized water.

In one embodiment of the present invention, ophthalmic formulation I comprises:

a the compound of the present invention or a pharmaceutically acceptable salt thereof;
b polyethylene glycol 400;
c hydroxypropyl-β-cyclodextrin, poloxamer 188, carbomer, tris(hydroxymethyl)aminomethane-hydrochloric acid buffer, deionized water.

In one embodiment of the present invention, ophthalmic formulation I comprises:

a the compound of the present invention or a pharmaceutically acceptable salt thereof;
b polyethylene glycol 400;
c hydroxypropyl-β-cyclodextrin, poloxamer 188, hydroxypropyl methylcellulose, tris(hydroxymethyl)aminomethane-hydrochloric acid buffer, deionized water.

In some embodiments of the present invention, in ophthalmic formulation I, the compound is compound I-1 to I-11, I-13 to I-139, for example:

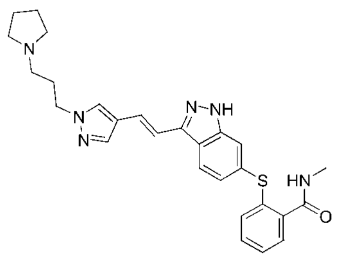
I-32,

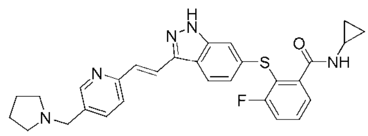
I-67,

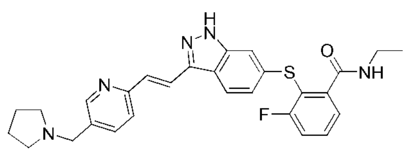
I-75,

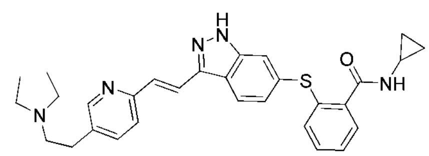
I-77,

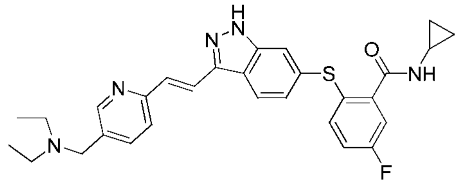
I-84,

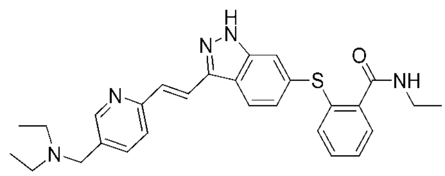
I-86,

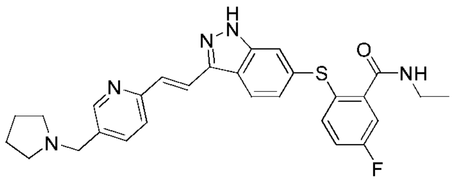 I-97, 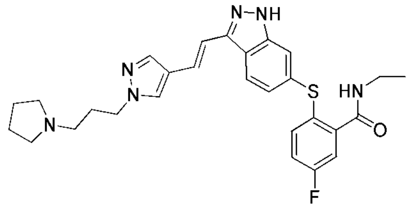 I-104.

Specifically, ophthalmic formulation I is a homogeneous formulation, such as a solution, emulsion, suspension, or gel, particularly a solution.

In one embodiment of the present invention, ophthalmic formulation I is an eye drop.

Specifically, the pH of ophthalmic formulation I is from 5 to 9 (e.g., 5, 5.5, 6, 6.5, 7, 7.5, 8, 8.5, 9), particularly from 6 to 8, e.g., 6.5-7.5.

Specifically, the osmotic pressure of ophthalmic formulation I is from 200 mOsm/kg to 500 mOsm/kg (e.g., 200, 250, 300, 350, 400, 450, 500 mOsm/kg), particularly from 250 mOsm/kg to 450 mOsm/kg.

In one aspect, the present invention provides a method for preparing an ophthalmic formulation I, comprising the steps of:

(1) adding a compound of the present invention or a pharmaceutically acceptable salt thereof into a co-solvent, and dispersing;
(2) adding the mixture obtained in step (1) into the pharmaceutically acceptable excipient, and mixing;

optionally, (3) adding a pH adjuster and/or an osmotic pressure adjuster to adjust the pH and osmotic pressure of the mixture obtained in step (2) to a desired level;
optionally, (4) adding a preservative.

Specifically, the dispersing in step (1) can be performed by means such as sonication, mechanical stirring, magnetic stirring, vortex shaking, or shearing, particularly sonication.

Specifically, the dispersing in step (1) is dispersing until the compound is dissolved or nearly dissolved, and heating (to 50°C) if necessary.

Specifically, the mixing in step (2) can be performed by means such as sonication, mechanical stirring, magnetic stirring, vortex shaking, or shearing, particularly vortex shaking.

In one embodiment of the present invention, step (2) comprises: adding the mixture obtained in step (1) into a buffer containing a solubilizer, a stabilizer, a surfactant, and a viscosity modifier, and mixing.

In one embodiment of the present invention, the viscosity modifier is gellan gum, and before step (2), the method further comprises adjusting the pH of the mixture obtained in step (1) to not less than 4 (e.g., 4, 5, 6, 7) to completely or partially dissolve the compound.

In one aspect, the present invention provides an ophthalmic formulation II, the raw materials for preparing the ophthalmic formulation II comprise:

a the compound of the present invention;
b a pharmaceutically acceptable excipient;
c an acidic pH adjuster;
d a basic pH adjuster;

wherein the pharmaceutically acceptable excipient comprises one or more of a solubilizer, a stabilizer, a surfactant, a viscosity modifier, and a buffer.

Specifically, the ophthalmic formulation II is prepared by mixing the raw materials, particularly prepared by the preparation method for ophthalmic formulation II as described below.

Specifically, the concentration of the compound in ophthalmic formulation II is from 0.01% w/v (0.1 mg/mL) to 2.5% w/v (25 mg/mL) (e.g., 0.01%, 0.05%, 0.1%, 0.2%, 0.4%, 0.5%, 0.6%, 0.8%, 1%, 2%, 2.2%, 2.4%, 2.5% w/v). In one embodiment of the present invention, the solubilizer is a cyclodextrin, including a β-cyclodextrin derivative, a γ-cyclodextrin derivative, or a combination thereof. Literature indicates that cyclodextrins can increase the contact time of compounds with the ocular surface and have the potential to reduce irritation, as listed in references such as P. Jansook etc. Eur J Pharm Biopharm, 76, 208-214 (2010). T. J"arvinen etc. J Ocul Pharmacol Ther, 11, 95-106(1995). P. Jarho etc. J Pharm Pharmacol, 48, 264-270 (1996). P. Suhonen etc. Pharmaceutical Research, 12, 529-533 (1995). A particularly preferred β-cyclodextrin derivative is a hydroxyalkyl-β-cyclodextrin, for example, hydroxypropyl-β-cyclodextrin (HP-β-CD). Another particularly preferred β-cyclodextrin derivative is a sulfoalkyl ether-β-cyclodextrin, for example, sulfobutyl ether-β-cyclodextrin (SBE-β-CD). A particularly preferred γ-cyclodextrin derivative is a hydroxyalkyl-γ-cyclodextrin, for example, hydroxypropyl-γ-cyclodextrin (HP-γ-CD). Typically, one of the aforementioned three is employed. Sulfobutyl-β-cyclodextrin has compatibility issues with the viscosity modifier gellan gum, but can be used in formulations containing other viscosity modifiers besides gellan gum. In some embodiments of the present invention, the solubilizer is hydroxypropyl-β-cyclodextrin (HP-β-CD). Specifically, the concentration of the solubilizer in ophthalmic formulation II is from 1% w/v to 30% w/v (e.g., 1%, 2%, 4%, 5%, 6%, 8%, 10%, 12%, 14%, 15%, 20%, 25%, 30% w/v).

Specifically, the stabilizer is a polymer, for example: poloxamer, polyethenyl alcohol, polyethenylpyrrolidone, or a combination thereof. Polymers at suitable concentrations can inhibit the precipitation of compounds from solution and increase the viscosity of the formulation composition; some amphiphilic polymers can also reduce surface tension and increase the solubility of compounds. In some preferred embodiments, poloxamer 188 is more advantageous due to its suitable viscosity.

In some embodiments of the present invention, the stabilizer is poloxamer 188.

Specifically, the concentration of the stabilizer in ophthalmic formulation II is from 0.1% w/v to 10% w/v (e.g., 0.1%, 0.5%, 0.8%, 1%, 1.2%, 1.5%, 2%, 3%, 4%, 5%, 6%, 8%, 10% w/v), particularly from 0.5% w/v to 2% w/v. Specifically, the viscosity modifier is selected from: cellulose derivatives (e.g., methylcellulose, hydroxypropyl cellulose, hydroxypropyl methylcellulose), biocompatible polysaccharides (e.g., gellan gum, xanthan gum, chitosan and its derivatives, sodium alginate, sodium hyaluronate), carbomer, or a combination thereof. A wide variety of viscosity modifiers are expected to be selectable. Depending on the type, the selection is primarily made from the aforementioned viscosity modifiers. Gellan gum is preferred as a viscosity modifier due to its excellent ion-sensitive gelling properties. In the presence of monovalent or divalent cations (such as sodium, potassium, calcium ions), it rapidly gels to form a film structure covering the surface, which is beneficial for prolonged drug retention and release.

In some embodiments of the present invention, the viscosity modifier is gellan gum, which is present in ophthalmic formulation II at a concentration ranging from 0.01% v/v to 1% v/v (e.g., 0.01%, 0.05%, 0.1%, 0.15%, 0.2%, 0.3%, 0.4%, 0.5%, 0.6%, 0.8%, 1% v/v), particularly from 0.1% v/v to 0.5% v/v.

In some embodiments of the present invention, the viscosity modifier is carbomer, which is present in ophthalmic formulation II at a concentration ranging from 0.01% v/v to 1% v/v (e.g., 0.01%, 0.05%, 0.1%, 0.15%, 0.2%, 0.3%, 0.4%, 0.5%, 0.6%, 0.8%, 1% v/v), particularly from 0.1% v/v to 0.6% v/v.

In some embodiments of the present invention, the viscosity modifier is hydroxypropyl methylcellulose, which is present in ophthalmic formulation II at a concentration ranging from 0.01% v/v to 1% v/v (e.g., 0.01%, 0.05%, 0.1%, 0.15%, 0.2%, 0.3%, 0.4%, 0.5%, 0.6%, 0.8%, 1% v/v).

Specifically, the buffer is selected from: borate, phosphate, bicarbonate, carbonate, citrate, tetraborate, hydrogen phosphate, tromethamine, 2-(2-hydroxyethyl)morpholine, tris(hydroxymethyl)aminomethane, L-carnosine. For formulation compositions containing ion-sensitive viscosity modifiers (e.g., gellan gum), components containing large amounts of monovalent or divalent cations, such as phosphates, should be avoided. In some embodiments of the present invention, the buffer is tris(hydroxymethyl)aminomethane-hydrochloric acid buffer.

Specifically, buffers have different buffering capacities, but the concentration is typically from 5 mM to 100 mM (e.g., 5, 10, 20, 30, 40, 50, 60, 70, 80, 90, 100 mM).

Specifically, the acidic pH adjuster is selected from: aspartic acid, glutamic acid, malonic acid, salicylic acid, maleic acid, fumaric acid, succinic acid, benzoic acid, propionic acid, acetic acid, decanoic acid, stearic acid, oleic acid, hexanoic acid, octanoic acid, tartaric acid, citric acid, malic acid, gluconic acid, glycolic acid, lactic acid, hydrobromic acid, hydrochloric acid, sulfuric acid, nitric acid, phosphoric acid, methanesulfonic acid, benzenesulfonic acid, p-toluenesulfonic acid, ethanesulfonic acid, hydroxyethanesulfonic acid, preferably selected from: methanesulfonic acid, lactic acid, acetic acid, particularly methanesulfonic acid.

Specifically, the alkaline pH adjuster is selected from: sodium hydroxide, tris(hydroxymethyl)aminomethane, particularly tris(hydroxymethyl)aminomethane.

Specifically, the amounts of the acidic pH adjuster and the alkaline pH adjuster should be based on compound dissolution and the final formulation composition's pH being within an acceptable range.

Specifically, the surfactant is selected from: polyoxyethylene castor oil, tyloxapol, polysorbate, polyoxyl hydrogenated castor oil, or a combination thereof. Polyoxyethylene castor oil EL has a more universal solubilizing effect. In some embodiments of the present invention, the surfactant is polyoxyethylene castor oil EL. Specifically, the concentration of the surfactant in ophthalmic formulation II is from 0.1% w/v to 10% w/v (e.g., 0.1%, 0.5%, 0.8%, 1%, 1.5%, 2%, 3%, 4%, 5%, 6%, 8%, 10% w/v), particularly from 0.5% w/v to 5% w/v.

In one embodiment of the present invention, the pharmaceutically acceptable excipient further comprises an osmotic pressure adjuster for adjusting the osmotic pressure of the ophthalmic formulation to a desired level (close to or slightly above physiological osmotic pressure is preferred). Specifically, the osmotic pressure adjuster is selected from one or more of: glucose, sodium chloride, potassium chloride, mannitol, sorbitol, sodium citrate, potassium citrate, and glycerol. In formulation compositions containing gellan gum, the amount used should be controlled or tonicity adjusters containing metal cations (e.g., sodium chloride) should be avoided.

In one embodiment of the present invention, the pharmaceutically acceptable excipient further comprises a preservative, including but not limited to polyhexamethylene biguanide, polymeric quaternary ammonium compounds, chlorine-containing preservatives, chlorite preservatives, or others.

In one embodiment of the present invention, the pharmaceutically acceptable excipient further comprises a solvent, particularly water, for example, deionized water.

In one embodiment of the present invention, the pharmaceutically acceptable excipient comprises: hydroxypropyl-β-cyclodextrin, poloxamer 188, gellan gum, tris(hydroxymethyl)aminomethane-hydrochloric acid buffer, deionized water.

In one embodiment of the present invention, the raw materials for ophthalmic formulation II comprise:

a the compound of the present invention;
b hydroxypropyl-β-cyclodextrin, poloxamer 188, gellan gum, tris(hydroxymethyl)aminomethane-hydrochloric acid buffer, deionized water;
c methanesulfonic acid;
d tris(hydroxymethyl)aminomethane.

In some embodiments of the present invention, in ophthalmic formulation II, the compound is compound I-1 to I-11, I-13 to I-139, for example:

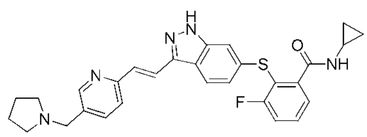 I-67, 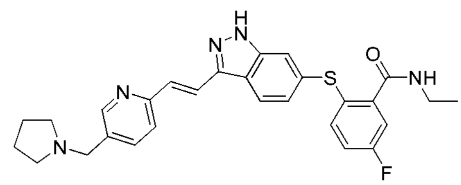 I-97,

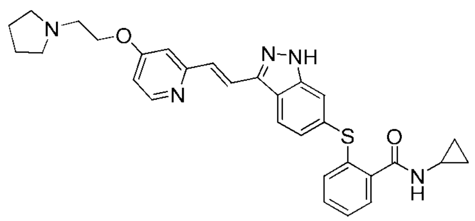 I-110, 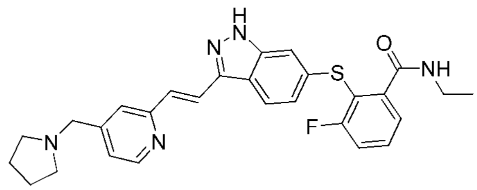 I-114.

Specifically, ophthalmic formulation II is a homogeneous formulation, e.g., a solution, emulsion, suspension, gel, particularly a solution.

In one embodiment of the present invention, ophthalmic formulation II is an eye drop.

Specifically, the pH of ophthalmic formulation II is from 5 to 9 (e.g., 5, 5.5, 6, 6.5, 7, 7.5, 8, 8.5, 9), particularly from 6 to 8, e.g., 6.5-7.5.

Specifically, the osmotic pressure of ophthalmic formulation II is from 200 mOsm/kg to 500 mOsm/kg (e.g., 200, 250, 300, 350, 400, 450, 500 mOsm/kg), particularly from 250 mOsm/kg to 450 mOsm/kg.

In one aspect, the present invention provides a method for preparing an ophthalmic formulation II, comprising the steps of:

(1) adding the compound of the present invention to a solution containing part of the pharmaceutically acceptable excipients, and dispersing;
(2) adding an acidic pH adjuster, and mixing;
(3) adding the remaining pharmaceutically acceptable excipients, and mixing;
(4) adding an alkaline pH adjuster.

Specifically, the part of the pharmaceutically acceptable excipients in step (1) is a solubilizer and a surfactant.

In one embodiment of the present invention, the solution containing part of the pharmaceutically acceptable excipients in step (1) is an aqueous solution containing a solubilizer and a surfactant.

In one embodiment of the present invention, the solubilizer is a cyclodextrin.

Specifically, the solution containing part of the pharmaceutically acceptable excipients in step (1) accounts for about 50% v/v of the total formulation volume.

In one embodiment of the present invention, the solution containing part of the pharmaceutically acceptable

excipients in step (1) accounts for 50% of the total formulation volume, the solution being an aqueous solution containing 2%-60% (w/v) (e.g., 2%, 10%, 30%, 40%, 50%, 60%) solubilizer and 0.2%-20% (w/v) surfactant. Specifically, the dispersing in step (1) can be performed by means such as sonication, mechanical stirring, magnetic stirring, vortex shaking, shearing, particularly sonication.

Specifically, the mixing in step (2) can be performed by means such as sonication, mechanical stirring, magnetic stirring, vortex shaking, shearing.

Specifically, the amount of the acidic pH adjuster added in step (2) can be such as to adjust the pH to 3-5 (3, 3.5, 4, 4.5, 5), to initially dissolve or nearly dissolve all of the compound.

Specifically, the part of the pharmaceutically acceptable excipients in step (3) comprises a stabilizer, a viscosity modifier, and a buffer; more specifically, step (3) is adding a buffer containing a stabilizer and a viscosity modifier, and mixing.

Specifically, the buffer containing the stabilizer and the viscosity modifier in step (3) accounts for about 50% v/v of the total formulation volume.

In one embodiment of the present invention, the volume ratio of the solution containing part of the pharmaceutically acceptable excipients in step (1) to the buffer containing the stabilizer and the viscosity modifier in step (3) is 50:50. Specifically, the mixing in step (3) can be performed by means such as sonication, mechanical stirring, magnetic stirring, vortex shaking, shearing, particularly vortex shaking.

Specifically, the amount of the alkaline pH adjuster added in step (4) can be such as to adjust the pH to the level required for the formulation, e.g., from 5 to 9 (e.g., 5, 5.5, 6, 6.5, 7, 7.5, 8, 8.5, 9), particularly from 6 to 8, e.g., 6.5-7.5.

In one aspect, the present invention provides an ophthalmic formulation, comprising:

a. the compound of the present invention or a pharmaceutically acceptable salt thereof;
b. a pharmaceutically acceptable excipient.

In some embodiments of the present invention, the pharmaceutically acceptable excipient comprises one or more of a solubilizer, a stabilizer, a surfactant, a viscosity modifier, and a buffer.

In some embodiments of the present invention, the ophthalmic formulation comprises: a. a pharmaceutically acceptable salt of the compound of the present invention; b. a pharmaceutically acceptable excipient.

In one aspect, the present invention provides a use of the compound of the present invention, or a pharmaceutically acceptable prodrug of the compound, a pharmaceutically active metabolite of the compound and/or a pharmaceutically acceptable salt of the compound, and/or the pharmaceutical composition of the present invention, in the manufacture of a medicament for treating a disease.

In one aspect, the present invention provides the compound of the present invention, or a pharmaceutically acceptable prodrug of the compound, a pharmaceutically active metabolite of the compound and/or a pharmaceutically acceptable salt of the compound, and/or the pharmaceutical composition of the present invention, for use in treating a disease.

In some embodiments of the present invention, the disease is a proliferative disease mediated by a protein tyrosine kinase.

In some embodiments of the present invention, the disease is an ocular disease, including, but not limited to, those accompanied by pathological neovascularization, retinal ischemia, retinal edema, and diabetic retinopathy.

In some embodiments of the present invention, the disease is an ocular disease, including, but not limited to, diabetic retinopathy (including simple (background) diabetic retinopathy, proliferative diabetic retinopathy, and diabetic macular edema); age-related macular degeneration (AMD) (including neovascular (wet/exudative) AMD, dry AMD, and geographic atrophy); pathological choroidal neovascularization (CNV) arising from any pathological mechanism (i.e., high myopia, trauma, sickle cell (anemia) disease; ocular histoplasmosis, angioid streaks, traumatic choroidal rupture, optic nerve drusen, and certain retinal dystrophies); pathological retinal neovascularization arising from any pathological mechanism (i.e., sickle cell retinopathy, Eales disease, ocular ischemic syndrome, internal carotid artery-cavernous sinus fistula, familial exudative vitreoretinopathy, hyperviscosity syndrome, idiopathic obliterative arteriolitis, birdshot retinochoroidopathy, retinal vasculitis, sarcoidosis, or toxoplasmosis); uveitis; retinal vein occlusion (central or branch); ocular trauma; surgically induced edema; surgically induced neovascularization; cystoid macular edema; ocular ischemia; retinopathy of prematurity; Coats' disease; sickle cell retinopathy and/or neovascular glaucoma.

In some embodiments of the present invention, the ocular disease is a posterior segment disease, i.e., a disease occurring in the vitreous, retina, choroid, sclera, or optic nerve.

In some embodiments of the present invention, the disease is diabetic retinopathy, including simple (background) diabetic retinopathy (non-proliferative diabetic retinopathy), proliferative diabetic retinopathy, and diabetic macular edema, particularly non-proliferative diabetic retinopathy.

In other embodiments of the present invention, the disease is age-related macular degeneration (AMD), including neovascular (wet/exudative) AMD, dry AMD, and geographic atrophy.

In some embodiments of the present invention, the disease is a tumor, particularly a malignant tumor (cancer), including, but not limited to, solid tumors and leukemias, such as breast cancer, lung cancer (particularly non-small cell lung cancer), adenocarcinoma, colorectal cancer, kidney cancer, liver cancer, pancreatic cancer, ovarian cancer, prostate cancer, glioma, glioblastoma, myeloma, acute myeloid leukemia, agnogenic myeloid metaplasia, mesothelioma, and myelodysplastic syndrome.

In some embodiments of the present invention, the disease is a hematologic malignancy including: leukemia, lymphoma, and multiple myeloma (MM).

Specifically, the leukemia can be chronic lymphocytic leukemia (CLL), chronic myeloid leukemia (CML), acute lymphoblastic leukemia (ALL), acute myeloid leukemia (AML), or acute monocytic leukemia.

Specifically, the lymphoma can be Hodgkin lymphoma (HL) and non-Hodgkin lymphoma (NHL) (e.g., diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphoma, primary mediastinal B-cell lymphoma, Burkitt lymphoma, lymphoplasmacytic lymphoma, hairy cell leukemia (HCL), immunoblastic large cell lymphoma, precursor B-lymphoblastic lymphoma, and primary central nervous system (CNS) lymphoma, and T-cell NHL, such as precursor T-lymphoblastic lymphoma/leukemia, peripheral T-cell lymphoma (PTCL), angioimmunoblastic T-cell lymphoma, extranodal natural killer/T-cell lymphoma, enteropathy-type T-cell lymphoma, subcutaneous panniculitis-like T-cell lymphoma, and anaplastic large cell lymphoma, NK/T-cell lymphoma, particularly diffuse large B-cell lymphoma (DLBCL). Specifically, in the use, the treatment of a tumor includes killing the tumor, preventing metastasis and spread of the tumor, and growth of micrometastases.

In one aspect, the present invention provides a use of the ophthalmic formulation of the present invention in the manufacture of a medicament for preventing and/or treating an ocular disease, the ocular disease being as described above.

In one aspect, the present invention provides a method for treating an ocular disease, comprising the step of administering the ophthalmic formulation of the present invention, the ocular disease being as described above.

In one aspect, the present invention provides a method for treating a disease, comprising administering the compound of the present invention, or a pharmaceutically acceptable prodrug, a pharmaceutically active metabolite, and/or a pharmaceutically acceptable salt thereof, and/or the pharmaceutical composition of the present invention.

As is well known to those skilled in the art, the dosage of a medicament depends on various factors, including but not limited to the following: the activity of the specific compound used, the age of the patient, the body weight of the patient, the health status of the patient, the behavior of the patient, the diet, the time of administration, the route of administration, the rate of excretion, drug combinations, etc.; furthermore, the optimal treatment regimen, such as the mode of treatment, the daily dosage of the compound of the present invention or its tautomer, mesomer, racemate, enantiomer, diastereomer, or mixture thereof, or a pharmaceutically acceptable salt thereof, and/or the type of pharmaceutically acceptable salt, can be verified according to conventional treatment regimens.

In one aspect, the present invention provides a method for modulating kinase receptor activity, comprising administering the compound of the present invention, or a pharmaceutically acceptable prodrug, a pharmaceutically active metabolite, and/or a pharmaceutically acceptable salt thereof, and/or the pharmaceutical composition of the present invention.

For example, the method inhibits kinase receptor activity. The compound of the present invention may have inhibitory activity against kinase receptor-mediated in vitro proliferation. The inhibitory activity may be a decrease in kinase receptor activity of more than 1%, more than 2%, more than 4%, more than 5%, more than 8%, more than 10%, more than 15%, more than 18%, more than 20%, more than 25%, more than 40%, more than 50%, more than 60%, more than 70%, more than 80%, more than 90%, or more than 95% after addition of the compound of the present invention, compared to addition of a negative control or a control drug. For example, the inhibitory activity may be an IC50 value (nM) for kinase receptor activity of 10000 or less, 5000 or less, 4000 or less, 3000 or less, 2000 or less, 1000 or less, 500 or less, 400 or less, 300 or less, 200 or less, 150 or less, 120 or less, 110 or less, 100 or less, 99 or less, 98 or less, 97 or less, 95 or less, 90 or less, 80 or less, 75 or less, 70 or less, 65 or less, 62 or less, 60 or less, 50 or less, 40 or less, 30 or less, 25 or less, 23 or less, 22 or less, 20 or less, 19 or less, 18 or less, 18.5 or less, 17 or less, 15 or less, 12 or less, 10 or less, 9 or less, 8.5 or less, 7 or less, 6.7 or less, 6 or less, 5.9 or less, 5.5 or less, 5.0 or less, 4.8 or less, 4.5 or less, 4.4 or less, 4 or less, 3.5 or less, 3 or less, 2.5 or less, 2 or less, 1.5 or less, 1.0 or less, 0.5 or less, 0.3 or less, 0.29 or less, 0.25 or less, 0.21 or less, 0.20 or less, 0.18 or less, 0.17 or less, 0.15 or less, 0.12 or less, 0.10 or less, 0.09 or less, 0.08 or less, 0.07 or less, 0.06 or less, 0.05 or less, 0.04 or less, 0.03 or less, 0.02 or less, or 0.01 or less. For example, the kinase receptor activity inhibition can be detected by a mobility shift assay.

For example, the kinase receptor comprises VEGFR (e.g., VEGFR1, VEGFR2, VEGFR3), EGFR, or a functionally active fragment thereof, particularly VEGFR. For example, the method is a non-therapeutic and/or non-diagnostic method. For example, the method is an in vitro and/or ex vivo method.

In some embodiments of the present invention, the method selectively inhibits VEGFR (e.g., VEGFR1, VEGFR2, VEGFR3, particularly VEGFR2) relative to EGFR.

In one aspect, the present invention provides a method for inhibiting ocular neovascularization and retinal vascular leakage, comprising administering the compound of the present invention, or a pharmaceutically acceptable prodrug of the compound, a pharmaceutically active metabolite of the compound and/or a pharmaceutically acceptable salt of the compound, and/or the pharmaceutical composition of the present invention.

Specifically, the administration can employ any suitable route of administration, particularly intraocular administration, such as eye drop administration, eye ointment administration, subconjunctival injection administration, intraocular injection administration, particularly eye drop administration.

Without wishing to be bound by any theory, the following examples are provided merely to illustrate the compounds, preparation methods, and uses of the present invention, and are not intended to limit the scope of the present invention.

Examples

Example 1: Compound Synthesis

I-10

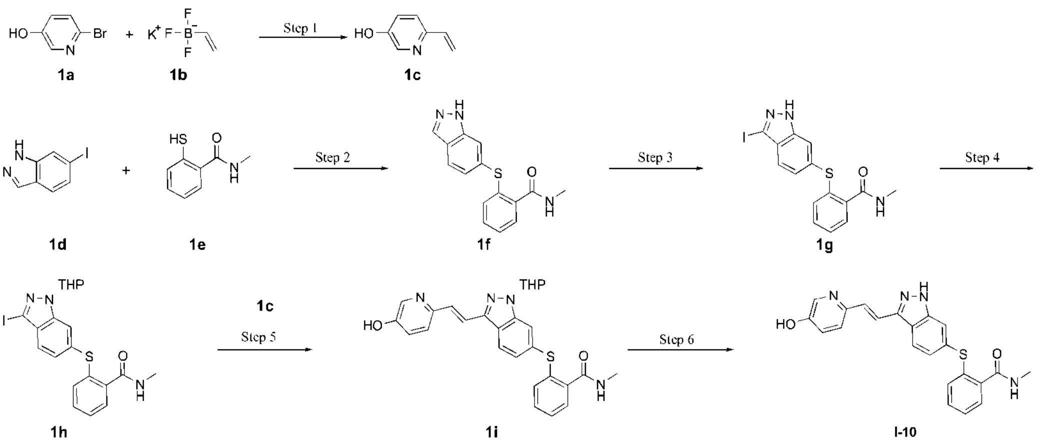


Step 1

At room temperature under nitrogen protection, potassium ethenyltrifluoroborate 1b (11.6 g, 86.21 mmol), Pd(dppf)Cl2 (1.3 g, 1.72 mmol), and triethylamine (8.7 g, 86.21 mmol) were added to a solution of 6-bromopyridin-3-ol la (10 g, 57.47 mmol) in N,N-dimethylformamide (150 mL). The mixture was heated to 85°C and stirred for about 16 hours. After thin-layer chromatography (petroleum ether/ethyl acetate = 1/1) indicated complete consumption of the starting material, the mixture was poured into water (100 mL) and extracted with ethyl acetate (50 mL X 3). The combined organic layers were washed with saturated brine (100 mL), dried over anhydrous sodium sulfate, and concentrated to afford the crude product. The crude product was purified by silica gel column chromatography (petroleum ether/ethyl acetate = 10/1-1/1) to give 6-ethenylpyridin-3-ol 1c as a white solid (4.3 g, 35.50 mmol, 61.76% yield).

The product was confirmed by LCMS and H-NMR.

MS-ESI calculated for [M+H]+ 122.1, found 122.1. $^1$H-NMR (400 MHz, CDCl3): $\delta$ (ppm) 8.17-8.18 (m, 1H), 7.40 (d, J = 8.4 Hz, 1H), 7.25-7.28 (m, 1H), 6.80 (dd, J = 11.2 Hz, 11.2 Hz, 1H), 5.93 (d, J = 17.6 Hz, 1H), 5.38 (d, J = 11.2 Hz, 1H).

Step 2

At room temperature, 6-iodo-1H-indazole 1d (13.3 g, 77.74 mmol), cesium carbonate (50.66 g, 155 mmol), and Pd(dppf)Cl2 (11.38 g, 15.55 mmol) were added to a solution of N-methyl-2-mercaptobenzamide 1e (13 g, 77.74 mmol) in N,N-dimethylformamide (250 mL). The mixture was heated to 80°C and stirred for about 2 hours. After thin-layer chromatography (dichloromethane/methanol = 15/1) indicated complete consumption of the starting material, the mixture was poured into water (1000 mL) and extracted with ethyl acetate (100 mL X 3). The combined organic layers were washed with saturated brine (200 mL), dried over anhydrous sodium sulfate, and concentrated to afford the crude product. The crude product was purified by silica gel column chromatography (petroleum ether/ethyl acetate = 2/1 to 0/1) to give 2-((1H-indazol-6-yl)thio)-N-methylbenzamide 1f as a yellow solid (8.3 g, 37.7% yield).

The product was confirmed by LCMS and H-NMR.

MS-ESI calculated for [M+H]+ 285.1, found 285.0.

$^{1}$H NMR (400 MHz, $CDCl_3$) δ: 8.07 (s, 1H), 7.72 (d, 1H, J=8.4Hz), 7.62-7.65 (m, 2H), 7.26-7.30 (m, 2H), 7.15-7.21 (m, 2H), 6.39 (s, 1H), 2.98 (d, 3H, J=4.8Hz).

Step 3

At 0°C, K2CO3 (6.83 g, 49.4 mmol) and I2 (10.66 g, 42.0 mmol) were added to a solution of 2-((1H-indazol-6-yl) thio)-N-methylbenzamide 1f in N,N-dimethylformamide. The mixture was warmed to room temperature and reacted for about 3.5 hours. After thin-layer chromatography (dichloromethane/methanol = 15/1) indicated complete consumption of the starting material, the mixture was poured into water (1000 mL) and extracted with ethyl acetate (100 mL X 3). The combined organic layers were washed with saturated brine (100 mL), dried over anhydrous sodium sulfate, and concentrated to afford the crude product 2-((3-iodo-1H-indazol-6-yl)thio)-N-methylbenzamide 1g (8.8 g, 87.0%).

The product was confirmed by LCMS and H-NMR.

MS-ESI calculated for [M+H]+ 411.0, found 410.8.

$^{1}$H NMR (400 MHz, DMSO-$d_6$) δ: 13.59 (s, 1H), 8.40 (d, 1H, J=4.8Hz), 7.58 (s, 1H), 7.44-7.50 (m, 2H), 7.28-7.32 (m, 2H), 7.22-7.27 (m, 1H), 7.14 (dd, 1H, J=8.4, 2.0Hz), 7.02 (dd, 1H, J = 8.0, 2.0Hz), 2.76 (d, 3H, J=4.4Hz).

Step 4

3,4-Dihydro-2H-pyran (DHP) (1.03 g, 12.22 mmol) and TsOH (63.12 mg, 366.53 μmol) were added at room temperature to a solution of 2-((3-iodo-1H-indazol-6-yl)thio)-N-methylbenzamide 1g (1 g, 2.44 mmol) in THF (20 mL). The mixture was stirred and refluxed for 16 hours. After thin-layer chromatography (petroleum ether/ethyl acetate = 1/2) indicated complete consumption of the starting material, the mixture was poured into water (100 mL) and extracted with ethyl acetate (50 mL x 3). The combined organic layers were washed with saturated brine (100 mL), dried over anhydrous sodium sulfate, and concentrated to afford the crude product. The crude product was purified by silica gel column chromatography (petroleum ether/ethyl acetate = 10/1-5/1-3/1-1/1) to give 2-((3-iodo-1-(tetrahydro-2H-pyran-2-yl)-1H-indazol-6-yl)thio)-N-methylbenzamide 1h as a white solid (650 mg, 1.32 mmol, 53.92% yield).

The product was confirmed by LCMS and H-NMR.

MS-ESI calculated for [M+H]+ 494.0, found 493.9.

$^{1}$H NMR (400MHz, CDCl3): δ (ppm) 7.66 (s, 1H), 7.58-7.65 (m, 1H), 7.42 (d, 1H, J = 8.4 Hz), 7.29-7.35 (m, 2H), 7.14-7.22 (m, 2H), 6.27 (s, 1H), 5.64 (dd, 1H, J = 9.4, 2.6 Hz), 4.13 -4.15 (m, 1H), 3.64-3.76 (m, 1H), 2.97 (d, 3H, J = 4.8 Hz), 2.46-2.54 (m, 1H), 2.02-2.18 (m, 2H), 1.66-1.84 (m, 3H).

Step 5

Under nitrogen atmosphere at room temperature, 6-ethenylpyridin-3-ol 1c (147.3 mg, 1.22 mmol), triethylamine (307.7 mg, 3.04 mmol), $Pd_2(dba)_3$ (464 mg, 506.73 μmol), and $P(o\text{-}tol)_3$ (308.5 mg, 1.01 mmol) were added to a solution of 2-((3-iodo-1-(tetrahydro-2H-pyran-2-yl)-1H-indazol-6-yl)thio)-N-methylbenzamide 1h (500 mg, 1.01 mmol) in 1,4-dioxane (10 mL). The reaction mixture was stirred at room temperature. After thin-layer chromatography (dichloromethane/methanol = 20/1) indicated complete consumption of the starting material, the mixture was poured into water (20 mL) and extracted with ethyl acetate (20 mL x 3). The combined organic layers were washed with saturated brine (30 mL), dried over anhydrous sodium sulfate, and concentrated to afford the crude product. The crude product was purified by silica gel column chromatography (petroleum ether/ethyl acetate = 30/1-1/1) to give 2-((3-((E)-2-(5-hydroxy-2-pyridyl)ethenyl)-1-(tetrahydro-2H-pyran-2-yl)-1H-indazol-6-yl)thio)-N-methylbenzamide 1i as a yellow solid (400 mg, 822.06 μmol, 81.11% yield). LCMS indicated the product purity was about 30%.

The product was confirmed by LCMS.

MS-ESI calculated for [M+H]+ 487.2, found 487.4.

Step 6

At room temperature, trifluoroacetic acid (35.2 mg, 308.27 μmol, 23.75 μL) was added to a solution of 2-((3-((E)-2-(5-hydroxy-2-pyridyl)ethenyl)-1-(tetrahydro-2H-pyran-2-yl)-1H-indazol-6-yl)thio)-N-methylbenzamide 1i (150 mg, 308.27 μmol) in dichloromethane (3 mL). The mixture was stirred at 35°C for 16 hours. After thin-layer chromatography (dichloromethane/methanol = 10/1) indicated complete consumption of the starting material, the mixture was poured into water (40 mL). The pH was adjusted to 8-9 with solid sodium carbonate, and the mixture was extracted with DCM (30 mL x 3). The combined organic layers were washed with saturated brine (20 mL), dried over anhydrous sodium

sulfate, and concentrated to afford the crude product. The crude product was purified by preparative thin-layer chromatography to give 2-({3-[(E)-2-(5-hydroxypyridin-2-yl)ethenyl]-1H-indazol-6-yl}thio)-N-methylbenzamide I-10 as a white solid (6.1 mg, 15.16 μmol, 4.92% yield).

The product was confirmed by LCMS and H-NMR.

MS-ESI calculated for [M+H]+ 403.1, found 403.1.

$^1H$ NMR (400MHz, $CDCl_3$&$CD_3OD$): δ (ppm) 8.05-8.04 (m, 1H), 7.90 (d, J=8.4Hz, 1H), 7.51-7.42 (m, 3H), 7.39-7.35 (m, 2H), 7.21-7.18 (m, 2H), 7.14-7.07 (m, 3H), 2.82 (s, 3H).

I-14

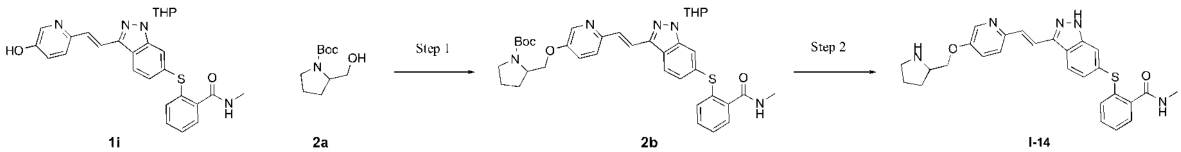


1i 2a 2b I-14

Step 1

Under nitrogen atmosphere at room temperature, tert-butyl 2-(hydroxymethyl)pyrrolidine-1-carboxylate 2a (198.5 mg, 986.47 μmol), $Ph_3P$ (323.4 mg, 1.23 mmol), and DIAD (249.3 mg, 1.23 mmol) were added to a solution of 2-((3-((E)-2-(5-hydroxy-2-pyridyl)ethenyl)-1-(tetrahydro-2H-pyran-2-yl)-1H-indazol-6-yl)thio)-N-methylbenzamide 1i (400 mg, 822.06 μmol) in tetrahydrofuran (10 mL). The temperature was raised to 40°C and the mixture was stirred for about 16 hours. After thin-layer chromatography (dichloromethane/methanol = 10/1) indicated complete consumption of the starting material, the mixture was poured into water (30 mL) and extracted with ethyl acetate (30 mL x 3). The combined organic layers were washed with saturated brine (40 mL), dried over anhydrous sodium sulfate, and concentrated to afford the crude product. The crude product was purified by preparative thin-layer chromatography (dichloromethane/methanol = 10/1) to give tert-butyl 2-(((6-((E)-2-(6-((2-(methylcarbamoyl)phenyl)thio)-2-(tetrahydro-2H-pyran-2-yl)-2H-indazol-3-yl)ethenyl)pyridin-3-yl)oxy)methyl)pyrrolidine-1-carboxylate 2b as a white solid (400 mg, 597.17 μmol, 72.64% yield). LCMS indicated the product purity was about 29%.

The product was confirmed by LCMS.

MS-ESI calculated for [M+H]+ 670.3, found 670.2.

Step 2

At room temperature, trifluoroacetic acid (340.4 mg, 2.99 mmol) was added to a solution of tert-butyl 2-(((6-((E)-2-(6-((2-(methylcarbamoyl)phenyl)thio)-2-(tetrahydro-2H-pyran-2-yl)-2H-indazol-3-yl)ethenyl)pyridin-3-yl)oxy)methyl)pyrrolidine-1-carboxylate 2b (0.4 g, 597.17 μmol) in dichloromethane (5 mL). The temperature was raised to 35°C and the mixture was stirred for about 16 hours. After thin-layer chromatography (dichloromethane/methanol = 10/1) indicated complete consumption of the starting material, the mixture was poured into water (40 mL). The pH was adjusted to 8-9 with solid sodium carbonate, and the mixture was extracted with dichloromethane (30 mL x 3). The combined organic layers were washed with saturated brine (20 mL), dried over anhydrous sodium sulfate, and concentrated to afford the crude product. The crude product was purified by preparative thin-layer chromatography to give N-methyl-2-({3-[(E)-2-{5-[(pyrrolidin-2-yl)methoxy]pyridin-2-yl}ethenyl]-1H-indazol-6-yl}thio)benzamide I-14 as a white solid (13.9 mg, 28.62 μmol, 4.79% yield).

The product was confirmed by LCMS, H-NMR, and C-NMR.

MS-ESI calculated for [M+H]+ 486.2, found 487.0.

$^1H$ NMR (400MHz, $CDCl_3$&$CD_3OD$): δ (ppm) 8.24-8.23 (m, 1H), 7.98 (d, J=8.8Hz, 1H), 7.65-7.61 (m, 1H), 7.55-7.52 (m, 1H), 7.50-7.48 (m, 2H), 7.44-7.39 (m, 1H), 7.29-7.22 (m, 3H), 7.19-7.15 (m, 2H), 4.05-4.02 (m, 1H), 3.94 (t, J=8.0Hz, 1H), 3.53-3.50 (m, 1H), 3.04-2.91 (m, 2H), 2.88 (s, 1H), 2.02-1.95 (m, 1H), 1.89-1.79 (m, 2H), 1.64-1.57 (m, 1H).

$^{13}C$ NMR (100 MHz, $CDCl_3$&$CD_3OD$): δ (ppm) 169.83, 154.32, 148.42, 142.19, 137.25, 136.56, 135.24, 133.46, 131.10, 130.65, 139.35, 128.04, 126.65, 125.39, 122.26, 122.03, 121.83, 121.47, 120.46, 114.42, 70.93, 57.08, 46.10, 46.06, 29.59, 27.64, 26.33, 25.07.

I-2

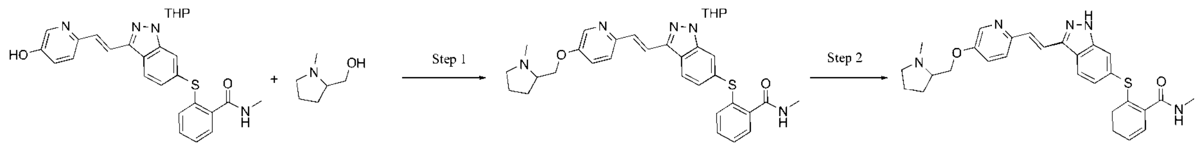


1i 3a 3b I-2

Step 1

Under nitrogen atmosphere at room temperature, (1-methylpyrrolidin-2-yl)methanol 3a (113.6 mg, 986.47 μmol), $Ph_3P$ (215.6 mg, 822.06 μmol), and DIAD (249.3 mg, 1.23 mmol) were added to a solution of 2-((3-((E)-2-(5-hydroxy-2-pyridyl)ethenyl)-1-(tetrahydro-2H-pyran-2-yl)-1H-indazol-6-yl)thio)-N-methylbenzamide 1i (400 mg, 822.06 μmol) in tetrahydrofuran (5 mL). The mixture was stirred at 30°C for about 16 hours. After thin-layer chromatography (dichloromethane/methanol = 10/1) indicated complete consumption of the starting material, the mixture was poured into water (30 mL) and extracted with ethyl acetate (20 mL x 3). The combined organic layers were washed with saturated brine (30 mL), dried over anhydrous sodium sulfate, and concentrated to afford the crude product. The crude product was purified by silica gel column chromatography (petroleum ether/ethyl acetate = 10/1 - dichloromethane/methanol = 10/1) to give N-methyl-2-((3-((E)-2-(5-((1-methylpyrrolidin-2-yl)methoxy)pyridin-2-yl)ethenyl)-2-(tetrahydro-2H-pyran-2-yl)-1H-indol-6-yl)thio)benzamide 3b as a yellow solid (240 mg, 411.14 μmol, 50.01% yield).

The product was confirmed by LCMS.

MS-ESI calculated for [M+H]+ 584.3, found 584.3.

Step 2

At room temperature, trifluoroacetic acid (46.9 mg, 411.14 μmol) was added to a solution of N-methyl-2-((3-((E)-2-(5-((1-methylpyrrolidin-2-yl)methoxy)pyridin-2-yl)ethenyl)-2-(tetrahydro-2H-pyran-2-yl)-1H-indol-6-yl)thio)benzamide 3b (240 mg, 411.14 μmol) in dichloromethane (5 mL). The mixture was stirred at 30°C for 16 hours. After thin-layer chromatography (dichloromethane/methanol = 10/1) indicated complete consumption of the starting material, the mixture was poured into water (10 mL). The pH was adjusted to 8-9 with solid sodium carbonate, and the mixture was extracted with DCM (10 mL x 3). The combined organic layers were washed with saturated brine (20 mL), dried over anhydrous sodium sulfate, and concentrated to afford the crude product. The crude product was purified by preparative thin-layer chromatography to give N-methyl-2-((3-((E)-2-(5-((1-methylpyrrolidin-2-yl)methoxy)pyridin-2-yl)ethenyl)-1H-indazol-6-yl)thio)benzamide I-2 as a white solid (31.6 mg, 63.25 μmol, 7.97% yield).

The product was confirmed by LCMS, H-NMR, and C-NMR.

MS-ESI calculated for [M+H]+ 500.2, found 500.1.

$^1H$ NMR (400MHz, $CDCl_3$&$CD_3OD$): δ (ppm) 8.23 (s, 1H), 7.94 (d, J = 8.0 Hz, 1H), 7.63 (s, 1H), 7.47 (dd, J = 39.8, 29.7 Hz, 4H), 7.24 (s, 2H), 4.02 (d, J = 6.4 Hz,2H), 3.08 (s, 3H), 2.85 (s, 1H), 2.70 (s, 3H), 2.28 - 2.32 (m, 1H), 2.63 (s, 1H), 1.98-2.02 (m, 1H), 1.80 (s, 1H), 0.82 (s, 1H).

$^{13}C$ NMR (100MHz, $CDCl_3$&$CD_3OD$): δ (ppm) 169.43, 154.32, 148.32, 137.43, 136.68, 134.67, 133.45, 131.85, 130.74, 129.50, 128.34, 126.93, 125.01, 122.20, 121.86, 121.79, 121.64, 121.49, 120.47, 113.93, 70.84, 64.29, 57.63, 41.61, 29.62, 28.32, 26.44, 22.71, 22.63.

I-3

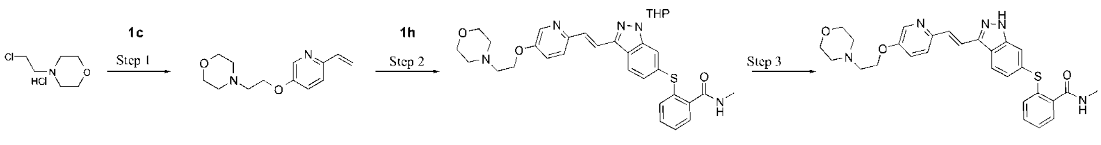


10a 10b 10c I-3

Step 1

At low temperature, sodium hydride (1.5 g, 37.15 mmol, 60% purity) was added portionwise to a solution of 6-ethenylpyridin-3-ol 1c (1.5 g, 12.38 mmol) in dry N,N-dimethylformamide (20 mL). The mixture was then stirred at 0°C for 1 hour. 4-(2-Chloroethyl)morpholine hydrochloride 10a (2.8 g, 14.86 mmol) was then added slowly. The mixture was stirred at 60°C for about 4 hours. After thin-layer chromatography (petroleum ether/ethyl acetate = 3/1) indicated complete consumption of the starting material, the mixture was poured into water (100 mL) and extracted with ethyl acetate (60 mL x 3). The combined organic layers were washed with saturated brine (100 mL), dried over anhydrous sodium sulfate, and concentrated to afford the crude product. The crude product was purified by preparative thin-layer chromatography to give 4-[2-[(6-ethenylpyridin-3-yl)oxy]ethyl]morpholine 10b as a yellow oily liquid (1.5 g, 6.40 mmol, 51.70% yield).

The product was confirmed by LCMS and H-NMR.

MS-ESI calculated for [M+H]+ 235.1, found 235.1. $^1$H-NMR (400MHz, CDCl3): δ (ppm) 8.29 (d, J = 2.8 Hz, 1H), 7.32-7.30 (m, 1H), 7.20-7.17 (m, 1H), 6.82-6.75 (m, 1H), 6.07-6.02 (m, 1H), 5.39-5.36 (m, 1H), 4.19-4.13 (m, 2H), 3.77-3.75 (m, 4H), 2.85-2.82 (m, 2H), 2.61-2.59 (m, 4H).

Step 2

At room temperature under nitrogen protection, 4-[2-[(6-ethenylpyridin-3-yl)oxy]ethyl]morpholine 10b (57 mg, 0.24 mmol), triethylamine (61.5 mg, 0.61 mmol), Pd2(dba)3 (45.5 mg, 0.20 mmol), and P(o-tol)3 (61.7 mg, 0.20 mmol) were added to a solution of 2-[(3-iodo-1-tetrahydropyran-2-yl-indazol-6-yl)thio]-N-methyl-benzamide 1h (100 mg, 0.20 mmol) in 1,4-dioxane (5 mL). The mixture was heated to 100°C and stirred for reaction. After thin-layer chromatography (dichloromethane/methanol = 20/1) indicated complete consumption of the starting material, the mixture was poured into water (10 mL) and extracted with ethyl acetate (10 mL x 3). The combined organic layers were washed with saturated brine (20 mL), dried over anhydrous sodium sulfate, and concentrated to give the crude product. The crude product was purified by preparative thin-layer chromatography (dichloromethane/methanol = 20/1) to give N-methyl-2-((3-((E)-2-(5-(2-(morpholin-4-yl)ethoxy)pyridin-2-yl)ethenyl)-1-(tetrahydro-2H-pyran-2-yl)-1H-indazol-6-yl)thio)benzamide 10c as a brownish-yellow solid (100 mg, 166.74 μmol, 82.26% yield).

The product was confirmed by LCMS.

MS-ESI calculated for [M+H]+ 600.2, found 600.1. Step 3

At room temperature, trifluoroacetic acid (380.2 mg, 3.33 mmol) was added to a solution of N-methyl-2-((3-((E)-2-(5-(2-(morpholin-4-yl)ethoxy)pyridin-2-yl)ethenyl)-1-(tetrahydro-2H-pyran-2-yl)-1H-indazol-6-yl)thio)benzamide 10c (400 mg, 0.67 mmol) in dichloromethane (2 mL). The mixture was stirred at 35°C for 16 hours. After thin-layer chromatography (dichloromethane/methanol = 10/1) indicated complete consumption of the starting material, the mixture was poured into water (40 mL). The pH was adjusted to 8-9 with solid sodium carbonate, and the mixture was extracted with dichloromethane (30 mL x 3). The combined organic layers were washed with saturated brine (20 mL), dried over anhydrous sodium sulfate, and concentrated to afford the crude product. The crude product was purified by preparative thin-layer chromatography to give N-methyl-2-((3-((E)-2-(5-(2-(morpholin-4-yl)ethoxy)pyridin-2-yl)ethenyl)-1H-indazol-6-yl)thio)benzamide I-3 as a white solid (53.7 mg, 104.15 μmol, 15.62% yield).

The product was confirmed by LCMS, H-NMR, and C-NMR.

MS-ESI calculated for [M+H]+ 516.2, found 516.2.

$^1$H NMR (400MHz, CDCl3): δ (ppm) 10.28 (s, 1H), 8.38-8.37 (m, 1H), 8.02 (d, J = 8.4 Hz, 1H), 7.78-7.74 (m, 1H), 7.68-7.66 (m, 1H), 7.57-7.52 (m, 2H), 7.44 (d, J = 8.8 Hz, 1H), 7.34-7.32 (m, 2H), 7.27-7.22 (m, 3H), 6.37-6.34 (m, 1H), 4.22 (t, J = 5.6 Hz, 2H), 3.78 (t, J = 4.4 Hz, 4H), 2.98 (d, J = 5.2 Hz, 3H), 2.87 (t, J = 5.6 Hz, 2H), 2.63 (s, 4H).

$^{13}$C NMR (100 MHz, $CDCl_3$&$CD_3OD$): δ (ppm) 170.16, 154.28, 148.42, 137.19, 136.59, 135.60, 133.54, 131.08, 130.51, 129.24, 127.79, 126.42, 125.60, 122.32, 122.21, 121.99, 121.40, 114.51, 66.52, 65.88, 57.34, 53.82, 26.09.

I-19

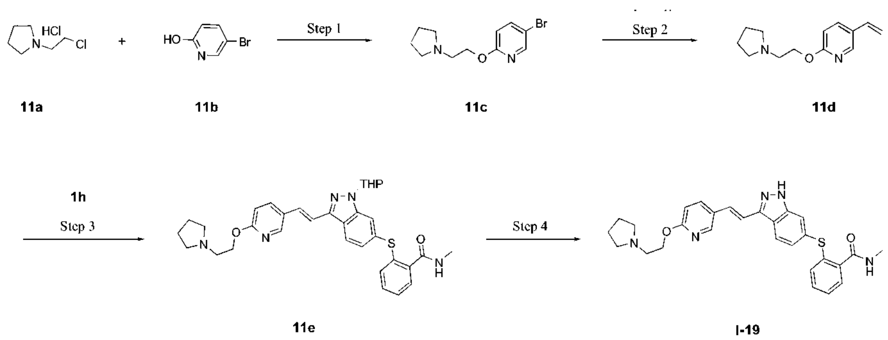


11a 11b 11c 11d

11e I-19

Step 1

At 0°C under nitrogen protection, sodium hydride (689.67 mg, 17.24 mmol, 60% purity) was added portionwise to a solution of 5-bromopyridin-2-ol 11b (1 g, 5.75 mmol) in N,N-dimethylformamide (20 mL). The mixture was stirred at 0°C for 1 h, then 1-(2-chloroethyl)pyrrolidine hydrochloride 11a (1.17 g, 6.90 mmol) was added at 0°C. The mixture was heated to 60°C and stirred for 16 h. After thin-layer chromatography (petroleum ether/ethyl acetate = 1/1) indicated complete consumption of the starting material, the mixture was poured into water (50 mL) and extracted with ethyl acetate (50 mL x 3). The combined organic layers were washed with saturated brine (100 mL), dried over anhydrous sodium sulfate, and concentrated to give the crude product 5-bromo-2-(2-(pyrrolidin-1-yl)methoxy)pyridine 11c as a brownish-yellow oily liquid (1 g, 3.69 mmol, 64.17% yield).
The product was confirmed by LCMS and H-NMR.
MS-ESI calculated for [M+H]+ 272.9, found 272.9.
$^1$H NMR (400 MHz, DMSO-$d_6$) δ 8.18 - 8.02 (m, 1H), 7.50 (dt, J = 13.8, 6.9 Hz, 1H), 7.41 - 7.21 (m, 1H), 4.12 (dd, J = 5.5, 2.9 Hz, 2H), 2.76 (dd, J = 5.5, 2.8 Hz, 2H), 2.48 (d, J = 1.7 Hz, 4H), 1.65 (d, J = 3.1 Hz, 4H).

Step 2

At room temperature, potassium ethenyltrifluoroborate lb (750.0 mg, 5.60 mmol), Pd(dppf)Cl2 (80.1 mg, 109.53 μmol), and triethylamine (560.0 mg, 5.53 mmol) were added to a solution of 5-bromo-2-(2-(pyrrolidin-1-yl)methoxy) pyridine 11c (1 g, 3.69 mmol) in dry N,N-dimethylformamide (20 mL). The mixture was heated to 85°C and stirred for about 16 hours. After thin-layer chromatography (dichloromethane/methanol = 10/1) indicated complete consumption of the starting material, the mixture was poured into water (50 mL) and extracted with ethyl acetate (50 mL x 3). The combined organic layers were washed with saturated brine (200 mL), dried over anhydrous sodium sulfate, and concentrated to afford the crude product. The crude product was purified by preparative thin-layer chromatography (dichloromethane/-methanol = 10/1) to give 2-(2-pyrrolidin-1-ylethoxy)-5-ethenylpyridine 11d as a yellow solid (340 mg, 1.56 mmol, 42.23% yield).
The product was confirmed by LCMS and H-NMR.
MS-ESI calculated for [M+H]+ 219.1, found 219.1.
$^1$H NMR (400 MHz, $CDCl_3$) δ 7.89 - 7.53 (m, 1H), 7.52 - 7.32 (m, 1H), 6.84 - 6.54 (m, 1H), 6.51 - 6.27 (m, 1H), 5.73 - 5.42 (m, 1H), 5.16 (dt, J = 22.5, 11.2 Hz, 1H), 4.06 (d, J = 17.0 Hz, 2H), 2.83 (d, J = 10.8 Hz, 2H), 2.60 (d, J = 7.4 Hz, 4H), 1.84 - 1.76 (m, 4H).

Step 3

At room temperature under nitrogen protection, 2-(2-pyrrolidin-1-ylethoxy)-5-ethenylpyridine 11d (280 mg, 1.28 mmol), triethylamine (300 mg, 2.96 mmol), Pd2(dba)3 (250 mg, 1.11 mmol), and P(o-tol)3 (250 mg, 821.40 μmol) were added to a solution of 2-((3-iodo-1-(tetrahydro-2H-pyran-2-yl)-1H-indazol-6-yl)thio)-N-methylbenzamide 1h (500 mg, 1.01 mmol) in 1,4-dioxane (20 mL). The mixture was heated to 100°C and stirred for about 2 hours. After thin-layer chromatography (dichloromethane/methanol = 20/1) indicated complete consumption of the starting material, the mixture was poured into water (30 mL) and extracted with ethyl acetate (30 mL x 3). The combined organic layers were washed with saturated brine (50 mL), dried over anhydrous sodium sulfate, and concentrated to afford the crude product. The crude product was purified by preparative thin-layer chromatography (dichloromethane/methanol = 10/1) to give N-methyl-2-((3-((E)-2-(6-(2-(pyrrolidin-1-yl)ethoxy)pyridin-3-yl)ethenyl)-1-(tetrahydro-2H-pyran-2-yl)-1H-indazol-6-yl)thio)benzamide 11e as a yellow solid (200 mg, 342.62 μmol, 33.81% yield).
The product was confirmed by LCMS.
MS-ESI calculated for [M+H]+ 584.3, found 584.4.

Step 4

At room temperature, trifluoroacetic acid (1.20 g, 10.52 mmol) was added to a solution of N-methyl-2-((3-((E)-2-(6-(2-(pyrrolidin-1-yl)ethoxy)pyridin-3-yl)ethenyl)-1-(tetrahydro-2H-pyran-2-yl)-1H-indazol-6-yl)thio)benzamide 11e (200 mg, 342.62 μmol) in dichloromethane (8 mL). The mixture was stirred at 40°C for 16 hours. After thin-layer chromatography (dichloromethane/methanol = 10/1) indicated complete consumption of the starting material, the mixture was poured into water (10 mL). The pH was adjusted to 8-9 with an aqueous sodium carbonate solution (10 mL), and the mixture was extracted with ethyl acetate (10 mL x 3). The combined organic layers were washed with saturated brine (20 mL), dried over anhydrous sodium sulfate, and concentrated to afford the crude product. The crude product was purified by preparative thin-layer chromatography to give N-methyl-2-((3-((E)-2-(6-(2-(pyrrolidin-1-yl)ethoxy)pyridin-3-yl)ethenyl)-1H-indazol-6-yl)thio)benzamide I-19 as a white solid (10.4 mg, 20.82 μmol, 6.08% yield).

The product was confirmed by LCMS, H-NMR, and C-NMR.

MS-ESI calculated for [M+H]+ 500.2, found 500.0.

$^{1}$H NMR (400 MHz, $CDCl_3$) δ 11.49 (s, 1H), 7.84 (d, J = 8.5 Hz, 1H), 7.65 (d, J = 5.0 Hz, 1H), 7.43 - 7.28 (m, 6H), 7.22 - 7.08 (m, 2H), 6.94 (dd, J = 41.7, 16.5 Hz, 2H), 6.19 (d, J = 9.4 Hz, 1H), 4.13 (s, 2H), 3.07 - 2.92 (m, 3H), 2.72 (s, 2H), 1.85 (s, 2H), 1.62 (s, 4H), 1.28 (d, J = 10.3 Hz, 2H).

$^{13}$C NMR (100MHz, CDCl3): δ (ppm) 170.29, 162.66, 137.38, 137.19, 136.91, 135.50, 133.68, 131.08, 130.36, 127.70, 126.39, 125.63, 125.28, 121.29, 119.95, 118.46, 54.22, 54.00, 25.76, 23.11.

I-32

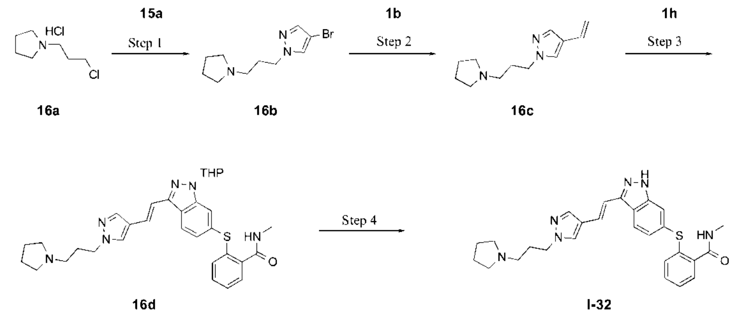


Step 1

At room temperature under nitrogen protection, 1-(3-chloropropyl)pyrrolidine hydrochloride 16a (2.4 g, 16.33 mmol) and cesium carbonate (13.3 g, 40.82 mmol) were added to a solution of 4-bromo-1 H-pyrazole 15a (2 g, 13.61 mmol) in DMSO (20 mL). The mixture was heated to 100°C and stirred for about 4 hours. After thin-layer chromatography (dichloromethane/methanol = 10/1) indicated complete consumption of the starting material, the mixture was poured into water (100 mL) and extracted with ethyl acetate (50 mL x 3). The combined organic layers were washed with saturated brine (120 mL), dried over anhydrous sodium sulfate, and concentrated to afford the crude product. The crude product was purified by silica gel column chromatography (petroleum ether/ethyl acetate = 20/1 to 0/1) to give 4-bromo-1-(3-(pyrrolidin-1-yl)propyl)-1H-pyrazole 16b as a yellow solid (3 g, 11.62 mmol, 85.40% yield).

The product was confirmed by LCMS and H-NMR.

MS-ESI calculated for [M+H]+ 259.9, found 260.1.

$^{1}$H NMR (400MHz, CDCl3): δ (ppm) 7.43 (d, J = 10.8 Hz, 2H), 4.17 (t, J = 6.8 Hz, 2H), 2.46-2.39 (m, 6H), 2.06-1.99 (m, 2H), 1.82-1.74 (m, 4H).

Step 2

At room temperature under nitrogen protection, potassium ethenyltrifluoroborate 1b (778.3 mg, 5.81 mmol), Pd(dppf)Cl2 (85 mg, 116.21 μmol), and triethylamine (587.9 mg, 5.81 mmol) were added to a solution of 4-bromo-1-(3-(pyrrolidin-1-yl)propyl)-1H-pyrazole 16b (1 g, 3.87 mmol) in N,N-dimethylformamide (10 mL). The mixture was heated to 100°C and stirred for about 4 hours. After thin-layer chromatography (dichloromethane/methanol = 10/1) indicated complete consumption of the starting material, the mixture was poured into water (50 mL) and extracted with ethyl acetate (50 mL x 3). The combined organic layers were washed with saturated brine (120 mL), dried over anhydrous sodium sulfate, and concentrated to afford the crude product. The crude product was purified by silica gel column chromatography (petroleum ether/ethyl acetate = 10/1 to 0/1, then dichloromethane/methanol = 20/1 to 10/1) to give 1-(3-(pyrrolidin-1-yl)propyl)-4-ethenyl-1H-pyrazole 16c as a yellow oil (300 mg, 1.46 mmol, 37.72% yield).

The product was confirmed by H-NMR. $^{1}$H-NMR (400 MHz, CDCl3): δ (ppm) 7.45-7.41 (m, 2H), 6.52 (dd, J = 9.8, 11.2 Hz, 1H), 5.44 (d, J = 16.4 Hz, 1H), 5.05 (d, J = 11.2 Hz, 1H), 4.17 (d, J = 6.8 Hz, 4H), 2.53 (s, 4H), 2.11-1.99 (m, 4H), 1.79 (d, J = 3.2 Hz, 4H).

Step 3

At room temperature under nitrogen protection, 1-(3-(pyrrolidin-1-yl)propyl)-4-ethenyl-1H-pyrazole 16c (124.8 mg, 608.08 μmol), triethylamine (92.3 mg, 912.11 μmol), Pd2(dba)3 (185.1 mg, 608.08 μmol), and P(o-tol)3 (835.2 mg, 912.11 μmol) were added to a solution of 2-[(3-iodo-1-(tetrahydro-2H-pyran-2-yl)-1H-indazol-6-yl)thio]-N-methylbenzamide 1h (300 mg, 608.08 μmol) in 1,4-dioxane (10 mL). The mixture was heated to 100°C and stirred for about 2 hours.

After thin-layer chromatography (dichloromethane/methanol = 10/1) indicated complete consumption of the starting material, the mixture was poured into water (30 mL) and extracted with ethyl acetate (30 mL x 3). The combined organic layers were washed with saturated brine (50 mL), dried over anhydrous sodium sulfate, and concentrated to afford the crude product, N-methyl-2-((3-((E)-2-(1-(3-(pyrrolidin-1-yl)propyl)-1H-pyrazol-4-yl)ethenyl)-1-(tetrahydro-2H-pyran-2-yl)-1H-indazol-6-yl)thio)benzamide 16d, as a yellow solid (300 mg, 525.63 μmol, 86.44% yield).

The product was confirmed by LCMS.

MS-ESI calculated for [M+H]+ 571.3, found 571.3.

Step 4

At room temperature, trifluoroacetic acid (100 mg, 876.05 μmol) was added to a solution of N-methyl-2-((3-((E)-2-(1-(3-(pyrrolidin-1-yl)propyl)-1H-pyrazol-4-yl)ethenyl)-1-(tetrahydro-2H-pyran-2-yl)-1H-indazol-6-yl)thio) benzamide 16d (500 mg, 876.05 μmol) in dichloromethane (10 mL). The mixture was stirred at room temperature for about 16 hours. After thin-layer chromatography (dichloromethane/methanol = 10/1) indicated complete consumption of the starting material, the mixture was poured into water (40 mL). The pH was adjusted to 8-9 with sodium carbonate, and the mixture was extracted with dichloromethane (30 mL x 3). The combined organic layers were washed with saturated brine (40 mL), dried over anhydrous sodium sulfate, and concentrated to afford the crude product. The crude product was purified by preparative thin-layer chromatography to give N-methyl-2-((3-((E)-2-(1-(3-(pyrrolidin-1-yl)propyl)-1H-pyrazol-4-yl)ethenyl)-1H-indazol-6-yl)thio)benzamide I-32 as a yellow solid (15.4 mg, 31.65 μmol, 3.61% yield).

The product was confirmed by LCMS, H-NMR, and C-NMR.

MS-ESI calculated for [M+H]+ 487.2, found 487.1.

$^1$H NMR (400 MHz, $CDCl_3$) δ (ppm) 7.91 (d, J = 8.0 Hz, 1H), 7.74 (s, 1H), 7.66-7.62 (m, 1H), 7.58 (s, 1H), 7.47 (s, 1H), 7.34-7.29 (m, 3H), 7.25-7.21 (m, 2H), 7.20-7.17 (m, 1H), 7.11 (d, J = 16.8 Hz, 1H), 6.33 (s, 1H), 4.22 (t, J = 6.8 Hz, 2H), 2.96 (d, J = 4.8 Hz, 3H), 2.59 (d, J = 19.2 Hz, 5H), 2.18-2.11 (m, 2H), 1.84 (s, 5H).

$^{13}$C NMR (100 MHz, $CDCl_3$) δ (ppm) 168.62, 144.36, 142.08, 137.46, 134.46, 131.96, 130.88, 128.81, 127.58, 127.17, 124.62, 121.80, 121.63, 120.49, 117.88, 113.31, 54.06, 53.00, 50.20, 29.71, 29.07, 26.82, 23.46.

## I-30

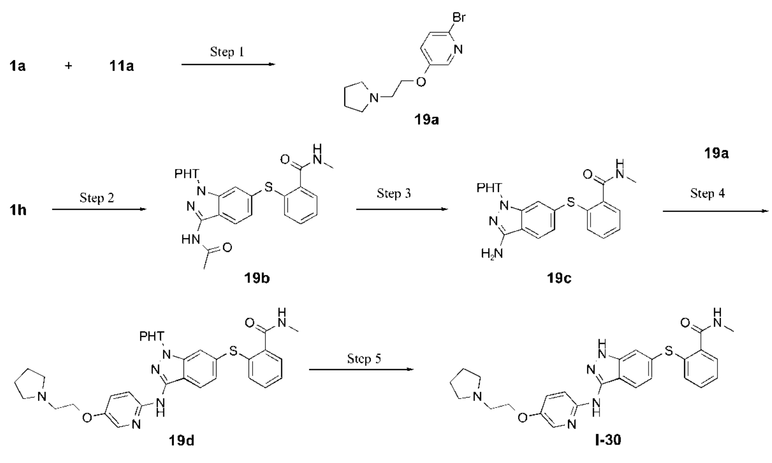


Step 1

Under nitrogen atmosphere at 0°C, sodium hydride (253 mg, 6.32 mmol, 60% purity) was added portionwise to a solution of 6-bromopyridin-3-ol 1a (1 g, 5.75 mmol) in N,N-dimethylformamide (20 mL) and the mixture was stirred for about 1 hour. Then, 1-(2-chloroethyl)pyrrolidine hydrochloride 11a (1.08 g, 6.32 mmol) was added slowly at low temperature. After stirring at low temperature for 1 hour, the mixture was warmed to 50°C and stirred for about 3 hours. After thin-layer chromatography (petroleum ether/ethyl acetate = 2/1) indicated complete consumption of the starting material, the reaction mixture was cooled to 0°C, quenched with water (about 10 mL), poured into water (100 mL), and extracted with ethyl acetate (50 mL X 3). The combined organic layers were washed with saturated brine (100 mL), dried over anhydrous sodium sulfate, and concentrated to afford the crude product 2-bromo-5-(2-(pyrrolidin-1-yl)methoxy) pyridine 19a as a yellow oily liquid (1.5 g, 96.3% yield).

The product was confirmed by LCMS and H-NMR.

MS-ESI calculated for [M+H]+ 272.9, found 273.1.

$^{1}$H NMR (400MHz, $CDCl_3$) δ (ppm) 8.09 (d, 1H, J = 3.2Hz), 7.38 (d, 1H, J = 8.8Hz), 7.16 (dd, 1H, J = 8.8, 2.8Hz), 4.21 (t, 2H, J = 5.6Hz), 3.01 (t, 2H, J = 5.6Hz), 2.75 (s, 4H), 1.87-1.90 (m, 4H).

Step 2

Under nitrogen atmosphere at room temperature, acetamide (71.83 mg, 1.22 mmol), (1R,2R)-N1,N2-dimethylcyclohexane-1,2-diamine (86.49 mg, 608.08 μmol), copper(I) iodide (115.81 mg, 608.08 μmol), and sodium tert-butoxide (116.88 mg, 1.22 mmol) were added to a solution of 2-((3-iodo-1-(tetrahydro-2H-pyran-2-yl)-1H-indazol-6-yl)thio)-N-methylbenzamide 1h (300 mg, 608.08 μmol) in 1,4-dioxane (9 mL). The mixture was heated to 110°C and stirred for about 6 hours. After thin-layer chromatography (ethyl acetate) indicated complete consumption of the starting material, the mixture was poured into water (30 mL) and extracted with ethyl acetate (20 mL X 3). The combined organic layers were washed with saturated brine (30 mL), dried over anhydrous sodium sulfate, and concentrated to afford the crude product. The crude product was purified by preparative thin-layer chromatography (ethyl acetate) to give 2-((3-acetamido-1-(tetrahydro-2H-pyran-2-yl)-1H-indazol-6-yl)thio)-N-methylbenzamide 19b as a white solid (0.17 g, 400.46 μmol, 65.86% yield).

The product was confirmed by LCMS.

MS-ESI calculated for [M+H]+ 425.2, found 425.0.

Step 3

At room temperature, NaOH (376.90 mg, 9.42 mmol) and H2O (2 mL) were added to a solution of 2-((3-acetamido-1-(tetrahydro-2H-pyran-2-yl)-1H-indazol-6-yl)thio)-N-methylbenzamide 19b (0.4 g, 942.25 μmol) in 1,4-dioxane (10 mL). The mixture was heated to 100°C and stirred for about 16 hours. After thin-layer chromatography (dichloromethane/methanol = 20/1) indicated complete consumption of the starting material, the mixture was poured into water (40 mL) and extracted with ethyl acetate (20 mL X 3). The combined organic layers were washed with saturated brine (20 mL), dried over anhydrous sodium sulfate, and concentrated to afford the crude product. The crude product was purified by preparative thin-layer chromatography (dichloromethane/methanol = 20/1, 0.1% NH3·H2O) to give 2-((3-amino-1-(tetrahydro-2H-pyran-2-yl)-1H-indazol-6-yl)thio)-N-methylbenzamide 19c as a white solid (0.152 g, 397.41 μmol, 42.18% yield).

The product was confirmed by LCMS.

MS-ESI calculated for [M+H]+ 383.1, found 383.1.

Step 4

Under nitrogen atmosphere at room temperature, XantPhos (378.20 mg, 653.63 μmol), Pd2(dba)3 (97.72 mg, 169.94 μmol), 2-bromo-5-(2-(pyrrolidin-1-yl)methoxy)pyridine 19a (124.8 mg, 608.08 μmol), and cesium carbonate (1.60 g, 4.92 mmol) were added to a solution of 2-((3-amino-1-(tetrahydro-2H-pyran-2-yl)-1H-indazol-6-yl)thio)-N-methylbenzamide 19c (0.1 g, 261.45 μmol) in 1,4-dioxane (5 mL). The mixture was heated to 100°C and stirred for about 6 hours. After thin-layer chromatography (dichloromethane/methanol = 10/1) indicated complete consumption of the starting material, the mixture was poured into water (30 mL) and extracted with ethyl acetate (10 mL X 3). The combined organic layers were washed with saturated brine (20 mL), dried over anhydrous sodium sulfate, and concentrated to afford the crude product as a yellow oily liquid. The crude product was purified by preparative thin-layer chromatography (dichloromethane/methanol = 10/1) to give N-methyl-2-((3-((5-(pyrrolidin-1-ylmethoxy)pyridin-2-yl)amino)-1-(tetrahydro-2H-pyran-2-yl)-1H-indazol-6-yl)thio)benzamide 19d as a yellow solid (0.1 g, 174.61 μmol, 66.78% yield).

The product was confirmed by LCMS.

MS-ESI calculated for [M+H]+ 573.3, found 573.1.

Step 5

At low temperature, HCL/MeOH (10 mL) was added to N-methyl-2-((3-((5-(pyrrolidin-1-ylmethoxy)pyridin-2-yl)amino)-1-(tetrahydro-2H-pyran-2-yl)-1H-indazol-6-yl)thio)benzamide 19d (86 mg, 150.16 μmol). The mixture was stirred at room temperature for about 4 hours. After thin-layer chromatography (dichloromethane/methanol = 10/1) indicated complete consumption of the starting material, the mixture was concentrated, poured into water (10 mL), and the aqueous phase was adjusted to neutral pH with sodium bicarbonate. The mixture was extracted with ethyl acetate (10 mL X 3). The combined organic layers were washed with saturated brine (20 mL), dried over anhydrous sodium sulfate, and concentrated to afford the crude product. The crude product was purified by preparative thin-layer chromatography (dichloromethane/methanol = 10/1, 0.1% NH3·H2O) to give N-methyl-2-((3-((5-(2-(pyrrolidin-1-yl)ethoxy)pyridin-2-yl)amino)-1H-indazol-6-yl)thio)benzamide I-30 as a pale yellow solid (20.3 mg, 41.55 μmol, 27.67% yield).

The product was confirmed by LCMS, H-NMR, and C-NMR.

MS-ESI calculated for [M+H]+ 489.2, found 489.1.

$^1$H NMR (400MHz, DMSO-d6): δ 12.18 (s,1H), 9.46 (s,1H), 8.37-8.38 (m,1H), 8.07 (d, 1H, J=8.4Hz), 7.91-7.95 (m, 2H), 7.49 (d, 1H, J=7.6Hz), 7.38-7.42 (m, 2H), 7.24-7.32 (m, 2H), 6.96-7.02 (m, 2H), 4.07-4.09 (m, 2H), 2.77-2.79 (m, 6H), 2.51-2.52 (m, 3H), 1.68 (s, 4H).

$^{13}$C NMR (100MHz, DMSO-d6): δ 168.36, 149.76, 149.30, 144.53, 141.58, 137.35, 136.32, 134.90, 133.12, 130.69, 130.35, 128.19, 126.48, 125.33, 123.37, 122.35, 114.54, 114.31, 110.61, 68.14, 54.88, 54.47, 26.56, 26.43, 23.62.

I-31

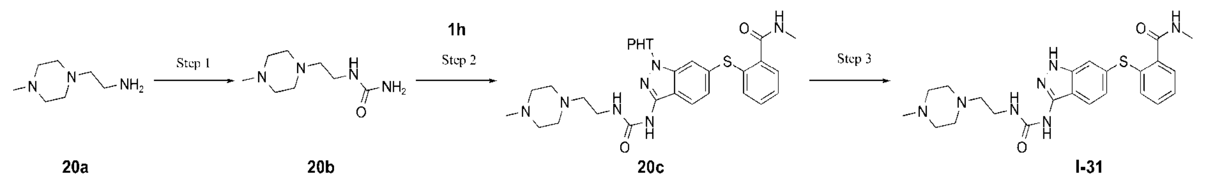


20a 20b 20c I-31

Step 1

At room temperature, acetic acid (55 mg, 915.88 μmol) and potassium cyanate (KOCN) (2.08 g, 24.44 mmol) were added to a mixture of 2-(4-methylpiperazin-1-yl)ethan-1-amine 20a (5 g, 34.91 mmol) and water (50 mL). The mixture was stirred at 25°C for about 2 hours. After thin-layer chromatography (dichloromethane/methanol = 10/1) indicated complete consumption of the starting material, the mixture was directly lyophilized to afford the crude product 1-(2-(4-methylpiperazin-1-yl)ethyl)urea 20b as a white solid of sufficient purity (6 g, 32.21 mmol, 92.28% yield).

The product was confirmed by LCMS and H-NMR.

MS-ESI calculated for [M+H]+ 187.1, found 187.1.

$^1$H NMR (400MHz, $CDCl_3$): δ (ppm) 5.44 (s, 1H), 4.70 (d, J = 78.3 Hz, 2H), 3.29 (m, J = 10.5, 5.1 Hz, 2H), 2.82 (t, J = 6.1 Hz, 2H), 2.31 (d, J = 1.4 Hz, 8H), 2.01 (s, 3H).

Step 2

Under nitrogen atmosphere at room temperature, potassium tert-butoxide (136.47 mg, 1.22 mmol) and copper(I) iodide (15.44 mg, 81.08 μmol) were added to a solution of 1-(2-(4-methylpiperazin-1-yl)ethyl)urea 20b (60.40 mg, 324.31 μmol) and 2-((3-iodo-1-(tetrahydro-2H-pyran-2-yl)-1H-indazol-6-yl)thio)-N-methylbenzamide 1h (200 mg, 405.38 μmol) in 1,4-dioxane (5 mL). The mixture was heated to 105°C and stirred for about 16 hours. After thin-layer chromatography (dichloromethane/methanol = 10/1) indicated complete consumption of the starting material, the mixture was poured into water (20 mL) and extracted with dichloromethane (20 mL × 3). The combined organic layers were washed with saturated brine (30 mL), dried over anhydrous sodium sulfate, and concentrated to afford the crude product. The crude product was purified by preparative thin-layer chromatography (dichloromethane/methanol = 10/1) to give N-methyl-2-((3-(2-(4-methylpiperazin-1-yl)ethylcarbamoylamino)-1-(tetrahydro-2H-pyran-2-yl)-1H-indazol-6-yl)thio)benzamide 20c as a green oily liquid (30 mg, 54.38 μmol, 13.41% yield).

The product was confirmed by LCMS.

MS-ESI calculated for [M+H]+ 552.3, found 552.1.

Step 3

At 0°C, trifluoroacetic acid (18.60 mg, 163.13 μmol) was added to a solution of N-methyl-2-((3-(2-(4-methylpiperazin-1-yl)ethylcarbamoylamino)-1-(tetrahydro-2H-pyran-2-yl)-1H-indazol-6-yl)thio)benzamide 20c (30 mg, 54.38 μmol) in dichloromethane (DCM) (5 mL). The mixture was stirred at room temperature for about 16 hours. After thin-layer chromatography (dichloromethane/methanol = 10/1) indicated complete consumption of the starting material, the mixture was poured into water (20 mL). The pH was adjusted to 8-9 with solid sodium carbonate, and the mixture was extracted with DCM (20 mL × 3). The combined organic layers were washed with saturated brine (20 mL), dried over anhydrous sodium sulfate, and concentrated to afford the crude product. The crude product was purified by preparative thin-layer chromatography to give N-methyl-2-((3-((2-(4-methylpiperazin-1-yl)ethyl)carbamoyl)amino)-1H-indazol-6-yl)thio)benzamide I-31 as a white solid (17.4 mg, 37.21 μmol, 68.43% yield).

The product was confirmed by LCMS, H-NMR, and C-NMR.

MS-ESI calculated for [M+H]+ 468.2, found 468.1.

$^1$H NMR (400MHz, $CDCl_3$&CD3OD): δ (ppm)7.74 (s, 1H), 7.46 (d, J = 19.2 Hz, 3H), 7.28 - 7.24 (m, 1H), 7.17 (s, 1H), 7.04 (d, J = 8.5 Hz, 1H), 3.47 (t, J = 6.2 Hz, 2H), 2.89 (s, 3H), 2.83 - 2.35 (m, 10H), 2.31 (s, 3H).

$^{13}$C NMR (100 MHz, $CDCl_3$&CD3OD) δ 170.12, 156.23, 142.10, 141.37, 131.23, 130.49, 127.83, 126.49, 123.83, 120.50, 113.71, 113.40, 57.02, 54.54, 52.32, 45.20, 37.01, 26.10.

I-16

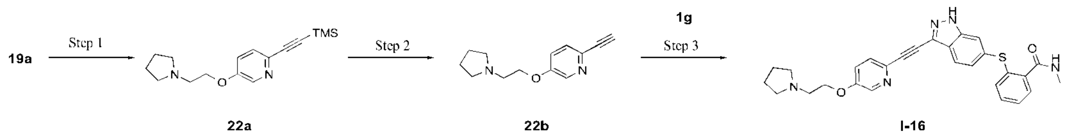


Step 1

Under nitrogen atmosphere at room temperature, ethynyl(trimethyl)silane (2.17 g, 22.13 mmol, 3.13 mL), bis(triphenylphosphine)palladium(II) dichloride (Pd(PPh3)2Cl2) (1.55 g, 2.21 mmol), copper(I) iodide (421 mg, 2.21 mmol), and triethylamine (3.36 g, 33.19 mmol) were added to a solution of 2-bromo-5-(2-(pyrrolidin-1-yl)methoxy)pyridine 19a (3.0 g, 11.06 mmol) in N,N-dimethylformamide (60 mL). The mixture was heated to 50°C and stirred for 12 hours. After thin-layer chromatography (dichloromethane/methanol = 10/1) indicated complete consumption of the starting material, the reaction mixture was poured into water (200 mL) and extracted with ethyl acetate (100 mL × 3). The combined organic layers were washed sequentially with water (100 mL × 3) and saturated brine (80 mL), dried over anhydrous sodium sulfate, and concentrated to afford the crude product. The crude product was purified by silica gel column chromatography (dichloromethane/methanol = 100/1 to 20/1) to give trimethyl-[2-[5-(2-pyrrolidin-1-ylmethoxy)-2-pyridyl]ethynyl]silane 22a as a black oily liquid (1.8 g, 56.4% yield).

The product was confirmed by LCMS and H-NMR.

MS-ESI calculated for [M+H]+ 289.2, found 289.2.

$^1$H NMR (400 MHz, $CDCl_3$): δ (ppm) 7.28 (d, J = 3.2 Hz, 1H), 7.40 (d, J = 8.8 Hz, 1H), 7.13-7.17 (m, 1H), 4.16 (t, J = 6.0 Hz, 2H), 2.89-2.97 (m, 2H), 2.62-2.66 (m, 4H), 1.81-1.84 (m, 4H), 0.26 (s, 9H).

Step 2

At room temperature, potassium carbonate (1.03 g, 7.49 mmol) was added to a solution of trimethyl(2-(5-(pyrrolidin-1-ylmethoxy)pyridin-2-yl)ethynyl)silane 22a (1.8 g, 6.24 mmol) in methanol (30 mL). The mixture was then stirred for about 2 hours. After thin-layer chromatography (dichloromethane/methanol = 10/1) indicated complete consumption of the starting material, the mixture was directly concentrated to afford the crude product. The crude product was purified by preparative thin-layer chromatography (dichloromethane/methanol = 10/1) to give 2-ethynyl-5-(2-(pyrrolidin-1-yl)ethoxy) pyridine 22b as a brownish-yellow oily liquid (0.6 g, 44.46% yield).

The product was confirmed by LCMS and H-NMR.

MS-ESI calculated for [M+H]+ 217.1, found 217.2.

$^1$H NMR (400 MHz, $CDCl_3$): δ (ppm) 8.29 (d, J = 2.8 Hz,1H), 7.42 (d, J = 8.4 Hz,1H), 7.15-7.18 (m, 1H), 4.17 (t, J = 5.6 Hz, 2H), 3.08 (s, 1H), 2.93 (t, J = 5.6 Hz, 2H), 2.62-2.65 (m, 4H), 1.80-1.84 (m, 4H).

Step 3

Under nitrogen atmosphere at room temperature, 2-ethynyl-5-(2-(pyrrolidin-1-yl)ethoxy)pyridine 22b (159 mg, 0.73 mmol), bis(triphenylphosphine)palladium(II) dichloride (10 mg, 0.01 mmol), copper(I) iodide (28 mg, 0.15 mmol), and triethylamine (184.6 mg, 1.82 mmol) were added to a solution of 2-((3-iodo-1H-indazol-6-yl)thio)-N-methylbenzamide 1g (300 mg, 0.73 mmol) in N,N-dimethylformamide (9 mL). The mixture was heated to 100°C and stirred for about 2 hours. After thin-layer chromatography (dichloromethane/methanol = 10/1) indicated complete consumption of the starting material, the mixture was poured into water (50 mL) and extracted with ethyl acetate (20 mL × 3). The combined organic layers were washed sequentially with water (30 mL × 3) and saturated brine (30 mL), dried over anhydrous sodium sulfate, and concentrated to afford the crude product. The crude product was purified by preparative thin-layer chromatography to give N-methyl-2-((3-(2-(5-(2-(pyrrolidin-1-ylethoxy)pyridin-2-yl)ethynyl)-1H-indazol-6-yl)thio)benzamide I-16 as a white solid (15.8 mg, 4.3% yield).

The product was confirmed by LCMS, H-NMR, and C-NMR.

MS-ESI calculated for [M+H]+ 498.2, found 498.1.

$^1$H NMR (400 MHz, $CDCl_3$) δ: 11.14 (br s, 1H), 8.39 (d, J=2.4Hz, 1H), 7.88 (d, J=8.4Hz, 1H), 7.59-7.64 (m, 3H), 7.30-7.31 (m, 2H), 7.19-7.27 (m, 3H), 6.37 (d, J=4.4Hz, 1H), 4.22 (t, J =6.0 Hz, 2H), 2.96-2.99 (m, 5H), 2.68 (s, 4H), 1.85 (s, 4H).

$^{13}$C NMR (100 MHz, DMSO-d6) δ: 168.30, 155.10, 141.06, 139.36, 137.86, 135.52, 134.19, 134.12, 130.92, 130.80, 128.78, 128.29, 128.02, 126.91, 126.36, 123.99, 121.72, 121.07, 115.10, 92.63, 79.46, 67.92, 54.57, 54.44, 26.56, 23.64.

I-8

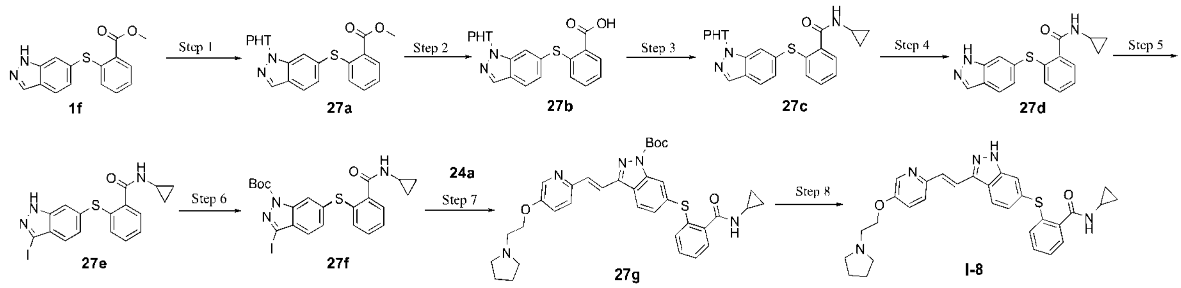


Step 1

3,4-Dihydro-2H-pyran (DHP) (1.8 g, 21.62 mmol) and p-toluenesulfonic acid (TsOH) (2.5 g, 14.78 mmol) were added at room temperature to a solution of 2-((1H-indazol-6-yl)thio)-N-methylbenzamide 1f (4 g, 14.07 mmol) in tetrahydrofuran (THF) (20 mL). The mixture was heated to 60°C and stirred for about 16 hours. After thin-layer chromatography (petroleum ether/ethyl acetate = 3/1) indicated complete consumption of the starting material, the mixture was concentrated to afford the crude product. The crude product was purified by preparative MPLC to give methyl 2-((1-(tetrahydro-2H-pyran-2-yl)-1H-indazol-6-yl)thio)benzoate 27a as a yellow solid (2.5 g, 6.79 mmol, 48.23% yield).

The product was confirmed by LCMS and H-NMR.

MS-ESI calculated for [M+H]+ 369.1, found 369.0.

$^1$H NMR (400 MHz, $CDCl_3$) δ 8.07 (d, J = 5.6 Hz, 1H), 8.02 (d, J = 7.6 Hz, 1H), 7.93 (d, J = 4.0 Hz, 1H), 7.77 (dd, J = 8.4, 4.0 Hz, 1H), 7.31 (d, J = 8.4 Hz, 1H), 7.25 (t, J = 7.6 Hz, 1H), 7.15 (t, J = 7.6 Hz, 1H), 6.85 (d, J = 8.0 Hz, 1H), 5.72 (dd, J = 9.6, 2.4 Hz, 1H), 4.13 - 4.03 (m, 1H), 3.99 (d, J = 2.0 Hz, 3H), 3.75 (dd, J = 11.2, 8.4 Hz, 1H), 2.59 - 2.39 (m, 1H), 2.13 (t, J = 10.4 Hz, 2H), 1.85 - 1.52 (m, 3H).

Step 2

At room temperature, methyl 2-((1-(tetrahydro-2H-pyran-2-yl)-1H-indazol-6-yl)thio)benzoate 27a (2.5 g, 6.79 mmol) was dissolved in a mixture of H2O (8 mL) and THF (20 mL). Lithium hydroxide (1.7 g, 40.71 mmol) was added, and the mixture was heated to 40°C and stirred for about 16 hours. After thin-layer chromatography (petroleum ether/ethyl acetate = 1/1) indicated complete consumption of the starting material, the reaction mixture was poured into water (20 mL). The pH was adjusted to 6-7 with 1 N hydrochloric acid solution, and the mixture was extracted with ethyl acetate (20 mL × 3). The combined organic layers were washed with saturated brine (20 mL), dried over anhydrous sodium sulfate, and concentrated to give 2-((1-(tetrahydro-2H-pyran-2-yl)-1H-indazol-6-yl)thio)benzoic acid 27b as a white solid (2 g, 5.64 mmol, 83.17% yield).

The product was confirmed by LCMS and H-NMR.

MS-ESI calculated for [M+H]+ 355.1, found 355.0.

$^1$H NMR (400 MHz, $CDCl_3$) δ 8.21 - 8.07 (m, 2H), 7.97 (s, 1H), 7.79 (dd, J = 8.4, 4.4 Hz, 1H), 7.40 - 7.30 (m, 1H), 7.28 - 7.25 (m, 1H), 7.18 (dd, J = 14.4, 7.2 Hz, 1H), 6.84 (dd, J = 8.0, 4.8 Hz, 1H), 5.74 (dd, J = 9.6, 2.4 Hz, 1H), 4.24 - 3.92 (m, 2H), 2.66 - 2.28 (m, 2H), 2.25 - 2.07 (m, 2H), 1.89 - 1.58 (m, 2H).

Step 3

At room temperature, cyclopropylamine (290 mg, 5.08 mmol), HATU (1.9 g, 5.08 mmol), and N,N-diisopropylethylamine (DIEA) (980 mg, 7.60 mmol) were added to a solution of 2-((1-(tetrahydro-2H-pyran-2-yl)-1H-indazol-6-yl)thio)benzoic acid 27b (900 mg, 2.54 mmol) in N,N-dimethylformamide (10 mL). The mixture was then stirred for about 2

hours. After thin-layer chromatography (petroleum ether/ethyl acetate = 1/1) indicated complete consumption of the starting material, the mixture was poured into water (50 mL), extracted with ethyl acetate (20 mL × 3), and the combined organic layers were washed with saturated brine (50 mL), dried over anhydrous sodium sulfate, and concentrated to give the crude product. The crude product was purified by silica gel column chromatography (petroleum ether/ethyl acetate = 2/1) to give N-cyclopropyl-2-((1-(tetrahydro-2H-pyran-2-yl)-1H-indazol-6-yl)thio)benzamide 27c as a white solid (700 mg, 1.78 mmol, 70.05% yield).

The product was confirmed by LCMS and H-NMR.

MS-ESI calculated for [M+H]+ 394.2, found 394.2.

$^1H$ NMR (400 MHz, $CDCl_3$) δ 8.11 - 7.90 (m, 2H), 7.71 - 7.58 (m, 3H), 7.30 (dd, J = 4.8, 3.6 Hz, 2H), 7.25 - 7.18 (m, 1H), 7.12 (dd, J = 8.4, 1.2 Hz, 1H), 5.66 (dd, J = 9.6, 2.4 Hz, 1H), 4.11 - 3.89 (m, 2H), 2.38 (ddd, J = 17.6, 13.2, 7.6 Hz, 1H), 2.20 - 2.04 (m, 2H), 1.72 (ddd, J = 62.4, 40.0, 27.2 Hz, 4H), 0.82 (dd, J = 12.8, 7.2 Hz, 2H), 0.54 - 0.44 (m, 2H).

Step 4

At room temperature, trifluoroacetic acid (3 g, 52.62 mmol) was added to a solution of N-cyclopropyl-2-((1-(tetrahydro-2H-pyran-2-yl)-1H-indazol-6-yl)thio)benzamide 27c (800 mg, 2.03 mmol) in dichloromethane (10 mL). The mixture was stirred at 40°C for about 16 hours. After thin-layer chromatography (dichloromethane/methanol = 10/1) indicated complete consumption of the starting material, the mixture was poured into water (20 mL). The pH was adjusted to 8-9 with sodium carbonate solution, and the mixture was extracted with dichloromethane (30 mL × 3). The combined organic layers were washed with saturated brine (20 mL), dried over anhydrous sodium sulfate, and concentrated to give N-cyclopropyl-2-((1H-indazol-6-yl)thio)benzamide 27d as a yellow solid (500 mg, 1.62 mmol, 79.49% yield).

The product was confirmed by LCMS and H-NMR.

MS-ESI calculated for [M+H]+ 310.1, found 310.0.

$^1H$ NMR (400 MHz, $CDCl_3$) δ 10.99 (s, 1H), 8.05 (s, 1H), 7.77 - 7.53 (m, 3H), 7.32 - 7.20 (m, 3H), 7.12 (d, J = 8.4 Hz, 1H), 6.60 (s, 1H), 2.90 - 2.80 (m, 1H), 0.81 (q, J = 6.4 Hz, 2H), 0.51 (d, J = 6.4 Hz, 2H).

Step 5

At room temperature, potassium carbonate (2.3 g, 16.81 mmol) and I2 (2.1 g, 8.40 mmol) were added to a solution ofN-cyclopropyl-2-((1H-indazol-6-yl)thio)benzamide 27d (1.3 g, 4.20 mmol) in N,N-dimethylformamide (10 mL). The mixture was stirred at 40°C for about 2 hours. After thin-layer chromatography (dichloromethane/methanol = 10/1) indicated complete consumption of the starting material, the mixture was poured into water (100 mL), extracted with ethyl acetate (100 mL × 3), and the combined organic layers were washed with saturated brine (200 mL), dried over anhydrous sodium sulfate, and concentrated to give the crude product. The crude product was purified by silica gel column chromatography (petroleum ether/ethyl acetate = 2/1 to 0/1) to give N-cyclopropyl-2-((3-iodo-1H-indazol-6-yl)thio) benzamide 27e as a yellow solid (1 g, 2.30 mmol, 54.67% yield).

The product was confirmed by LCMS and H-NMR.

MS-ESI calculated for [M+H]+ 436.0, found 435.9.

$^1H$ NMR (400 MHz, $CDCl_3$) δ 11.17 (s, 1H), 8.04 (s, 1H), 7.68 - 7.55 (m, 2H), 7.46 (d, J = 8.4 Hz, 1H), 7.32 (dd, J = 9.2, 5.6 Hz, 2H), 7.25 - 7.20 (m, 1H), 7.17 (d, J = 8.4 Hz, 1H), 2.88 - 2.80 (m, 1H), 0.85 (q, J = 6.8 Hz, 2H), 0.53 (q, J = 6.8 Hz, 2H).

Step 6

At room temperature, di-tert-butyl dicarbonate (250 mg, 1.15 mmol) and triethylamine (232 mg, 2.30 mmol) were added to a solution of N-cyclopropyl-2-((3-iodo-1H-indazol-6-yl)thio)benzamide 27e (1.3 g, 4.20 mmol) in THF (10 mL). The mixture was then stirred for about 2 hours. After thin-layer chromatography (dichloromethane/methanol = 10/1) indicated complete consumption of the starting material, the mixture was poured into water (20 mL), extracted with ethyl acetate (20 mL × 3), and the combined organic layers were washed with saturated brine (30 mL), dried over anhydrous sodium sulfate, and concentrated to give the crude product. The crude product was purified by preparative thin-layer chromatography (dichloromethane/methanol = 10/1) to give tert-butyl 6-((2-(cyclopropylcarbamoyl)phenyl)thio)-3-iodo-1H-indazole-1-carboxylate 27f as a white solid (280 mg, 522.98 μmol, 45.53% yield).

The product was confirmed by LCMS and H-NMR.

MS-ESI calculated for [M-Boc+H]+ 436.0, found 435.9.

$^1H$ NMR (400 MHz, $CDCl_3$) δ 8.04 (s, 1H), 7.75 - 7.62 (m, 1H), 7.40 (dd, J = 13.6, 5.6 Hz, 4H), 7.31 - 7.19 (m, 2H), 2.81 (d, J = 4.4 Hz, 1H), 1.64 (s, 9H), 0.79 (t, J = 6.0 Hz, 2H), 0.47 (d, J = 2.4 Hz, 2H).

Step 7

At room temperature under nitrogen protection, 5-(2-(pyrrolidin-1-yl)ethoxy)-2-ethenylpyridine 24a (97 mg, 448.27 μmol), triethylamine (113 mg, 1.12 mmol), Pd2(dba)3 (171 mg, 186.78 μmol), and P(o-tol)3 (113 mg, 373.55 μmol) were added to a solution of tert-butyl 6-((2-(cyclopropylcarbamoyl)phenyl)thio)-3-iodo-1H-indazole-1-carboxylate 27f (200 mg, 373.55 μmol) in 1,4-dioxane (5 mL). The mixture was heated to 100°C and stirred for about 2 hours. After thin-layer chromatography (dichloromethane/methanol = 10/1) indicated complete consumption of the starting material, the mixture was poured into water (20 mL) and extracted with ethyl acetate (10 mL × 3). The combined organic layers were washed with saturated brine (20 mL), dried over anhydrous sodium sulfate, and concentrated to give the crude product. The crude product was purified by preparative thin-layer chromatography (dichloromethane/methanol = 10/1) to give tert-butyl 6-((2-(cyclopropylcarbamoyl)phenyl)thio)-3-((E)-2-(5-(2-(pyrrolidin-1-yl)ethoxy)pyridin-2-yl)ethenyl)-1H-indazole-1-carboxylate 27g as a yellow oil (150 mg, 239.70 μmol, 64.17% yield).

The product was confirmed by LCMS.

MS-ESI calculated for [M+H]+ 626.3, found 626.2.

Step 8

At room temperature, trifluoroacetic acid (1 g, 8.77 mmol) was added to a solution of tert-butyl 6-((2-(cyclopropylcarbamoyl)phenyl)thio)-3-((E)-2-(5-(2-(pyrrolidin-1-yl)ethoxy)pyridin-2-yl)ethenyl)-1H-indazole-1-carboxylate 27g (150 mg, 239.70 μmol) in dichloromethane (5 mL). The mixture was then stirred for about 4 hours. After thin-layer chromatography (dichloromethane/methanol = 10/1) indicated complete consumption of the starting material, the mixture was poured into water (20 mL). The pH was adjusted to 8-9 with sodium carbonate solution, and the mixture was extracted with ethyl acetate (20 mL × 3). The combined organic layers were washed with saturated brine (30 mL), dried over anhydrous sodium sulfate, and concentrated to give the crude product. The crude product was purified by preparative thin-layer chromatography to give N-cyclopropyl-2-((3-((E)-2-(5-(2-(pyrrolidin-1-yl)ethoxy)pyridin-2-yl)ethenyl)-1H-indazol-6-yl)thio)benzamide I-8 as a white solid (15.4 mg, 29.30 μmol, 12.22% yield).

The product was confirmed by LCMS and H-NMR.

MS-ESI calculated for [M+H]+ 526.2, found 526.2.

$^1$H NMR (400 MHz, $CDCl_3$) δ 8.38 (d, J = 2.8 Hz, 1H), 8.01 (d, J = 8.4 Hz, 1H), 7.75 (d, J = 16.4 Hz, 1H), 7.67 (dd, J = 5.6, 3.2 Hz, 1H), 7.58 - 7.41 (m, 2H), 7.35 (dd, J = 24.0, 19.6 Hz, 2H), 7.25 (dd, J = 8.8, 2.8 Hz, 2H), 7.18 (d, J = 8.4 Hz, 1H), 6.54 (s, 1H), 4.24 (t, J = 5.6 Hz, 2H), 3.00 (t, J = 5.6 Hz, 2H), 2.89 - 2.80 (m, 1H), 2.72 (s, 4H), 1.87 (d, J = 3.2 Hz, 4H), 0.86 - 0.75 (m, 2H), 0.50 (t, J = 8.0 Hz, 2H).

I-38

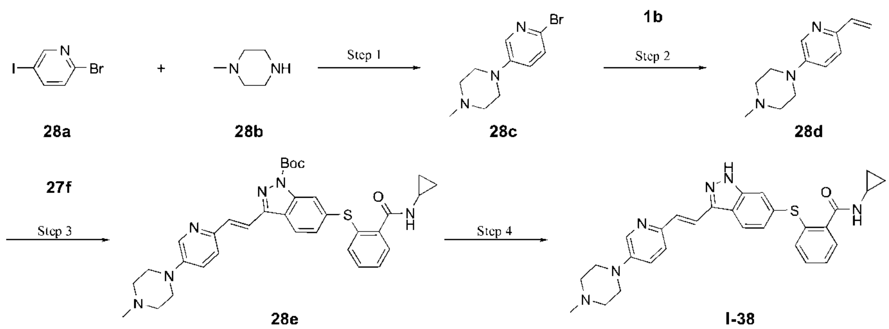


Step 1

At room temperature under nitrogen protection, 1-methylpiperazine 28b (2.1 g, 21.13 mmol), sodium tert-butoxide (5.1 g, 52.84 mmol), Pd2(dba)3 (806.4 mg, 880.62 μmol), and Xantphos (254.8 mg, 440.31 μmol) were added to a solution of 2-bromo-5-iodopyridine 28a (5 g, 17.61 mmol) in 1,4-dioxane (50 mL). The mixture was heated to 60°C and stirred for about 3 hours. After thin-layer chromatography (dichloromethane/methanol = 10/1) indicated complete consumption of the starting material, the mixture was poured into water (100 mL) and extracted with ethyl acetate (50 mL × 3). The combined organic layers were washed with saturated brine (100 mL), dried over anhydrous sodium sulfate, and concentrated to give the crude product. The crude product was purified by neutral alumina column chromatography (petroleum ether/ethyl acetate = 10/1) to give 1-(6-bromopyridin-3-yl)-4-methylpiperazine 28c as a yellow solid (2.1 g, 8.20

mmol, 46.55% yield).

The product was confirmed by LCMS and H-NMR.

MS-ESI calculated for [M+H]+ 257.9, found 258.0.

$^1$H NMR (400 MHz, $CDCl_3$) δ (ppm) 8.01 (d, J = 3.2 Hz, 1H), 7.30 (d, J = 8.8 Hz, 1H), 7.07 (dd, J = 8.8, 3.2 Hz, 1H), 3.21 (t, J = 4.8 Hz, 4H ), 2.57 (t, J = 5.2 Hz, 4H ), 2.35 (s, 3H).

Step 2

At room temperature under nitrogen protection, potassium ethenyltrifluoroborate 1b (1.3 g, 9.37 mmol), Pd(dppf) Cl2 (571.3 mg, 780.82 μmol), and triethylamine (2.4 g, 23.42 mmol) were added to a solution of 1-(6-bromopyridin-3-yl)-4-methylpiperazine 28c (2 g, 7.81 mmol) in N,N-dimethylformamide (20 mL). The mixture was heated to 85°C and stirred for about 2 hours. After thin-layer chromatography (dichloromethane/methanol = 10/1) indicated complete consumption of the starting material, the mixture was poured into water (100 mL) and extracted with ethyl acetate (50 mL × 3). The combined organic layers were washed with saturated brine (100 mL), dried over anhydrous sodium sulfate, and concentrated to give the crude product. The crude product was purified by neutral alumina column chromatography (petroleum ether/ethyl acetate = 10/1) to give 1-methyl-4-(6-ethenylpyridin-3-yl)piperazine 28d as a yellow oily liquid (1.2 g, 5.90 mmol, 75.60% yield).

The product was confirmed by LCMS and H-NMR.

MS-ESI calculated for [M+H]+ 204.1, found 204.1.

$^1$H NMR (400 MHz, $CDCl_3$) δ (ppm) 8.27 (d, J = 3.2 Hz, 1H), 7.24 (s, 1H), 7.16-7.13 (m, 1H), 6.75 (dd, J = 17.5, 10.8 Hz, 1H), 5.98 (d, J = 17.6 Hz, 1H), 5.31 (d, J = 10.8 Hz, 1H), 3.28 - 3.20 (m, 4H), 2.62 - 2.55 (m, 4H), 2.36 (s, 3H).

Step 3

At room temperature under nitrogen protection, 1-methyl-4-(6-ethenylpyridin-3-yl)piperazine 28d (223.3 mg, 1.10 mmol), triethylamine (277.8 mg, 2.75 mmol), Pd2(dba)3 (502.8 mg, 549.13 μmol), and P(o-tol)3 (557.1 mg, 1.83 mmol) were added to a solution of tert-butyl 6-((2-(cyclopropylcarbamoyl)phenyl)thio)-3-iodo-1H-indazole-1-carboxylate 27f (490 mg, 915.21 μmol) in 1,4-dioxane (10 mL). The mixture was heated to 85°C and stirred for about 2 hours. After thin-layer chromatography (dichloromethane/methanol = 10/1) indicated complete consumption of the starting material, the mixture was poured into water (40 mL) and extracted with ethyl acetate (30 mL × 3). The combined organic layers were washed with saturated brine (40 mL), dried over anhydrous sodium sulfate, and concentrated to give the crude product. The crude product was purified by preparative MPLC to give tert-butyl 6-((2-(cyclopropylcarbamoyl)phenyl) thio)-3-((E)-2-(5-(4-methylpiperazin-1-yl)pyridin-2-yl)ethenyl)-1H-indazole-1-carboxylate 28e as a white solid (110 mg, 180.10 μmol, 19.68% yield).

The product was confirmed by LCMS.

MS-ESI calculated for [M+H]+ 611.3, found 611.2.

Step 4

At room temperature, trifluoroacetic acid (186.7 mg, 1.64 mmol) was added to a solution of tert-butyl 6-((2-(cyclopropylcarbamoyl)phenyl)thio)-3-((E)-2-(5-(4-methylpiperazin-1-yl)pyridin-2-yl)ethenyl)-1H-indazole-1-carboxylate 28e (100 mg, 163.73 μmol) in dichloromethane (2 mL), and the mixture was stirred for about 16 hours. After thin-layer chromatography (dichloromethane/methanol = 10/1) indicated complete consumption of the starting material, the mixture was poured into water (20 mL). The pH was adjusted to 8-9 with a sodium carbonate solution, and the mixture was extracted with dichloromethane (20 mL × 3). The combined organic layers were washed with saturated brine (20 mL), dried over anhydrous sodium sulfate, and concentrated to give the crude product. The crude product was purified by preparative thin-layer chromatography to give N-cyclopropyl-2-({3-[(E)-2-[5-(4-methylpiperazin-1-yl)pyridin-2-yl]ethenyl]-1H-indazol-6-yl}thio)benzamide I-38 as a yellow solid (50 mg, 97.91 μmol, 59.80% yield).

The product was confirmed by LCMS, H-NMR, and C-NMR.

MS-ESI calculated for [M+H]+ 511.2, found 511.0.

$^1$H NMR (400MHz, CD30D): δ (ppm) 8.22 (d, J = 2.8 Hz, 1H), 8.02 (d, J = 8.8 Hz, 1H), 7.64 - 7.54 (m, 3H), 7.49-7.43 (m, 2H), 7.40 - 7.29 (m, 3H), 7.25 - 7.23 (m, 1H), 7.17 (d, J = 8.4 Hz, 1H), 3.33 (s, 4H), 2.79-2.75 (m, 1H), 2.65-2.62 (m, 4H), 2.36 (s, 3H), 0.77-0.72 (m, 2H), 0.55 - 0.51 (m, 2H).

$^{13}$C NMR (100 MHz, $CDCl_3$& CD3OD) δ: 172.68, 147.59, 147.39, 138.66, 137.99, 135.33, 131.77, 130.85, 128.00, 126.48, 124.48, 123.29, 122.73, 122.17, 121.60, 55.71, 23.95, 6.80.

I-40

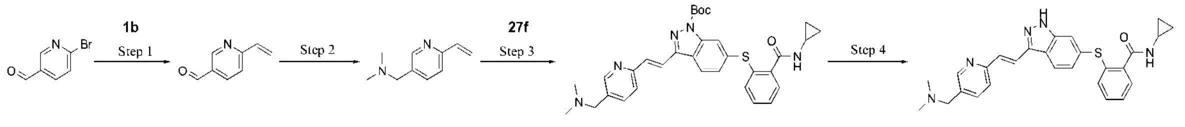


29a 29b 29c 29d I-40

Step 1

At room temperature under nitrogen protection, potassium ethenyltrifluoroborate 1b (5.4 g, 40.32 mmol), Pd(dppf)Cl2 (975.3 mg, 1.34 mmol), and triethylamine (8.2 g, 80.64 mmol) were added to a solution of 6-bromopyridine-3-carbaldehyde 29a (5 g, 26.88 mmol) in N,N-dimethylformamide (50 mL). The mixture was heated to 85°C and stirred for about 16 hours. After thin-layer chromatography (petroleum ether/ethyl acetate = 10/1) indicated complete consumption of the starting material, the mixture was poured into water (250 mL) and extracted with ethyl acetate (100 mL × 3). The combined organic layers were washed sequentially with water (300 mL) and saturated brine (300 mL), dried over anhydrous sodium sulfate, and concentrated to give the crude product. The crude product was purified by silica gel column chromatography (petroleum ether/ethyl acetate = 10/1) to give 6-ethenylpyridine-3-carbaldehyde 29b as a yellow oily liquid (1.7 g, 12.77 mmol, 47.50% yield).

The product was confirmed by LCMS and H-NMR.

MS-ESI calculated for [M+H]+ 134.1, found 134.1.

$^1$H NMR (400 MHz, $CDCl_3$): δ 10.08 (s, 1H), 9.01 (s, 1H), 8.13 (d, J = 8.0 Hz, 1H), 7.48 (d, J = 8.4 Hz, 1H), 6.88 (dd, J=17.6 Hz, 10.8 Hz, 1H),6.41 (d, J=17.6 Hz, 1H), 5.68 (d, J=10.8 Hz, 1H).

Step 2

At room temperature, N-methylmethanamine hydrochloride (612.4 mg, 7.51 mmol) and NaBH(OAc)3 (2.4 g, 11.27 mmol) were added to a solution of 6-ethenylpyridine-3-carbaldehyde 29b (500 mg, 3.76 mmol) in THF (20 mL), and the mixture was stirred for about 16 hours. After thin-layer chromatography (dichloromethane/methanol = 10/1) indicated complete consumption of the starting material, the mixture was poured into water (20 mL). The pH was adjusted to 8-9 with sodium bicarbonate, and the mixture was extracted with ethyl acetate (20 mL × 3). The combined organic layers were washed with saturated brine (30 mL), dried over anhydrous sodium sulfate, and concentrated to give the crude product. The crude product was purified by silica gel column chromatography (DCM) to give N,N-dimethyl-1-(6-ethenylpyridin-3-yl) methanamine 29c as a white solid (400 mg, 2.47 mmol, 65.66% yield).

The product was confirmed by LCMS and H-NMR.

MS-ESI calculated for [M+H]+ 163.1, found 163.1.

$^1$H NMR (400MHz, $CDCl_3$ ): δ 8.45 (s, 1H), 7.62 (dd, J = 8.0 Hz, 1.6 Hz, 1H), 7.32 (d, J = 8.0 Hz, 1H), 6.81 (dd, J = 17.6 Hz, 10.8 Hz, 1H), 6.16 (d, J = 17.6 Hz, 1H), 5.46 (dd, J = 10.8 Hz, 4.4 Hz, 1H), 3.41 (s, 2H), 2.23 (s, 6H).

Step 3

At room temperature under nitrogen protection, N,N-dimethyl-1-(6-ethenylpyridin-3-yl)methanamine 29c (303 mg, 1.87 mmol), triethylamine (472.5 mg, 4.67 mmol), Pd2(dba)3 (427.6 mg, 466.94 μmol), and P(o-tol)3 (426.4 mg, 1.40 mmol) were added to a solution of tert-butyl 6-((2-(cyclopropylcarbamoyl)phenyl)thio)-3-iodo-1H-indazole-1-carboxylate 27f (500 mg, 933.89 μmol) in 1,4-dioxane (10 mL). The mixture was heated to 85°C and stirred for about 2 hours. After thin-layer chromatography (petroleum ether/ethyl acetate = 1/1) indicated complete consumption of the starting material, the mixture was poured into water (20 mL) and extracted with ethyl acetate (20 mL × 3). The combined organic layers were washed with saturated brine (50 mL), dried over anhydrous sodium sulfate, and concentrated to give the crude product. The crude product was purified by preparative MPLC to give tert-butyl 6-((2-(cyclopropylcarbamoyl)phenyl) thio)-3-((E)-2-(5-((dimethylamino)methyl)pyridin-2-yl)ethenyl)-1H-indazole-1-carboxylate 29d as a yellow oily liquid (155 mg, 272.07 μmol, 29.13% yield).

The product was confirmed by LCMS.

MS-ESI calculated for [M+H]+ 570.2, found 570.2.

Step 4

At room temperature, trifluoroacetic acid (300.2 mg, 2.63 mmol) was added to a solution of tert-butyl 6-((2-(cyclopropylcarbamoyl)phenyl)thio)-3-((E)-2-(5-((dimethylamino)methyl)pyridin-2-yl)ethenyl)-1H-indazole-1-carboxylate 29d (150 mg, 263.29 μmol) in dichloromethane (5 mL), and the mixture was stirred for about 16 hours. After thin-layer chromatography (dichloromethane/methanol = 10/1) indicated complete consumption of the starting material, the mixture was poured into water (20 mL). The pH was adjusted to 8-9 with a sodium carbonate solution, and the mixture was extracted with dichloromethane (20 mL × 3). The combined organic layers were washed with saturated brine (30 mL), dried over anhydrous sodium sulfate, and concentrated to give the crude product. The crude product was purified by preparative thin-layer chromatography to give N-cyclopropyl-2-((3-((E)-2-(5-((dimethylamino)methyl)pyridin-2-yl)ethenyl)-1H-indazol-6-yl)thio)benzamide I-40 as a white solid (29 mg, 61.75 μmol, 23.46% yield).

The product was confirmed by LCMS, H-NMR, and C-NMR.

MS-ESI calculated for [M+H]+ 470.2, found 470.1.

$^1$H NMR (400MHz, CD3OD): δ 8.49 (s, 1H), 8.05 (d, J = 8.64 Hz, 1H), 7.86 (d, J = 16.8 Hz, 1H), 7.80 (dd, J = 8.0 Hz, 1.6 Hz, 1H), 7.68 (d, J = 8.0 Hz, 1H), 7.56 (d, J = 16.6 Hz, 2H), 7.49 - 7.44 (m, 1H), 7.37 - 7.27 (m, 2H), 7.24-7.2 (m, 2H), 3.57 (s, 2H), 2.63-2.8 (m,1H), 2.3 (s, 6H), 0.76 (q, J = 7.0 Hz, 2H), 0.60 - 0.51 (m, 2H).

13CNMR (100MHz, CD3OD&$CDCl_3$): δ 172.66, 156.23, 151.27, 139.82, 133.39, 132.97, 131.82, 130.51, 129.21, 128.05, 126.72, 122.94, 122.64, 121.78, 61.87, 23.98, 6.88.

I-33

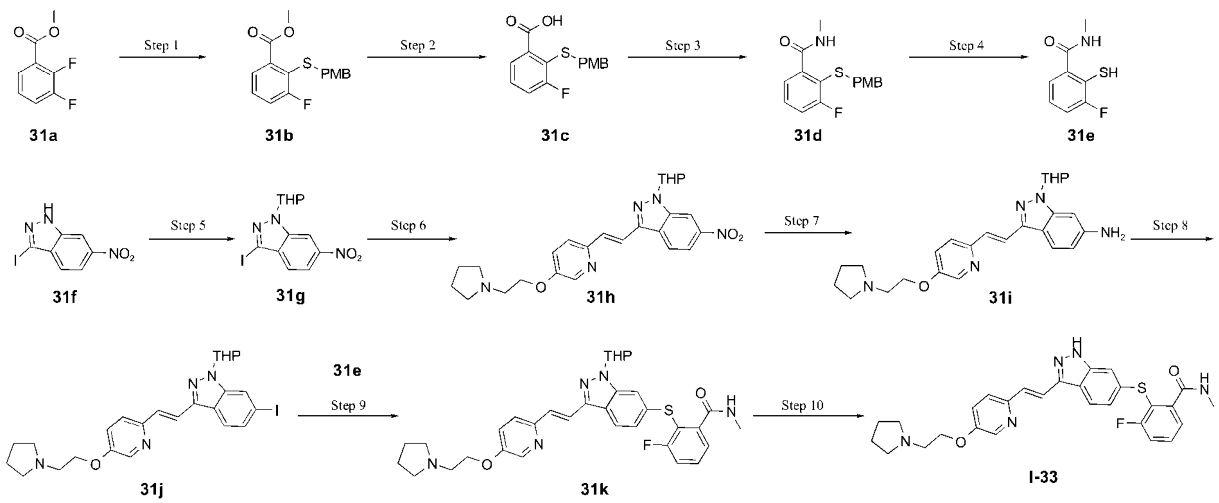


31a 31b 31c 31d 31e 31f 31g 31h 31i 31j 31k I-33

Step 1

To a 250 mL single-necked flask, methyl 2,3-difluorobenzoate 31a (5.00 g, 29.1 mmol) was dissolved in N,N-dimethylformamide (50 mL). (4-Methoxyphenyl)methanethiol (4.564 g, 29.1 mmol) was added, followed by cesium carbonate (18.94 g, 58.2 mmol). The mixture was stirred at room temperature for 2 hours. The reaction mixture was filtered, and the filtrate was collected. Ethyl acetate (200 mL) was added to the filtrate for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 3:1). Concentration afforded methyl 3-fluoro-2-[(4-methoxyphenyl)methylthio] benzoate 31b as a white solid (4.5 g, 14.7 mmol, 50.5% yield).

MS (ESI) M/Z: 329.1 [M+Na+]

Step 2

To a 250 mL single-necked flask, methyl 3-fluoro-2-[(4-methoxyphenyl)methylthio]benzoate 31b (4.50 g, 14.7 mmol) was dissolved in tetrahydrofuran (45 mL). Lithium hydroxide (1.853 g, 44.1 mmol) was added, followed by water (15 mL). The mixture was heated to 50°C and stirred for 5 hours. The reaction mixture was filtered, and the filtrate was collected and concentrated under reduced pressure. Dilute hydrochloric acid (1 mol/L) was added to the system to adjust the pH to 5.

After filtration and drying, 3-fluoro-2-[(4-methoxyphenyl)methylthio]benzoic acid 31c was obtained as a white solid (3.80 g, 13.0 mmol, 88.4% yield).

MS (ESI) M/Z: 315.2 [M+Na+]

Step 3

To a 250 mL single-necked flask, 3-fluoro-2-[(4-methoxyphenyl)methylthio]benzoic acid 31c (3.80 g, 13.0 mmol) was dissolved in N,N-dimethylformamide (38 mL). Methylamine (506 mg, 19.5 mmol) was added, followed by N-methylmorpholine (3.943 g, 39.0 mmol), 1-hydroxybenzotriazole (2.64 g, 19.5 mmol), and 1-(3-dimethylaminopropyl)-3-ethylcarbodiimide hydrochloride (3.72 g, 19.5 mmol). The mixture was maintained at room temperature and stirred for 3 hours. Ethyl acetate (200 mL) was added to the reaction system for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 4:1). Concentration afforded 3-fluoro-2-[(4-methoxyphenyl)methylthio]-N-methylbenzamide 31d as a white solid (2.95 g, 9.66 mmol, 74.3% yield).

Step 4

To a 250 mL three-necked flask, 3-fluoro-2-[(4-methoxyphenyl)methylthio]-N-methylbenzamide 31d (2.95 g, 9.66 mmol) was dissolved in trifluoroacetic acid (32 mL). The mixture was heated to 70°C and stirred for 3 hours. After concentration under reduced pressure, the solid residue was purified by silica gel column chromatography (eluent: dichloromethane/methanol = 30:1). Concentration afforded 3-fluoro-N-methyl-2-mercaptobenzamide 31e as a white solid (1.05 g, 5.68 mmol, 58.8% yield).

Step 5

At room temperature, 3,4-dihydro-2H-pyran (2.2 g, 25.95 mmol) and p-toluenesulfonic acid (357.5 mg, 2.08 mmol) were added to a solution of 3-iodo-6-nitro-1H-indazole 31f (5 g, 17.30 mmol) in ethyl acetate (100 mL). The mixture was heated to 75°C and stirred for 16 hours. After thin-layer chromatography (petroleum ether/ethyl acetate = 10/1) indicated complete consumption of the starting material, the reaction mixture was concentrated to give the crude product. The crude product was purified by column chromatography (petroleum ether/ethyl acetate = 20/1) to give compound 3-iodo-6-nitro-1-tetrahydropyran-2-ylindazole 31g as a yellow solid (6.1 g, 16.35 mmol, 94.50% yield). The product was confirmed by H-NMR.

$^1$H NMR (400 MHz, $CDCl_3$) δ 8.58 (s, 1H), 8.11 (dd, J = 8.8, 1.2 Hz, 1H), 7.63 (d, J = 8.8 Hz, 1H), 5.81 (dd, J = 9.2, 2.8 Hz, 1H), 4.06 (d, J = 10.4 Hz, 1H), 3.84-3.79 (m, 1H), 2.56-2.53 (m, 1H), 2.18-2.15 (m 2H), 1.81-1.73 (m, 3H).

Step 6

At room temperature under nitrogen, compound 5-(2-pyrrolidin-1-ylethoxy)-2-ethenylpyridine 24a (3.5 g, 16.08 mmol), tris(dibenzylideneacetone)dipalladium (6.1 g, 6.70 mmol), triethylamine (4.1 g, 40.20 mmol), and tri(o-tolyl) phosphine (4.1 g, 13.40 mmol) were added to a solution of compound 3-iodo-6-nitro-1-tetrahydropyran-2-ylindazole 31g (5 g, 13.40 mmol) in 1,4-dioxane (60 mL). The mixture was heated to 100°C and stirred for 2 hours. After thin-layer chromatography (dichloromethane/methanol = 10/1) indicated complete consumption of the starting material, the mixture was poured into water (120 mL) and filtered. The filtrate was extracted with dichloromethane (120 mL × 3). The combined organic layers were dried over anhydrous sodium sulfate and concentrated to give the crude product. The crude product was purified by column chromatography (dichloromethane/methanol = 20/1) to give compound 6-nitro-3-[(E)-2-[5-(2-pyrrolidin-1-ylethoxy)pyridin-2-y1]ethenyl]-1-tetrahydropyran-2-ylindazole 31h as a yellow solid (2.2 g, 4.64 mmol, 34.62% yield).

The product was confirmed by LCMS and H-NMR.

MS-ESI calculated for [M+H]+ 464.2, found 464.1.

$^1$H NMR (400 MHz, $CDCl_3$) δ 8.57 (s, 1H), 8.40 (d, J = 2.8 Hz, 1H), 8.15-8.10 (m, 2H), 7.79-7.74 (m, 1H), 7.62-7.58 (m, 1H), 7.27-7.24 (m, 1H), 7.14-7.10 (m, 1H), 5.84 (dd, J = 9.6, 2.8 Hz, 1H), 4.22 (t, J = 6.0 Hz, 2H), 4.10-4.08 (m, 1H), 3.86-3.81 (m, 1H), 2.98 (t, J = 5.6 Hz, 2H), 2.68-2.59 (m, 4H), 2.22-2.15 (m, 2H), 1.86-1.74 (m, 8H).

Step 7

At room temperature under nitrogen, sodium sulfide nonahydrate (4 g, 16.20 mmol, 98.0% purity) and water (20 mL) were added to a solution of compound 6-nitro-3-[(E)-2-[5-(2-pyrrolidin-1-ylethoxy)pyridin-2-yl]ethenyl]-1-tetrahydropyran-2-ylindazole 31h (2.2 g, 4.63 mmol) in methanol (20 mL) and tetrahydrofuran (20 mL). The mixture was heated to 60°C and stirred for 2 hours. After thin-layer chromatography (dichloromethane/methanol = 10/1) indicated complete consumption of the starting material, the mixture was poured into water (80 mL) and filtered. The filtrate was extracted with dichloromethane (80 mL × 3). The combined organic layers were washed with saturated brine (100 mL), dried over anhydrous sodium sulfate, and concentrated to give the crude product. The crude product was purified by column chromatography (DCM/MeOH = 20/1-3:1) to give compound 3-[(E)-2-[5-(2-pyrrolidin-1-ylethoxy)pyridin-2-yl]ethenyl]-1-tetrahydropyran-2-yl-indazol-6-amine 31i as a brown oil (0.9 g, 2.05 mmol, 44.36% yield).

The product was confirmed by LCMS and H-NMR.

MS-ESI calculated for [M+H]+ 434.2, found 434.1.

$^{1}$H NMR (400 MHz, $CDCl_3$) δ 8.36 (d, J = 2.8 Hz, 1H), 7.83 (d, J = 8.4 Hz, 1H), 7.65 (d, J = 16.4 Hz, 1H), 7.52-7.45 (m, 2H), 7.24 (dd, J = 8.4, 2.8 Hz, 1H), 6.76 (d, J = 1.6 Hz, 1H), 6.66 (dd, J = 8.4, 1.6 Hz, 1H), 5.60 (dd, J = 9.6, 2.8 Hz, 1H), 4.20 (t, J = 6.0 Hz, 2H), 4.09-4.07 (m, 1H), 3.78-3.20 (m, 1H), 2.96 (t, J = 5.6 Hz, 2H), 2.66 (s, 4H), 2.20 (s, 1H), 2.09-2.05 (m, 1H), 1.86-1.84 (m, 4H), 1.78-1.63 (m, 4H).

Step 8

At 0-5°C, a solution of sodium nitrite (221.8 mg, 3.21 mmol) in water (6 mL) was added to a solution of compound 3-[(E)-2-[5-(2-pyrrolidin-1-ylethoxy)pyridin-2-yl]ethenyl]-1-tetrahydropyran-2-yl-indazol-6-amine 31i (1.3 g, 2.92 mmol) in acetic acid (12 mL) and acetonitrile (15 mL). The mixture was stirred at 0-5°C for 1 hour. A solution of sodium iodide (884.8 mg, 5.90 mmol) and iodine (370.9 mg, 1.46 mmol) in water (6 mL) was added, and the mixture was stirred at 0°C for 3 hours. After thin-layer chromatography (dichloromethane/methanol = 10/1) indicated complete consumption of the starting material, the mixture was poured into water (50 mL). The pH of the mixture was adjusted to 8-9 with sodium carbonate, and it was extracted with dichloromethane (50 mL × 3). The combined organic layers were dried over anhydrous sodium sulfate and concentrated to give the crude product. The crude product was purified by column chromatography (DCM/MeOH = 20/1) to give compound 6-iodo-3-[(E)-2-[5-(2-pyrrolidin-1-ylmethoxy)pyridin-2-yl]ethenyl]-1-(tetrahydro-2H-pyran-2-yl)-1H-indazole 31j as a red solid (0.85 g, 1.56 mmol, 53.30% yield).

The product was confirmed by LCMS and H-NMR.

MS-ESI calculated for [M+H]+ 545.1, found 546.0.

$^{1}$H NMR (400 MHz, $CDCl_3$) δ 8.35 (d, J = 3.6 Hz, 1H), 8.02 (s, 1H), 7.78 (d, J = 8.4 Hz, 1H), 7.73 (d, J = 16.4 Hz, 1H), 7.55 (d, J = 2.0 Hz, 1H), 7.53-7.51 (m, 1H), 7.46 (d, J = 8.4 Hz, 1H), 7.33-7.31 (m, 1H), 5.69 (d, J = 6.8 Hz, 1H), 4.06 (s, 2H), 3.78 (s, 2H), 2.57 (s, 2H), 2.16 (s, 4H), 1.80 (s, 2H), 1.60 (s, 8H).

Step 9

At room temperature under nitrogen, cesium carbonate (418.9 mg, 1.29 mmol), 3-fluoro-N-methyl-2-mercaptobenzamide 31e (142.9 mg, 771.45 μmol), and 1,1'-bis(diphenylphosphino)ferrocene palladium(II) dichloride (233.3 mg, 321.44 μmol) were added to a solution of compound 6-iodo-3-[(E)-2-[5-(2-pyrrolidin-1-ylmethoxy)pyridin-2-yl]ethenyl]-1-(tetrahydro-2H-pyran-2-yl)-1H-indazole 31j

(350 mg, 642.88 μmol) in N,N-dimethylformamide (5 mL). The mixture was heated to 80°C and stirred for 5 hours. After thin-layer chromatography (dichloromethane/methanol = 10/1) indicated complete consumption of the starting material, the mixture was poured into water (30 mL) and extracted with ethyl acetate (20 mL × 3). The combined organic layers were washed with saturated brine (30 mL), dried over anhydrous sodium sulfate, and concentrated to give the crude product. The crude product was purified by column chromatography on neutral alumina (petroleum ether/ethyl acetate = 1/1) to give compound 3-fluoro-N-methyl-2-({3-[(E)-2-[5-(2-pyrrolidin-1-ylethoxy)pyridin-2-yl]ethenyl]-1-(tetrahydro-2H-pyran-2-yl)-1H-indazol-6-yl}sulfanyl)benzamide 31k as a yellow solid (250 mg, 415.47 μmol, 64.63% yield).

The product was confirmed by LCMS.

MS-ESI calculated for [M+H]+ 602.3, found 602.2.

Step 10

At room temperature, trifluoroacetic acid (473.7 mg, 4.15 mmol) was added to a solution of compound 3-fluoro-N-methyl-2-({3-[(E)-2-[5-(2-pyrrolidin-1-ylethoxy)pyridin-2-yl]ethenyl]-1-(tetrahydro-2H-pyran-2-yl)-1H-indazol-6-yl}sulfanyl)benzamide 31k (250 mg, 415.47 μmol) in dichloromethane (5 mL). Under a nitrogen atmosphere, the mixture was stirred at 30°C for 16 hours. After thin-layer chromatography (dichloromethane/methanol = 10/1) indicated complete consumption of the starting material, the mixture was poured into water (40 mL), and the pH was adjusted to 8-9 with sodium carbonate. The mixture was extracted with ethyl acetate (30 mL × 3). The combined organic layers were washed with saturated brine (40 mL), dried over anhydrous sodium sulfate, and concentrated to give the crude product. The crude product was purified by preparative high-performance liquid chromatography to give compound 3-fluoro-N-methyl-2-({3-[(E)-2-{5-[2-(pyrrolidin-1-yl)ethoxy]pyridin-2-yl}ethenyl]-1H-indazol-6-yl}sulfanyl)benzamide I-33 as a white solid (78 mg, 150.69 μmol, 36.27% yield).

The product was confirmed by LCMS, H-NMR, and C-NMR.

MS-ESI calculated for [M+H]+ 518.2, found 518.1.

$^1$H NMR (400MHz, $CDCl_3$&CD3OD): δ (ppm) 8.26 (d, J = 2.8 Hz, 1H), 8.01 (d, J = 8.4 Hz, 1H), 7.67 (d, J = 16.8 Hz, 1H), 7.61 (d, J = 8.8 Hz, 1H), 7.54 - 7.48 (m, 2H), 7.40 (dd, J = 8.8, 2.8 Hz, 1H), 7.29-7.23 (m, 2H), 7.17 (dd, J = 8.4, 1.2 Hz, 1H), 7.11-7.06 (m, 1H), 4.23 (t, J = 5.6 Hz, 2H), 2.98 (t, J = 5.2 Hz, 2H), 2.87 (s, 3H), 2.71 (s, 4H), 1.86 (t, J = 3.2 Hz, 4H).

$^{13}$C NMR (100 MHz, $CDCl_3$&CD3OD) δ (ppm) 168.80, 161.97, 160.22, 154.36, 148.28, 137.19, 134.26, 134.19, 130.02, 129.21, 124.79, 122.07, 120.25, 117.44, 115.31, 115.11, 114.88, 113.64, 66.98, 54.61, 54.41, 25.96, 223.06.

I-34

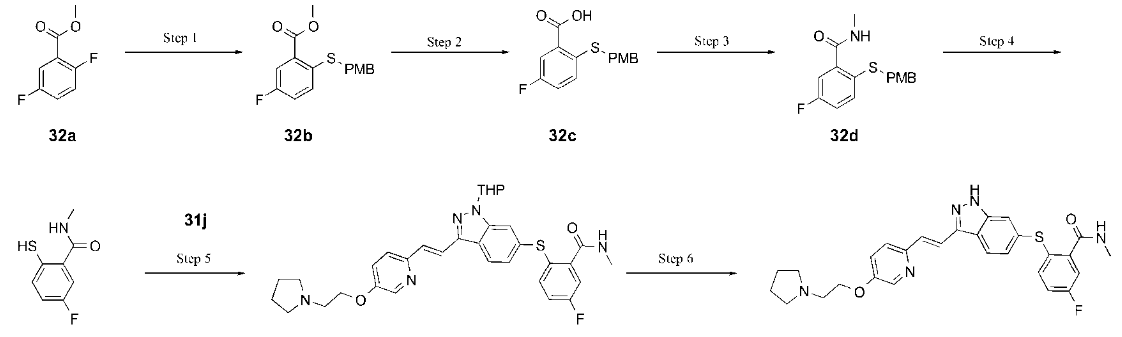


32a 32b 32c 32d

32e 32f I-34

Step 1

A 250 mL single-necked flask was charged with methyl 2,5-difluorobenzoate 32a (5.00 g, 29.1 mmol) dissolved in N,N-dimethylformamide (50 mL). (4-Methoxyphenyl)methanethiol (4.564 g, 29.1 mmol) was added, followed by cesium carbonate (18.94 g, 58.2 mmol). The mixture was stirred at room temperature for 2 hours. The reaction mixture was filtered, and the filtrate was collected. Ethyl acetate (200 mL) was added to the filtrate for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 3:1). Concentration afforded methyl 5-fluoro-2-((4-methoxyphenyl)methylthio) benzoate 32b as a white solid (4.9 g, 16 mmol, 54.9% yield).

MS (ESI) M/Z: 329.1 [M+Na+]

Step 2

A 250 mL single-necked flask was charged with methyl 5-fluoro-2-((4-methoxyphenyl)methylthio)benzoate 32b (4.9 g, 16 mmol) dissolved in tetrahydrofuran (45 mL). Lithium hydroxide (2.017 g, 48 mmol) was added, followed by water (15 mL). The mixture was heated to 50°C and stirred for 5 hours. The reaction mixture was filtered, and the filtrate was collected and concentrated under reduced pressure. Dilute hydrochloric acid (1 mol/L) was added to the system to adjust the pH to 5. Filtration and drying afforded 5-fluoro-2-[(4-methoxyphenyl)methylthio]benzoic acid 32c as a white solid (3.94 g, 13.5 mmol, 84.38% yield).

MS (ESI) M/Z: 315.2 [M+Na+]

Step 3

A 250 mL single-necked flask was charged with 5-fluoro-2-[(4-methoxyphenyl)methylthio]benzoic acid 32c (3.94 g, 13.5 mmol) dissolved in N,N-dimethylformamide (38 mL). Methylamine (532 mg, 20.5 mmol) was added, followed by N-methylmorpholine (4.095 g, 40.5 mmol), 1-hydroxybenzotriazole (2.78 g, 20.5 mmol), and 1-(3-dimethylaminopropyl)-3-ethylcarbodiimide hydrochloride (3.91 g, 20.5 mmol). The mixture was maintained at room temperature and stirred for 3 hours. Ethyl acetate (200 mL) was added to the reaction system for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 4:1). Concentration afforded 5-fluoro-2-[(4-methoxyphenyl)methylthio]-N-methylbenzamide 32d as a white solid (2.96 g, 9.7 mmol, 71.9% yield).

Step 4

A 250 mL three-necked flask was charged with 5-fluoro-2-[(4-methoxyphenyl)methylthio]-N-methylbenzamide 32d (2.96 g, 9.7 mmol) dissolved in trifluoroacetic acid (32 mL). The mixture was heated to 70°C and stirred for 3 hours. The mixture was concentrated under reduced pressure. The solid residue was purified by silica gel column chromatography (eluent: dichloromethane/methanol = 30:1). Concentration afforded 5-fluoro-N-methyl-2-mercaptobenzamide 32e as a white solid (1.1 g, 5.95 mmol, 61.3% yield).

MS (ESI) M/Z: 186.1[M+H+]

$^1$H NMR (400 MHz, MeOD) δ 7.40 (dd, J = 8.8, 5.2 Hz, 1H), 7.23 (dd, J = 8.8, 2.8 Hz, 1H), 7.08 (td, J = 8.4, 2.8 Hz, 1H), 2.89 (s, 3H).

Step 5

Under nitrogen atmosphere at room temperature, to a solution of compound 6-iodo-3-[(E)-2-(5-(2-(pyrrolidin-1-yl)methoxy)pyridin-2-yl)ethenyl]-1-(tetrahydropyran-2-yl)indazole 31j (350 mg, 642.88 μmol) in N,N-dimethylformamide (5 mL) were added cesium carbonate (314.2 mg, 964.32 μmol), 5-fluoro-N-methyl-2-mercaptobenzamide 32e (148.8 mg, 803.60 μmol, synthesized according to the method for compound 31e using methyl 2,5-difluorobenzoate as starting material), and [1,1'-bis(diphenylphosphino)ferrocene]dichloropalladium(II) (350 mg, 482.32 μmol). The mixture was heated to 80°C and stirred for 5 hours. After thin-layer chromatography (dichloromethane/methanol = 10/1) indicated complete consumption of the starting material, the mixture was poured into water (30 mL) and extracted with ethyl acetate (20 mL × 3). The combined organic layers were washed with saturated brine (30 mL), dried over anhydrous sodium sulfate, and concentrated to afford the crude product. The crude product was purified by neutral alumina column chromatography (petroleum ether/ethyl acetate = 1/1) to give compound 5-fluoro-N-methyl-2-[3-[(E)-2-[5-(2-pyrrolidin-1-ylethoxy)-2-pyridyl]ethenyl]-1-tetrahydropyran-2-yl-indazol-6-yl]sulfanylbenzamide 32f as a yellow solid (250 mg, 415.47 μmol, 64.63% yield).

The product was confirmed by LCMS.

MS-ESI calculated for [M+H]+ 602.3, found 602.2.

Step 6

At room temperature, to a solution of compound 5-fluoro-N-methyl-2-[3-[(E)-2-[5-(2-pyrrolidin-1-ylethoxy)-2-pyridyl]ethenyl]-1-tetrahydropyran-2-yl-indazol-6-yl]sulfanylbenzamide 32f (250 mg, 415.47 μmol) in dichloromethane (10 mL) was added trifluoroacetic acid (473.7 mg, 4.15 mmol). Under nitrogen atmosphere, the mixture was stirred at 30°C for 16 hours. After thin-layer chromatography (dichloromethane/methanol = 10/1) indicated complete consumption of the starting material, the mixture was poured into water (40 mL), and the pH was adjusted to 8-9 with sodium carbonate. The mixture was extracted with ethyl acetate (30 mL × 3). The combined organic layers were washed with saturated brine (40 mL), dried over anhydrous sodium sulfate, and concentrated to afford the crude product. The crude product was purified by preparative high-performance liquid chromatography to give compound 5-fluoro-N-methyl-2-({3-[(E)-2-{5-[2-(pyrrolidin-1-yl)ethoxy]pyridin-2-yl}ethenyl]-1H-indazol-6-yl}sulfanyl)benzamide I-34 as a white solid (33 mg, 63.75 μmol, 15.35% yield).

The product was confirmed by LCMS, H-NMR, and C-NMR.

MS-ESI calculated for [M+H]+ 518.2, found 518.0.

$^1$H NMR (400MHz, $CDCl_3$&CD3OD): δ (ppm) 8.28 (d, J = 2.4 Hz, 1H), 8.13 (d, J = 8.4 Hz, 1H), 7.74 - 7.70 (m, 2H), 7.68-7.63 (m, 1H), 7.57 - 7.50 (m, 2H), 7.44 (dd, J = 8.8, 2.8 Hz, 1H), 7.28 (d, J = 9.6 Hz, 1H), 6.97 - 6.93 (m, 1H), 6.70 (dd, J = 10.0, 2.4 Hz, 1H), 4.26 (t, J = 5.2 Hz, 2H), 3.03 (s, 2H), 2.91 (s, 3H), 2.76 (s, 4H), 1.89 (s, 4H).

$^{13}$C NMR (100 MHz, $CDCl_3$&CD3OD) δ (ppm) 169.22, 162.37, 154.39, 148.26, 142.24, 137.19, 131.46, 129.35, 126.55, 122.41, 122.07, 121.84, 120.94, 115.97, 115.73, 112.59, 112.37, 67.00, 54.61, 54.40, 26.00, 23.06.

I-35

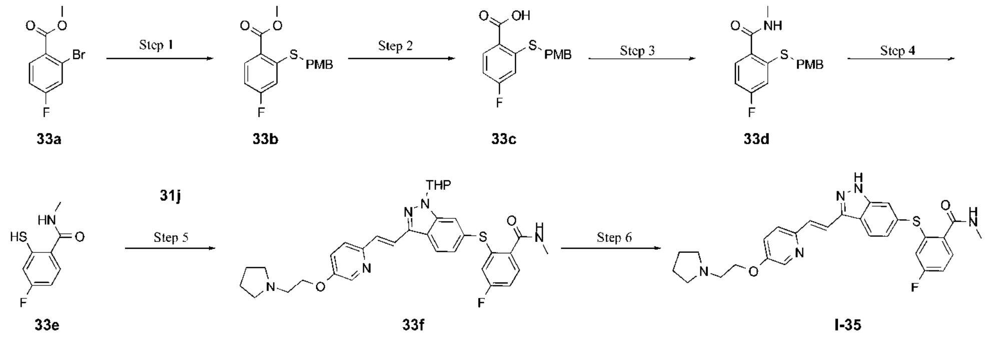


Step 1

A 250 mL single-necked flask was charged with methyl 2-bromo-4-fluorobenzoate 33a (5.00 g, 21.5 mmol) dissolved in N,N-dimethylformamide (50 mL). PMBSH (3.98 g, 25.8 mmol) and cesium carbonate (10.51 g, 32.3 mmol) were added, followed by Pd(dppf)C12 (0.79 g, 1.08 mmol). The mixture was stirred at 120°C overnight. Work-up: The reaction mixture was filtered, and the filtrate was collected. Ethyl acetate (200 mL) was added to the filtrate for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 3:1). Concentration afforded methyl 4-fluoro-2-[(4-methoxyphenyl)methylthio]benzoate 33b as a white solid (5 g).
MS (ESI) M/Z: 329.1 [M+Na+]

Step 2

A 250 mL single-necked flask was charged with methyl 4-fluoro-2-[(4-methoxyphenyl)methylthio]benzoate 33b (4.50 g, 14.7 mmol) dissolved in tetrahydrofuran (45 mL). Lithium hydroxide (1.853 g, 44.1 mmol) was added, followed by water (15 mL). The mixture was heated to 50°C and stirred for 5 hours. The reaction mixture was filtered, and the filtrate was collected and concentrated under reduced pressure. Dilute hydrochloric acid (1 mol/L) was added to the system to adjust the pH to 5. Filtration and drying afforded 4-fluoro-2-[(4-methoxyphenyl)methylthio]benzoic acid 33c as a white solid (3.80 g, 13.0 mmol, 88.4% yield).
MS (ESI) M/Z: 315.2 [M+Na+]

Step 3

A 250 mL single-necked flask was charged with 4-fluoro-2-[(4-methoxyphenyl)methylthio]benzoic acid 33c (3.80 g, 13.0 mmol) dissolved in N,N-dimethylformamide (38 mL). Methylamine (506 mg, 19.5 mmol) was added, followed by N-methylmorpholine (3.943 g, 39.0 mmol), 1-hydroxybenzotriazole (2.64 g, 19.5 mmol), and 1-(3-dimethylaminopropyl)-3-ethylcarbodiimide hydrochloride (3.72 g, 19.5 mmol). The mixture was maintained at room temperature and stirred for 3 hours. Ethyl acetate (200 mL) was added to the reaction system for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 4:1). Concentration afforded 4-fluoro-2-[(4-methoxyphenyl)methylthio]-N-methylbenzamide 33d as a white solid (2.95 g, 9.66 mmol, 74.3% yield).

Step 4

A 250 mL three-necked flask was charged with 4-fluoro-2-[(4-methoxyphenyl)methylthio]-N-methylbenzamide 33d (2.95 g, 9.66 mmol) dissolved in trifluoroacetic acid (32 mL). The mixture was heated to 70°C and stirred for 3 hours. The mixture was concentrated under reduced pressure. The solid residue was purified by silica gel column chromatography (eluent: dichloromethane/methanol = 30:1). Concentration afforded 4-fluoro-N-methyl-2-mercaptobenzamide 33e as a white solid (1.05 g, 5.68 mmol, 58.8% yield).
MS (ESI) M/Z: 186.1 [M+H+]
$^1$H NMR (400 MHz, CD3OD) δ 7.50 (dd, J = 8.6, 5.8 Hz, 1H), 7.18 (dd, J = 9.6, 2.5 Hz, 1H), 7.01 - 6.76 (m, 1H), 2.89

(s, 3H).

Step 5

Under nitrogen atmosphere at room temperature, to a solution of compound 6-iodo-3-[(E)-2-(5-(2-(pyrrolidin-1-yl)methoxy)pyridin-2-yl)ethenyl]-1-(tetrahydropyran-2-yl)indazole 31j (300 mg, 551.04 μmol) in N,N-dimethylformamide (5 mL) were added cesium carbonate (359.1 mg, 1.10 mmol), 4-fluoro-N-methyl-2-mercaptobenzamide 33e (122.5 mg, 661.25 μmol), and [1,1'-bis(diphenylphosphino)ferrocene]dichloropalladium(II) (199.9 mg, 275.52 μmol). The mixture was heated to 80°C and stirred for 5 hours. After thin-layer chromatography (dichloromethane/methanol = 10/1) indicated complete consumption of the starting material, the mixture was poured into water (30 mL) and extracted with ethyl acetate (20 mL × 3). The combined organic layers were washed with saturated brine (30 mL), dried over anhydrous sodium sulfate, and concentrated to afford the crude product. The crude product was purified by neutral alumina column chromatography (petroleum ether/ethyl acetate = 1/1) to give compound 4-fluoro-N-methyl-2-[3-[(E)-2-[5-(2-pyrrolidin-1-ylethoxy)-2-pyridyl]ethenyl]- 1 -tetrahydropyran-2-yl-indazol-6-yl]sulfanylbenzamide 33f as a yellow solid (250 mg, 415.47 μmol, 75.40% yield).

The product was confirmed by LCMS.

MS-ESI calculated for [M+H]+ 602.3, found 602.1.

Step 6

At room temperature, to a solution of compound 4-fluoro-N-methyl-2-[3-[(E)-2-[5-(2-pyrrolidin-1-ylethoxy)-2-pyridyl]ethenyl]-1-tetrahydropyran-2-yl-indazol-6-yl]sulfanylbenzamide 33f (250 mg, 415.47 μmol) in dichloromethane (5 mL) was added trifluoroacetic acid (473.6 mg, 4.15 mmol). Under nitrogen atmosphere, the mixture was stirred at 30°C for 16 hours. After thin-layer chromatography (dichloromethane/methanol = 10/1) indicated complete consumption of the starting material, the mixture was poured into water (40 mL), and the pH was adjusted to 8-9 with sodium carbonate. The mixture was extracted with ethyl acetate (30 mL × 3). The combined organic layers were washed with saturated brine (40 mL), dried over anhydrous sodium sulfate, and concentrated to afford the crude product. The crude product was purified by preparative high-performance liquid chromatography to give compound 4-fluoro-N-methyl-2-({3-[(E)-2-{5-[2-(pyrrolidin-1-yl)ethoxy]pyridin-2-yl}ethenyl]-1H-indazol-6-yl}sulfanyl)benzamide I-35 as a yellow solid (20 mg, 38.64 μmol, 9.30% yield).

The product was confirmed by LCMS, H-NMR, and C-NMR.

MS-ESI calculated for [M+H]+ 518.2, found 518.1.

$^1$H NMR (400MHz, $CDCl_3$&CD3OD): δ (ppm) δ 8.29 (d, J = 2.8 Hz, 1H), 8.13 (d, J = 8.4 Hz, 1H), 7.75 - 7.70 (m, 2H), 7.68-7.63 (m, 1H), 7.57-7.54 (m, 1H), 7.53 - 7.50 (m, 1H), 7.44 (dd, J = 8.8, 2.8 Hz, 1H), 7.28 (d, J = 10.0 Hz, 1H), 6.98-6.93 (m, 1H), 6.71 (dd, J = 9.6, 2.4 Hz, 1H), 4.27 (t, J = 5.6 Hz, 2H), 3.05 (t, J = 5.2 Hz, 2H), 2.91 (s, 3H), 2.78 (s, 4H), 1.89 (s, 4H).

$^{13}$C NMR (100 MHz, $CDCl_3$&CD3OD) δ (ppm) 170.59, 166.30, 163.79, 155.74, 149.73, 144.24, 143.69, 14.18, 142.10, 138.66, 136.12, 132.82, 132.34, 131.18, 131.09, 128.00, 125.86, 123.53, 123.14, 122.40, 117.94, 117.45, 117.21, 114.07, 113.85, 68.36, 56.02, 55.87, 30.93, 27.58, 24.52.

I-36

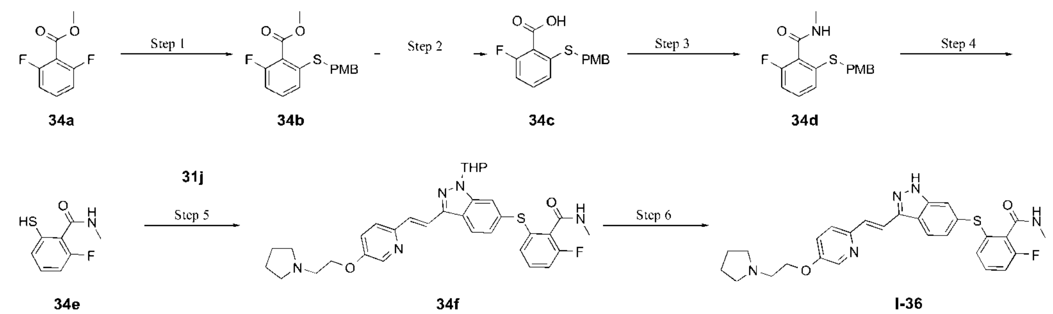


34a 34b 34c 34d

34e 34f I-36

Step 1

A 250 mL single-necked flask was charged with methyl 2,6-difluorobenzoate 34a (5.00 g, 29.1 mmol) dissolved in

N,N-dimethylformamide (50 mL). (4-Methoxyphenyl)methanethiol (4.564 g, 29.1 mmol) was added, followed by cesium carbonate (18.94 g, 58.2 mmol). The mixture was stirred at room temperature for 2 hours. The reaction mixture was filtered, and the filtrate was collected. Ethyl acetate (200 mL) was added to the filtrate for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 3:1). Concentration afforded methyl 2-fluoro-6-[(4-methoxyphenyl)methylthio] benzoate 34b as a white solid (4.5 g, 14.7 mmol, 54.9% yield).

MS (EST) M/Z: 329.1 [M+Na+]

Step 2

A 250 mL single-necked flask was charged with methyl 2-fluoro-6-[(4-methoxyphenyl)methylthio]benzoate 34b (4.50 g, 14.7 mmol) dissolved in tetrahydrofuran (45 mL). Lithium hydroxide (1.853 g, 44.1 mmol) was added, followed by water (15 mL). The mixture was heated to 50°C and stirred for 5 hours. The reaction mixture was filtered, and the filtrate was collected and concentrated under reduced pressure. Dilute hydrochloric acid (1 mol/L) was added to the system to adjust the pH to 5. After filtration and drying, 2-fluoro-6-[(4-methoxyphenyl)methylthio]benzoic acid 34c was obtained as a white solid (3.80 g, 13.0 mmol, 88.4% yield).

MS (ESI) M/Z: 315.2 [M+Na+]

Step 3

A 250 mL single-necked flask was charged with 2-fluoro-6-[(4-methoxyphenyl)methylthio]benzoic acid 34c (3.80 g, 13.0 mmol) dissolved in N,N-dimethylformamide (38 mL). Methylamine (506 mg, 19.5 mmol) was added, followed by N-methylmorpholine (3.943 g, 39.0 mmol), 1-hydroxybenzotriazole (2.64 g, 19.5 mmol), and 1-(3-dimethylaminopropyl)-3-ethylcarbodiimide hydrochloride (3.72 g, 19.5 mmol). The mixture was maintained at room temperature and stirred for 3 hours. Ethyl acetate (200 mL) was added to the reaction system for dilution, and the mixture was washed with water (200 mL × 3). The layers were separated, and the organic phase was collected and dried over anhydrous sodium sulfate. After concentration under reduced pressure, the solid residue was purified by silica gel column chromatography (eluent: petroleum ether:ethyl acetate = 4:1). Concentration afforded 2-fluoro-6-[(4-methoxyphenyl)methylthio]-N-methylbenzamide 34d as a white solid (2.95 g, 9.66 mmol, 74.3% yield).

Step 4

A 250 mL three-necked flask was charged with 2-fluoro-6-[(4-methoxyphenyl)methylthio]-N-methylbenzamide 34d (2.95 g, 9.66 mmol) dissolved in trifluoroacetic acid (32 mL). The mixture was heated to 70°C and stirred for 3 hours. The mixture was concentrated under reduced pressure. The solid residue was purified by silica gel column chromatography (eluent: dichloromethane/methanol = 30:1). Concentration afforded 2-fluoro-N-methyl-6-mercaptobenzamide 34e as a white solid (1.05 g, 5.68 mmol, 58.8% yield).

MS (ESI) M/Z: 186.0[M+H+]

$^1$H NMR (400 MHz, CD3OD) δ 7.34 - 7.32 (m, 1H), 7.24 - 7.20 (m, 2H), 2.92 (s, 3H).

Step 5

Under nitrogen atmosphere at room temperature, to a solution of compound 6-iodo-3-[(E)-2-(5-(2-(pyrrolidin-1-yl)methoxy)pyridin-2-yl)ethenyl]-1-(tetrahydropyran-2-yl)indazole 31j (350 mg, 642.88 μmol) in N,N-dimethylformamide (8 mL) were added cesium carbonate (314.2 mg, 964.32 μmol), 2-fluoro-N-methyl-6-mercaptobenzamide 34e (148.8 mg, 803.60 μmol, synthesized according to the method for compound 31e using methyl 2,6-difluorobenzoate as starting material), and [1,1'-bis(diphenylphosphino)ferrocene]dichloropalladium(II) (350 mg, 482.32 μmol). The mixture was heated to 80°C and stirred for 5 hours. After thin-layer chromatography (dichloromethane/methanol = 10/1) indicated complete consumption of the starting material, the mixture was poured into water (25 mL) and extracted with dichloromethane (25 mL × 3). The combined organic layers were washed with water (20 mL × 2), dried over anhydrous sodium sulfate, and concentrated to afford the crude product. The crude product was purified by preparative thin-layer chromatography (dichloromethane/methanol = 20/1) to give compound 2-fluoro-N-methyl-6-[3-[(E)-2-[5-(2-pyrrolidin-1-ylethoxy)-2-pyridyl]ethenyl]-1-tetrahydropyran-2-yl-indazol-6-yl]sulfanylbenzamide 34f as a red solid (197.9 mg, 328.88 μmol, 51.16% yield).

The product was confirmed by LCMS.

MS-ESI calculated for [M+H]+ 602.3, found 602.2.

Step 6

At room temperature, to a solution of compound 2-fluoro-N-methyl-6-[3-[(E)-2-[5-(2-pyrrolidin-1-ylethoxy)-2-pyridyl]ethenyl]-1-tetrahydropyran-2-yl-indazol-6-yl]sulfanylbenzamide 34f (197.9 mg, 328.88 μmol) in dichloromethane (5 mL) was added trifluoroacetic acid (1.2 g, 10.42 mmol). Under nitrogen atmosphere, the mixture was stirred at room temperature for 16 hours. After thin-layer chromatography (dichloromethane/methanol = 10/1) indicated complete consumption of the starting material, the mixture was poured into water (5 mL), and the pH was adjusted to 8-9 with sodium carbonate. The mixture was extracted with dichloromethane (5 mL × 3). The combined organic layers were washed with water (5 mL × 2), dried over anhydrous sodium sulfate, and concentrated to afford the crude product. The crude product was purified by preparative high-performance liquid chromatography to give compound 2-fluoro-N-methyl-6-({3-[(E)-2-{5-[2-(pyrrolidin-1-yl)ethoxy]pyridin-2-yl}ethenyl]-1H-indazol-6-yl}sulfanyl)benzamide I-36 as a dark yellow solid (18 mg, 34.77 μmol, 10.57% yield).

The product was confirmed by LCMS, H-NMR, and C-NMR.

MS-ESI calculated for [M+H]+ 518.2, found 518.1.

$^1$H NMR (400 MHz, CD3OD) δ 8.29 (d, J = 2.8 Hz, 1H), 8.09 (d, J = 8.4 Hz, 1H), 7.74 - 7.64 (m, 3H), 7.56-7.45 (m, 2H), 7.37-7.31 (m, 1H), 7.26 (d, J = 8.4 Hz, 1H), 7.12-7.05 (m, 2H), 4.27 (t, J = 5.2 Hz, 2H), 3.03 (t, J = 6.0 Hz, 2H), 2.89 (s, 3H), 2.77 (s, 4H), 1.89 (s, 4H).

$^{13}$C NMR (100 MHz, CD3OD&CDCl$_3$) δ: 165.56, 160.40, 154.46, 148.23, 142.74, 142.13, 137.16, 132.81,130.84, 130.75, 129.19, 126.78, 125.41, 122.41, 122.02, 121.64, 121.39, 120.47, 114.55, 113.84, 113.62, 66.96, 54.54, 54.28, 25.55, 22.96.

I-29

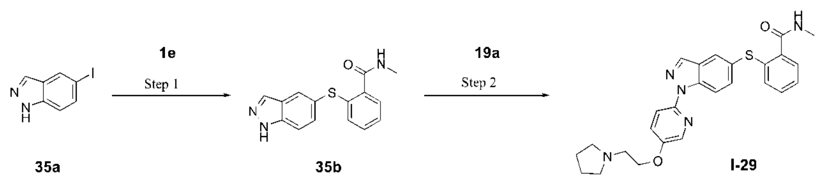


35a 35b I-29

Step 1

Under nitrogen atmosphere at room temperature, to a solution of 5-iodo-1H-indazole 35a (1.02 g, 4.19 mmol) and N-methyl-2-mercaptobenzamide 1e (1 g, 5.98 mmol) in N,N-dimethylformamide (10 mL) were added cesium carbonate (3.90 g, 11.96 mmol) and [1,1'-bis(diphenylphosphino)ferrocene]dichloropalladium(II) (0.88 g, 1.20 mmol). The mixture was heated to 80°C and stirred for 2 hours. After thin-layer chromatography (dichloromethane/methanol = 20/1) indicated complete consumption of the starting material, the mixture was poured into water (30 mL) and extracted with ethyl acetate (10 mL × 3). The combined organic layers were washed with saturated brine (20 mL), dried over anhydrous sodium sulfate, and concentrated to afford the crude product. The crude product was purified by column chromatography (petroleum ether/ethyl acetate = 2/1-0/1) to give compound 2-(1H-indazol-5-ylsulfanyl)-N-methylbenzamide 35b as a yellow oil (0.8 g, 47.2% yield).

The product was confirmed by LCMS.

MS-ESI calculated for [M+H]+ 284.1, found 284.1.

Step 2

Under nitrogen atmosphere at room temperature, to a solution of compound 2-(1H-indazol-5-ylsulfanyl)-N-methylbenzamide 35b (0.5 g, 1.76 mmol) and 2-bromo-5-(2-pyrrolidin-1-ylethoxy)pyridine 19a (478 mg, 1.76 mmol) in dimethyl sulfoxide (20 mL) were added cesium carbonate (1.15 g, 3.53 mmol), 1,10-phenanthroline (95 mg, 0.53 mmol), and copper(I) iodide (101 mg, 0.53 mmol). The mixture was heated to 100°C and stirred for 16 hours. Thin-layer chromatography (dichloromethane/methanol = 20/1) and LCMS indicated the presence of product. The mixture was poured into water (80 mL) and extracted with ethyl acetate (20 mL × 3). The combined organic layers were washed with saturated brine (30 mL), dried over anhydrous sodium sulfate, and concentrated to afford the crude product. The crude product was purified by preparative high-performance liquid chromatography to give compound N-methyl-2-[(1-{5-[2-(pyrrolidin-1-yl)ethoxy]pyridin-2-yl}-1H-indazol-5-yl)sulfanyl]benzamide I-29 as a white solid (18 mg, 2.2% yield).

The product was confirmed by LCMS, H-NMR, and C-NMR.

MS-ESI calculated for [M+H]+ 474.2, found 474.2.

$^1H$ NMR (400 MHz, CD3OD) δ: 8.73 (d, 1H, J=8.8Hz), 8.23 (d, 1H, J=2.8Hz), 8.16 (d, 1H, J= 0.4Hz), 7.93-7.98 (m, 2H), 7.58-7.60 (m, 1H), 7.55 (dd, 1H, J=8.8, 1.6Hz), 7.46 (dd, 1H, J =8.8, 2.8Hz), 7.21-7.23 (m, 2H), 7.01-7.04 (m, 1H), 6.30 (s, 1H), 4.23 (t, 2H, J=6.0Hz), 3.04 (d, 3H, J=4.8Hz), 2.98 (t, 2H, J=6.0Hz), 2.66-2.68 (m, 4H), 1.84-1.88 (m, 4H).

$^{13}C$ NMR (100 MHz, $CDCl_3$) δ: 168.68, 152.93, 147.72, 138.17, 137.07, 135.65, 135.04, 134.32, 133.06, 130.66, 129.81, 128.41, 126.67, 126.23, 125.95, 125.08, 115.85, 114.51, 68.05, 55.04, 54.77, 26.80, 23.52.

I-21

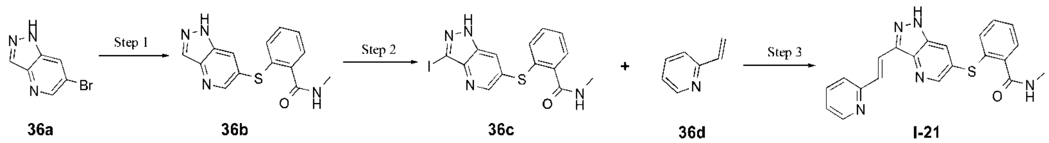


36a 36b 36c 36d I-21

Step 1

Under nitrogen atmosphere at room temperature, to a solution of 6-bromo-1H-pyrazolo[4,3-b]pyridine 36a (2 g, 10.10 mmol) in N,N-dimethylformamide (20 mL) were added compound N-methyl-2-mercaptobenzamide 1e (2.03 g, 12.12 mmol), cesium carbonate (6.58 g, 20.20 mmol), and [1,1'-bis(diphenylphosphino)ferrocene]dichloropalladium(II) (1.48 g, 2.02 mmol). The mixture was heated to 100°C and stirred for 1 hour. After thin-layer chromatography (dichloromethane/methanol = 20/1) indicated complete consumption of the starting material, the mixture was poured into water (40 mL) and extracted with ethyl acetate (20 mL × 3). The combined organic layers were washed with saturated brine (20 mL), dried over anhydrous sodium sulfate, and concentrated to afford the crude product. The crude product was purified by column chromatography (dichloromethane/methanol = 10/1) to give compound N-methyl-2-(1H-pyrazolo[4,3-b]pyridin-6-ylsulfanyl)benzamide 36b as a brown solid (1.59 g, 5.59 mmol, 55.37% yield).

The product was confirmed by LCMS and H-NMR.

MS-ESI calculated for [M+H]+ 285.1, found 285.0.

$^1H$ NMR (400MHz, DMSO-d6): δ (ppm) 13.43 (s, 1H), 8.44 (d, J = 2.0 Hz, 2H), 8.33 (s, 1H), 8.08 (s, 1H), 7.53-7.50 (m, 1H), 7.37-7.26 (m, 2H), 7.04-6.96 (m, 1H), 2.77 (d, J = 4.6 Hz, 3H).

Step 2

At room temperature, to a solution of compound N-methyl-2-(1H-pyrazolo[4,3-b]pyridin-6-ylsulfanyl)benzamide 36b (1.6 g, 5.63 mmol) in N,N-dimethylformamide (5 mL) were added iodine (2.43 g, 9.57 mmol) and potassium carbonate (1.56 g, 11.25 mmol). The mixture was stirred at 25°C for 3.0 hours. After thin-layer chromatography (dichloromethane/methanol = 20/1) indicated complete consumption of the starting material, the mixture was poured into water (10 mL) and extracted with ethyl acetate (10 mL × 3). The combined organic layers were washed with saturated brine (20 mL), dried over anhydrous sodium sulfate, and concentrated to afford the crude product of 2-[(3-iodo-1H-pyrazolo[4,3-b]pyridin-6-yl)sulfanyl]-N-methylbenzamide 36c as a brown solid (2.1 g, 5.12 mmol, 90.97% yield).

The product was confirmed by LCMS and H-NMR.

MS-ESI calculated for [M+H]+ 411.0, found 410.9.

$^1H$ NMR (400MHz, DMSO-d6): δ (ppm) 13.84 (s, 1H), 8.45 (d, J = 1.6 Hz, 2H), 8.06 (d, J = 1.6 Hz, 1H), 7.54-7.49 (m, 1H), 7.42-7.24 (m, 2H), 7.08-6.98 (m, 1H), 2.76 (d, J = 4.6 Hz, 3H).

Step 3

Under nitrogen atmosphere at room temperature, to a solution of 2-[(3-iodo-1H-pyrazolo[4,3-b]pyridin-6-yl)sulfanyl]-N-methylbenzamide 36c (100 mg, 243.76 μmol) in N,N-dimethylformamide (5 mL) were added 2-ethenylpyridine 36d (38.44 mg, 365.65 μmol), N,N-diisopropylethylamine (63.03 mg, 487.53 μmol), palladium(II) acetate (21.89 mg, 97.51 μmol), and 1,1'-bis(diphenylphosphino)ferrocene (16.56 mg, 195.01 μmol). The reaction mixture was heated to 100°C and stirred for 16 hours. After thin-layer chromatography (dichloromethane/methanol = 10/1) indicated complete consumption of the starting material, the mixture was poured into water (20 mL) and extracted with ethyl acetate (10 mL × 3). The combined organic layers were washed with saturated brine (50 mL), dried over anhydrous sodium sulfate, and concentrated to afford the crude product. The crude product was purified by preparative high-performance liquid chromatography to give compound N-methyl-2-({3-[(E)-2-(pyridin-2-yl)ethenyl]-1H-pyrazolo[4,3-b]pyridin-6-yl}sulfanyl)benzamide I-21 as a white solid (15.8 mg, 40.78 μmol, 16.73% yield).

The product was confirmed by LCMS, H-NMR, and C-NMR.

MS-ESI calculated for [M+H]+ 388.1, found 388.1.

$^1$H NMR (400MHz, DMSO-d6): δ (ppm) 13.55 (s, 1H), 8.62 (d, J = 4.4 Hz, 1H), 8.55 (s, 1H), 8.46 (d, J = 4.4 Hz, 1H), 8.20-8.07 (m, 2H), 7.93 (d, J = 16.4 Hz, 1H), 7.81 (t, J = 7.6 Hz, 1H), 7.65 (d, J = 7.6 Hz, 1H), 7.53 (d, J = 8.4 Hz, 1H), 7.39-7.26 (m, 3H), 7.07 (d, J = 7.6 Hz, 1H), 2.78 (d, J = 4.4 Hz, 3H).

$^{13}$C NMR (100MHz, DMSO-d6): δ (ppm) 168.26, 155.29, 150.13, 149.16, 142.32, 138.48, 137.50, 137.42, 135.41, 134.52, 132.05, 131.06, 130.67, 129.41, 128.39, 127.05, 123.41, 123.27, 123.12, 122.84, 26.58.

I-26

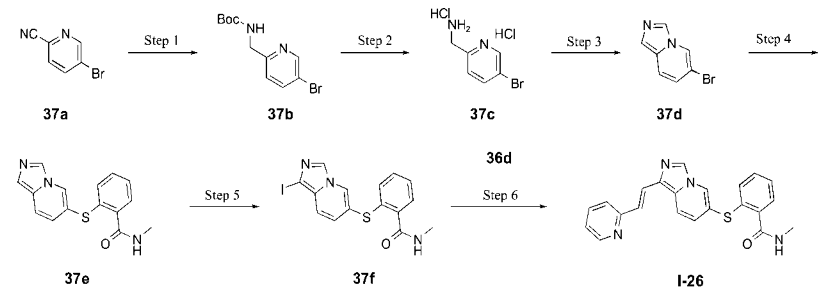


37a 37b 37c 37d 36d 37e 37f I-26

Step 1

At 0°C, to a solution of 5-bromopyridine-2-carbonitrile 37a (1 g, 5.46 mmol) in methanol (10 mL) were added di-tert-butyl dicarbonate (2.39 g, 10.93 mmol) and nickel(II) chloride (129.88 mg, 546.43 μmol). Sodium borohydride (1.45 g, 38.25 mmol) was added in portions slowly over 2.0 hours. The mixture was stirred at 25°C for 16 hours. After thin-layer chromatography (petroleum ether/ethyl acetate = 2/1) indicated complete consumption of the starting material, the mixture was poured into water (50 mL) and extracted with ethyl acetate (10 mL × 3). The combined organic layers were washed with saturated brine (20 mL), dried over anhydrous sodium sulfate, and concentrated to afford the crude product. The crude product was purified by column chromatography (petroleum ether/ethyl acetate = 10/1-5/1) to give compound tert-butyl N-[(5-bromo-2-pyridyl)methyl]carbamate 37b as a white solid (700 mg, 2.44 mmol, 44.61% yield).

The product was confirmed by LCMS and H-NMR.

MS-ESI calculated for [M+H]+ 287.0, found 286.9.

$^1$H NMR (400 MHz, $CDCl_3$) δ 8.60 (d, J = 2.0 Hz, 1H), 7.79 (dd, J = 8.4, 2.4Hz, 1H), 7.21 (d, J = 8.4 Hz, 1H), 5.51 (s, 1H), 4.40 (d, J = 5.4 Hz, 2H), 1.47 (s, 9H).

Step 2

Compound tert-butyl N-[(5-bromo-2-pyridyl)methyl]carbamate 37b (500 mg, 1.74 mmol) was dissolved in a hydrochloric acid/methanol solution (15 mL) and stirred at 25°C for 2.0 hours. After LCMS indicated complete consumption of the starting material, the reaction mixture was concentrated under reduced pressure to give compound (5-bromo-2-pyridyl)methanamine dihydrochloride 37c as a white solid (290 mg, 1.55 mmol, 89.05% yield).

The product was confirmed by LCMS and H-NMR.

MS-ESI calculated for [M+H]+ 188.9, found 189.0.

$^1$H NMR (400MHz, DMSO-d6): δ 8.74 (d, J = 2.0 Hz, 1H), 8.66 (s, 3H), 8.14 (dd, J = 8.4, 2.4 Hz, 1H), 7.55 (d, J = 8.4 Hz, 1H), 4.13-4.17 (m, 2H).

Step 3

Compound (5-bromo-2-pyridyl)methanamine dihydrochloride 37c (400 mg, 1.79 mmol) was dissolved in acetic anhydride (2 mL) and formic acid (1.6 mL), and stirred at 100°C for 16 hours. After thin-layer chromatography (petroleum ether/ethyl acetate = 2/1) indicated complete consumption of the starting material, the reaction mixture was concentrated under reduced pressure. Saturated aqueous sodium bicarbonate solution (10 mL) was added, and the mixture was extracted with ethyl acetate (10 mL × 3). The combined organic layers were washed with saturated brine (20 mL), dried over anhydrous sodium sulfate, and concentrated to afford the crude product. The crude product was purified by column chromatography (petroleum ether/ethyl acetate = 10/1-5/1) to give compound 6-bromoimidazo[1,5-a]pyridine 37d as a yellow solid (300 mg, 1.52 mmol, 85.07% yield).

The product was confirmed by LCMS and H-NMR.

MS-ESI calculated for [M+H]+ 197.0, found 197.0.

$^1$H NMR (400 MHz, DMSO-d6) δ 9.40 (s, 1H), 8.96 (s, 1H), 8.05 (s, 1H), 7.80 (d, J = 9.6 Hz, 1H), 7.26 (dd, J =10.4Hz ,9.6 Hz, 1H).

Step 4

At room temperature, to a solution of compound 6-bromoimidazo[1,5-a]pyridine 37d (0.3 g, 1.52 mmol) in N,N-dimethylformamide (10 mL) were added compound N-methyl-2-mercaptobenzamide 1e (254.62 mg, 1.52 mmol), cesium carbonate (992.18 mg, 3.05 mmol), and [1,1'-bis(diphenylphosphino)ferrocene]dichloropalladium(II) (222.82 mg, 304.52 μmol). The mixture was heated to 80°C and stirred for 2 hours. After thin-layer chromatography (dichloromethane/-methanol = 15/1) indicated complete consumption of the starting material, the mixture was poured into water (40 mL) and extracted with ethyl acetate (20 mL × 3). The combined organic layers were washed with saturated brine (20 mL), dried over anhydrous sodium sulfate, and concentrated to afford the crude product. The crude product was purified by preparative high-performance liquid chromatography to give compound 2-(imidazo[1,5-a]pyridin-6-ylthio)-*N*-methylbenzamide 37e as a yellow solid (87 mg, 307.04 μmol, 20.17% yield).

The product was confirmed by LCMS and H-NMR.

MS-ESI calculated for [M+H]+ 284.1, found 284.1.

$^1$H NMR (400 MHz, $CDCl_3$) δ 8.20 (d, J = 0.8 Hz, 1H), 8.13 (s, 1H), 7.54 (dd, J = 7.6, 1.6 Hz, 1H), 7.42 - 7.45 (m, 2H), 7.29-7.32 (m, 1H), 7.22-7.26 (m, 1H), 7.14 (dd, J = 7.6, 1.2 Hz, 1H), 6.66 (dd, J = 9.2, 1.2 Hz, 1H), 6.21 (s, 1H), 3.04 (d, J = 5.2 Hz, 3H).

Step 5

At room temperature, to a solution of compound 2-(imidazo[1,5-a]pyridin-6-ylthio)-N-methylbenzamide 37e (110 mg, 388.22 μmol) in N,N-dimethylformamide (5 mL) were added iodine (167.50 mg, 659.97 μmol) and potassium carbonate (95.60 mg, 776.43 μmol). The mixture was stirred at 25°C for 3.0 hours. After thin-layer chromatography (dichloromethane/methanol = 20/1) indicated complete consumption of the starting material, the mixture was poured into water (10 mL) and extracted with ethyl acetate (10 mL × 3). The combined organic layers were washed with saturated brine (20 mL), dried over anhydrous sodium sulfate, and concentrated to afford the crude product. The crude product was purified by preparative thin-layer chromatography to give compound 2-[(1-iodoimidazo[1,5-a]pyridin-6-yl)thio]-*N*-methylbenzamide 37f as a yellow solid (89 mg, 217.47 μmol, 56.02% yield).

The product was confirmed by LCMS and H-NMR.

MS-ESI calculated for [M+H]+ 410.0, found 410.0.

$^1$H NMR (400 MHz, $CDCl_3$) δ 8.15 (t, J = 10.8 Hz 2H), 7.53-7.55 (m, 1H), 7.27 - 7.33 (m, 3H), 7.13-7.15 (m, 1H), 6.72-6.75 (m, 1H), 6.10 (s, 1H), 3.05 (d, J = 5.2 Hz, 3H).

Step 6

At room temperature under a nitrogen atmosphere, to a solution of compound 2-[(1-iodoimidazo[1,5-a]pyridin-6-yl)thio]-*N*-methylbenzamide 37f (200 mg, 488.71 μmol) in N,N-dimethylformamide (10 mL) were added 2-ethenylpyridine 36d (77.07 mg, 733.06 μmol), N,N-diisopropylethylamine (126.32 mg, 977.41 μmol), palladium(II) acetate (43.89 mg, 195.48 μmol), and 1,1'-bis(diphenylphosphino)ferrocene (33.22 mg, 390.96 μmol). The reaction mixture was heated to 100°C and stirred for 16 hours. After thin-layer chromatography (dichloromethane/methanol = 20/1) indicated complete consumption of the starting material, the mixture was poured into water (10 mL) and extracted with ethyl acetate (10 mL × 3). The combined organic layers were washed with water (10 mL × 2) and saturated brine (20 mL), dried over anhydrous sodium sulfate, and concentrated to afford the crude product. The crude product was purified by preparative high-performance liquid chromatography. The purified product was triturated with methanol (2 mL) and filtered. The filter cake was dried to give compound N-methyl-2-({1-[(E)-2-(pyridin-2-yl)ethenyl]imidazo[1,5-a]pyridin-6-yl}thio)benzamide I-26 as a yellow solid (6.3 mg, 16.30 μmol, 3.34% yield).

The product was confirmed by LCMS, H-NMR, and C-NMR.

MS-ESI calculated for [M+H]+ 387.1, found 387.1.

$^1$H NMR (400 MHz, CD3OD+CD3Cl) δ 8.47 (d, J = 3.6 Hz, 1H), 8.20 (d, J = 16.0 Hz, 2H), 7.77 (d, J = 15.6 Hz, 1H) 7.64-7.70 (m, 2H), 7.47 (d, J = 7.2 Hz, 1H), 7.34-7.40 (m, 2H), 7.20-7.30 (m, 2H), 7.15 (d, J =7.2 Hz, 1H), 6.74 (d, J = 9.6 Hz, 1H), 2.92 (s, 3H).

$^{13}$C NMR (100 MHz, CD3OD+CD3Cl) δ 169.50, 155.69, 148.95, 137.23, 135.69, 135.15, 130.76, 129.82, 128.99, 127.91, 127.83, 126.63, 126.43, 125.64, 124.85, 122.80, 122.20, 121.82, 120.66, 118.12, 26.36.

I-28

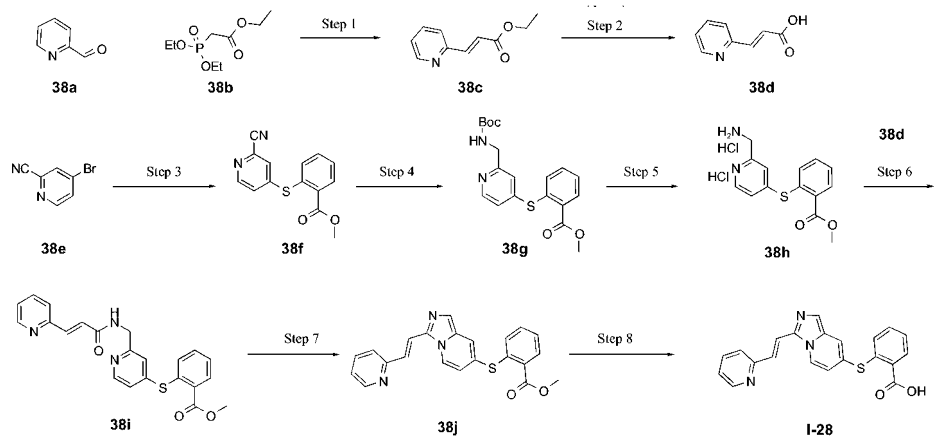


Step 1

At 0°C under a nitrogen atmosphere, to a solution of ethyl 2-(diethoxyphosphoryl)acetate 38b (460.5 mg, 2.05 mmol) in tetrahydrofuran (10 mL) was added sodium hydride (89.6 mg, 3.73 mmol). The mixture was stirred at 0°C for 1.0 hour, and then pyridine-2-carbaldehyde 38a (0.2 g, 1.87 mmol) was added slowly. The reaction mixture was stirred at 25°C for 2 hours. After thin-layer chromatography (petroleum ether/ethyl acetate = 3/1) indicated complete consumption of the starting material, the mixture was poured into water (10 mL) and extracted with ethyl acetate (10 mL × 3). The combined organic layers were washed with saturated brine (20 mL), dried over anhydrous sodium sulfate, and concentrated to afford the crude product ethyl (E)-3-(pyridin-2-yl)prop-2-enoate 38c as a yellow oil (0.15 g, 846.50 μmol, 45.33% yield).

The product was confirmed by LCMS and H-NMR.

MS-ESI calculated for [M+H]+ 178.1, found 178.0.

$^1$H NMR (400MHz, $CDCl_3$): δ (ppm) 8.65 (d, J=4.0Hz, 1H), 7.67-7.74 (m, 2H), 7.42 (d, J=8.0Hz, 1H), 7.25-7.29 (m, 1H), 6.90-6.94 (m, 1H), 1.17-1.31 (m, 3H).

Step 2

At room temperature, to a solution of compound ethyl (E)-3-(pyridin-2-yl)prop-2-enoate 38c (0.2 g, 1.13 mmol) in tetrahydrofuran (8 mL) and water (2 mL) was added lithium hydroxide (81.1 mg, 3.39 mmol). The mixture was stirred at 30°C for 16 hours. After thin-layer chromatography (dichloromethane/methanol = 10/1) indicated complete consumption of the starting material, the reaction mixture was concentrated. Water (10 mL) was added, and the pH was adjusted to 5-6 with aqueous citric acid solution. The mixture was concentrated under reduced pressure, ethanol (20 mL) was added, and the mixture was stirred for 30 minutes and then filtered. The filtrate was concentrated under reduced pressure to give the crude product (*E*)-3-(pyridin-2-yl)prop-2-enoic acid 38d as a yellow solid (0.15 g, 1.01 mmol, 89.11% yield).

The product was confirmed by LCMS and H-NMR.

MS-ESI calculated for [M+H]+ 150.0, found 150.0.

$^1$H NMR (400MHz, DMSO-d6): δ (ppm) 8.62 (d, J=4.4Hz, 1H), 7.82-7.86 (m, 1H), 7.68-7.70 (m, 1H), 7.50-7.54 (m, 1H), 7.36-7.39 (m, 1H), 6.83 (d, J=15.6Hz, 1H).

Step 3

At room temperature, to a solution of 4-bromopyridine-2-carbonitrile 38e (0.2 g, 1.09 mmol) and methyl 2-mercaptobenzoate (202.2 mg, 1.20 mmol) in *N*-methylpyrrolidone (10 mL) were added potassium carbonate (302.1 mg, 2.19 mmol) and copper(I) iodide (41.6 mg, 218.57 μmol). Under a nitrogen atmosphere, the mixture was heated to 100°C and stirred for about 16 hours. After thin-layer chromatography (petroleum ether/ethyl acetate = 5/1) indicated complete consumption of the starting material, the mixture was poured into water (30 mL) and extracted with ethyl acetate (10 mL × 3). The combined organic layers were washed with saturated brine (20 mL), dried over anhydrous sodium sulfate, and concentrated to afford the crude product. The crude product was purified by preparative thin-layer chromatography (petroleum ether/ethyl acetate = 5/1) to give methyl 2-[(2-cyano-4-pyridyl)thio]benzoate 38f as a white solid (0.19 g, 702.91 μmol, 64.32% yield).

The product was confirmed by LCMS and H-NMR.

MS-ESI calculated for [M+H]+ 271.0, found 271.0.

$^1H$ NMR (400MHz, $CDCl_3$): δ (ppm) 8.50 (d, J=8Hz, 1H), 8.00-8.02 (m, 1H), 7.51-7.59 (m, 3 H), 7.36 (s, 1H), 7.26-7.28 (m, 1H), 3.87-3.89 (m, 3H).

Step 4

At room temperature, to a solution of methyl 2-[(2-cyano-4-pyridyl)thio]benzoate 38f (200 mg, 739.90 μmol) in methanol (5 mL) were added di-tert-butyl dicarbonate (193.8 mg, 887.88 μmol) and Raney nickel (100 mg). Under a hydrogen atmosphere, the mixture was stirred at 25°C for 16 hours. After thin-layer chromatography (ethyl acetate/petroleum ether = 3/1) indicated complete consumption of the starting material, the mixture was filtered, and the filtrate was concentrated to afford the crude product. The crude product was purified by preparative thin-layer chromatography (ethyl acetate/petroleum ether = 5/1-3/1) to give methyl 2-[[2-[(tert-butoxycarbonylamino)methyl]-4-pyridyl]thio]benzoate 38g as a yellow oil (110 mg, 293.76 μmol, 39.70% yield). The product was confirmed by LCMS and H-NMR.

MS-ESI calculated for [M+H]+ 375.1, found 375.2.

$^1H$ NMR (400 MHz, $CDCl_3$) δ 8.34 (d, J = 5.6 Hz, 1H), 7.90 (dd, J = 6.0, 1.6 Hz, 1H), 7.26 - 7.40 (m, 3H), 7.14 (s, 1H), 7.00 (d, J = 4.8 Hz, 1H), 5.68 (s, 1H), 4.35 (d, J = 5.4 Hz, 2H), 3.84 (s, 3H), 1.40 (d, J = 2.8 Hz, 9H).

Step 5

Compound methyl 2-[[2-[(tert-butoxycarbonylamino)methyl]-4-pyridyl]thio]benzoate 38g (140 mg, 373.88 μmol) was dissolved in a solution of hydrogen chloride in dioxane (3 mL, 9 mol/L) and stirred at 25°C for 16 hours. After LCMS indicated complete consumption of the starting material, the reaction mixture was concentrated under reduced pressure to afford the crude product of methyl 2-[[2-(aminomethyl)-4-pyridyl]thio]benzoate dihydrochloride 38h.

The product was confirmed by LCMS.

MS-ESI calculated for [M+H]+ 275.1, found 275.0.

Step 6

To a solution of compound (E)-3-(pyridin-2-yl)prop-2-enoic acid 38d (24.0 mg, 160.88 μmol) in N,N-dimethylformamide (5 mL) were added compound methyl 2-[[2-(aminomethyl)-4-pyridyl]thio]benzoate dihydrochloride 38h (50.0 mg, 160.88 μmol), 1-(3-dimethylaminopropyl)-3-ethylcarbodiimide hydrochloride (45.9 mg, 241.31 μmol), 1-hydroxybenzotriazole (32.6 mg, 241.31 μmol), and triethylamine (81.4 mg, 804.38 μmol). The mixture was stirred at 25°C for 3.0 hours. After thin-layer chromatography (petroleum ether/ethyl acetate = 3/1) indicated complete consumption of the starting material, the mixture was poured into water (20 mL) and extracted with ethyl acetate (10 mL × 3). The combined organic layers were washed with saturated brine (20 mL), dried over anhydrous sodium sulfate, and concentrated to afford the crude product. Purification by preparative thin-layer chromatography (ethyl acetate/petroleum ether = 3/1) gave methyl 2-[3-[(*E*)-2-(pyridin-2-yl)ethenyl]imidazo[1,5-a]pyridin-7-yl]thiobenzoate 38i as a yellow oil (37 mg, 91.25 μmol, 56.72% yield).

The product was confirmed by LCMS and H-NMR.

MS-ESI calculated for [M+H]+ 406.1, found 406.1.

$^1H$ NMR (400 MHz, $CDCl_3$) δ 8.61 (d, J = 4.0 Hz, 1H), 8.38 (d, J = 5.6 Hz, 1H), 7.93 (dd, J = 8.0, 1.6 Hz, 1H), 7.62-7.71 (m, 2H), 7.34 - 7.44 (m, 4H), 7.08 - 7.25 (m, 4H), 7.05 (dd, J = 5.2, 1.6 Hz, 1H), 4.63 (s, 2H), 3.87 (s, 3H).

Step 7

At room temperature, to a solution of compound methyl 2-[3-[(*E*)-2-(pyridin-2-yl)ethenyl]imidazo[1,5-a]pyridin-7-yl]thiobenzoate 38i (13 mg, 32.06 μmol) in 1,2-dichloroethane (1.5 mL) was added phosphorus oxychloride (14.8 mg, 96.18 μmol). The mixture was stirred at 80°C for 16 hours. After LCMS indicated complete consumption of the starting material, the mixture was poured into water (10 mL) and extracted with ethyl acetate (10 mL × 3). The combined organic layers were washed with saturated brine (20 mL), dried over anhydrous sodium sulfate, and concentrated to afford the crude product. Purification by preparative thin-layer chromatography (ethyl acetate/petroleum ether = 3/1) gave compound methyl 2-[[2-[[(*E*)-3-(pyridin-2-yl)prop-2-enyl]amino]methyl]-4-pyridyl]thio]benzoate 38j as a yellow solid (5 mg, 12.90 μmol, 40.25% yield).

The product was confirmed by LCMS.

MS-ESI calculated for [M+H]+ 388.1, found 388.1.

Step 8

At room temperature, to a solution of compound methyl 2-[[2-[[(*E*)-3-(pyridin-2-yl)prop-2-enyl]amino]methyl]-4-pyridyl]thio]benzoate 38j (80 mg, 206.48 μmol) in methanol (5 mL) was added sodium hydroxide (16.5 mg, 412.95 μmol). The mixture was stirred at 25°C for 1.0 hour. After thin-layer chromatography (petroleum ether/ethyl acetate = 1/1) indicated complete consumption of the starting material, the mixture was poured into water (10 mL), and the pH was adjusted to 4-5 with IN aqueous hydrochloric acid solution. The mixture was filtered, and the filter cake was washed with water and dried to give compound 2-({3-[(E)-2-(pyridin-2-yl)ethenyl]imidazo[1,5-a]pyridin-7-yl}thio)benzoic acid I-28 as a red solid (48 mg, 128.54 μmol, 62.25% yield).

The product was confirmed by LCMS, H-NMR, and C-NMR.

MS-ESI calculated for [M+H]+ 374.1, found 374.1.

$^1$H NMR (400 MHz, $CDCl_3$&CD3OD) δ 8.59-8.71 (m, 3H), 8.13 (t, J = 7.2 Hz, 1H), 8.04 (d, J = 7.6 Hz, 1H), 7.83 (d, J = 7.6 Hz, 1H), 7.76 (s, 1H), 7.66 (s, 1H), 7.51-7.55 (m, 2H), 7.34 (t, J = 7.2 Hz, 1H), 7.23 (t, J = 7.2 Hz, 1H), 7.06 (d, J = 8.0 Hz, 1H), 6.82 (d, J = 6.8 Hz, 1H).

$^{13}$C NMR (100 MHz, $CDCl_3$&CD3OD) δ 168.38, 151.61, 144.03, 142.61, 139.59, 135.70, 132.80, 132.52, 131.63, 128.56, 125.73, 123.67, 123.29, 123.05 (s, 3H), 120.92, 119.86.

I-27

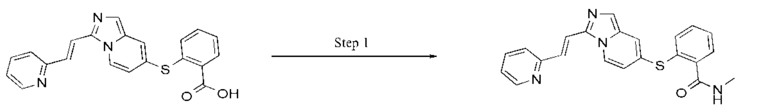


I-28 I-27

Step 1

At room temperature, to a solution of compound 2-({3-[(E)-2-(pyridin-2-yl)ethenyl]imidazo[1,5-a]pyridin-7-yl}thio)benzoic acid I-28 (23 mg, 61.59 μmol) in N,N-dimethylformamide (2 mL) were added methylamine (8.3 mg, 123.18 μmol), benzotriazol-1-yl-oxytripyrrolidinophosphonium hexafluorophosphate (48.1 mg, 92.39 μmol), and 1,8-diazabicyclo[5.4.0]undec-7-ene (18.8 mg, 123.18 μmol). The mixture was stirred at 25°C for 16 hours. After thin-layer chromatography (dichloromethane/methanol = 15/1) indicated complete consumption of the starting material, the mixture was poured into water (10 mL) and extracted with ethyl acetate (10 mL × 3). The combined organic layers were washed with saturated brine (20 mL), dried over anhydrous sodium sulfate, and concentrated to afford the crude product. The crude product was purified by preparative high-performance liquid chromatography to give N-methyl-2-({3-[(E)-2-(pyridin-2-yl)ethenyl]imidazo[1,5-a]pyridin-7-yl}thio)benzamide I-27 as a white solid (8 mg, 20.70 μmol, 33.61% yield).

The product was confirmed by LCMS and H-NMR.

MS-ESI calculated for [M+H]+ 387.1, found 387.0.

$^1$H NMR (400 MHz, CD3OD) δ 8.57 (d, J = 4.4 Hz, 1H), 8.42 (d, J = 7.2 Hz, 1H), 7.96 (d, J = 15.6 Hz, 1H), 7.86 - 7.90 (m, 1H), 7.68 (d, J = 7.6 Hz, 1H), 7.63 (s, 1H), 7.51-7.56 (m, 3H), 7.92-7.44 (m, 3H), 7.32-7.35 (m, 1H), 6.67 (dd, J = 7.6, 1.6 Hz, 1H), 2.88 (s, 3H).

I-44

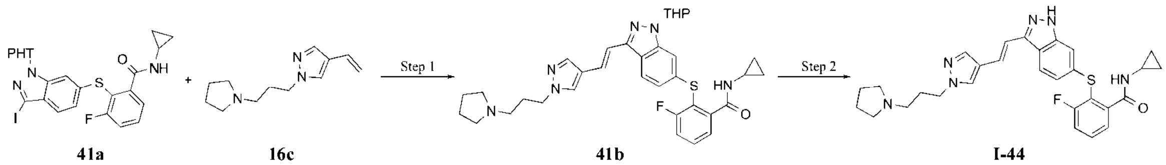


41a 16c 41b I-44

Step 1

At room temperature, N-cyclopropyl-3-fluoro-2-(3-iodo-1-tetrahydropyran-2-yl-indazol-6-yl)sulfanylbenzamide 41a (2 g, 3.72 mmol) was dissolved in dioxane (20 mL). 1-(3-Pyrrolidin-1-ylpropyl)-4-ethenylpyrazole 16c (458 mg, 2.23 mmol), palladium(II) chloride (198 mg, 1.12 mmol), tris(o-methylphenyl)phosphine (679 mg, 2.23 mmol), and triethylamine (1.1 g, 11.17 mmol) were added. The reaction system was stirred at 90°C for 16 hours under nitrogen protection. The

reaction mixture was poured into water (50 mL) and extracted with ethyl acetate (50 mL × 3). The combined organic phases were washed with saturated brine (30 mL), dried over anhydrous sodium sulfate, and concentrated under reduced pressure to afford the crude product. The crude product was purified by medium-pressure preparative chromatography to give the yellow solid product N-cyclopropyl-3-fluoro-2-[3-[(E)-2-[5-(pyrrolidin-1-ylmethyl)-2-pyridyl]ethenyl]-1-tetrahydropyran-2-yl-indazol-6-yl]sulfanylbenzamide 41b (700 mg, 1.14 mmol, 50.99% yield).

The product was confirmed by LCMS.

MS-ESI calculated for $[M+H]^+$ 615.3, found 615.1.

Step 2

At room temperature, N-cyclopropyl-3-fluoro-2-[3-[(E)-2-[5-(pyrrolidin-1-ylmethyl)-2-pyridyl]ethenyl]-1-tetrahydropyran-2-yl-indazol-6-yl]sulfanylbenzamide 41b (700 mg, 1.14 mmol) was dissolved in dichloromethane (16 mL), and trifluoroacetic acid (4 mL) was added. The reaction system was stirred at 25°C for 16 hours. The reaction mixture was poured into water (50 mL), the pH was adjusted to 8-9 with a saturated aqueous sodium carbonate solution, and then extracted with dichloromethane (50 mL × 3). The combined organic phases were washed with saturated brine (30 mL), dried over anhydrous sodium sulfate, and concentrated under reduced pressure to afford the crude product. The crude product was purified by high-performance liquid preparative chromatography to give the yellow solid product N-cyclopropyl-3-fluoro-2-({3-[(E)-2- {1-[3-(pyrrolidin-1-yl)propyl]-1H-pyrazol-4-yl}ethenyl]-1H-indazol-6-yl}sulfanyl)benzamide I-44 (90.15 mg, 169.88 μmol, 14.92% yield).

The product was confirmed by LCMS, $^1$H-NMR, and $^{19}$F-NMR.

MS-ESI calculated for $[M+H]^+$ 531.2, found 531.1.

$^1$H NMR (400MHz,$CD_3OD$): δ (ppm) 7.92-7.89 (m, 2H), 7.78 (s, 1H), 7.54-7.49 (m, 1H), 7.33-7.26 (m, 4H), 7.16-7.05 (m, 2H), 4.11 (t, *J* = 6.8 Hz, 2H), 2.78-2.73 (m, 1H), 2.75 (s, 4H), 2.49 (t, *J* = 7.6 Hz, 2H), 2.13-2.05 (m, 2H), 1.82-1.80 (m, 4H), 0.75-0.70 (m, 2H), 0.52-0.48 (m, 2H).

$^{19}$F NMR (400MHz,$CD_3OD$): δ (ppm)-105.90.

I-51

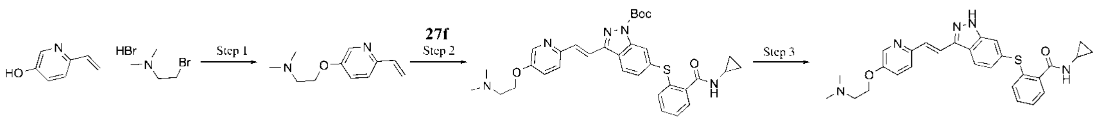


1c 43a 43b 43c I-51

Step 1

At room temperature, 6-ethenylpyridin-3-ol 1c (1 g, 8.26 mmol) was dissolved in anhydrous tetrahydrofuran (10 mL). The mixture was cooled to 0°C, and sodium hydride (991 mg, 24.77 mmol, 60%) was added. The reaction system was stirred at 0°C for 0.5 hours. 2-Bromo-N,N-dimethylethanolamine hydrobromide 43a (2.9 g, 12.38 mmol) was added at room temperature. The reaction system was stirred at 50°C for 2 hours. The reaction mixture was poured into water (30 mL) and extracted with ethyl acetate (20 mL × 3). The combined organic phases were washed with saturated brine (20 mL), dried over anhydrous sodium sulfate, and concentrated under reduced pressure to afford the crude product. The crude product was purified by column chromatography (petroleum ether:ethyl acetate = 10/1-3/1) to give the white solid product N,N-dimethyl-2-[(6-ethenylpyridin-3-yl)oxy]ethanamine 43b (900 mg, 4.68 mmol, 56.71% yield).

The product was confirmed by LCMS.

MS-ESI calculated for $[M+H]^+$ 193.1, found 193.1.

Step 2

At room temperature, N,N-dimethyl-2-[(6-ethenylpyridin-3-yl)oxy]ethanamine 43b (216 mg, 1.12 mmol) was dissolved in dioxane (10 mL). tert-Butyl 6-((2-(cyclopropylcarbamoyl)phenyl)sulfanyl)-3-iodo-1H-indazole-1-carboxylate 27f (500 mg, 933.89 μmol), tris(o-methylphenyl)phosphine (500 mg, 1.64 mmol), tris(dibenzylideneacetone)dipalladium(0) (500 mg, 546.02 μmol), and triethylamine (284 mg, 2.80 mmol) were added. The reaction system was stirred at 80°C under nitrogen protection for 1.5 hours. The reaction mixture was poured into water (30 mL) and extracted with ethyl acetate (20 mL × 3). The combined organic phases were washed with saturated brine (30 mL), dried over anhydrous sodium sulfate, and concentrated under reduced pressure to afford the yellow solid crude product 6-[2-(cyclopropylcarbamoyl)phenyl]sulfanyl-3-[(E)-2-[5-[2-(dimethylamino)ethoxy]-2-pyridyl]ethenyl]indazole-1-carboxylic acid tert-butyl ester 43c (300 mg, 500.22 μmol, 53.56% yield).

The product was confirmed by LCMS.

MS-ESI calculated for $[M+H]^+$ 600.3, found 600.4.

Step 3

At room temperature, 6-[2-(cyclopropylcarbamoyl)phenyl]sulfanyl-3-[(E)-2-[5-[2-(dimethylamino)ethoxy]-2-pyridyl]ethenyl]indazole-1-carboxylic acid tert-butyl ester 43c (300 mg, 500.22 μmol) was dissolved in dichloromethane (4 mL), and trifluoroacetic acid (4 mL) was added. The reaction mixture was stirred at 25°C for 16 hours. The reaction mixture was poured into water (30 mL), the pH was adjusted to 9 with a saturated sodium carbonate solution, and then extracted with dichloromethane (20 mL × 3). The combined organic phases were washed with saturated brine (30 mL), dried over anhydrous sodium sulfate, and concentrated under reduced pressure to afford the crude product. The crude product was purified by high-performance liquid preparative chromatography to give the yellow solid product N-cyclopropyl-2-({3-[(E)-2-{5-[2-(dimethylamino)ethoxy]pyridin-2-yl}ethenyl]-1H-indazol-6-yl}sulfanyl)benzamide I-51 (2.8 mg, 5.60 μmol, 1.12% yield).

The product was confirmed by LCMS, H-NMR, and C-NMR.

MS-ESI calculated for $[M+H]^+$ 500.2, found 500.2.

$^1$H NMR (400MHz, $CDCl_3$): δ (ppm) 8.36 (d, *J* = 2.8 Hz, 1H), 7.99 (d, *J* = 8.6 Hz, 1H), 7.75-7.63 (m, 2H), 7.73 (d, *J* = 16.0 Hz, 1H), 7.68-7.65 (m, 1H), 7.50-7.46 (m, 1H), 7.43-7.41 (m, 2H), 7.35-7.31 (m, 3H), 7.17 (d, *J* = 8.0 Hz, 1H), 6.51 (s, 1H), 4.25 (s, 2H), 2.92 (s, 2H), 2.85-2.78 (m, 1H), 2.48 (s, 6H), 0.80-0.78 (m, 2H), 0.49-0.45 (m, 2H). $^{13}$C NMR (100 MHz, $CDCl_3$): δ (ppm) 169.23, 148.94, 148.78, 142.07, 138.03, 136.79, 133.94, 133.59, 132.75, 132.60, 131.00, 130.53, 129.95, 129.11, 127.54, 124.27, 122.43, 121.69, 121.62, 121.52, 120.80, 57.68, 45.21, 31.93, 29.70, 23.04, 22.69, 14.12, 6.72.

I-55

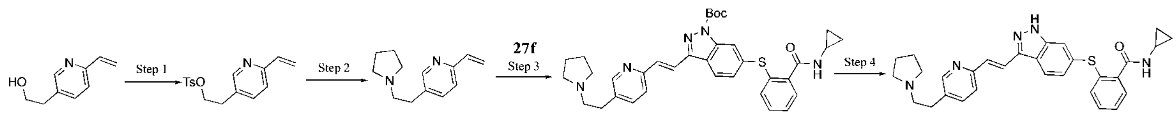


46a 46b 46c 46d I-55

Step 1

At room temperature, 2-(6-ethenylpyridin-3-yl)ethanol46a (1 g, 6.70 mmol) was dissolved in dichloromethane (10 mL) and acetonitrile (5 mL). p-Toluenesulfonyl chloride (1.7 g, 8.71 mmol) and triethylamine (1.7 g, 16.76 mmol) were added. The reaction system was stirred at 25°C for 15 hours. The reaction mixture was poured into water (30 mL) and extracted with dichloromethane (30 mL × 3). The combined organic phases were washed with saturated brine (40 mL), dried over anhydrous sodium sulfate, and concentrated under reduced pressure to afford the crude product. The crude product was purified by preparative TLC (petroleum ether:ethyl acetate = 3/1) to give the yellow oily product 2-(6-ethenylpyridin-3-yl)ethyl 4-methylbenzenesulfonate 46b (945 mg, 3.11 mmol, 46.47% yield).

The product was confirmed by LCMS and H-NMR.

MS-ESI calculated for $[M+H]^+$ 304.1, found 304.1.

$^1$H NMR (400MHz, $CDCl_3$): δ (ppm) 8.30 (d, *J* = 2.0 Hz, 1H), 7.66 (d, *J*= 8.0 Hz, 2H), 7.42 (dd, *J* = 8.0, 2.4 Hz, 1H), 7.28 (s, 1H), 7.26-7.22 (m, 2H), 6.78 (dd, *J* = 17.2, 10.8 Hz, 1H), 6.16 (dd, *J* = 17.6, 1.2 Hz, 1H), 5.47 (dd, *J* = 10.8, 0.8 Hz, 1H), 4.22 (t, *J* = 6.8 Hz, 2H), 2.94 (t, *J* = 6.8 Hz, 2H), 2.43 (s, 3H).

Step 2

At room temperature, 2-(6-ethenylpyridin-3-yl)ethyl 4-methylbenzenesulfonate46b (940 mg, 3.10 mmol) was dissolved in acetonitrile (15 mL). Pyrrolidine (1.1 g, 15.49 mmol) and triethylamine (784 mg, 7.75 mmol) were added. The reaction system was stirred at 6°C for 1.5 hours. The reaction mixture was poured into water (30 mL) and extracted with ethyl acetate (30 mL × 3). The combined organic phases were washed with saturated brine (30 mL), dried over anhydrous sodium sulfate, and concentrated under reduced pressure to afford the crude product. The crude product was purified by preparative TLC (dichloromethane:methanol = 10/1) to give the yellow oily product 5-(2-pyrrolidin-1-yl)-2-ethenylpyridine46c (396.7 mg, 1.96 mmol, 63.29% yield).

The product was confirmed by LCMS and H-NMR.

MS-ESI calculated for $[M+H]^+$ 203.2, found 203.2.

$^1H$ NMR (400MHz, $CDCl_3$): δ (ppm) 8.43 (d, *J* = 2.0 Hz, 1H), 7.51 (dd, *J* = 8.0, 2.4 Hz, 1H), 7.28 (d, *J* = 8.0 Hz, 1H), 6.80 (dd, *J* = 17.6, 10.8 Hz, 1H), 6.14 (dd, *J* = 17.6, 1.2Hz, 1H), 5.44 (dd, *J* = 10.8, 1.2 Hz, 1H), 2.87-2.83 (m, 2H), 2.74-2.70 (m, 2H), 2.62 (s, 4H), 1.84-1.81 (m, 4H).

Step 3

At room temperature, 5-(2-pyrrolidin-1-yl)-2-ethenylpyridine 46c (189 mg, 933.89 μmol) was dissolved in dioxane (10 mL). tert-butyl 6-((2-(cyclopropylcarbamoyl)phenyl)sulfanyl)-3-iodo-1H-indazole-1-carboxylate 27f (500 mg, 933.89 μmol), tris(o-methylphenyl)phosphine (284 mg, 933.89 μmol), palladium chloride (83 mg, 466.94 μmol), and triethylamine (473 mg, 4.67 mmol) were added. The reaction system was stirred at 95°C under nitrogen protection for 1.5 hours. The reaction mixture was poured into water (20 mL) and extracted with ethyl acetate (20 mL × 3). The combined organic phases were washed with saturated brine (20 mL), dried over anhydrous sodium sulfate, and concentrated under reduced pressure to afford the brown solid crude product tert-butyl 6-{[2-(cyclopropylcarbamoyl)phenyl]sulfanyl}-3-[(E)-2-{5-[(pyrrolidin-1-yl)methyl]pyridin-2-yl} ethenyl]-1H-indazole-1-carboxylate 46d (300 mg, 491.98 μmol, 52.68% yield).

The product was confirmed by LCMS.

MS-ESI calculated for $[M+H]^+$ 610.3, found 610.3.

Step 4

At room temperature, tert-butyl 6-{[2-(cyclopropylcarbamoyl)phenyl]sulfanyl}-3-[(E)-2-{5-[(pyrrolidin-1-yl) methyl]pyridin-2-yl}ethenyl]-1H-indazole-1-carboxylate 46d (300 mg, 491.98 μmol) was dissolved in dichloromethane (12 mL). Trifluoroacetic acid (4 mL) was added. The reaction mixture was stirred at 25°C for 2 hours. The reaction mixture was poured into water (30 mL). The pH was adjusted to 9 with saturated sodium carbonate solution, and then extracted with dichloromethane (20 mL × 3). The combined organic phases were washed with saturated brine (30 mL), dried over anhydrous sodium sulfate, and concentrated under reduced pressure to afford the crude product. The crude product was purified by preparative HPLC to give the white solid product N-cyclopropyl-2-({3-[(E)-2-{5-[2-(pyrrolidin-1-yl)ethyl]pyridin-2-yl}ethenyl]-1H-indazol-6-yl}thio)benzamide I-55 (17 mg, 33.36 μmol, 6.78% yield).

The product was confirmed by LCMS, H-NMR, and C-NMR.

MS-ESI calculated for $[M+H]^+$ 512.2, found 512.2.

$^1H$ NMR (400MHz, $CD_3OD$): δ (ppm) 8.53 (s, 1H), 8.49 (s, 1H), 8.03 (d, *J* = 8.4 Hz, 1H), 7.81 (dd, *J* = 26.2, 12.4 Hz, 2H), 7.64 (d, *J* = 8.0 Hz, 1H), 7.57-7.42 (m, 3H), 7.39-7.28 (m, 2H), 7.24 (d, *J* = 7.2 Hz, 1H), 7.18 (d, *J* = 8.4 Hz, 1H), 3.48-3.32 (m, 6H), 3.15-3.04 (m, 2H), 2.84 -2.73 (m, 1H), 2.09 (s, 4H), 0.82-0.66 (m, 2H), 0.61-0.47 (m, 2H).

$^{13}C$ NMR (100 MHz, $CD_3OD$) δ (ppm) 172.67, 155.80, 150.59, 143.76, 143.61, 139.29, 138.94, 135.82, 133.25, 131.72, 130.27, 129.17, 128.27, 126.41, 125.36, 123.26, 122.50, 121.58, 114.86, 56.57, 55.26, 30.25, 24.12, 23.82, 6.51.

I-67

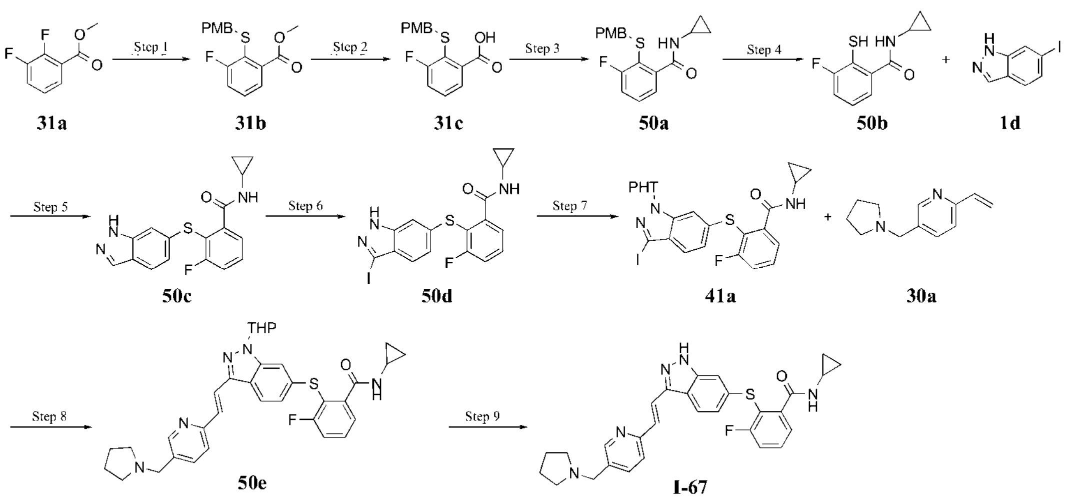


31a 31b 31c 50a 50b 1d

50c 50d 41a 30a

50e I-67

Step 1

At room temperature, methyl 2,3-difluorobenzoate 31a (50 g, 290.48 mmol) was dissolved in N,N-dimethylformamide (500 mL). (4-Methoxyphenyl)methanethiol (44.8 g, 290.48 mmol) and cesium carbonate (189.3 g, 580.96 mmol) were added. The reaction system was stirred at 25°C for 2 hours. The reaction mixture was filtered. The filtrate was poured into an aqueous solution (1000 mL) and extracted with ethyl acetate (500 mL × 3). The combined organic phases were washed with saturated brine (500 mL), dried over anhydrous sodium sulfate, and concentrated under reduced pressure to afford the crude product. The crude product was purified by medium pressure preparative chromatography to give the white solid product methyl 3-fluoro-2-[(4-methoxyphenyl)methylthio]benzoate 31b (50 g, 163.21 mmol, 56.19% yield).
The product was confirmed by LCMS and H-NMR.
MS-ESI calculated for $[M+Na]^+$ 329.1, found 328.9.
$^1H$ NMR (400MHz, $CDCl_3$): δ (ppm) 7.37 (d, *J*= 7.6 Hz, 1H), 7.30-7.25 (m, 1H), 7.18-7.13 (m, 3H), 6.76 (d, *J*= 8.8 Hz, 2H), 4.08 (s, 2H), 3.89 (s, 3H), 3.76 (s, 3H)

Step 2

At room temperature, methyl 3-fluoro-2-[(4-methoxyphenyl)methylthio]benzoate 31b (50 g, 163.21 mmol) was dissolved in tetrahydrofuran (360 mL) and water (102 mL). Lithium hydroxide (11.7 g, 489.63 mmol) was added. The reaction system was stirred at 50°C for 16 hours. Tetrahydrofuran was removed under reduced pressure. The remaining mixture was poured into an aqueous solution (1000 mL). The pH was adjusted to 5 with dilute hydrochloric acid (1 N), and the solid was collected by filtration. The filter cake was dried to give the white solid product 3-fluoro-2-[(4-methoxyphenyl) methylthio]benzoic acid 31c (41 g, 140.25 mmol, 85.93% yield).
The product was confirmed by LCMS and H-NMR.
MS-ESI calculated for $[M+H_2O]^+$ 310.1, found 310.0.
$^1H$ NMR (400MHz, $CDCl_3$): δ (ppm) 7.86 (d, *J*= 7.6 Hz, 1H), 7.42-7.37 (m, 1H), 7.29 (d, *J*= 8.4 Hz, 1H), 7.09 (d, *J*= 8.4 Hz, 2H), 6.76 (d, *J*= 8.0 Hz, 2H), 4.12 (s, 2H), 3.76 (s, 3H).

Step 3

At room temperature, 3-fluoro-2-[(4-methoxyphenyl)methylthio]benzoic acid 31c (20 g, 68.42 mmol) was dissolved in N,N-dimethylformamide (200 mL). Cyclopropylamine (9.6 g, 102.63 mmol), 1-ethyl-3-(3-dimethylaminopropyl)carbodiimide hydrochloride (19.7 g, 102.63 mmol), 1-hydroxybenzotriazole (13.9 g, 102.63 mmol), and N,N-diisopropylethylamine (26.5 g, 205.25 mmol) were added. The reaction system was stirred at 25°C for 2 hours. The reaction mixture was poured into water (1000 mL) and extracted with ethyl acetate (500 mL × 3). The combined organic phases were washed with saturated brine (300 mL), dried over anhydrous sodium sulfate, and concentrated under reduced pressure to afford the crude product. The crude product was purified by medium pressure preparative chromatography to give the white solid product N-cyclopropyl-3-fluoro-2-[(4-methoxyphenyl)methylthio]benzamide 50a (15 g, 45.26 mmol, 66.16% yield).
The product was confirmed by LCMS and H-NMR.
MS-ESI calculated for $[M+H]^+$ 332.1, found 332.0.
$^1H$ NMR (400 MHz, $CDCl_3$) δ (ppm) 7.34-7.31 (m, 2H), 7.17-7.12 (m, 1H), 6.97 (d, *J*= 8.4 Hz, 2H), 6.74 (d, *J*= 8.4 Hz, 2H), 4.00 (s, 2H), 3.76 (s, 3H), 2.77-2.73 (m, 1H), 0.81-0.76 (m, 2H), 0.48-0.44 (m, 2H).

Step 4

At room temperature, N-cyclopropyl-3-fluoro-2-[(4-methoxyphenyl)methylthio]benzamide 50a (24 g, 72.42 mmol) was dissolved in trifluoroacetic acid (240 mL). The reaction system was stirred at 70°C under nitrogen protection for 5 hours. The reaction mixture was concentrated under reduced pressure to afford the crude product. The crude product was purified by medium pressure preparative chromatography to give the yellow solid product N-cyclopropyl-3-fluoro-2-mercaptobenzamide 50b (7 g, 33.14 mmol, 45.75% yield).
The product was confirmed by LCMS and H-NMR.
MS-ESI calculated for $[M+H]^+$ 212.1, found 212.1.
$^1H$ NMR (400 MHz, $CDCl_3$) δ (ppm) 7.24-7.18 (m, 1H), 7.15-7.06 (m, 2H), 6.24 (s, 1H), 4.73 (s, 1H), 2.91-2.86 (m, 1H), 0.91-0.82 (m, 2H), 0.70-0.63 (m, 2H).

Step 5

At room temperature, N-cyclopropyl-3-fluoro-2-mercaptobenzamide 50b (7 g, 33.14 mmol) was dissolved in

N,N-dimethylformamide (100 mL). 6-Todo-1H-indazole 1d (8.1 g, 33.14 mmol), tris(dibenzylideneacetone)dipalladium (3 g, 3.31 mmol), 4,5-bis(diphenylphosphino)-9,9-dimethylxanthene (3.8 g, 6.62 mmol), and cesium carbonate (32.4 g, 99.41 mmol) were added. The reaction system was stirred at 90°C under nitrogen protection for 16 hours. The reaction mixture was poured into water (500 mL), then extracted with ethyl acetate (100 mL x 3). The combined organic phases were washed with saturated brine (100 mL), dried over anhydrous sodium sulfate, and concentrated under reduced pressure to afford the crude product. The crude product was purified by medium pressure preparative chromatography to give the yellow solid product N-cyclopropyl-3-fluoro-2-[(1H-indazol-6-yl)sulfanyl]benzamide 50c (5.6 g, 17.11 mmol, 51.62% yield).

The product was confirmed by LCMS and H-NMR.

MS-ESI calculated for $[M+H]^+$ 328.1, found 328.0.

$^1$H NMR (400MHz,$CD_3OD$): δ (ppm) 7.97 (s, 1H), 7.62 (d, *J* = 8.8 Hz, 1H), 7.54-7.46 (m, 1H), 7.38 (s, 1H), 7.28-7.25 (m, 2H), 7.00 (dd, *J* = 9.6, 1.2 Hz, 1H), 2.78-2.73 (m, 1H), 0.74-0.69 (m, 2H), 0.51-0.47 (m, 2H).

Step 6

At room temperature, N-cyclopropyl-3-fluoro-2-[(1H-indazol-6-yl)sulfanyl]benzamide 50c (2.5 g, 7.64 mmol) was dissolved in N,N-dimethylformamide (30 mL). Potassium carbonate (3.2 g, 22.91 mmol) and iodine (3.9 g, 15.27 mmol) were added. The reaction system was stirred at 40°C for 2 hours. The reaction mixture was poured into water (200 mL), then extracted with ethyl acetate (100 mL x 3). The combined organic phases were washed with saturated brine (100 mL), dried over anhydrous sodium sulfate, and concentrated under reduced pressure to give the yellow solid product N-cyclopropyl-3-fluoro-2-[(3-iodo-1H-indazol-6-yl)sulfanyl]benzamide 50d (2.5 g, 5.52 mmol, 72.23% yield).

The product was confirmed by LCMS and H-NMR.

MS-ESI calculated for $[M+H]^+$ 454.0, found 453.9.

$^1$H NMR (400MHz, DMSO_*d*6): δ (ppm) 13.38 (s, 1H), 8.53 (d, *J*= 4.0 Hz, 1H), 7.62-7.54 (m, 1H), 7.41-7.30 (m, 4H), 7.04 (d, *J*= 8.4 Hz, 1H), 2.79-2.74 (m, 1H), 0.68-0.63 (m, 2H), 0.48-0.45 (m, 2H).

Step 7

At room temperature, N-cyclopropyl-3-fluoro-2-[(3-iodo-1H-indazol-6-yl)sulfanyl]benzamide 50d (6 g, 13.24 mmol) was dissolved in ethyl acetate (80 mL). p-Toluenesulfonic acid (1.1 g, 6.62 mmol) and 3,4-dihydro-2H-pyran (3.3 g, 39.71 mmol) were added. The reaction system was stirred at 70°C for 3 hours. The reaction mixture was concentrated under reduced pressure to afford the crude product. The crude product was purified by medium pressure preparative chromatography to give the yellow solid product N-cyclopropyl-3-fluoro-2-[(3-iodo-1-(tetrahydro-2H-pyran-2-yl)-1H-indazol-6-yl)sulfanyl]benzamide 41a (4.2 g, 7.82 mmol, 59.04% yield).

The product was confirmed by LCMS and H-NMR.

MS-ESI calculated for $[M+H]^+$ 538.0, found 537.9.

$^1$H NMR (400 MHz, DMSO_*d*6) δ (ppm) 8.53 (d, J = 4.0 Hz, 1H), 7.69 (s, 1H), 7.60-7.52 (m, 1H), 7.41-7.28 (m, 3H), 6.95 (d, *J* = 8.8 Hz, 1H), 5.85-5.73 (m, 1H), 3.85 (d, *J* = 11.6 Hz, 1H), 3.70 (t, *J* = 12.8 Hz, 1H), 2.79-2.73 (m, 1H), 2.31 (dd, *J* = 21.2, 8.8 Hz, 2H), 1.99-1.91 (m, 2H), 1.72 (s, 1H), 1.55 (s, 2H), 0.65 (dd, *J* = 6.8, 2.0 Hz, 2H), 0.51 - 0.42 (m, 2H).

Step 8

At room temperature, N-cyclopropyl-3-fluoro-2-[(3-iodo-1-(tetrahydro-2H-pyran-2-yl)-1H-indazol-6-yl)sulfanyl]benzamide 41a(2 g, 3.72 mmol) was dissolved in 1,4-dioxane (20 mL). 5-(Pyrrolidin-1-ylmethyl)-2-ethenylpyridine 30a (841 mg, 4.47 mmol), palladium(II) chloride (330 mg, 1.86 mmol), tri(o-tolyl)phosphine (1.1 g, 3.72 mmol), and triethylamine (1.9 g, 18.61 mmol) were added. The reaction system was stirred at 90°C for 16 hours under nitrogen protection. The reaction mixture was poured into water (100 mL) and extracted with ethyl acetate (100 mL x 3). The combined organic phases were washed with saturated brine (50 mL), dried over anhydrous sodium sulfate, and concentrated under reduced pressure to afford the crude product. The crude product was purified by medium pressure preparative chromatography to give the yellow solid product N-cyclopropyl-3-fluoro-2-({3-[(E)-2-{5-[(pyrrolidin-1-yl) methyl]pyridin-2-yl}ethenyl]-1-(tetrahydro-2H-pyran-2-yl)-1H-indazol-6-yl}sulfanyl)benzamide 50e (1.4 g, 2.34 mmol, 62.93% yield).

The product was confirmed by LCMS.

MS-ESI calculated for $[M+H]^+$ 598.3, found 598.1.

Step 9

At room temperature, N-cyclopropyl-3-fluoro-2-({3-[(E)-2-{5-[(pyrrolidin-1-yl)methyl]pyridin-2-yl}ethenyl]-1-(tetrahydro-2H-pyran-2-yl)-1H-indazol-6-yl}sulfanyl)benzamide 50e (1.4 g, 2.34 mmol) was dissolved in dichloromethane (18 mL). Trifluoroacetic acid (6 mL) was added. The reaction system was stirred at 25°C for 16 hours. The reaction mixture was poured into water (100 mL). The pH was adjusted to 8-9 with a saturated aqueous sodium carbonate solution, then extracted with dichloromethane (100 mL x 3). The combined organic phases were washed with saturated brine (50 mL), dried over anhydrous sodium sulfate, and concentrated under reduced pressure to afford the crude product. The crude product was purified by high pressure preparative chromatography to give the yellow solid product N-cyclopropyl-3-fluoro-2-({3-[(E)-2-{5-[(pyrrolidin-1-yl)methyl]pyridin-2-yl}ethenyl]-1H-indazol-6-yl}thio)benzamide I-67 (214.47 mg, 417.56 μmol, 17.83% yield).

The product was confirmed by LCMS, F-NMR, and H-NMR.

MS-ESI calculated for $[M+H]^+$ 514.2, found 514.2.

$^1H$ NMR (400MHz,$CD_3OD$): δ (ppm) 8.51 (d, *J* = 1.6 Hz, 1H), 7.98 (d, *J* = 8.8 Hz, 1H), 7.88-7.79 (m, 2H), 7.67 (d, *J* = 8.0 Hz, 1H), 7.58-7.47 (m, 2H), 7.39 (s, 1H), 7.30-7.26 (m, 2H), 7.13-7.10 (m, 1H), 3.70 (s, 2H), 2.78-2.74 (m, 1H), 2.59 (s, 4H), 1.84 (s, 4H), 0.78-0.68 (m, 2H), 0.52-0.48 (m, 2H).

$^{19}F$ NMR (400MHz,$CD_3OD$): δ (ppm)-105.88.

I-68

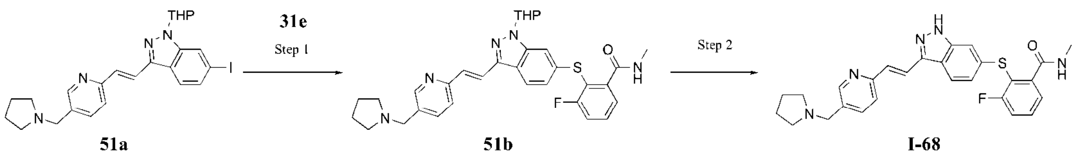


Step 1

At room temperature, 6-iodo-3-[(E)-2-{5-[(pyrrolidin-1-yl)methyl]pyridin-2-yl}ethenyl]-1-(tetrahydro-2H-pyran-2-yl)-1H-indazole 51a (190.0 mg, 369.36 μmol) was dissolved in N,N-dimethylformamide (5 mL). 3-Fluoro-N-methyl-2-mercaptobenzamide 31e (68.4 mg, 369.36 μmol), [1,1'-bis(diphenylphosphino)ferrocene]dichloropalladium(II) (107.2 mg, 147.74 μmol), and cesium carbonate (240.7 mg, 738.72 μmol) were then added. The reaction system was stirred at 80°C under nitrogen protection for 16 hours. The reaction mixture was poured into water (20 mL) and extracted with ethyl acetate (10 mL x 3). The combined organic phases were washed with saturated brine (30 mL), dried over anhydrous sodium sulfate, and concentrated under reduced pressure to afford the crude product. The crude product was purified by preparative TLC (DCM/MeOH = 10/1) to give the brown solid product 3-fluoro-N-methyl-2-({3-[(E)-2-{5-[(pyrrolidin-1-yl)methyl]pyridin-2-yl}ethenyl]-1-(tetrahydro-2H-pyran-2-yl)-1H-indol-6-yl}sulfanyl)benzamide 51b (70 mg, 122.44 μmol, 33.15% yield).

The product was confirmed by LCMS.

MS-ESI calculated for $[M+H]^+$ 572.2, found 572.1.

Step 2

At room temperature, 3-fluoro-N-methyl-2-({3-[(E)-2-{5-[(pyrrolidin-1-yl)methyl]pyridin-2-yl}ethenyl]-1-(tetrahydro-2H-pyran-2-yl)-1H-indol-6-yl}sulfanyl)benzamide 51b (70.0 mg, 122.44 μmol) was dissolved in dichloromethane (5 mL). Trifluoroacetic acid (139.6 mg, 1.22 mmol) was added, and the reaction system was stirred at 25°C for 4 hours. The reaction mixture was poured into water (20 mL). The pH was adjusted to 8-9 with a saturated sodium carbonate solution, then extracted with dichloromethane (20 mL x 3). The combined organic phases were washed with saturated brine (20 mL), dried over anhydrous sodium sulfate, and concentrated under reduced pressure to afford the crude product. The crude product was purified by preparative HPLC to give the yellow solid product 3-fluoro-N-methyl-2-({3-[(E)-2-{5-[(pyrrolidin-1-yl)methyl]pyridin-2-yl}ethenyl]-1H-indazol-6-yl}sulfanyl)benzamide I-68 (10.02 mg, 20.55 μmol, 16.78% yield).

The product was confirmed by LCMS, F-NMR, H-NMR, and C-NMR.

MS-ESI calculated for $[M+H]^+$ 488.2, found 488.3.

$^1H$ NMR (400MHz, $CD_3OD$): δ (ppm) 8.49 (d, J = 1.2 Hz, 1H), 7.97 (d, J= 8.8 Hz, 1H), 7.84-7.79 (m, 2H), 7.64 (d, J= 8.0 Hz, 1H), 7.54-7.50 (m, 2H), 7.39 (s, 1H), 7.32-7.26 (m, 2H), 7.12 (d, J=8.4 Hz, 1H), 3.69 (s, 2H), 2.83 (s, 3H), 2.59 (s, 4H), 1.83 (s, 4H).

$^{13}C$ NMR (100MHz, $CD_3OD$): δ (ppm) 170.76, 170.74, 165.40, 162.92, 155.98, 151.01, 145.43, 143.73, 143.47, 139.57, 136.56, 134.17, 132.62, 132.53, 130.32, 125.35, 124.63, 124.79, 124.04, 122.10, 121.12, 119.98, 118.56, 118.32, 111.71, 58.15, 54.92, 26.69, 24.19.

$^{19}F$ NMR (400MHz, $CD_3OD$): δ (ppm) -105.58.

I-73

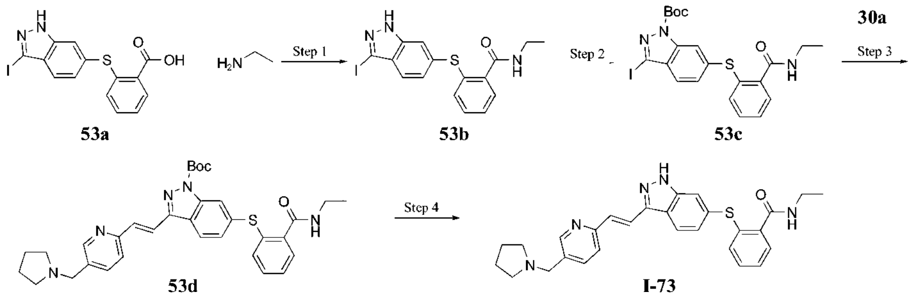


53a 53b 53c 53d I-73

Step 1

At room temperature, 2-[(3-iodo-1H-indazol-6-yl)sulfanyl]benzoic acid 53a (1.5 g, 3.79 mmol) was dissolved in N,N-dimethylformamide (20 mL). Ethylamine (617 mg, 7.57 mmol), HATU (2.2 g, 5.68 mmol), and N,N-diisopropylethylamine (2.4 g, 18.93 mmol) were added, and the reaction system was stirred at 25°C for 2 hours. The reaction mixture was poured into water (50 mL) and extracted with dichloromethane (50 mL x 3). The combined organic phases were washed with saturated brine (40 mL), dried over anhydrous sodium sulfate, and concentrated under reduced pressure to afford the crude product. The crude product was purified by medium pressure preparative chromatography to give the brown solid product N-ethyl-2-[(3-iodo-1H-indazol-6-yl)sulfanyl]benzamide 53b (1.3 g, 3.07 mmol, 81.12% yield).

The product was confirmed by LCMS and H-NMR.

MS-ESI calculated for $[M+H]^+$ 424.0, found 424.0.

$^1$H NMR (400MHz, $CDCl_3$): δ (ppm) 12.93 (s, 1H), 7.99 (d, *J* = 8.2 Hz, 1H), 7.55 (d, *J* = 9.0 Hz, 2H), 7.44-7.38 (m, 1H), 7.27-7.07 (m, 4H), 4.19-4.00 (m, 2H), 1.34 (d, *J* = 9.0 Hz, 3H).

Step 2

At room temperature, N-ethyl-2-[(3-iodo-1H-indazol-6-yl)sulfanyl]benzamide 53b (1.3 g, 3.07 mmol) was dissolved in tetrahydrofuran (10 mL). Triethylamine (1.2 g, 12.29 mmol) and di-tert-butyl dicarbonate (1.3 g, 6.14 mmol) were added, and the reaction system was stirred at 60°C for 16 hours. The reaction mixture was poured into water (50 mL) and extracted with ethyl acetate (50 mL x 3). The combined organic phases were washed with saturated brine (50 mL), dried over anhydrous sodium sulfate, and concentrated under reduced pressure to afford the crude product. The crude product was purified by medium pressure preparative chromatography to give the white solid producttert-butyl 6-{[2-(ethylcarbamoyl)phenyl]sulfanyl}-3-iodo-1H-indazole-1-carboxylate 53c (1.5 g, 2.87 mmol, 93.31% yield).

The product was confirmed by LCMS and H-NMR.

MS-ESI calculated for $[M+H]^+$ 524.0, found $[M-100]^+$ 424.0.

$^1$H NMR (400MHz, $CDCl_3$): δ (ppm) 8.05 (s, 1H), 7.66 (dd, *J* = 4.8, 1.6 Hz, 1H), 7.42-7.31 (m, 4H), 7.27 (d, *J* = 1.2 Hz, 1H), 6.29 (s, 1H), 2.80 (s, 2H), 1.63 (s, 9H), 1.14 (t, *J* = 7.4 Hz, 3H).

Step 3

At room temperature, tert-butyl 6-{[2-(ethylcarbamoyl)phenyl]sulfanyl}-3-iodo-1H-indazole-1-carboxylate 53c (500 mg, 955.32 μmol) was dissolved in 1,4-dioxane (10 mL). 5-[(Pyrrolidin-1-yl)methyl]-2-ethenylpyridine 30a (180 mg, 955.32 μmol), tris(2-methylphenyl)phosphine (290 mg, 955.32 μmol), palladium(II) chloride (85 mg, 477.66 μmol), and triethylamine (483 mg, 4.78 mmol) were added. The reaction system was stirred at 95°C under nitrogen protection for 1.5 hours. The reaction mixture was poured into water (10 mL) and extracted with ethyl acetate (10 mL x 3). The combined organic phases were washed with saturated brine (20 mL), dried over anhydrous sodium sulfate, and concentrated under reduced pressure to afford the brown solid crude product tert-butyl (E)-6-{[2-(ethylcarbamoyl)phenyl]sulfanyl}-3-[2-(5-[(pyrrolidin-1-yl)methyl]pyridin-2-yl)ethenyl]-1H-indazole-1-carboxylate 53d (200 mg, 342.62 μmol, 35.86% yield).

The product was confirmed by LCMS.

MS-ESI calculated for $[M+H]^+$ 584.3, found 584.3.

Step 4

At room temperature, tert-butyl (E)-6-{[2-(ethylcarbamoyl)phenyl]sulfanyl}-3-[2-(5-[(pyrrolidin-1-yl)methyl]pyridin-2-yl)ethenyl]-1H-indazole-1-carboxylate 53d (350 mg, 599.58 μmol) was dissolved in dichloromethane (9 mL). Trifluoroacetic acid (3 mL) was added, and the reaction mixture was stirred at 25°C for 2 hours. The reaction mixture was poured into water (30 mL). The pH was adjusted to 9 with a saturated sodium carbonate solution, then extracted with dichloromethane (20 mL x 3). The combined organic phases were washed with saturated brine (30 mL), dried over anhydrous sodium sulfate, and concentrated under reduced pressure to afford the crude product. The crude product was purified by preparative HPLC to give the yellow solid product N-ethyl-2-({3-[(E)-2-{5-[(pyrrolidin-1-yl)methyl]pyridin-2-yl}ethenyl]-1H-indazol-6-yl}thio)benzamide I-73 (53 mg, 109.59 μmol, 18.28% yield).

The product was confirmed by LCMS, H-NMR, and C-NMR.

MS-ESI calculated for $[M+H]^+$ 484.2, found 484.2.

$^1$H NMR (400MHz, $CD_3OD$): δ (ppm) 8.66 (d, *J* = 1.6 Hz, 1H), 8.48 (s, 1H), 8.05 (d, *J* = 8.6 Hz, 1H), 7.99-7.88 (m, 2H), 7.72 (d, *J* = 8.2 Hz, 1H), 7.61-7.52 (m, 2H), 7.51-7.44 (m, 1H), 7.39-7.28 (m, 2H), 7.28-7.17 (m, 2H), 4.32 (s, 2H), 3.34 (dd, *J* = 14.2, 6.8 Hz, 2H), 3.25 (s, 4H), 2.06 (s, 4H), 1.16 (t, *J* = 7.4 Hz, 3H).

$^{13}$C NMR (100 MHz, $CD_3OD$) δ (ppm) 171.00, 169.53, 157.82, 151.96, 143.62, 140.34, 139.56, 135.84, 133.17, 131.62, 129.10, 128.13, 126.62, 126.54, 123.38, 122.45, 121.65, 114.98, 56.51, 54.85, 35.80, 23.94, 14.70.

I-75

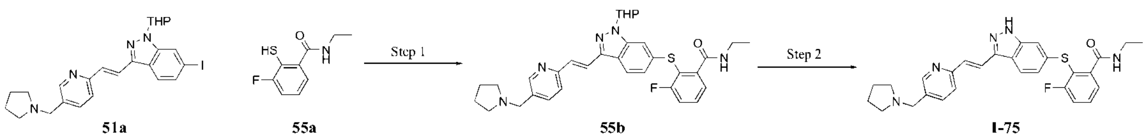


51a 55a 55b I-75

Step 1

At room temperature, 6-iodo-3-[(E)-2-{5-[(pyrrolidin-1-yl)methyl]pyridin-2-yl}ethenyl]-1-(tetrahydro-2H-pyran-2-yl)-1H-indol 51a (180 mg, 349.92 μmol) was dissolved in DMF (6 mL). N-Ethyl-3-fluoro-2-mercaptobenzamide 55a (69.7 mg, 349.92 μmol), cesium carbonate (228.2 mg, 700.23 μmol), and Pd(dppf)Cl, (90 mg, 124.03 μmol) were added under nitrogen protection. The reaction system was stirred at 80°C for 5.0 hours. The reaction mixture was poured into water (30 mL) and extracted with EA (30 mL x 3). The combined organic phases were washed with saturated brine (30 mL), dried over anhydrous sodium sulfate, and concentrated under reduced pressure to afford the crude product. The crude product was purified by preparative thin-layer chromatography (DCM/MeOH = 10/1) to give the brown solid crude product N-ethyl-3-fluoro-2-({3-[(E)-2-{5-[(pyrrolidin-1-yl)methyl]pyridin-2-yl}ethenyl]-1-(tetrahydro-2H-pyran-2-yl)-1H-indol-6-yl}sulfanyl)benzamide 55b (400 mg, crude).

The product was confirmed by LCMS.

MS-ESI calculated for $[M+H]^+$ 586.3, found 586.3.

Step 2

N-Ethyl-3-fluoro-2-({3-[(E)-2-{5-[(pyrrolidin-1-yl)methyl]pyridin-2-yl}ethenyl]-1-(tetrahydro-2H-pyran-2-yl)-1H-indol-6-yl}sulfanyl)benzamide 55b (80 mg, 136.58 μmol)was dissolved in DCM (10 mL). Trifluoroacetic acid (155.7 mg, 1.37 mmol) was added. The reaction system was stirred at 25°C for 2 hours. The reaction mixture was poured into water (20 mL). The pH was adjusted to 8-9 with a saturated sodium bicarbonate aqueous solution, then extracted with DCM (20 mL x 3). The combined organic phases were washed with saturated brine (30 mL), dried over anhydrous sodium sulfate, and concentrated under reduced pressure to afford the crude product. The crude product was purified by preparative high-performance liquid chromatography to give the yellow solid product N-ethyl-3-fluoro-2-({3-[(E)-2-{5-[(pyrrolidin-1-yl)methyl]pyridin-2-yl}ethenyl]-1H-indazol-6-yl}sulfanyl)benzamide I-75 (21.51 mg, 42.88 μmol, 31.40% yield).

The product was confirmed by LCMS, F-NMR, H-NMR, and C-NMR.

MS-ESI calculated for $[M+H]^+$ 502.2, found 502.2.

$^1$H NMR (400 MHz, $CD_3OD$): δ (ppm) 8.63 (s, 1H), 7.98-7.83 (m, 3H), 7.70 (d, J=8.0, 1H), 7.56-7.51 (m, 2H), 7.38-7.27 (m, 3H), 7.13 (d, J=8.4, 1H), 4.26 (s, 2H), 3.35-3.29 (m, 2H), 3.19 (s, 4H), 2.03 (s, 4H), 1.13 (t, J=7.2, 3H).

$^{13}$C NMR (100 MHz, $CD_3OD$): δ (ppm) 169.99, 169.97, 165.45, 162.97, 157.67, 151.86, 145.65, 143.56, 143.49,140.29, 136.77, 132.75, 132.67, 128.52, 126.49, 123.31, 122.06, 121.10, 119.85, 119.65, 118.50, 118.26,

111.52, 56.58, 54.82, 35.82, 23.95, 14.62

$^{19}F$ NMR (400 MHz, $CD_3OD$): δ (ppm) -105.69.

I-77

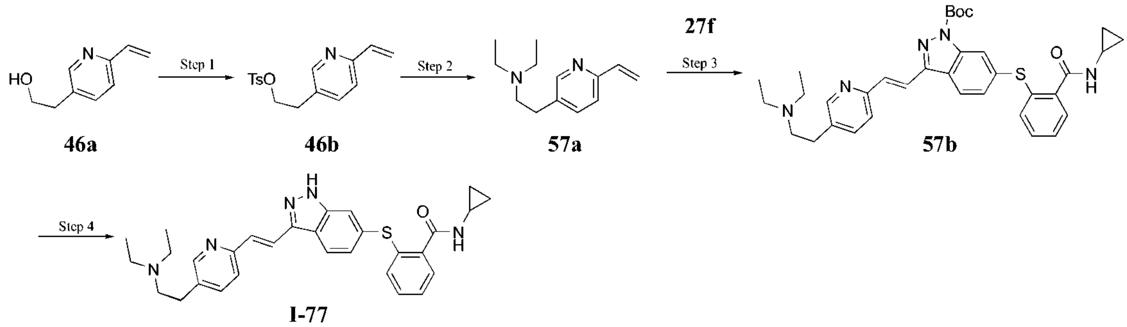


46a 46b 57a 57b

I-77

Step 1

At room temperature, 2-(6-ethenylpyridin-3-yl)ethanol 46a (1 g, 6.70 mmol) was dissolved in acetonitrile (10 mL). p-Toluenesulfonyl chloride (1.7 g, 8.71 mmol) and triethylamine (1.7 g, 16.76 mmol) were added, and the reaction system was stirred at 25°C for 16 hours. The reaction mixture was poured into water (30 mL) and extracted with dichloromethane (30 mL × 3). The combined organic phases were washed with saturated brine (40 mL), dried over anhydrous sodium sulfate, and concentrated under reduced pressure to afford the crude product. The crude product was purified by preparative thin-layer chromatography (petroleum ether: ethyl acetate = 3/1) to give the brown oily product 2-(6-ethenylpyridin-3-yl)ethyl 4-methylbenzenesulfonate 46b (500 mg, 1.65 mmol, 24.59% yield).

The product was confirmed by LCMS and H-NMR.

MS-ESI calculated for $[M+H]^+$ 304.1, found 304.1.

$^1H$ NMR (400MHz, $CDCl_3$): δ (ppm) 8.30 (d, *J* = 2.0 Hz, 1H), 7.66 (d, *J* = 8.4 Hz, 2H), 7.42 (dd, *J* = 8.0, 2.4 Hz, 1H), 7.26 (dd, *J* = 9.4, 4.6 Hz, 3H), 6.78 (dd, *J* = 17.6, 10.8 Hz, 1H), 6.16 (dd, *J* = 17.6, 1.2 Hz, 1H), 5.48 (dd, *J* = 10.8, 1.2 Hz, 1H), 4.22 (t, *J* = 6.6 Hz, 2H), 2.96 (s, 2H), 2.42 (s, 3H)..

Step 2

At room temperature, 2-(6-ethenylpyridin-3-yl)ethyl 4-methylbenzenesulfonate 46b (400 mg, 1.32 mmol) was dissolved in acetonitrile (10 mL). Diethylamine (482 mg, 6.59 mmol) was added, and the reaction system was stirred at 55°C for 16 hours. The reaction mixture was poured into water (20 mL) and extracted with ethyl acetate (20 mL x 3). The combined organic phases were washed with saturated brine (20 mL), dried over anhydrous sodium sulfate, and concentrated under reduced pressure to give the brown oily product N,N-diethyl-2-(6-ethenylpyridin-3-yl)ethanamine 57a (200 mg, 978.90 μmol, 74.24% yield).

The product was confirmed by LCMS and H-NMR.

MS-ESI calculated for $[M+H]^+$ 205.2, found 205.1.

$^1H$ NMR (400MHz, $CDCl_3$): δ (ppm) 8.40 (d, *J* = 2.2 Hz, 1H), 7.77 (d, *J* = 8.2 Hz, 2H), 6.79 (dd, *J* = 17.6, 10.8 Hz, 1H), 6.16 (dd, *J* = 17.6, 1.2 Hz, 1H), 5.47 (dd, *J* = 10.8, 1.2 Hz, 1H), 3.06-2.98 (m, 1H), 1.28-1.23 (m, 6H).

Step 3

At room temperature, N,N-diethyl-2-(6-ethenylpyridin-3-yl)ethanamine 57a (191 mg, 933.89 μmol) was dissolved in 1,4-dioxane (10 mL). tert-Butyl 6-((2-(cyclopropylcarbamoyl)phenyl)thio)-3-iodo-1H-indazole-1-carboxylate 27f (500 mg, 933.89 μmol), tris(2-methylphenyl)phosphine (284 mg, 933.89 μmol), palladium(II) chloride (83 mg, 466.94 μmol), and triethylamine (473 mg, 4.67 mmol) were added. The reaction system was stirred at 95°C under nitrogen protection for 2 hours. The reaction mixture was poured into water (30 mL) and extracted with ethyl acetate (30 mL x 3). The combined organic phases were washed with saturated brine (30 mL), dried over anhydrous sodium sulfate, and concentrated under reduced pressure to give the brown solid crude product 6-[2-(cyclopropylcarbamoyl)phenyl]sulfanyl-3-[(E)-2-{5-[2-(diethylamino)ethyl]pyridin-2-yl}ethenyl]-1H-indazole-1-carboxylic acid tert-butyl ester 57b (200 mg, 326.91 μmol, 35.00% yield).

The product was confirmed by LCMS.

MS-ESI calculated for $[M+H]^+$ 612.3, found 612.2.

Step 4

At room temperature, 6-[2-(cyclopropylcarbamoyl)phenyl]sulfanyl-3-[(E)-2-{5-[2-(diethylamino)ethyl]pyridin-2-yl}ethenyl]-1H-indazole-1-carboxylic acid tert-butyl ester 57b (150 mg, 245.18 μmol) was dissolved in dichloromethane (9 mL). Trifluoroacetic acid (3 mL) was added, and the reaction mixture was stirred at 25°C for 2 hours. The reaction mixture was poured into water (30 mL). The pH was adjusted to 9 with a saturated sodium carbonate solution, then extracted with dichloromethane (20 mL x 3). The combined organic phases were washed with saturated brine (30 mL), dried over anhydrous sodium sulfate, and concentrated under reduced pressure to afford the crude product. The crude product was purified by preparative high-performance liquid chromatography to give the yellow solid product N-cyclopropyl-2-({3-[(E)-2-{5-[2-(diethylamino)ethyl]pyridin-2-yl}ethenyl]-1H-indazol-6-yl}sulfanyl)benzamide I-77 (16 mg, 31.27 μmol, 12.75% yield).

The product was confirmed by LCMS, H-NMR, and C-NMR.

MS-ESI calculated for $[M+H]^+$ 512.2, found 512.2.

$^1H$ NMR (400MHz, $CD_3OD$): δ (ppm) 8.52 (s, 1H), 8.34 (s, 2H), 8.05 (d, *J* = 8.4 Hz, 1H), 7.84 (dd, *J* = 19.6, 12.4 Hz, 2H), 7.67 (d, *J* = 8.2 Hz, 1H), 7.60-7.45 (m, 3H), 7.39-7.17 (m, 4H), 3.41 (dd, *J* = 10.4, 6.6 Hz, 2H), 3.35 (d, *J* = 7.4 Hz, 2H), 3.32-3.25 (m, 2H), 3.12 (dd, *J* = 10.4, 6.4 Hz, 2H), 2.85-2.74 (m, 1H), 1.38 (t, *J* = 7.2 Hz, 6H), 0.83-0.69 (m, 2H), 0.61-0.49 (m, 2H).

$^{13}C$ NMR (100 MHz, $CD_3OD$) δ (ppm) 172.68, 167.46, 155.86, 150.62, 143.75, 143.62, 139.29, 139.02, 135.80, 133.27, 132.57, 131.72, 130.22, 129.17, 128.28, 126.40, 125.43, 123.28, 122.49, 121.57, 114.85, 53.42, 28.11, 23.82, 9.16, 6.50.

I-81

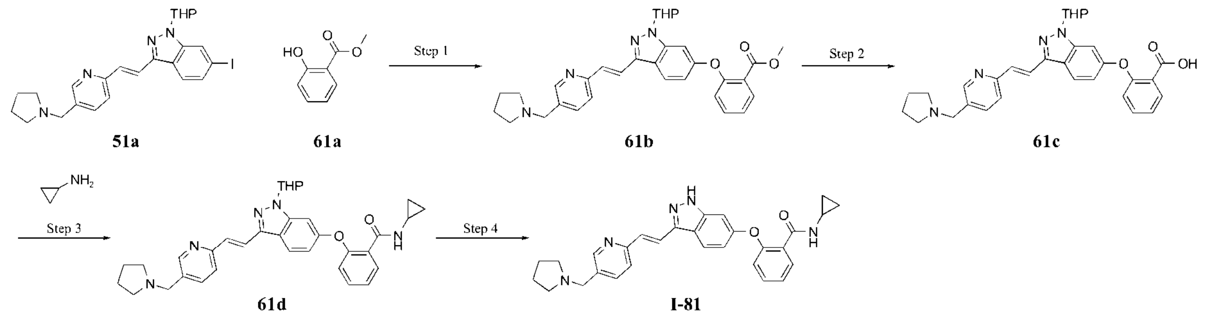


51a 61a 61b 61c 61d I-81

Step 1

At room temperature, 6-iodo-3-[(E)-2-{5-[(pyrrolidin-1-yl)methyl]pyridin-2-yl}ethenyl]-1-(tetrahydro-2H-pyran-2-yl)-1H-indazole 51a (200 mg, 388.80 μmol) was dissolved in dimethyl sulfoxide (5 mL). Methyl 2-hydroxybenzoate 61a (88.7 mg, 583.20 μmol), picolinic acid (47.9 mg, 388.80 μmol), potassium phosphate (247.6 mg, 1.17 mmol), and copper(I) iodide (37.0 mg, 194.40 μmol) were added. The reaction system was stirred at 100°C for 16 hours under nitrogen protection. The reaction mixture was poured into water (20 mL) and extracted with ethyl acetate (10 mL x 3). The combined organic phases were washed with saturated brine (30 mL), dried over anhydrous sodium sulfate, and concentrated under reduced pressure to afford the crude product. The crude product was purified by preparative thin-layer chromatography (DCM/MeOH = 10/1) to give the yellow solid product methyl 2-({3-[(E)-2-{5-[(pyrrolidin-1-yl)methyl]pyridin-2-yl}ethenyl]-1-(tetrahydro-2H-pyran-2-yl)-1H-indazol-6-yl}oxy)benzoate 61b (200 mg, 371.31 μmol, 95.50% yield).

The product was confirmed by LCMS.

MS-ESI calculated for $[M+H]^+$ 539.3, found 539.1.

Step 2

Methyl 2-({3-[(E)-2-{5-[(pyrrolidin-1-yl)methyl]pyridin-2-yl}ethenyl]-1-(tetrahydro-2H-pyran-2-yl)-1H-indazol-6-yl}oxy)benzoate 61b (200 mg, 371.31 μmol) was dissolved in tetrahydrofuran (5 mL) and water (1 mL). Sodium hydroxide (74.3 mg, 1.86 mmol) was added to the above mixture, and the mixture was stirred at 60°C for 3 hours. Water (20 mL) was added to the reaction mixture. The mixture was adjusted to pH 6-7 with dilute hydrochloric acid (3 N) and then extracted

with ethyl acetate (20 mL x 3). The combined organic phases were washed with saturated brine (20 mL), dried over anhydrous sodium sulfate, and concentrated under reduced pressure to afford the brown solid crude product. The crude product was purified by preparative thin-layer chromatography (DCM/MeOH = 10/1) to give the yellow solid product 2-({3-[(E)-2-{5-[(pyrrolidin-1-yl)methyl]pyridin-2-yl}ethenyl]-1-(tetrahydro-2H-pyran-2-yl)-1H-indazol-6-yl}oxy)benzoic acid 61c (30 mg, 57.19 μmol, 12.32% yield).

The product was confirmed by LCMS.

MS-ESI calculated for $[M+H]^+$ 525.2, found 525.2.

Step 3

At room temperature, 2-({3-[(E)-2-{5-[(pyrrolidin-1-yl)methyl]pyridin-2-yl}ethenyl]-1-(tetrahydro-2H-pyran-2-yl)-1H-indazol-6-yl}oxy)benzoic acid 61c (20 mg, 38.12 μmol) was dissolved in N,N-dimethylformamide (2 mL). Cyclopropylamine (2.2 mg, 38.12 μmol), HATU (21.7 mg, 57.19 μmol), and N,N-diisopropylethylamine (14.8 mg, 114.37 μmol) were added. The reaction system was stirred at 25°C for 2 hours. The reaction mixture was poured into water (10 mL) and extracted with ethyl acetate (10 mL x 3). The combined organic phases were washed with saturated brine (20 mL), dried over anhydrous sodium sulfate, and concentrated under reduced pressure to afford the crude product N-cyclopropyl-2-({3-[(E)-2-{5-[(pyrrolidin-1-yl)methyl]pyridin-2-yl}ethenyl]-1-(tetrahydro-2H-pyran-2-yl)-1H-indazol-6-yl}oxy)benzamide 61d (20 mg, 35.48 μmol, 93.07% yield).

The product was confirmed by LCMS.

MS-ESI calculated for [M+H]+ 564.3, found 564.2.

Step 4

N-Cyclopropyl-2-({3-[(E)-2-{5-[(pyrrolidin-1-yl)methyl]pyridin-2-yl}ethenyl]-1-(tetrahydro-2H-pyran-2-yl)-1H-indazol-6-yl}oxy)benzamide 61d (20 mg, 35.48 μmol) was dissolved in trifluoroacetic acid (2 mL) and dichloromethane (6 mL). The reaction system was stirred at 25°C for 16 hours. The reaction mixture was poured into water (10 mL). The pH was adjusted to 8 with saturated sodium carbonate solution and extracted with dichloromethane (10 mL x 3). The combined organic phases were washed with saturated brine (50 mL), dried over anhydrous sodium sulfate, and concentrated under reduced pressure to afford the crude product. The crude product was purified by preparative high-performance liquid chromatography to give the white solid product N-cyclopropyl-2-({3-[(E)-2-{5-[(pyrrolidin-1-yl)methyl]pyridin-2-yl}ethenyl]-1H-indazol-6-yl}oxy)benzamide I-81 (8.73 mg, 18.20 μmol, 51.31% yield).

The product was confirmed by LCMS, H-NMR, and C-NMR.

MS-ESI calculated for $[M+H]^+$ 479.2, found 480.3.

$^1$H NMR (400MHz, $CD_3OD$): δ (ppm) 8.52 (s, 1H), 8.10 (d, J= 9.2 Hz, 1H), 7.88-7.84 (m, 2H), 7.76 (d, J= 7.6 Hz, 1H), 7.69 (d, J= 7.6 Hz, 1H), 7.59-7.49 (m, 2H), 7.30 (t, J= 7.6 Hz, 1H), 7.07 (d, J= 8.4 Hz, 1H), 7.01 (d, J= 8.8 Hz, 1H), 6.93 (s, 1H), 3.71 (s, 2H), 2.73-2.70 (m, 1H), 2.60 (s, 4H), 1.84 (s, 4H), 0.71-0.66 (m, 2H), 0.39-0.35 (m, 2H). $^{13}$C NMR (100 MHz, $CD_3OD$) δ (ppm) 170.02, 158.30, 156.01, 155.08, 151.03, 143.86, 139.56, 134.34, 133.58, 131.42, 130.43, 128.86, 125.61, 123.25, 122.88, 121.57, 118.89, 115.61, 99.01, 58.19, 54.93, 24.21, 23.75, 6.69.

I-82

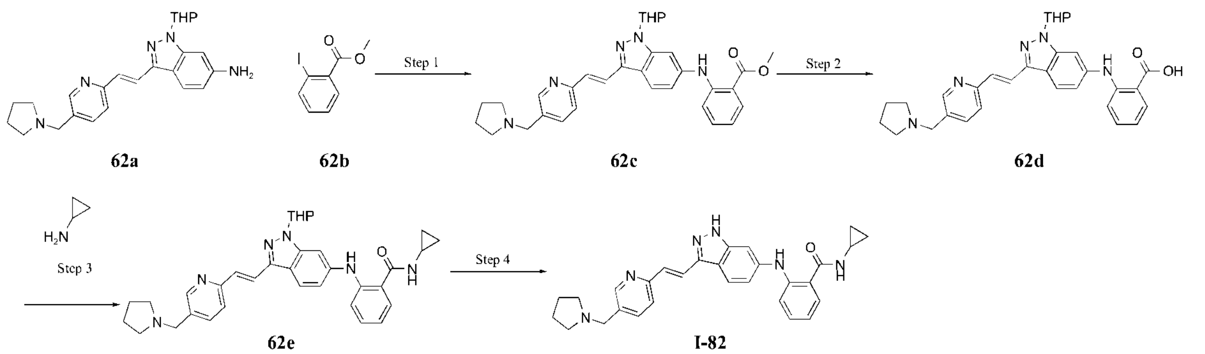


62a 62b 62c 62d 62e I-82

Step 1

At room temperature, 3-[(E)-2-{5-[(pyrrolidin-1-yl)methyl]pyridin-2-yl}ethenyl]-1-(tetrahydro-2H-pyran-2-yl)-1H-indazol-6-amine 62a (400 mg, 991.28 μmol) was dissolved in toluene (8 mL). Methyl 2-iodobenzoate 62b (260 mg, 991.28 μmol), tris(dibenzylideneacetone)dipalladium(0) (91 mg, 99.13 μmol), 4,5-bis(diphenylphosphino)-9,9-dimethylxanthene (573 mg, 991.28 μmol), and cesium carbonate (970 mg, 2.97 mmol) were added. The reaction system was stirred at 100°C for 6 hours under nitrogen protection. The reaction mixture was poured into water (50 mL) and extracted with ethyl acetate (50 mL x 3). The combined organic phases were washed with saturated brine (50 mL), dried over anhydrous sodium sulfate, and concentrated under reduced pressure to afford the crude product. The crude product was purified by medium-pressure preparative chromatography to give the yellow solid product methyl 2-({3-[(E)-2-{5-[(pyrrolidin-1-yl)methyl]pyridin-2-yl}ethenyl]-1-(tetrahydro-2H-pyran-2-yl)-1H-indazol-6-yl}amino)benzoate 62c (500 mg, 929.97 μmol, 93.82% yield).

The product was confirmed by LCMS and H-NMR.

MS-ESI calculated for $[M+H]^+$ 538.3, found 538.4.

$^1$H NMR (400 MHz, $CDCl_3$): δ (ppm) 9.66 (s, 1H), 8.55 (d, *J* = 1.8 Hz, 1H), 7.99 (dd, *J* = 8.4, 3.6 Hz, 2H), 7.85 (d, *J* = 16.5 Hz, 1H), 7.74-7.66 (m, 1H), 7.57 (d, *J* = 16.6 Hz, 1H), 7.48 (d, *J* = 8.0 Hz, 1H), 7.41 (d, *J* = 1.4 Hz, 1H), 7.36 (d, *J* = 3.4 Hz, 2H), 6.77-6.81 (m, 1H), 5.65 (dd, *J* = 9.4, 2.6 Hz, 1H), 4.06 (d, *J* = 10.4 Hz, 1H), 3.93 (s, 3H), 3.77-3.70 (m, 1H), 3.66 (s, 2H), 2.56 (s, 4H), 2.21-2.05 (m, 2H), 1.82 (s, 4H), 1.68 (s, 5H).

Step 2

At room temperature, methyl 2-({3-[(E)-2-{5-[(pyrrolidin-1-yl)methyl]pyridin-2-yl}ethenyl]-1-(tetrahydro-2H-pyran-2-yl)-1H-indazol-6-yl}amino)benzoate 62c (470 mg, 874.17 μmol) was dissolved in tetrahydrofuran (5 mL) and water (1 mL). Sodium hydroxide (210 mg, 5.25 mmol) was added. The reaction system was stirred at 60°C for 16 hours. The reaction mixture was poured into water (20 mL). The pH was adjusted to 6-7 with dilute hydrochloric acid (1 N) and extracted with ethyl acetate (20 mL x 3). The combined organic phases were washed with saturated brine (50 mL), dried over anhydrous sodium sulfate, and concentrated under reduced pressure to afford the brown solid product2-({3-[(E)-2-{5-[(pyrrolidin-1-yl)methyl]pyridin-2-yl}ethenyl]-1-(tetrahydro-2H-pyran-2-yl)-1H-indazol-6-yl}amino)benzoic acid 62d (400 mg, 763.91 μmol, 87.39% yield).

The product was confirmed by LCMS.

MS-ESI calculated for $[M+H]^+$ 524.3, found 524.4.

Step 3

At room temperature, 2-({3-[(E)-2-{5-[(pyrrolidin-1-yl)methyl]pyridin-2-yl}ethenyl]-1-(tetrahydro-2H-pyran-2-yl)-1H-indazol-6-yl}amino)benzoic acid 62d (180 mg, 343.76 μmol) was dissolved in N,N-dimethylformamide (6 mL). Cyclopropylamine (20 mg, 343.76 μmol), O-(7-azabenzotriazol-1-yl)-N,N,N',N'-tetramethyluronium hexafluorophosphate (196 mg, 515.64 μmol), and N,N-diisopropylethylamine (88.7 mg, 687.52 μmol) were added. The reaction system was stirred at 25°C for 2 hours. The reaction mixture was poured into water (30 mL) and extracted with ethyl acetate (30 mL x 3). The combined organic phases were washed with saturated brine (20 mL), dried over anhydrous sodium sulfate, and concentrated under reduced pressure to afford the crude product. The crude product was purified by preparative plate (dichloromethane/methanol = 10/1) to give the brown solid product N-cyclopropyl-2-({3-[(E)-2-{5-[(pyrrolidin-1-yl)methyl]pyridin-2-yl}ethenyl]-1-(tetrahydro-2H-pyran-2-yl)-1H-indazol-6-yl}amino)benzamide 62e (160 mg, 284.34 μmol, 82.72% yield).

The product was confirmed by LCMS and H-NMR.

MS-ESI calculated for $[M+H]^+$ 563.3, found 563.5.

$^1$H NMR (400MHz, $CDCl_3$): δ (ppm) 9.68 (s, 1H), 8.55 (d, *J* = 1.8 Hz, 1H), 7.96-7.71 (m, 3H), 7.64-7.28 (m, 6H), 7.15 (dd, *J* = 8.8, 1.8 Hz, 1H), 6.80 (s, 1H), 6.30 (s, 1H), 5.62 (d, *J* = 6.8 Hz, 1H), 4.06 (d, *J* = 9.6 Hz, 1H), 3.76 (s, 2H), 2.88 (s, 1H), 2.69 (s, 5H), 1.80-1.55 (m, 10H), 0.89 (d, *J* = 5.6 Hz, 2H), 0.64 (d, *J* = 2.4 Hz, 2H).

Step 4

At room temperature, N-cyclopropyl-2-({3-[(E)-2-{5-[(pyrrolidin-1-yl)methyl]pyridin-2-yl}ethenyl]-1-(tetrahydro-2H-pyran-2-yl)-1H-indazol-6-yl}amino)benzamide 62e (150 mg, 266.57 μmol) was dissolved in dichloromethane (6 mL), and trifluoroacetic acid (2 mL) was added. The reaction system was stirred at 25°C for 2 hours. The reaction mixture was poured into water (30 mL), the pH was adjusted to 8-9 with a saturated aqueous sodium carbonate solution, and then extracted with dichloromethane (30 mL x 3). The combined organic phases were washed with saturated brine (20 mL), dried over anhydrous sodium sulfate, and concentrated under reduced pressure to afford the crude product. The crude product was purified by high-pressure preparative chromatography to give the white solid product N-cyclopropyl-2-({3-[(E)-2-{5-[(pyrrolidin-1-yl)methyl]pyridin-2-yl}ethenyl]-1H-indazol-6-yl}amino)benzamide I-82 (40 mg, 83.58 μmol, 31.35% yield).

The product was confirmed by LCMS, H-NMR, and C-NMR.

MS-ESI calculated for $[M+H]^+$ 479.3, found 479.1.

$^1H$ NMR (400MHz, $CD_3OD$): δ (ppm) 8.68 (d, *J* = 1.6 Hz, 1H), 8.03-7.89 (m, 3H), 7.74 (d, *J* = 8.2 Hz, 1H), 7.61-7.52 (m, 2H), 7.45 (d, *J* = 8.2 Hz, 1H), 7.40-7.33 (m, 1H), 7.21 (d, *J* = 1.6 Hz, 1H), 7.05 (dd, *J* = 8.8, 1.6 Hz, 1H), 6.92 (t, *J* = 7.6 Hz, 1H), 4.41 (s, 2H), 3.33 (dd, *J* = 11.0, 4.2 Hz, 4H), 2.84-2.75 (m, 1H), 2.10 (t, *J* = 6.6 Hz, 4H), 0.80-0.72 (m, 2H), 0.59-0.51 (m, 2H).

$^{13}C$ NMR (100MHz, $CD_3OD$): δ (ppm) 173.19, 168.09, 158.28, 152.05, 144.85, 144.51, 143.57, 143.51, 140.42, 133.18, 130.25, 129.44, 127.24, 127.15, 123.32, 122.59, 122.22, 120.84, 118.36, 117.88, 117.83, 98.12, 56.29, 54.85, 23.89, 23.79, 6.59

I-83

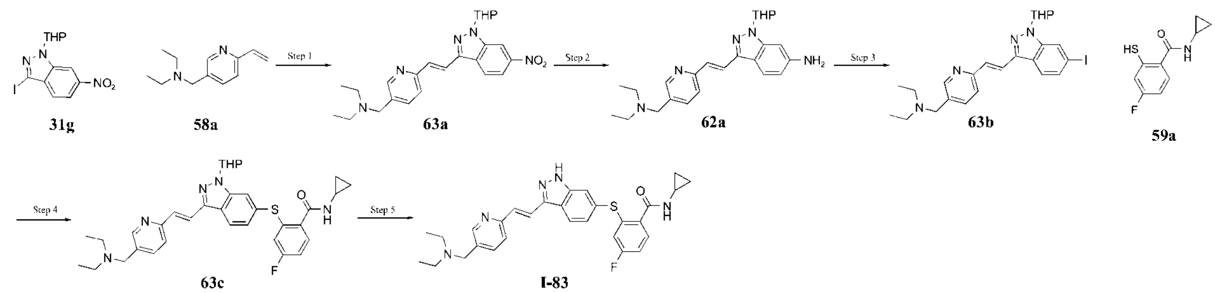


Step 1

At room temperature, 3-iodo-6-nitro-1-(tetrahydro-2H-pyran-2-yl)-1H-indazole 31g (5 g, 13.40 mmol) was dissolved in 1,4-dioxane (100 mL). N-Ethyl-N-[(6-ethenylpyridin-3-yl)methyl]ethanamine 58a (2.6 g, 13.40 mmol), tris(dibenzylideneacetone)dipalladium(0) (5 g, 5.46 mmol), tri(o-tolyl)phosphine (5 g, 16.45 mmol), and triethylamine (6.8 g, 67.00 mmol) were added. The reaction system was stirred at 95°C under nitrogen protection for 2 hours. The reaction mixture was poured into water (100 mL) and extracted with ethyl acetate (100 mL x 3). The combined organic phases were washed with saturated brine (100 mL), dried over anhydrous sodium sulfate, and concentrated under reduced pressure to afford the crude product. The crude product was purified by medium-pressure preparative chromatography to give the brown solid product N-ethyl-N-{[6-[(E)-2-(6-nitro-1-(tetrahydro-2H-pyran-2-yl)-1H-indazol-3-yl)ethenyl]pyridin-3-yl]methyl}ethanamine 63a (2.0 g, 4.59 mmol, 34.27% yield). The product was confirmed by LCMS and H-NMR.

MS-ESI calculated for $[M+H]^+$ 436.2, found 436.3.

$^1H$ NMR (400 MHz, $CDCl_3$): δ (ppm) 8.67 (d, *J* = 2.2 Hz, 1H), 8.62-8.55 (m, 2H), 8.15-8.05 (m, 2H), 7.98 (d, *J* = 16.2 Hz, 1H), 7.64-7.51 (m, 2H), 5.84 (dd, *J* = 9.2, 2.6 Hz, 1H), 4.26 (d, *J* = 16.8 Hz, 2H), 4.08 (d, *J* = 11.6 Hz, 1H), 3.90-3.78 (m, 1H), 3.28-3.14 (m, 4H), 2.57 (dt, *J* = 13.2, 6.8 Hz, 1H), 2.19 (dd, *J* = 16.4, 8.6 Hz, 2H), 1.87-1.75 (m, 3H), 1.55 (t, *J* = 7.4 Hz, 6H)

Step 2

At room temperature, N-ethyl-N-{[6-[(E)-2-(6-nitro-1-(tetrahydro-2H-pyran-2-yl)-1H-indazol-3-yl)ethenyl]pyridin-3-yl]methyl}ethanamine 63a (2 g, 4.59 mmol) was dissolved in methanol (10 mL), tetrahydrofuran (10 mL), and water (10 mL). Sodium sulfide nonahydrate (3.3 g, 13.78 mmol) was added. The reaction system was stirred at 60°C for 4 hours. The reaction mixture was poured into water (50 mL) and extracted with ethyl acetate (50 mL x 3). The combined organic phases were washed with saturated brine (50 mL), dried over anhydrous sodium sulfate, and concentrated under reduced pressure to afford the yellow solid crude product 3-[(E)-2-{5-[(diethylamino)methyl]pyridin-2-yl}ethenyl]-1-(tetrahydro-2H-pyran-2-yl)-1H-indazol-6-amine 62a (1.6 g, 3.95 mmol, 85.91% yield).

The product was confirmed by LCMS and H-NMR.

MS-ESI calculated for $[M+H]^+$ 406.3, found 406.3.

$^1H$ NMR (400MHz, $CDCl_3$):δ(ppm) 8.52 (s, 1H), 7.91-7.60 (m, 4H), 7.49 (dd, *J* = 17.2, 12.4 Hz, 2H), 6.75 (t, *J* = 6.6 Hz, 1H), 6.73-6.63 (m, 1H), 5.58 (dd, *J* = 9.4, 2.6 Hz, 1H), 3.83-3.63 (m, 2H), 3.60 (s, 2H), 2.59-2.54 (m, 4H), 2.13 (dd, *J* = 27.8, 22.2 Hz, 4H), 1.74 (dd, *J* = 21.2, 10.8 Hz, 3H), 1.07 (t, *J* = 7.2 Hz, 6H).

Step 3

At room temperature, 3-[(E)-2-{5-[(diethylamino)methyl]pyridin-2-yl}ethenyl]-1-(tetrahydro-2H-pyran-2-yl)-1H-indazol-6-amine 62a (1.9 g, 4.69 mmol) was dissolved in acetic acid (10 mL) and acetonitrile (10 mL). The mixture was cooled to 0-5°C, and an aqueous solution (5 mL) of sodium nitrite (485 mg, 7.03 mmol) was added. The reaction system was stirred at 0-5°C for 1 hour. An aqueous solution (5 mL) of sodium iodide (1.4 g, 9.37 mmol) and iodine (595 mg, 2.34 mmol) was added. The reaction system was stirred at 0-5°C for an additional 1 hour. The reaction mixture was poured into water (50 mL), the pH was adjusted to 8-9 with a saturated sodium carbonate solution, and extracted with dichloromethane (50 mL x 3). The combined organic phases were dried over anhydrous sodium sulfate and concentrated under reduced pressure to afford the crude product. The crude product was purified by medium-pressure preparative chromatography to give the brown solid product N-ethyl-N-{[6-[(E)-2-(6-iodo-1-(tetrahydro-2H-pyran-2-yl)-1H-indazol-3-yl)ethenyl]pyridin-3-yl]methyl}ethanamine 63b (1 g, 1.94 mmol, 41.33% yield).

The product was confirmed by LCMS and H-NMR.

MS-ESI calculated for $[M+H]^+$ 517.1, found 517.2.

$^1$H NMR (400MHz, $CDCl_3$): δ (ppm) 8.56 (d, *J* = 16.0 Hz, 1H), 8.05-7.93 (m, 1H), 7.88-7.64 (m, 3H), 7.57-7.44(m, 3H), 5.68 (dd, *J* = 9.4, 2.6 Hz, 1H), 4.10-3.73 (m, 3H), 3.62 (d, *J* = 7.2 Hz, 2H), 2.60-2.53 (m, 4H), 2.27-2.00 (m, 3H), 1.68 (s, 2H), 1.08 (dd, *J* = 8.8, 5.4 Hz, 6H).

Step 4

At room temperature, N-ethyl-N-{[6-[(E)-2-(6-iodo-1-(tetrahydro-2H-pyran-2-yl)-1H-indazol-3-yl)ethenyl]pyridin-3-yl]methyl}ethanamine 63b (200 mg, 387.28 μmol) was dissolved in N,N-dimethylformamide (6 mL). N-Cyclopropyl-4-fluoro-2-mercaptobenzamide 59a (82 mg, 387.28 μmol), [1,1'-bis(diphenylphosphino)ferrocene]dichloropalladium(II) (57 mg, 77.46 μmol), and cesium carbonate (253 mg, 774.57 μmol) were added. The reaction system was stirred at 80°C under nitrogen protection for 6 hours. The reaction mixture was poured into water (50 mL) and extracted with ethyl acetate (50 mL x 3). The combined organic phases were washed with saturated brine (50 mL), dried over anhydrous sodium sulfate, and concentrated under reduced pressure to afford the brown solid crude product N-cyclopropyl-4-fluoro-2-({3-[(E)-2-{5-[(diethylamino)methyl]pyridin-2-yl}ethenyl]-1-(tetrahydro-2H-pyran-2-yl)-1H-indazol-6-yl}sulfanyl) benzamide 63c (120 mg, 200.08 μmol, 51.66% yield).

The product was confirmed by LCMS.

MS-ESI calculated for $[M+H]^+$ 600.3, found 600.1.

Step 5

At room temperature, N-cyclopropyl-4-fluoro-2-({3-[(E)-2-{5-[(diethylamino)methyl]pyridin-2-yl}ethenyl]-1-(tetrahydro-2H-pyran-2-yl)-1H-indazol-6-yl}thio)benzamide 63c (120 mg, 200.08 μmol) was dissolved in dichloromethane (9 mL), and trifluoroacetic acid (3 mL) was added. The reaction mixture was stirred at 25°C for 2 hours. The reaction mixture was poured into water (30 mL), and the pH was adjusted to 8-9 with saturated sodium carbonate solution. It was then extracted with dichloromethane (20 mL x 3). The combined organic phases were washed with saturated brine (30 mL), dried over anhydrous sodium sulfate, and concentrated under reduced pressure to afford the crude product. The crude product was purified by preparative high-performance liquid chromatography to give the yellow solid product N-cyclopropyl-4-fluoro-2-({3-[(E)-2-{5-[(diethylamino)methyl]pyridin-2-yl}ethenyl]-1H-indazol-6-yl}thio)benzamide I-83 (12 mg, 23.27 μmol, 11.63% yield).

The product was confirmed by LCMS, F-NMR, H-NMR, and C-NMR.

MS-ESI calculated for $[M+H]^+$ 516.2, found 516.3.

$^1$H NMR (400MHz, $CD_3OD$): δ (ppm) 8.68 (s, 1H), 8.43 (s, 1H), 8.15 (d, *J* = 8.6 Hz, 1H), 7.99 (t, *J* = 10.2 Hz, 2H), 7.80-7.69 (m, 2H), 7.61 (d, *J* = 16.4 Hz, 1H), 7.50 (dd, *J* = 8.6, 5.8 Hz, 1H), 7.27 (dd, *J* = 8.6, 1.2 Hz, 1H), 6.99 (d, *J* = 2.4 Hz, 1H), 6.75 (dd, *J* = 9.8, 2.4 Hz, 1H), 4.35 (s, 2H), 3.18 (q, *J* = 7.2 Hz, 4H), 2.81 (s, 1H), 1.35 (t, *J* = 7.2 Hz, 6H), 0.83-0.73 (m, 2H), 0.59 (dd, *J*= 3.8, 2.2 Hz, 2H).

$^{13}$C NMR (100 MHz, $CD_3OD$) δ (ppm) 171.66, 166.17, 163.68, 157.97, 152.50, 143.62, 140.92, 133.64, 129.93, 126.60, 123.48, 122.23, 117.84, 117.60, 116.93, 114.34, 114.12, 54.38, 23.89, 9.31, 6.53

$^{19}$F NMR (400 MHz, $CD_3OD$) δ (ppm) -111.39

I-84

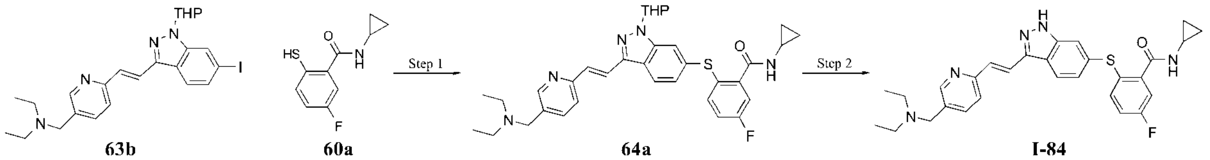


63b 60a 64a I-84

Step 1

At room temperature, N-ethyl-N-{[6-[(E)-2-(6-iodo-1-(tetrahydro-2H-pyran-2-yl)-1H-indazol-3-yl)ethenyl]pyridin-3-yl]methyl}ethanamine 63b (200 mg, 387.28 μmol) was dissolved in N,N-dimethylformamide (8 mL). N-Cyclopropyl-5-fluoro-2-mercaptobenzamide 60a (82 mg, 387.28 μmol), [1,1'-bis(diphenylphosphino)ferrocene]dichloropalladium(II) (100 mg, 136.80 μmol), and cesium carbonate (126 mg, 387.28 μmol) were added. The reaction system was stirred at 80°C under nitrogen protection for 6 hours. The reaction mixture was poured into water (30 mL) and extracted with ethyl acetate (30 mL x 3). The combined organic phases were washed with saturated brine (30 mL), dried over anhydrous sodium sulfate, and concentrated under reduced pressure to afford the brown solid crude product N-cyclopropyl-5-fluoro-2-({3-[(E)-2-{5-[(diethylamino)methyl]pyridin-2-yl}ethenyl]-1-(tetrahydro-2H-pyran-2-yl)-1H-indazol-6-yl}thio)benzamide 64a (100 mg, 166.73 μmol, 43.05% yield).

The product was confirmed by LCMS.

MS-ESI calculated for $[M+H]^+$ 600.3, found 600.3.

Step 2

At room temperature, N-cyclopropyl-5-fluoro-2-({3-[(E)-2-{5-[(diethylamino)methyl]pyridin-2-yl}ethenyl]-1-(tetrahydro-2H-pyran-2-yl)-1H-indazol-6-yl}thio)benzamide 64a (100 mg, 166.73 μmol) was dissolved in dichloromethane (9 mL) and trifluoroacetic acid (3 mL). The reaction system was stirred at 25°C for 2 hours. The reaction mixture was poured into water (30 mL), and the pH was adjusted to 8-9 with saturated sodium carbonate solution. It was extracted with dichloromethane (20 mL x 3). The combined organic phases were washed with saturated brine (30 mL), dried over anhydrous sodium sulfate, and concentrated under reduced pressure to afford the crude product. The crude product was purified by preparative high-performance liquid chromatography to give the brown solid product N-cyclopropyl-5-fluoro-2-({3-[(E)-2-{5-[(diethylamino)methyl]pyridin-2-yl} ethenyl]-1H-indazol-6-yl}thio)benzamide I-84 (18 mg, 34.91 μmol, 20.94% yield).

The product was confirmed by LCMS, F-NMR, H-NMR, and C-NMR.

MS-ESI calculated for $[M+H]^+$ 516.2, found 516.0.

$^1H$ NMR (400MHz, $CD_3OD$): δ (ppm) 8.63 (s, 1H), 8.51 (s, 1H), 8.04 (d, *J* = 8.6 Hz, 1H), 7.97-7.90 (m, 2H), 7.72 (d, *J* = 8.0 Hz, 1H), 7.60-7.46 (m, 2H), 7.38 (dd, *J* = 8.8, 5.2 Hz, 1H), 7.25 (dd, *J* = 8.6, 2.8 Hz, 1H), 7.18-7.14 (m, 2H), 4.18 (s, 2H), 3.04 (q, *J* = 7.2 Hz, 4H), 2.85-2.70 (m, 1H), 1.29 (t, *J* = 7.2 Hz, 6H), 0.74-0.71 (m, 2H), 0.58-0.47 (m, 2H).

$^{13}C$ NMR (100 MHz, $CD_3OD$) δ (ppm) 171.18, 162.04, 157.48, 152.17, 142.18, 140.62, 136.78, 136.70, 136.48, 129.94, 126.35, 125.69, 123.29, 122.47, 121.48, 118.84, 118.62, 116.42, 116.18, 113.86, 54.60, 23.79, 9.84, 6.45 $^{19}F$ NMR (400 MHz, $CD_3OD$) δ (ppm) -115.35.

I-86

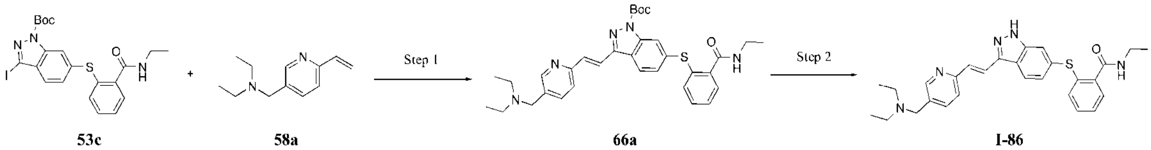


53c 58a 66a I-86

Step 1

At room temperature under nitrogen protection, to a solution of tert-butyl 6-{[2-(ethylcarbamoyl)phenyl]sulfanyl}-3-iodo-1H-indazole-1-carboxylate 53c (400 mg, 764.25 μmol) in 1,4-dioxane (5 mL) was added N-ethyl-N-[(6-ethenylpyridin-3-yl)methyl]ethanamine 58a (145.4 mg, 764.25 μmol), $PdCl_2$ (67.6 mg, 382.13 μmol), $P(o\text{-}tol)_3$ (232.3 mg, 764.25 μmol), and triethylamine (386.7 mg, 3.82 mmol). The mixture was heated to 95°C and stirred for about 2 hours.

After thin-layer chromatography (dichloromethane/methanol = 10/1) indicated complete consumption of the starting material, the mixture was poured into water (10 mL) and extracted with ethyl acetate (10 mL x 3). The combined organic layers were washed with saturated brine (20 mL), dried over anhydrous sodium sulfate, and concentrated to afford the brownish-yellow solid crude product 3-[(E)-2-[5-(diethylaminomethyl)pyridin-2-yl]ethenyl]-6-{2-(ethylcarbamoyl)phenylthio}-1H-indazole-1-carboxylate salt 66a

(200 mg, 341.44 μmol, 44.68% yield).

The product was confirmed by LCMS.

Step 2

At room temperature, to a solution of 3-[(E)-2-[5-(diethylaminomethyl)pyridin-2-yl]ethenyl]-6-{[2-(ethylcarbamoyl)phenyl]sulfanyl}-1H-indazole-1-carboxylate salt 66a (150 mg, 256.08 μmol) in dichloromethane (9 mL) was added trifluoroacetic acid (3 mL), and the mixture was stirred for about 2 hours. After LC-MS indicated complete consumption of the starting material, the mixture was poured into water (30 mL), and the pH was adjusted to 8-9 with sodium carbonate solution. It was extracted with dichloromethane (20 mL x 3). The combined organic layers were washed with saturated brine (30 mL), dried over anhydrous sodium sulfate, and concentrated to afford the crude product. The crude product was purified by preparative thin-layer chromatography to give the yellow solid 2-({3-[(E)-2-{5-[(diethylamino)methyl]pyridin-2-yl}ethenyl]-1H-indazol-6-yl}thio)-N-ethylbenzamide I-86 (15 mg, 30.89 μmol, 12.06% yield).

The product was confirmed by LCMS, H-NMR, and C-NMR.

MS-ESI calculated for $[M+H]^+$ 486.2, found 486.1.

$^1$H NMR (400MHz, $CD_3OD$): δ (ppm) 8.50 (d, J = 1.8 Hz, 1H), 8.06 (d, J = 8.4 Hz, 1H), 7.90 -7.77 (m, 2H), 7.67 (d, J = 8.2 Hz, 1H), 7.62-7.46 (m, 3H), 7.39-7.30 (m, 2H), 7.28-7.17 (m, 2H), 3.65 (s, 2H), 3.40-3.34 (m, 2H), 2.61-2.55 (m, 4H), 1.18 (t, J = 7.4 Hz, 3H), 1.10 (t, J = 7.2 Hz, 6H).

$^{13}$C NMR (100 MHz, $CD_3OD$) δ (ppm) 171.03, 155.69, 151.06, 143.82, 143.63, 139.64, 139.48, 135.95, 135.72, 134.93, 133.11, 130.48, 128.18, 126.54, 125.11, 122.83, 122.52, 121.61, 115.03, 55.37, 35.80, 14.70, 11.67.

I-87

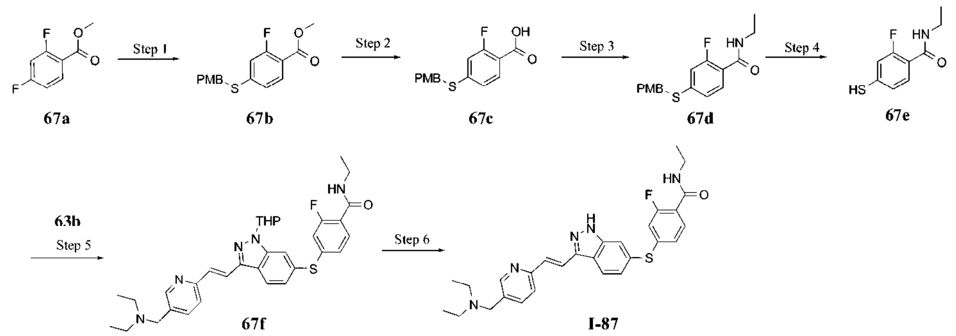


Step 1

At room temperature, methyl 2,4-difluorobenzoate 67a (10.0 g, 58.10 mmol) was dissolved in N,N-dimethylformamide (100 mL). (4-Methoxyphenyl)methanethiol (9.0 g, 58.10 mmol) and cesium carbonate (37.9 g, 116.19 mmol) were added. The reaction system was stirred at 25°C for 2 hours. The reaction mixture was poured into water (200 mL) and extracted with ethyl acetate (200 mL x 3). The combined organic phases were washed with saturated brine (200 mL), dried over anhydrous sodium sulfate, and concentrated under reduced pressure to afford the yellow solid crude product methyl 2-fluoro-4-{[(4-methoxyphenyl)methyl]sulfanyl}benzoate 67b (15.0 g, 48.96 mmol, 84.28% yield).

The product was confirmed by LCMS and H-NMR.

MS-ESI calculated for $[M+H]^+$ 307.1, found 307.0.

$^1$H NMR (400 MHz, $CDCl_3$): δ (ppm)7.80 (t, J = 8.0 Hz, 1H), 7.28 (s, 1H), 7.03 (dd, J = 18.8, 8.4 Hz, 2H), 6.84 (d, J = 8.4 Hz, 3H), 4.14 (s, 2H), 3.89 (s, 3H), 3.78 (s, 3H).

Step 2

At room temperature, methyl 2-fluoro-4-{[(4-methoxyphenyl)methyl]sulfanyl}benzoate 67b (18.0 g, 58.76 mmol)

was dissolved in tetrahydrofuran (200 mL) and water (50 mL). Lithium hydroxide (4.2 g, 176.28 mmol) was added. The reaction system was stirred at 50°C for 5 hours. The reaction mixture was concentrated under reduced pressure to afford the crude product. Water (300 mL) was added, and dilute hydrochloric acid solution (2 N) was slowly added dropwise to adjust the pH to 3. The mixture was filtered, and the filter cake was collected to give the white solid product 2-fluoro-4-{[(4-methoxyphenyl)methyl]sulfanyl}benzoic acid 67c (15.0 g, 51.31 mmol, 87.33% yield).

The product was confirmed by LCMS and H-NMR.

MS-ESI calculated for $[M+H]^+$ 293.1, found 293.0.

$^1$H NMR (400MHz, $CDCl_3$): δ (ppm)7.88 (t, J = 8.0 Hz, 1H), 7.29 (d, J = 8.4 Hz, 1H), 7.17 (d, J = 8.0 Hz, 1H), 7.07 (dd, J = 20.0, 8.4 Hz, 2H), 6.87-6.84 (m, 3H), 4.18 (s, 2H), 3.80 (s, 3H).

Step 3

At room temperature, 2-fluoro-4-{[(4-methoxyphenyl)methyl]sulfanyl}benzoic acid 67c (10.0 g, 34.21 mmol) was dissolved in N,N-dimethylformamide (100 mL). EDCI (13.1 g, 68.42 mmol), HOBt (9.2 g, 68.42 mmol), N,N-diisopropylethylamine (22.1 g, 171.04 mmol), and ethylamine hydrochloride (4.2 g, 51.31 mmol) were added. The reaction system was stirred at 25°C for 3 hours. The reaction mixture was poured into water (200 mL) and extracted with ethyl acetate (200 mL x 3). The combined organic phases were washed with saturated brine (300 mL), dried over anhydrous sodium sulfate, and concentrated under reduced pressure to afford the crude product. The crude product was purified by medium-pressure preparative chromatography to give the yellow solid product N-ethyl-2-fluoro-4-{[(4-methoxyphenyl)methyl]sulfanyl}benzamide 67d (7 g, 21.92 mmol, 64.07% yield).

The product was confirmed by LCMS and H-NMR.

MS-ESI calculated for $[M+H]^+$320.1, found 320.1.

$^1$H NMR (400MHz, $CDCl_3$): δ (ppm) 7.99 (t, J = 8.4 Hz, 1H), 7.28 (s, 1H), 7.12 (d, J = 8.4 Hz, 1H), 6.97 (d, J = 12.8 Hz, 1H), 6.91-6.77 (m, 3H), 6.63 (s, 1H), 4.15 (s, 2H), 3.79 (s, 3H), 3.53-3.46 (m, 2H), 1.24 (t, J = 7.2 Hz, 3H).

Step 4

At room temperature, N-ethyl-2-fluoro-4-{[(4-methoxyphenyl)methyl]sulfanyl} benzamide 67d (7.0 g, 21.92 mmol) was dissolved in trifluoroacetic acid (40 mL). The reaction system was stirred at 70°C for 6 hours. The reaction mixture was concentrated under reduced pressure to afford the crude product. The crude product was purified by medium-pressure preparative chromatography to give the yellow solid product N-ethyl-2-fluoro-4-aminobenzamide 67e (3 g, 15.06 mmol, 68.70% yield).

The product was confirmed by LCMS and H-NMR.

MS-ESI calculated for $[M+H]^+$200.1, found 200.0.

$^1$H NMR (400MHz, $CDCl_3$): δ (ppm) 7.97 (t, J = 8.4 Hz, 1H), 7.10 (d, J = 8.4 Hz, 1H), 7.00 (d, J = 12.4 Hz, 1H), 6.62 (s, 1H), 3.64 (s, 1H), 3.54-3.47 (m, 2H), 1.25 (t, J = 7.2 Hz, 3H).

Step 5

At room temperature, N-ethyl-N-{[6-[(E)-2-(6-iodo-1-tetrahydropyran-2-yl-indazol-3-yl)ethenyl]-3-pyridyl]methyl}ethanamine 63b (130 mg, 251.73 μmol) was dissolved in N,N-dimethylformamide (6 mL). N-ethyl-2-fluoro-4-aminobenzamide 67e (50 mg, 251.73 μmol), Pd(dppf)$Cl_2$ (65 mg, 89.62 μmol), and cesium carbonate (164 mg, 503.47 μmol) were added. The reaction system was stirred at 80°C for 6 hours under nitrogen protection. The reaction mixture was poured into water (50 mL) and extracted with ethyl acetate (50 mL x 3). The combined organic phases were washed with saturated brine (50 mL), dried over anhydrous sodium sulfate, and concentrated under reduced pressure to afford the crude product. The crude product was purified by preparative thin-layer chromatography (DCM/MeOH=10/1) to give the brown solid product 4-{3-[(E)-2-[5-(diethylaminomethyl)-2-pyridyl]ethenyl]-1-tetrahydropyran-2-yl-indazol-6-yl}sulfanyl-N-ethyl-2-fluorobenzamide 67f (110 mg, 187.15 μmol, 74.35% yield).

The product was confirmed by LCMS.

MS-ESI calculated for $[M+H]^+$588.3, found 588.2.

Step 6

At room temperature, 4-{3-[(E)-2-[5-(diethylaminomethyl)-2-pyridyl]ethenyl]-1-tetrahydropyran-2-yl-indazol-6-yl}sulfanyl-N-ethyl-2-fluorobenzamide 67f (90 mg, 153.13 μmol) was dissolved in dichloromethane (9 mL). Trifluoroacetic acid (3 mL) was added. The reaction mixture was stirred at 25°C for 2 hours. The reaction mixture was poured into water (30 mL). The pH was adjusted to 9 with sodium carbonate, and then the mixture was extracted with dichloromethane (50 mL x 3). The combined organic phases were washed with saturated brine (30 mL), dried over anhydrous sodium sulfate,

and concentrated under reduced pressure to afford the crude product. The crude product was purified by high-pressure preparative chromatography to give the yellow solid product 4-({3-[(E)-2-{5-[(diethylamino)methyl]pyridin-2-yl}ethenyl]-1H-indazol-6-yl}sulfanyl)-N-ethyl-2-fluorobenzamide I-87 (26.52 mg, 52.66 μmol, 34.39% yield).

The product was confirmed by LCMS, F-NMR, H-NMR, and C-NMR.

MS-ESI calculated for $[M+H]^+$ 504.2, found 504.3.

$^1$H NMR (400MHz, $CDCl_3$): δ (ppm) 8.58 (s, 1H), 8.02 (d, J = 8.4 Hz, 1H), 7.95-7.88 (m, 2H), 7.71 (d, J = 8.0 Hz, 1H), 7.65-7.60 (m, 2H), 7.44 (d, J = 8.0 Hz, 1H), 7.23 (d, J = 8.4 Hz, 1H), 6.96 (d, J = 8.4 Hz, 1H), 6.80 (d, J = 12.4 Hz, 1H), 6.70-6.67 (m, 1H), 3.62 (s, 2H), 3.52-3.45 (m, 2H), 2.57 (q, J = 7.2 Hz, 4H), 1.22 (t, J = 7.2 Hz, 3H), 1.07 (t, J = 7.2 Hz, 6H).

$^{13}$C NMR (100 MHz, $CDCl_3$) δ (ppm) 162.90, 162.87, 161.84, 159.36, 154.20, 150.04, 144.96, 144.87, 143.39, 142.12, 137.44, 133.99, 132.21, 130.26, 129.94, 126.50, 122.05, 121.73, 121.65, 118.47, 118.35, 116.08, 114.79, 114.51, 54.64, 46.72, 34.98, 14.73, 11.66

$^{19}$F (400MHz, $CDCl_3$): δ (ppm) -112.83

I-97

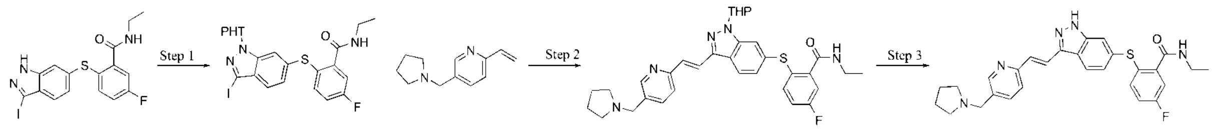


75a 75b 30a 75c I-97

Step 1

At room temperature, N-ethyl-5-fluoro-2-[(3-iodo-1H-indazol-6-yl)sulfanyl]benzamide 75a (7 g, 15.86 mmol) was dissolved in ethyl acetate (70 mL). p-Toluenesulfonic acid (546 mg, 3.17 mmol) and 3,4-dihydro-2H-pyran (2.7 g, 31.73 mmol) were added. The reaction system was stirred at 70°C for 16 hours. The reaction mixture was concentrated under reduced pressure to afford the crude product. The crude product was purified by column chromatography (petroleum ether/ethyl acetate=10/1-1/1) to give the yellow solid product N-ethyl-5-fluoro-2-(3-iodo-1-tetrahydropyran-2-yl-indazol-6-yl)sulfanylbenzamide 75b (7 g, 13.32 mmol, 83.99% yield).

The product was confirmed by LCMS and H-NMR.

MS-ESI calculated for [M+H]+ 526.0, found 525.9.

$^1$H NMR (400MHz, $CDCl_3$): δ (ppm) δ 7.49 (s, 1H), 7.43 (dd, *J* = 8.8, 2.8 Hz, 1H), 7.38 (d, *J* = 8.4 Hz, 1H), 7.32 (dd, *J* = 8.4, 5.2 Hz, 1H), 7.09-7.04 (m, 2H), 6.39 (s, 1H), 5.60 (dd, *J* = 9.6, 2.8 Hz, 1H), 3.98 (d, *J* = 12.0 Hz, 1H), 3.71-3.65 (m, 1H), 3.42-3.35 (m, 2H), 2.50-2.42 (m, 1H), 2.10-2.04 (m, 2H), 1.75-1.65 (m, 3H), 1.11 (t, *J* = 7.2 Hz, 3H).

Step 2

At room temperature, N-ethyl-5-fluoro-2-(3-iodo-1-tetrahydropyran-2-yl-indazol-6-yl)sulfanylbenzamide 75b (3.5 g, 6.66 mmol) was dissolved in 1,4-dioxane (40 mL). 5-(Pyrrolidin-1-ylmethyl)-2-ethenylpyridine 30a (1.3 g, 6.66 mmol), Xphos Pd G3 (564 mg, 666.19 μmol), and triethylamine (2 g, 19.99 mmol) were added. The reaction system was stirred at 90°C for 16 hours under nitrogen protection. The reaction mixture was poured into water (100 mL) and extracted with ethyl acetate (30 mL x 3). The combined organic phases were washed with saturated brine (50 mL), dried over anhydrous sodium sulfate, and concentrated under reduced pressure to afford the crude product. The crude product was purified by column chromatography (dichloromethane/methanol= 100/1-10/1) to give the yellow solid product N-ethyl-5-fluoro-2-{3-[(E)-2-[5-(pyrrolidin-1-ylmethyl)-2-pyridyl]ethenyl]-1-tetrahydropyran-2-yl-indazol-6-yl}sulfanylbenzamide 75c (2.1 g, 3.59 mmol, 53.82% yield). The product was confirmed by LCMS.

MS-ESI calculated for [M+H]+ 586.3, found 586.2.

Step 3

At room temperature, N-ethyl-5-fluoro-2-{3-[(E)-2-[5-(pyrrolidin-1-ylmethyl)-2-pyridyl]ethenyl]-1-tetrahydropyran-2-yl-indazol-6-yl}sulfanylbenzamide 75c (2.1 g, 3.59 mmol) was dissolved in dichloromethane (30 mL), and trifluoroacetic acid (10 mL) was added. The reaction system was stirred at 25°C for 16 hours. The reaction mixture was poured into water (50 mL), the pH was adjusted to 8 with saturated sodium carbonate, and extracted with dichloromethane (20 mL x 3). The combined organic phases were washed with saturated brine (50 mL), dried over anhydrous sodium sulfate, and concentrated under reduced pressure to afford the crude product. The crude product was purified by preparative high-performance liquid chromatography to give the yellow solid product N-ethyl-5-fluoro-2-({3-[(E)-2-{5-[(pyrrolidin-1-yl)methyl]pyridin-2-yl}ethenyl]-1H-indazol-6-yl}sulfanyl)benzamide I-97 (157 mg, 312.99 μmol, 8.73% yield).

The product was confirmed by LCMS, F-NMR, and H-NMR.

MS-ESI calculated for [M+H]+ 502.2, found 502.0.

$^1$H NMR (400MHz, $CD_3OD$): δ (ppm) 8.51 (d, *J* = 1.6 Hz, 1H), 8.04 (d, *J* =8.4 Hz, 1H), 7.87-7.80 (m, 2H), 7.66 (d, *J* =8.0 Hz, 1H), 7.57-7.50 (m, 2H), 7.38-7.35 (m, 1H), 7.25 (dd, *J* =8.8, 2.8 Hz, 1H), 7.18-7.13 (m, 2H), 3.69 (s, 2H), 3.33-3.29 (m, 2H), 2.58 (s, 4H), 1.85-1.81 (m, 4H), 1.14 (t, *J* = 7.2Hz, 3H).

$^{19}$F NMR (400MHz, $CD_3OD$): δ (ppm) -115.65

I-98

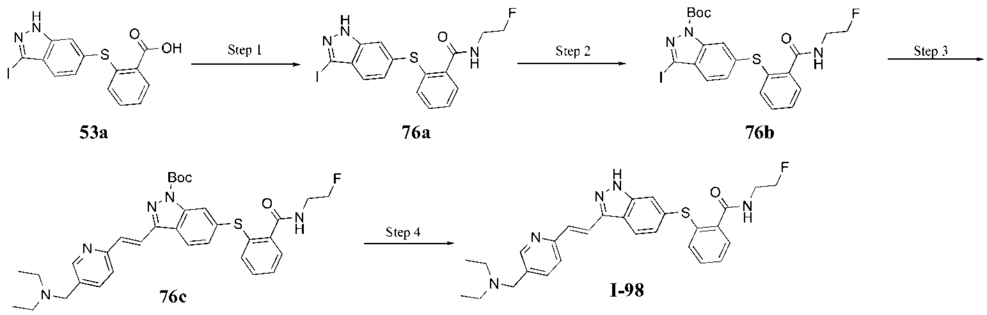


53a 76a 76b 76c I-98

Step 1

At room temperature, 2-[(3-iodo-1H-indazol-6-yl)sulfanyl]benzoic acid 53a (1.0 g, 2.52 mmol) was dissolved in N,N-dimethylformamide (10 mL), followed by addition of 2-fluoroethylamine hydrochloride (502.4 mg, 5.05 mmol), 1-hydroxybenzotriazole (511.6 mg, 3.79 mmol), 1-(3-dimethylaminopropyl)-3-ethylcarbodiimide hydrochloride (725.8 mg, 3.79 mmol), and N,N-diisopropylethylamine (1.3 g, 10.10 mmol). The reaction system was stirred for 2 hours. The mixture was poured into water (30 mL) and extracted with ethyl acetate (30 mL x 3). The combined organic phases were washed with brine (50 mL), dried over anhydrous sodium sulfate, and concentrated to afford the crude product. The crude product was purified by preparative high-performance liquid chromatography to give the white solid product N-(2-fluoroethyl)-2-((3-iodo-1H-indazol-6-yl)sulfanyl)benzamide 76a (750.0 mg, 1.70 mmol, 67.34% yield).

The product was confirmed by LCMS and H-NMR.

MS-ESI calculated for $[M+H]^+$ 442.0, found 442.1.

$^1$H NMR (400 MHz, $CD_3OD$) δ (ppm) 8.48 (s, 0H), 7.63-7.53 (m, 1H), 7.41 (dd, J = 8.4, 3.8 Hz, 1H), 7.34-7.12 (m, 1H), 7.10-6.93 (m, 0H), 4.76-4.46 (m, 2H), 3.80-3.45 (m, 2H).

Step 2

At room temperature, N-(2-fluoroethyl)-2-((3-iodo-1H-indazol-6-yl)sulfanyl)benzamide 76a (700 mg, 1.59 mmol) was dissolved in tetrahydrofuran (7 mL), followed by addition of triethylamine (642.1 mg, 6.35 mmol) and di-tert-butyl dicarbonate (692.4 mg, 3.17 mmol). The system was stirred at 60°C for 16 hours, then poured into water (20 mL) and extracted with ethyl acetate (30 mL x 3). The combined organic phases were washed with brine (20 mL), dried over anhydrous sodium sulfate, and concentrated to afford the crude product. The crude product was purified by preparative TLC(PE/EA=3/1) to give the white solid product tert-butyl 6-((2-((2-fluoroethyl)carbamoyl)phenyl)sulfanyl)-3-iodo-1H-indazole-1-carboxylate 76b (655.0 mg, 1.21 mmol, 76.27% yield).

The product was confirmed by LCMS and H-NMR.

MS-ESI calculated for $[M+H]^+$ 542.0, found 442.1.

$^1$H NMR (400MHz, $CDCl_3$): δ (ppm) 8.06 (s, 1H), 7.69 (dd, J = 4.8, 2.0 Hz, 1H), 7.61-7.47 (m, 1H), 7.42-7.35 (m, 4H), 6.72 (s, 1H), 4.57 (t, J = 4.8 Hz, 1H), 4.45 (t, J = 4.8 Hz, 1H), 3.74 (dd, J = 10.4, 5.2 Hz, 1H), 3.67 (dd, J = 10.4, 5.2 Hz, 1H), 1.63 (s, 9H).

Step 3

At room temperature, tert-butyl 6-((2-((2-fluoroethyl)carbamoyl)phenyl)sulfanyl)-3-iodo-1H-indazole-1-carboxylate 76b (500 mg, 923.57 μmol) was dissolved in dioxane (10 mL), followed by addition of N-ethyl-N-((6-ethenylpyridin-3-yl)methyl)ethanamine (175.8 mg, 923.57 μmol), tris(dibenzylideneacetone)dipalladium (422.9 mg, 461.79 μmol), tri(o-

tolyl)phosphine (421.6 mg, 1.39 mmol), and triethylamine (467.3 mg, 4.62 mmol). The system was stirred at 95°C under nitrogen protection for 2 hours. The reaction mixture was poured into water (10 mL) and extracted with ethyl acetate (10 mL x 3). The combined organic phases were washed with brine (20 mL), dried over anhydrous sodium sulfate, and concentrated to afford the yellow solid crude product tert-butyl (E)-3-(2-(5-((diethylamino)methyl)pyridin-2-yl)ethenyl)-6-((2-((2-fluoroethyl)carbamoyl)phenyl)sulfanyl)-1H-indole-1-carboxylate 76c (70 mg, 115.94 μmol, 12.55% yield).

The product was confirmed by LCMS.

MS-ESI calculated for $[M+H]^+$ 604.3, found 604.4.

Step 4

At room temperature, tert-butyl (E)-3-(2-(5-((diethylamino)methyl)pyridin-2-yl)ethenyl)-6-((2-((2-fluoroethyl) carbamoyl)phenyl)sulfanyl)-1H-indole-1-carboxylate 76c (300 mg, 496.90 μmol) was dissolved in dichloromethane (10 mL), followed by addition of trifluoroacetic acid (56.7 mg, 496.90 μmol). The system was stirred at room temperature for 2 hours. The mixture was poured into water (50 mL), the pH was adjusted to 8-9 with saturated sodium carbonate solution, and extracted with dichloromethane (10 mL x 3). The combined organic phases were washed with brine (20 mL), dried over anhydrous sodium sulfate, and concentrated to afford the crude product. The crude product was purified by preparative high-performance liquid chromatography to give the yellow solid product 2-({3-[(E)-2-{5-[(diethylamino) methyl]pyridin-2-yl}ethenyl]-1H-indazol-6-yl}sulfanyl)-N-(2-fluoroethyl)benzamide I-98 (16.57 mg, 32.90 μmol, 6.62% yield).

The product was confirmed by LCMS, F-NMR, H-NMR, and C-NMR.

MS-ESI calculated for $[M+H]^+$ 504.2, found 504.3.

$^1$H NMR (400 MHz, $CD_3OD$) δ (ppm) 8.50 (d, J = 1.8 Hz, 1H), 8.07 (d, J = 8.4 Hz, 1H), 7.86 (d, J = 16.6 Hz, 2H), 7.69 (s, 1H), 7.58 (dd, J = 11.4, 8.6 Hz, 2H), 7.52-7.49 (m, 1H), 7.34 (d, J = 1.0 Hz, 2H), 7.27-7.19 (m, 2H), 4.57 (s, 1H), 4.45 (s, 1H), 3.69-3.54 (m, 4H), 2.57 (q, J = 7.2 Hz, 4H), 1.10 (m, J = 7.2 Hz, 6H).

$^{13}$C NMR (100 MHz, $CD_3OD$): δ (ppm) 171.41, 161.443, 155.85, 151.04, 139.59, 131.81, 129.14. 126.66, 122.75, 121.84, 116.03, 83.94, 82.27, 79.66, 79.33, 79.01, 55.44, 39.28, 30.79, 11.81.

$^{19}$F NMR (400MHz, $CD_3OD$): δ (ppm) -123.62.

I-104

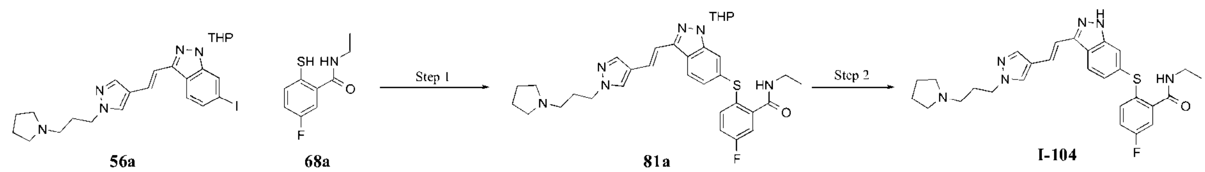


56a 68a 81a I-104

Step 1

At room temperature, 6-iodo-3-[(E)-2-[1-(3-pyrrolidin-1-ylpropyl)pyrazol-4-yl]ethenyl]-1-tetrahydropyran-2-ylindazole 56a (500 mg, 940.86 μmol) was dissolved in DMF (8 mL), followed by addition of N-ethyl-5-fluoro-2-sulfanylbenzamide 68a (187.5 mg, 940.86 μmol), cesium carbonate (613.1 mg, 1.88 mmol), and $Pd(dppf)Cl_2$ (341.4 mg, 470.43 μmol) under nitrogen protection. The reaction system was stirred at 80°C for 5.0 hours. The reaction mixture was poured into water (50 mL) and extracted with EA (50 mL x 3). The combined organic phases were washed with saturated brine (30 mL), dried over anhydrous sodium sulfate, and concentrated under reduced pressure to afford the crude product. The crude product was purified by preparative thin-layer chromatography (DCM/MeOH=10/1) to give the brown solid crude product ethyl 5-fluoro-2-({3-[(E)-2-[1-(3-pyrrolidin-1-ylpropyl)-1H-pyrazol-4-yl]ethenyl]-1-(tetrahydro-2H-pyran-2-yl)-1H-indol-6-yl} sulfanyl)benzamide 81a (400 mg, crude).

The product was confirmed by LCMS.

MS-ESI calculated for [M+H]+ 603.3, found 603.2.

Step 2

Ethyl 5-fluoro-2-({3-[(E)-2-[1-(3-pyrrolidin-1-ylpropyl)-1H-pyrazol-4-yl]ethenyl]-1-(tetrahydro-2H-pyran-2-yl)-1H-indol-6-yl}sulfanyl)benzamide 81a (350 mg, 580.66 μmol) was dissolved in DCM (12 mL), and trifluoroacetic acid (4 mL) was added. The reaction system was stirred at 25°C for 2 hours. The reaction mixture was poured into water (50 mL), adjusted to pH=8-9 with saturated aqueous sodium bicarbonate solution, extracted with DCM (50 mL x 3). The combined organic phases were washed with saturated brine (20 mL), dried over anhydrous sodium sulfate, and

concentrated under reduced pressure to afford the crude product. The crude product was purified by preparative high-performance liquid chromatography to give the brown solid product N-ethyl-5-fluoro-2-({3-[(E)-2-{1-[3-(pyrrolidin-1-yl) propyl]-1H-pyrazol-4-yl}ethenyl]-1H-indazol-6-yl}sulfanyl)benzamide I-104 (52.07 mg, 100.40 μmol, 17.3% yield).

The product was confirmed by LCMS, F-NMR, H-NMR, and C-NMR.

MS-ESI calculated for [M+H]+ 519.2, found 519.1.

$^{1}$H NMR (400MHz, $CD_3OD$): δ (ppm) 7.95 (d, J=8.8 Hz, 1H), 7.91 (s, 1H), 7.84 (s, 1H), 7.46 (s, 1H), 7.36-7.24 (m, 3H), 7.18-7.10 (m, 3H), 4.29 (t, J=6.4, 2H), 3.66 (s, 2H), 3.35-3.10 (m, 2H), 3.23 -3.19 (m, 2H), 3.06-3.03 (m, 2H), 2.33-2.26 (m, 2H), 2.13 (s, 2H), 2.02-1.99 (m, 2H), 1.14 (t, J=7.2, 3H).

$^{13}$C NMR (100MHz, $CD_3OD$) δ (ppm) 169.55, 164.43, 161.96, 161.88, 1616.51, 144.65, 143.49, 142.26, 142.19, 138.96, 136.48, 136.40, 136.20, 130.60, 130.56, 129.96, 125.31, 122.66, 122.62, 122.45, 121.00, 119.14, 118.74, 118.52, 116.32, 116.09, 113.98, 55.27, 53.72, 35.84, 27.86, 23.98, 14.60.

$^{19}$F NMR (400MHz, $CD_3OD$): δ (ppm) -77.23, -115.62.

I-110

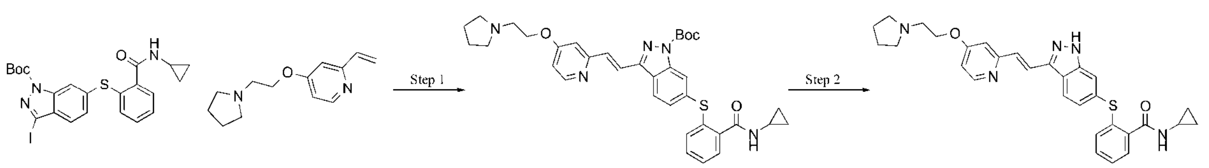


27f 23c 86a I-110

Step 1

At room temperature, tert-butyl 6-((2-(cyclopropylcarbamoyl)phenyl)thio)-3-iodo-1H-indazole-1-carboxylate 27f (1.0 g, 1.87 mmol) was dissolved in 1,4-dioxane (10 mL), followed by addition of 4-(2-pyrrolidin-1-ylethoxy)-2-ethenylpyridine 23c (489 mg, 2.24 mmol), tris(o-methylphenyl)phosphine (569 mg, 1.87 mmol), triethylamine (567 mg, 5.60 mmol), and palladium chloride (166 mg, 933.89 μmol). The reaction system was stirred at 90°C for 16 hours under nitrogen protection. The reaction mixture was poured into water (50 mL) and extracted with ethyl acetate (20 mL x 3). The combined organic phases were washed with saturated brine (50 mL), dried over anhydrous sodium sulfate, and concentrated under reduced pressure to afford the crude product. The crude product was purified by column chromatography (DCM/MeOH=10/1) to give the yellow solid product 6-[2-(cyclopropylcarbamoyl)phenyl]sulfanyl-3-[(E)-2-[4-(2-pyrrolidin-1-ylethoxy)pyridin-2-yl]ethenyl]indazole-1-carboxylic acid tert-butyl ester 86a (400 mg, 639.20 μmol, 34.22% yield).

The product was confirmed by LCMS.

MS-ESI calculated for [M+H]+ 625.3, found 626.2.

Step 2

At room temperature, 6-[2-(cyclopropylcarbamoyl)phenyl]sulfanyl-3-[(E)-2-[4-(2-pyrrolidin-1-ylethoxy)pyridin-2-yl]ethenyl]indazole-1-carboxylic acid tert-butyl ester 86a (400 mg, 639.20 μmol) was dissolved in dichloromethane (10 mL), and trifluoroacetic acid (364 mg, 3.20 mmol) was added. The reaction system was stirred at 25°C for 16 hours. The reaction mixture was poured into water (30 mL), adjusted to pH 8 with saturated sodium carbonate solution, extracted with dichloromethane (10 mL x 3). The combined organic phases were washed with saturated brine (50 mL), dried over anhydrous sodium sulfate, and concentrated under reduced pressure to afford the crude product. The crude product was purified by preparative high-performance liquid chromatography to give the yellow solid product N-cyclopropyl-2-({3-[(E)-2-{4-[2-(pyrrolidin-1-yl)ethoxy]pyridin-2-yl}ethenyl]-1H-indazol-6-yl}sulfanyl)benzamide I-110 (67.53 mg, 128.47 μmol, 20.10% yield).

The product was confirmed by LCMS, H-NMR, and C-NMR.

MS-ESI calculated for $[M+H]^+$ 526.2, found 526.1.

$^{1}$H NMR (400MHz, $CD_3OD$): δ (ppm) 8.35 (d, J=5.6 Hz, 1H), 8.06 (d, J= 8.4 Hz, 1H), 7.85 (d, J= 16.8 Hz, 1H), 7.56-7.44 (m, 3H), 7.37-7.30 (m, 2H), 7.20 (m, 2H), 7.20-7.18 (dd, J= 8.8, 1.6 Hz, 1H), 6.91 (dd, J= 5.6, 2.4 Hz, 1H), 4.29 (t, J= 5.6 Hz, 2H), 2.97 (t, J=5.6 Hz, 2H), 2.81-2.75 (m, 1H), 2.69 (s, 4H), 1.85 (s, 4H), 0.77-0.72 (m, 2H), 0.55-0.51 (m, 2H).

$^{13}$C NMR (100 MHz, $CD_3OD$) δ (ppm) 172.67, 167.42, 158.51, 151.50, 143.64, 136.31, 135.49, 133.01, 130.76, 129.19, 128.05, 126.67, 125.63, 122.62, 121.80, 115.35, 110.60, 109.51, 68.06, 55.74, 24.40, 23.94, 6.78.

I-113

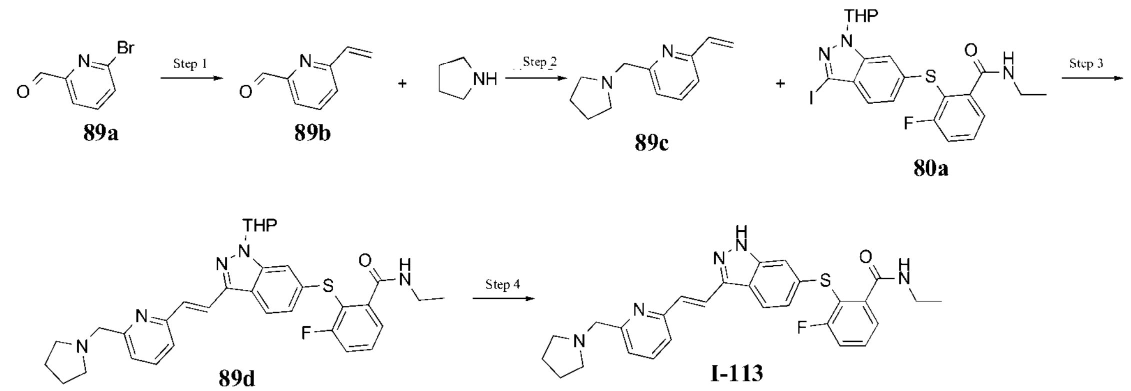


89a 89b 89c 80a 89d I-113

Step 1

At room temperature, 6-bromopyridine-2-carbaldehyde 89a (20 g, 107.52 mmol) was dissolved in N,N-dimethylformamide (200 mL), followed by addition of $PdCl_2$(dppf) (3.9 g, 5.38 mmol), potassium ethenyltrifluoroborate (21.6 g, 161.29 mmol), and triethylamine (32.6 g, 322.57 mmol). The reaction system was stirred at 90°C for 16 hours under nitrogen protection. The reaction mixture was poured into water (1000 mL) and extracted with ethyl acetate (200 mL x 3). The combined organic phases were washed with saturated brine (500 mL), dried over anhydrous sodium sulfate, and concentrated under reduced pressure to afford the crude product. The crude product was purified by column chromatography (petroleum ether/ethyl acetate = 1/0-10/1) to give the yellow oily product 6-ethenylpyridine-2-carbaldehyde 89b (6 g, 45.06 mmol, 41.91% yield).

The product was confirmed by LCMS and H-NMR.

MS-ESI calculated for [M+H]+ 134.1, found 134.2.

$^1$H NMR (400MHz, $CDCl_3$): δ (ppm) 10.08 (s, 1H), 7.83 (d, *J* = 4.4 Hz, 2H), 7.58-7.54 (m, 1H), 6.90 (dd, *J* = 17.6, 10.8 Hz, 1H), 6.35 (d, *J* =17.2 Hz, 1H), 5.61 (d, *J* = 10.8 Hz, 1H).

Step 2

At room temperature, 6-ethenylpyridine-2-carbaldehyde 89b (6 g, 45.06 mmol) was dissolved in tetrahydrofuran (250 mL), followed by addition of pyrrolidine (3.9 g, 54.08 mmol) and sodium triacetoxyborohydride (28.7 g, 135.19 mmol). The reaction system was stirred at 25°C for 16 hours. The reaction mixture was poured into water (500 mL), adjusted to pH 8-9 with aqueous sodium carbonate solution, extracted with ethyl acetate (100 mL x 3). The combined organic phases were washed with saturated brine (300 mL), dried over anhydrous sodium sulfate, and concentrated under reduced pressure to afford the crude product. The crude product was purified by column chromatography (dichloromethane/-methanol=1/0-20/1) to give the yellow oily product 2-(pyrrolidin-1-ylmethyl)-6-ethenylpyridine 89c (4 g, 21.25 mmol, 47.15% yield).

The product was confirmed by LCMS and H-NMR.

MS-ESI calculated for [M+H]+ 189.1, found 189.1.

$^1$H NMR (400MHz, $CDCl_3$): δ (ppm) 7.61 (t, *J* = 7.8 Hz, 1H), 7.31-7.24 (m, 2H), 6.83 (dd, *J* = 17.6, 10.8Hz, 1H), 6.16 (d, *J* = 17.6 Hz, 1H), 5.46 (d, *J* = 10.8 Hz, 1H), 3.81 (s, 2H), 2.63 (s, 4H), 1.85-1.78 (m, 4H).

Step 3

At room temperature, N-ethyl-3-fluoro-2-(3-iodo-1-tetrahydropyran-2-yl-indazol-6-yl)sulfanylbenzamide 80a (600 mg, 1.14 mmol) was dissolved in 1,4-dioxane (10 mL), followed by addition of 2-(pyrrolidin-1-ylmethyl)-6-ethenylpyridine 89c (258 mg, 1.37 mmol), tris(o-methylphenyl)phosphine (348 mg, 1.14 mmol), $Pd_2(dba)_3$ (523 mg, 571.02 μmol), and triethylamine (231 mg, 2.28 mmol). The reaction system was stirred at 90°C for 16 hours under nitrogen protection. The reaction mixture was poured into water (30 mL) and extracted with ethyl acetate (10 mL x 3). The combined organic phases were washed with saturated brine (30 mL), dried over anhydrous sodium sulfate, and concentrated under reduced pressure to afford the crude product. The crude product was purified by column chromatography (dichloromethane/methanol = 1/0-20/1) to give the yellow solid product N-ethyl-3-fluoro-2-{3-[(E)-2-[6-(pyrrolidin-1-ylmethyl)pyridin-2-yl]ethenyl]-1-tetrahydropyran-2-yl-indazol-6-yl} sulfanylbenzamide 89d (400 mg, 682.90 μmol, 59.80% yield).

The product was confirmed by LCMS.

MS-ESI calculated for [M+H]+ 586.3, found 586.1.

Step 4

At room temperature, N-ethyl-3-fluoro-2-{3-[(E)-2-[6-(pyrrolidin-1-ylmethyl)pyridin-2-yl]ethenyl]-1-tetrahydropyran-2-yl-indazol-6-yl}sulfanylbenzamide 89d (400 mg, 682.90 μmol) was dissolved in dichloromethane (15 mL) and trifluoroacetic acid (5 mL). The reaction system was stirred at 25°C for 16 hours. The reaction mixture was poured into water (30 mL), adjusted to pH 8 with saturated aqueous sodium carbonate solution, and extracted with dichloromethane (10 mL x 3). The combined organic phases were washed with saturated brine (50 mL), dried over anhydrous sodium sulfate, and concentrated under reduced pressure to afford the crude product. The crude product was purified by preparative high-performance liquid chromatography to give the yellow solid product N-ethyl-3-fluoro-2-({3-[(E)-2-{6-[(pyrrolidin-1-yl)methyl]pyridin-2-yl}ethenyl]-1H-indazol-6-yl}sulfanyl)benzamide I-113 (99.99 mg, 199.34 μmol, 30.03% yield).

The product was confirmed by LCMS, F-NMR, H-NMR, and C-NMR.

MS-ESI calculated for [M+H]+ 502.2, found 502.1.

$^{1}$H NMR (400MHz, $CD_3OD$): δ (ppm) 7.89 (d, *J* = 8.4 Hz, 1H), 7.78 (d, *J* = 16.4 Hz, 1H), 7.66 (t, *J* = 7.6 Hz, 1H), 7.46-7.40 (m, 3H), 7.30 (s, 1H), 7.23-7.16 (m, 3H), 7.03 (d, *J* = 8.4 Hz, 1H), 3.71 (s, 2H), 3.25-3.20 (m, 2H), 2.56 (s, 4H), 1.73 (s, 4H), 1.04 (t, *J* = 7.6 Hz, 3H).

$^{19}$F NMR (400MHz, $CD_3OD$): δ (ppm) -105.64

$^{13}$C NMR (100 MHz, $CD_3OD$) δ (ppm) 170.00, 165.45, 162.98, 159.48, 156.46, 145.61, 143.91, 143.50, 138.74, 136.63, 132.65, 132.57, 131.05, 125.28, 124.79, 124.75, 123.93, 123.29, 122,20, 121.48, 121.05, 120.00, 119.81, 118.48, 118.24, 111.62, 62.73, 55.17, 35.82, 24.30, 14.62.

I-116

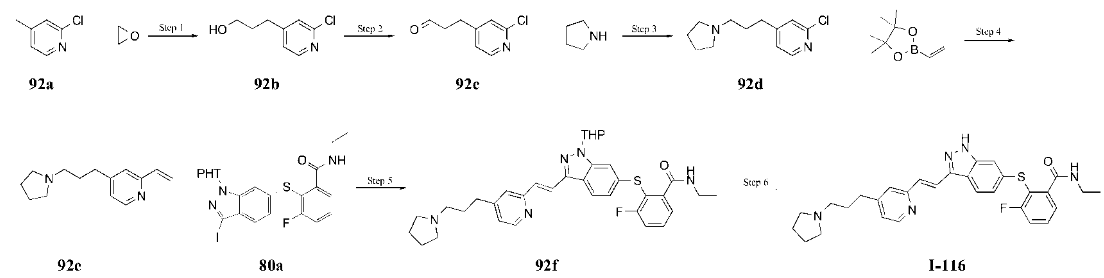


92a 92b 92c 92d

92e 80a 92f I-116

Step 1

At room temperature, 2-chloro-4-methylpyridine 92a (20 g, 156.78 mmol) was dissolved in tetrahydrofuran (200 mL), cooled to -70°C, and lithium diisopropylamide (2 mol/L, 156.78 mL, 313.55 mmol) was added. The reaction system was stirred at -70°C for 1 hour. At the same temperature, ethylene oxide (3 mol/L, 104.52 mL, 313.55 mmol) was added, and the reaction system was stirred at -70°C for 3 hours. The reaction mixture was poured into saturated aqueous ammonium chloride solution (300 mL) and extracted with ethyl acetate (300 mL x 3). The combined organic phases were washed with saturated brine (500 mL), dried over anhydrous sodium sulfate, and concentrated under reduced pressure to afford the crude product. The crude product was purified by column chromatography (petroleum ether/ethyl acetate - petroleum ether/ethyl acetate = 1/0-1/1) to give the yellow oily product 3-(2-chloropyridin-4-yl)propan-1-ol 92b (14 g, 81.57 mmol, 52.03% yield).

The product was confirmed by LCMS and H-NMR.

MS-ESI calculated for $[M+H]^+$ 172.1, found $[M+H]^+$ 172.0.

$^{1}$H NMR (400MHz, $CDCl_3$): δ (ppm) 8.26 (d, *J* = 5.2 Hz, 1H), 7.19 (s, 1H), 7.08 (d, *J* = 4.8 Hz, 1H), 3.69 (t, *J* = 6.0 Hz, 2H), 2.73 (t, *J* = 7.6 Hz, 2H), 1.93-1.86 (m, 2H).

Step 2

At room temperature, 3-(2-chloropyridin-4-yl)propan-1-ol 92b (14 g, 81.57 mmol) was dissolved in dichloromethane (140 mL), and Dess-Martin periodinane (51.9 g, 122.36 mmol) was added. The reaction system was stirred at

25°C for 16 hours. The reaction mixture was poured into aqueous sodium bicarbonate solution (200 mL) and extracted with dichloromethane (100 mL x 3). The combined organic phases were washed with saturated brine (300 mL), dried over anhydrous sodium sulfate, and concentrated under reduced pressure to afford the crude product. The crude product was purified by column chromatography (petroleum ether/ethyl acetate - petroleum ether/ethyl acetate = 1/0-1/1) to give the yellow oily product 3-(2-chloropyridin-4-yl)propanal 92c (8 g, 47.17 mmol, 57.82% yield).

The product was confirmed by LCMS and H-NMR.

MS-ESI calculated for $[M+H]^+$ 170.0, found $[M+H]^+$ 170.0.

$^1$H NMR (400MHz, $CDCl_3$): δ (ppm) 9.82 (s, 1H), 8.29 (d, *J* = 5.2 Hz, 1H), 7.19 (s, 1H), 7.08- 7.05 (m, 1H), 2.96-2.93 (m, 2H), 2.87-2.83 (m, 2H).

Step 3

At room temperature, 3-(2-chloropyridin-4-yl)propanal 92c (7.5 g, 44.22 mmol) was dissolved in tetrahydrofuran (75 mL), and pyrrolidine (4.7 g, 66.33 mmol) and sodium triacetoxyborohydride (28.1 g, 132.66 mmol) were added. The reaction system was stirred at 25°C for 16 hours. The reaction mixture was poured into water (300 mL) and extracted with ethyl acetate (200 mL x 3). The combined organic phases were washed with saturated brine (300 mL), dried over anhydrous sodium sulfate, and concentrated under reduced pressure to afford the crude product. The crude product was purified by column chromatography (petroleum ether/ethyl acetate - petroleum ether/ethyl acetate = 1/0-1/1) to give the yellow oily liquid product 2-chloro-4-(3-(pyrrolidin-1-yl)propyl)pyridine 92d (6.9 g, 30.70 mmol, 69.43% yield).

The product was confirmed by LCMS and H-NMR.

MS-ESI calculated for $[M+H]^+$ 225.1, found $[M+H]^+$ 225.1.

$^1$H NMR (400 MHz, $CDCl_3$) δ(ppm) 8.23 (d, *J* = 8.0 Hz, 1H), 7.17 (s, 1H), 7.03 (d, *J* = 4.8 Hz, 1H), 2.67 (t, *J* = 7.6 Hz, 2H), 2.50-2.44 (m, 6H), 1.88-1.83 (m, 2H), 1.80-1.77 (m, 4H).

Step 4

At room temperature, 2-chloro-4-(3-(pyrrolidin-1-yl)propyl)pyridine 92d (5.9 g, 26.25 mmol) was dissolved in 1,4-dioxane (60 mL), and 4,4,5,5-tetramethyl-2-ethenyl-1,3,2-dioxaborolane (6.1 g, 39.38 mmol), tetrakis(triphenylphosphine)palladium(0) (738 mg, 638.22 μmol), and cesium carbonate (25.7 g, 78.76 mmol) were added. The reaction system was stirred at 100°C for 16 hours under nitrogen protection. The reaction mixture was poured into water (300 mL) and extracted with ethyl acetate (100 mL x 3). The combined organic phases were washed with saturated brine (300 mL), dried over anhydrous sodium sulfate, and concentrated under reduced pressure to afford the crude product. The crude product was purified by medium-pressure preparative chromatography to give the brown oily product 4-(3-(pyrrolidin-1-yl)propyl)-2-ethenylpyridine 92e (2 g, 9.25 mmol, 35.22% yield).

The product was confirmed by LCMS and H-NMR.

MS-ESI calculated for $[M+H]^+$ 217.2, found $[M+H]^+$ 217.1.

$^1$H NMR (400 MHz, $CDCl_3$) δ(ppm) 8.45 (d, *J* = 4.8 Hz, 1H), 7.05 (d, *J* = 4.4 Hz, 1H), 7.01-7.00 (m, 1H), 6.79 (dd, *J* = 17.2, 10.8 Hz, 1H), 6.19 (dd, *J* = 17.2, 1.2 Hz, 1H), 5.46 (dd, *J* = 10.8, 0.8 Hz, 1H), 2.68-2.64 (m, 2H), 2.49-2.43 (m, 6H), 1.90-1.82(m, 2H), 1.80-1.76 (m, 4H).

Step 5

At room temperature, 4-(3-(pyrrolidin-1-yl)propyl)-2-ethenylpyridine 92e (247 mg, 1.14 mmol) was dissolved in 1,4-dioxane (10 mL), and N-ethyl-3-fluoro-2-(3-iodo-1-tetrahydropyran-2-yl-indazol-6-yl)sulfanylbenzamide 80a (500 mg, 951.70 μmol), tris(o-methylphenyl)phosphine (290 mg, 951.70 μmol), palladium(II) chloride (85 mg, 475.85 μmol), and triethylamine (289 mg, 2.86 mmol) were added. The reaction system was stirred at 90°C for 16 hours. The reaction mixture was poured into water (100 mL) and extracted with ethyl acetate (30 mL x 3). The combined organic phases were washed with saturated brine (30 mL), dried over anhydrous sodium sulfate, and concentrated under reduced pressure to afford the crude product. The crude product was purified by preparative TLC (dichloromethane/methanol = 10/1) to give the yellow solid product N-ethyl-3-fluoro-2-{3-[(E)-2-[4-(3-(pyrrolidin-1-yl)propyl)pyridin-2-yl]ethenyl]-1-tetrahydropyran-2-yl-indazol-6-yl}sulfanylbenzamide 92f (170 mg, 276.97 μmol, 29.10% yield).

The product was confirmed by LCMS.

MS-ESI calculated for $[M+H]^+$ 614.3, found 614.2.

Step 6

At room temperature, N-ethyl-3-fluoro-2-{3-[(E)-2-[4-(3-(pyrrolidin-1-yl)propyl)pyridin-2-yl]ethenyl]-1-tetrahydropyran-2-yl-indazol-6-yl}sulfanylbenzamide 92f (160 mg, 260.68 μmol) was dissolved in dichloromethane (3 mL),

and trifluoroacetic acid (0.6 mL) was added. The reaction system was stirred at 25°C for 16 hours. The reaction mixture was poured into water (30 mL) and extracted with ethyl acetate (10 mL x 3). The combined organic phases were washed with saturated brine (30 mL), dried over anhydrous sodium sulfate, and concentrated under reduced pressure to afford the crude product. The crude product was purified by high-pressure preparative chromatography to give the white solid product N-ethyl-3-fluoro-2-({3-[(E)-2-{4-[3-(pyrrolidin-1-yl)propyl]pyridin-2-yl}ethenyl]-1H-indazol-6-yl}sulfanyl)benzamide I-116.

The product was confirmed by LCMS, F-NMR, H-NMR, and C-NMR.

MS-ESI calculated for $[M+H]^+$ 530.2, found 530.1.

$^1H$ NMR (400MHz,$CD_3OD$): δ (ppm) 8.41 (d, *J* = 5.2 Hz, 1H), 7.98 (d, *J* = 8.8 Hz, 1H), 7.83 (d, *J* = 16.8 Hz, 1H), 7.57-7.49 (m, 3H), 7.39 (s, 1H), 7.32-7.26 (m, 2H), 7.18 (d, *J* = 5.2 Hz, 1H), 7.13 (dd, *J* = 8.8, 1.2 Hz, 1H), 3.34-3.31 (m, 2H), 2.73 (t, *J* = 7.6 Hz, 2H), 2.60-2.54 (m, 6H), 1.97-1.89 (m, 2H), 1.84-1.80 (m, 4H), 1.13 (t, *J* = 7.2 Hz, 3H).

$^{19}F$ NMR (400MHz,$CD_3OD$): δ (ppm) -105.78

$^{13}C$ NMR (400MHz,$CD_3OD$): δ (ppm) 169.95, 165.45, 162.97, 156.77, 154.21, 150.09, 145.64, 143.75, 143.48, 136.65, 132.66, 132.58, 130.66, 125.35, 124.75, 123.96, 123.36, 122.11, 121.11, 119.99, 119.79, 118.47, 118.23, 111.60, 56.82, 55.00, 35.81, 34.04, 30.17, 24.18, 14.62

I-124

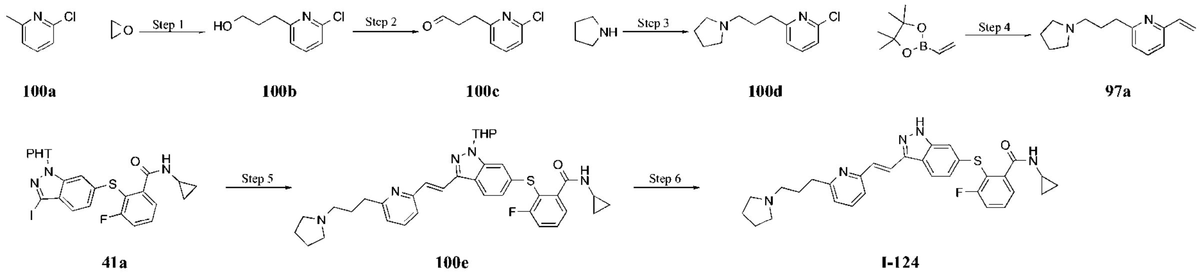

100a 100b 100c 100d 97a

41a 100e I-124

Step 1

At room temperature, 2-chloro-6-methylpyridine 100a (50 g, 391.94 mmol) was dissolved in tetrahydrofuran (500 mL), cooled to -78°C under nitrogen protection, and lithium diisopropylamide (2 M, 392 mL, 783.88 mmol) was added. The reaction system was stirred at -78°C for 2 hours. Then, ethylene oxide (3 M, 261 mL, 783.88 mmol) was slowly added at -78°C, and the reaction system was stirred at -78°C for 2 hours. The reaction mixture was poured into saturated ammonium chloride solution (2000 mL) and extracted with ethyl acetate (1000 mL x 3). The combined organic phases were washed with saturated brine (1000 mL), dried over anhydrous sodium sulfate, and concentrated under reduced pressure to afford the crude product. The crude product was purified by column chromatography (petroleum ether:ethyl acetate = 1/0-0/1) to give the yellow solid product 3-(6-chloropyridin-2-yl)propan-1-ol 100b (16 g, 93.23 mmol, 23.79% yield).

The product was confirmed by LCMS and H-NMR.

MS-ESI calculated for $[M+H]^+$ 172.1, found 172.2.

$^1H$ NMR (400 MHz,$CDCl_3$): δ (ppm) 7.57 (t, *J* = 7.6 Hz, 1H), 7.17 (d, *J* = 8.0 Hz, 1H), 7.11 (d, *J* = 7.6 Hz, 1H), 3.70 (s, 2H), 2.90 (t, *J* = 7.6 Hz, 2H), 2.71 (s, 1H), 2.02-1.95 (m, 2H).

Step 2

At room temperature, 3-(6-chloropyridin-2-yl)propan-1-ol 100b (11 g, 64.09 mmol) was dissolved in dichloromethane (200 mL), and Dess-Martin periodinane (40.8 g, 96.14 mmol) was added. The reaction system was stirred at 25°C for 16 hours. The reaction mixture was poured into saturated aqueous sodium carbonate solution (300 mL) and extracted with dichloromethane (100 mL x 3). The combined organic phases were dried over anhydrous sodium sulfate and concentrated under reduced pressure to afford the crude product. The crude product was purified by column chromatography (petroleum ether:ethyl acetate = 1/0-10/1) to give the yellow solid product 3-(6-chloropyridin-2-yl) propanal 100c (2.6 g, 15.33 mmol, 23.92% yield).

The product was confirmed by LCMS and H-NMR.

MS-ESI calculated for $[M+H]^+$ 170.0, found 170.0.

$^1H$ NMR (400MHz, $CDCl_3$): δ(ppm) 9.85 (s, 1H), 7.58-7.55 (m, 1H), 7.18-7.13 (m, 2H), 3.10 (t, *J* = 7.2 Hz, 2H), 2.98 (t, *J* = 6.8 Hz, 2H).

Step 3

At room temperature, 3-(6-chloropyridin-2-yl)propanal 100c (2.7 g, 15.92 mmol) was dissolved in tetrahydrofuran (30 mL), and pyrrolidine (1.7 g, 23.88 mmol) and sodium triacetoxyborohydride (10.1 g, 47.76 mmol) were added. The reaction system was stirred at 25°C for 16 hours. The reaction mixture was poured into water (300 mL) and extracted with ethyl acetate (100 mL x 3). The combined organic phases were washed with saturated brine (300 mL), dried over anhydrous sodium sulfate, and concentrated under reduced pressure to afford the crude product. The crude product was purified by column chromatography (petroleum ether:ethyl acetate = 20/1-1/1) to give the yellow solid product 2-chloro-6-[3-(pyrrolidin-1-yl)propyl]pyridine 100d (2 g, 8.90 mmol, 55.91% yield).
The product was confirmed by LCMS and H-NMR.
MS-ESI calculated for $[M+H]^+$ 225.1, found 225.0.
$^1$H NMR (400MHz, $CDCl_3$): δ (ppm) 7.57-7.53 (m, 1H), 7.16-7.12 (m, 1H), 7.09 (d, J = 7.6 Hz, 1H), 2.81 (t, J = 7.6 Hz, 2H), 2.61-2.48 (m, 6H), 1.99-1.90 (m, 2H), 1.80-1.70 (m, 4H).

Step 4

At room temperature, 2-chloro-6-[3-(pyrrolidin-1-yl)propyl]pyridine 100d (2.7 g, 12.01 mmol) was dissolved in dioxane (30 mL), and ethenylboronic acid pinacol ester (2.8 g, 18.02 mmol), tetrakis(triphenylphosphine)palladium(0) (0.3 g, 259.61 μmol), and cesium carbonate (11.7 g, 36.04 mmol) were added. The reaction system was stirred at 100°C under nitrogen protection for 16 hours. The reaction mixture was poured into water (300 mL) and extracted with ethyl acetate (100 mL x 3). The combined organic phases were washed with saturated brine (300 mL), dried over anhydrous sodium sulfate, and concentrated under reduced pressure to afford the brown oily crude product 2-[3-(pyrrolidin-1-yl)propyl]-6-ethenylpyridine 97a (1.2 g, 5.55 mmol, 46.17% yield).
The product was confirmed by LCMS.
MS-ESI calculated for $[M+H]^+$ 217.2, found 217.1.

Step 5

At room temperature, N-cyclopropyl-3-fluoro-2-(3-iodo-1-tetrahydropyran-2-yl-indazol-6-yl)sulfanylbenzamide 41a (600 mg, 1.12 mmol) was dissolved in dioxane (10 mL), and 2-[3-(pyrrolidin-1-yl)propyl]-6-ethenylpyridine 97a (362 mg, 1.67 mmol), tris(o-methylphenyl)phosphine (339 mg, 1.12 mmol), triethylamine (565 mg, 5.58 mmol), and palladium(II) chloride (99 mg, 558.26 μmol) were added. The reaction system was stirred at 90°C under nitrogen protection for 16 hours. The reaction mixture was poured into water (20 mL) and extracted with ethyl acetate (20 mL x 3). The combined organic phases were washed with saturated brine (30 mL), dried over anhydrous sodium sulfate, and concentrated under reduced pressure to afford the crude product. The crude product was purified by medium-pressure preparative chromatography to give the yellow solid N-cyclopropyl-3-fluoro-2-{3-[(E)-2-[6-(3-(pyrrolidin-1-yl)propyl)pyridin-2-yl]ethenyl]-1-tetrahydropyran-2-yl-indazol-6-yl}sulfanylbenzamide 100e (500 mg, 798.98 μmol, 71.56% yield).
The product was confirmed by LCMS.
MS-ESI calculated for $[M+H]^+$ 626.3, found 626.1.

Step 6

At room temperature, N-cyclopropyl-3-fluoro-2-{3-[(E)-2-[6-(3-(pyrrolidin-1-yl)propyl)pyridin-2-yl]ethenyl]-1-tetrahydropyran-2-yl-indazol-6-yl}sulfanylbenzamide 100e (500 mg, 798.98 μmol) was dissolved in dichloromethane (12 mL) and trifluoroacetic acid (4 mL). The reaction system was stirred at 25°C for 16 hours. The reaction mixture was poured into water (30 mL), adjusted to pH 8-9 with saturated sodium carbonate solution, and extracted with dichloromethane (30 mL x 3). The combined organic phases were washed with saturated brine (30 mL), dried over anhydrous sodium sulfate, and concentrated under reduced pressure to afford the crude product. The crude product was purified by high-performance liquid chromatography to give the yellow solid product N-cyclopropyl-3-fluoro-2-({3-[(E)-2-{6-[3-(pyrrolidin-1-yl)propyl]pyridin-2-yl}ethenyl]-1H-indazol-6-yl}sulfanyl)benzamide I-124 (51.74 mg, 95.52 μmol, 11.95% yield).
The product was confirmed by LCMS, F-NMR, H-NMR, and C-NMR.
MS-ESI calculated for $[M+H]^+$ 542.2, found 542.3.
$^1$H NMR (400MHz, $CD_3OD$): δ (ppm) 8.00 (d, *J* = 8.4 Hz, 1H), 7.83 (d, *J* = 16.8 Hz, 1H), 7.72 (t, *J* = 8.0 Hz, 1H), 7.56-7.50 (m, 3H), 7.39 (s, 1H), 7.30-7.26 (m, 2H), 7.17 (d, *J* = 7.6 Hz, 1H), 7.12 (dd, *J* = 8.4, 1.2 Hz, 1H), 2.85 (t, *J* = 7.6 Hz, 2H), 2.79-2.74 (m, 1H), 2.63-2.57 (m, 6H), 2.05-1.98 (m, 2H), 1.84-1.80 (m, 4H), 0.75-0.70 (m, 2H), 0.52-0.48 (m, 2H).
$^{13}$C NMR (100 MHz, $CD_3OD$): δ (ppm) 171.64, 165.43, 162.96, 162.76, 156.50, 145.34, 143.92, 143.51, 138.78, 136.57, 132.67, 132.58, 131.18, 123.90, 122.93, 122.22, 121.00, 120.34, 120.01, 119.82, 118.54, 118.31, 111.63, 57.11, 55.02, 36.88, 33.10, 30.78, 30.50, 29.90, 24.18, 23.76, 14.47, 6.44.

$^{19}F$ NMR (400MHz, $CD_3OD$): δ (ppm) -105.86.

I-134

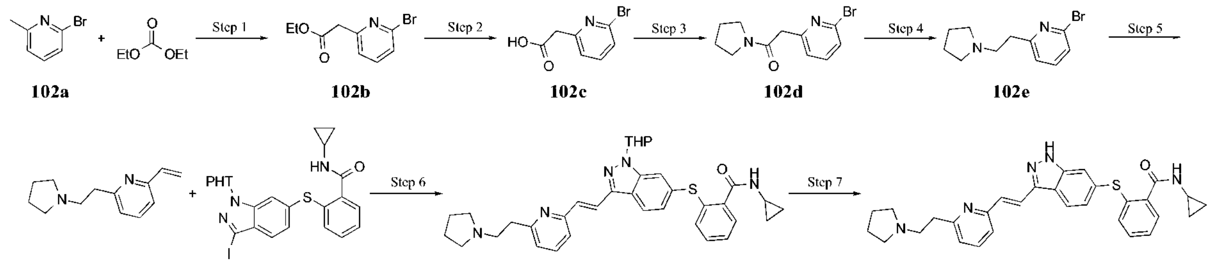


102a 102b 102c 102d 102e

102f 93a 102g I-134

Step 1

At room temperature, 2-bromo-6-methylpyridine 102a (50 g, 290.66 mmol) was dissolved in tetrahydrofuran (500 mL), cooled to -70°C under nitrogen protection, and lithium diisopropylamide (2 M, 291 mL, 581.32 mmol) was added. The reaction system was stirred at -70°C for 1 hour. Then, diethyl carbonate (41.2 g, 348.79 mmol) was added dropwise at -70°C, and the reaction system was stirred at -70°C for 4 hours. The reaction mixture was poured into water (1000 mL) and extracted with ethyl acetate (500 mL x 3). The combined organic phases were washed with saturated brine (300 mL), dried over anhydrous sodium sulfate, and concentrated under reduced pressure to afford the crude product. The crude product was purified by medium-pressure preparative chromatography to give the yellow oily product ethyl 2-(6-bromopyridin-2-yl) acetate 102b (38 g, 155.68 mmol, 53.56% yield).

The product was confirmed by LCMS and H-NMR.

MS-ESI calculated for $[M+H]^+$ 244.0, found 243.9.

$^1H$ NMR (400 MHz,$CDCl_3$): δ (ppm) 7.53 (t, *J* = 7.8 Hz, 1H), 7.40 (d, *J* = 8.0 Hz, 1H), 7.31-7.26 (m, 1H), 4.19 (q, *J* = 7.2 Hz, 2H), 3.83 (s, 2H), 1.30-1.23 (m, 3H).

Step 2

At room temperature, ethyl 2-(6-bromopyridin-2-yl)acetate 102b (40 g, 163.88 mmol) was dissolved in water (100 mL), tetrahydrofuran (100 mL), and methanol (200 mL), and lithium hydroxide (11.8 g, 491.63 mmol) was added. The reaction system was stirred at 25°C for 2 hours. Tetrahydrofuran and methanol were removed by rotary evaporation. The mixture was poured into water (500 mL), adjusted to pH 3 with 1N dilute hydrochloric acid, and then extracted with ethyl acetate (500 mL x 3). The combined organic phases were washed with saturated brine (300 mL), dried over anhydrous sodium sulfate, and concentrated under reduced pressure to give the yellow solid product 2-(6-bromopyridin-2-yl)acetic acid 102c (33 g, 152.76 mmol, 93.21% yield).

The product was confirmed by LCMS and H-NMR.

MS-ESI calculated for $[M+H]^+$ 216.0, found 215.9.

$^1H$ NMR (400MHz, $CDCl_3$):δ(ppm) 7.58 (t, *J* = 7.6 Hz, 1H), 7.44 (d, *J* = 8.0 Hz, 1H), 7.27 (d, *J* = 7.2 Hz, 1H), 3.89 (s, 2H).

Step 3

At room temperature, 2-(6-bromopyridin-2-yl)acetic acid 102c (26 g, 120.35 mmol) was dissolved in N,N-dimethylformamide (400 mL), and pyrrolidine (12.8 g, 180.53 mmol), 1-hydroxybenzotriazole (24.4 g, 180.53 mmol), N,N-diisopropylethylamine (46.6 g, 361.06 mmol), and 1-(3-dimethylaminopropyl)-3-ethylcarbodiimide hydrochloride (34.7 g, 180.53 mmol) were added. The reaction system was stirred at 25°C for 16 hours. The reaction mixture was poured into water (500 mL) and extracted with ethyl acetate (500 mL x 3). The combined organic phases were washed with saturated brine (300 mL), dried over anhydrous sodium sulfate, and concentrated under reduced pressure to afford the crude product. The crude product was purified by medium-pressure preparative chromatography to give the yellow solid product 2-(6-bromopyridin-2-yl)-1-(pyrrolidin-1-yl)ethan-1-one 102d (28 g, 104.04 mmol, 86.44% yield).

The product was confirmed by LCMS and H-NMR.

MS-ESI calculated for $[M+H]^+$ 269.0, found 268.9.

$^1H$ NMR (400MHz, $CDCl_3$): δ (ppm) 7.51 (t, *J* = 7.6 Hz, 1H), 7.39-7.36 (m, 2H), 3.83 (s, 2H), 3.56 (t, *J* = 6.8 Hz, 2H), 3.48 (t, *J* = 6.8 Hz, 2H), 2.00-1.93 (m, 2H), 1.90-1.84 (m, 2H).

Step 4

At room temperature, 2-(6-bromopyridin-2-yl)-1-(pyrrolidin-1-yl)ethan-1-one 102d (26 g, 96.61 mmol) was dissolved in tetrahydrofuran (250 mL), and borane dimethyl sulfide complex (10 M, 19 mL) was added. The reaction system was stirred at 70°C for 3 hours. The reaction mixture was cooled to room temperature, then methanol (50 mL) was added dropwise to the reaction mixture. After stirring under reflux for an additional 4 hours, the mixture was concentrated under reduced pressure to afford the crude product. The crude product was purified by medium-pressure preparative chromatography to give the yellow oily product 2-bromo-6-[2-(pyrrolidin-1-yl)ethyl]pyridine 102e (20 g, 78.38 mmol, 81.14% yield).
The product was confirmed by LCMS and H-NMR.
MS-ESI calculated for $[M+H]^+$ 255.0, found 255.0.
$^1$H NMR (400MHz, $CDCl_3$): δ (ppm) 7.46 (t, *J* = 7.6 Hz, 1H), 7.32 (d, *J* = 7.6 Hz, 1H), 7.17 (d, *J* = 7.2 Hz, 1H), 3.08-3.01 (m, 2H), 2.94-2.88 (m, 2H), 2.67-2.60 (m, 4H), 1.84-1.81 (m, 4H).

Step 5

At room temperature, 2-bromo-6-[2-(pyrrolidin-1-yl)ethyl]pyridine 102e (20 g, 78.38 mmol) was dissolved in N,N-dimethylformamide (200 mL), and [1,1'-bis(diphenylphosphino)ferrocene]dichloropalladium(II) (2.0 g, 2.74 mmol), triethylamine (23.8 g, 235.15 mmol), and potassium trifluoro(ethenyl)borate (15.8 g, 117.58 mmol) were added. The reaction system was stirred at 85°C under nitrogen protection for 16 hours. The reaction mixture was poured into water (500 mL) and extracted with ethyl acetate (500 mL x 3). The combined organic phases were washed with saturated brine (300 mL), dried over anhydrous sodium sulfate, and concentrated under reduced pressure to afford the crude product. The crude product was purified by medium-pressure preparative chromatography to give the brown oily product 2-[2-(pyrrolidin-1-yl)ethyl]-6-ethenylpyridine 102f (9.5 g, 46.96 mmol, 59.91% yield).
The product was confirmed by LCMS and H-NMR.
MS-ESI calculated for $[M+H]^+$ 203.1, found 203.1.
$^1$H NMR (400MHz, $CDCl_3$): 7.55 (t, *J* = 8 Hz, 1H), 7.18 (d, *J* = 8.0 Hz, 1H), 7.07 (d, *J* = 7.6 Hz, 1H), 6.84-6.75 (m, 1H), 6.21-6.14 (m, 1H), 5.45 (dd, *J* = 10.8, 1.6 Hz, 1H), 3.06-3.02 (m, 2H), 2.94-2.88 (m, 2H), 2.65-2.58 (m, 4H), 1.85-1.76 (m, 4H).

Step 6

At room temperature, N-cyclopropyl-2-(3-iodo-1-tetrahydropyran-2-yl-indazol-6-yl)sulfanylbenzamide 93a (600 mg, 1.16 mmol) was dissolved in dioxane (10 mL), and 2-[2-(pyrrolidin-1-yl)ethyl]-6-ethenylpyridine 102f (234 mg, 1.16 mmol), palladium(II) chloride (102 mg, 577.59 μmol), triethylamine (585 mg, 5.78 mmol), and tris(o-methylphenyl) phosphine (351 mg, 1.16 mmol) were added. The reaction system was stirred at 90°C under nitrogen protection for 16 hours. The reaction mixture was poured into water (40 mL) and extracted with ethyl acetate (40 mL x 3). The combined organic phases were washed with saturated brine (40 mL), dried over anhydrous sodium sulfate, and concentrated under reduced pressure to afford the crude product. The crude product was purified by medium-pressure preparative chromatography to give N-cyclopropyl-2-{3-[(E)-2-{6-[2-(pyrrolidin-1-yl)ethyl]pyridin-2-yl}ethenyl]-1-tetrahydropyran-2-yl-indazol-6-yl}sulfanylbenzamide 102g (300 mg, 505.24 μmol, 43.74% yield).
The product was confirmed by LCMS.
MS-ESI calculated for $[M+H]^+$ 594.3, found 594.1.

Step 7

At room temperature, N-cyclopropyl-2-{3-[(E)-2-{6-[2-(pyrrolidin-1-yl)ethyl]pyridin-2-yl}ethenyl]-1-tetrahydropyran-2-yl-indazol-6-yl}sulfanylbenzamide 102g (300 mg, 505.24 μmol) was dissolved in dichloromethane (12 mL) and trifluoroacetic acid (4 mL). The reaction system was stirred at 25°C for 16 hours. The reaction mixture was poured into water (50 mL), and the pH was adjusted to 8 with saturated sodium carbonate solution. It was extracted with dichloromethane (50 mL x 3). The combined organic phases were washed with saturated brine (50 mL), dried over anhydrous sodium sulfate, and concentrated under reduced pressure to afford the crude product. The crude product was purified by high-performance liquid chromatography to give the yellow solid product N-cyclopropyl-2-({3-[(E)-2-{4-[2-(pyrrolidin-1-yl)ethyl]pyridin-2-yl}ethenyl]-1H-indazol-6-yl}sulfanyl)benzamide I-134 (72.04 mg, 141.35 μmol, 27.98% yield).
The product was confirmed by LCMS, H-NMR, and C-NMR.
MS-ESI calculated for $[M+H]^+$ 510.2, found 510.3.
$^1$H NMR (400MHz, $CD_3OD$): 8.09 (d, *J* = 8.8 Hz, 1H), 7.87 (d, *J* = 16.8 Hz, 1H), 7.73 (t, *J* = 8.0 Hz, 1H), 7.61-7.41 (m, 4H), 7.39-7.15 (m, 5H), 3.08-3.04 (m, 2H), 2.96-2.93 (m, 2H) , 2.82-2.74 (m, 1H), 2.68 (s, 4H), 1.93-1.78 (m, 4H),

0.81-0.66 (m, 2H), 0.58-0.49 (m, 2H).

$^{13}C$ NMR (100 MHz, $CD_3OD$) δ (ppm) 172.69, 160.99, 156.50, 144.00, 143.65, 139.29, 138.81, 135.76, 133.25, 131.25, 128.22, 125.16, 123.22, 122.63, 121.47, 120.70, 114.86, 57.26, 55.05, 38.04, 24.25, 23.78, 6.47.

I-103

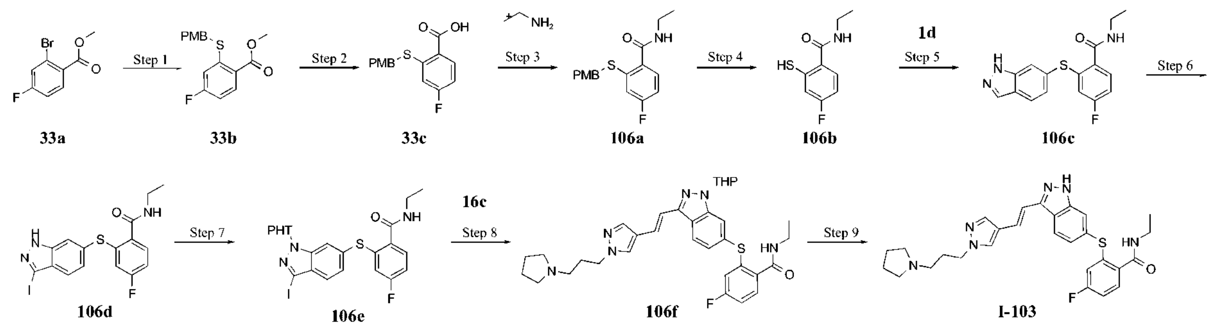


Step 1

At room temperature, methyl 2-bromo-4-fluorobenzoate 33a (25 g, 107.28 mmol) was dissolved in N,N-dimethylformamide (250 mL), and (4-methoxyphenyl)methanethiol (16.6 g, 107.28 mmol), triethylamine (32.5 g, 321.84 mmol), and [1,1'-bis(diphenylphosphino)ferrocene]palladium(II) dichloride (2.5 g, 107.28 mmol) were added. The reaction system was stirred at 120°C for 16 hours under nitrogen protection. The reaction mixture was poured into water (1000 mL) and extracted with ethyl acetate (500 mL x 3). The combined organic phases were washed with saturated brine (500 mL), dried over anhydrous sodium sulfate, and concentrated under reduced pressure to afford the crude product. The crude product was purified by column chromatography (petroleum ether/ethyl acetate = 50/1-20/1) to give the yellow solid product methyl 4-fluoro-2-[(4-methoxyphenyl)methylthio]benzoate 33b (13 g, 42.43 mmol, 39.56% yield).

The product was confirmed by LCMS and H-NMR.

MS-ESI calculated for $[M+Na]^+$ 329.1, found 328.9.

$^1$HNMR (400MHz, $CDCl_3$): δ (ppm) 8.02 (dd, *J*=8.8, 6.0 Hz, 1H), 7.36-7.32 (m, 2H), 7.05 (dd, *J* = 10.4, 2.4 Hz, 1H), 6.88-6.84 (m, 2H), 6.82-6.80 (m, 1H), 4.08 (s, 2H), 3.88 (s, 3H), 3.79 (s, 3H).

Step 2

At room temperature, methyl 4-fluoro-2-[(4-methoxyphenyl)methylthio]benzoate 33b (13 g, 42.43 mmol) was dissolved in tetrahydrofuran (120 mL) and water (40 mL), and lithium hydroxide (3.05 g, 127.30 mmol) was added. The reaction system was stirred at 50°C for 5 hours. The reaction mixture was poured into water (300 mL), and the pH was adjusted to 5 with 2N dilute hydrochloric acid. It was extracted with ethyl acetate (300 mL x 3). The combined organic phases were washed with saturated brine (300 mL), dried over anhydrous sodium sulfate, and concentrated under reduced pressure to afford the yellow solid product 4-fluoro-2-[(4-methoxyphenyl)methylthio]benzoic acid 33c (11 g, 37.63 mmol, 88.68% yield).

The product was confirmed by LCMS and H-NMR.

MS-ESI calculated for $[M+Na]^+$ 315.1, found 314.9.

$^1$HNMR (400 MHz, $CD_3OD$): δ(ppm) 8.14 (dd, *J*=8.8, 6.0 Hz, 1H), 7.34 (d, *J*=8.4 Hz, 2H), 7.08 (dd, *J*=10.4, 2.4 Hz, 1H), 6.89-6.84 (m, 3H), 4.10 (s, 2H), 3.81 (s, 3H).

Step 3

At room temperature, 4-fluoro-2-[(4-methoxyphenyl)methylthio]benzoic acid 33c (15 g, 51.31 mmol) was dissolved in N,N-dimethylformamide (150 mL), and ethylamine (5 g, 61.58 mmol, 6.25 mL), N,N-diisopropylethylamine(19.9 g, 153.94 mmol), 1-hydroxybenzotriazole (10.4 g, 76.97 mmol), and 1-(3-dimethylaminopropyl)-3-ethylcarbodiimide hydrochloride(14.8 g, 76.97 mmol) were added. The reaction system was stirred at 25°C for 2 hours. The reaction mixture was poured into water (500 mL) and filtered. The filter cake was dissolved in ethyl acetate, dried over anhydrous sodium sulfate, and concentrated under reduced pressure to afford the white solid crude product N-ethyl-4-fluoro-2-[(4-methoxyphenyl)methylthio]benzamide 106a (15 g, 46.96 mmol, 91.53% yield).

The product was confirmed by LCMS and H-NMR.

MS-ESI calculated for $[M+H]^+$ 320.1, found 320.1.

$^1$HNMR (400MHz, $CDCl_3$): δ (ppm) 7.60 (dd, *J*=8.8, 6.0 Hz, 1H), 7.16 (d, *J*=8.8 Hz, 2H), 7.07 (dd, *J*=9.6, 2.8 Hz, 1H), 6.94-6.89 (m, 1H), 6.83-6.79 (m, 2H), 6.32 (s, 1H), 4.06 (s, 2H), 3.78 (s, 3H), 3.47-3.40 (m, 2H), 1.21 (t, *J*=7.2 Hz, 3H).

Step 4

At room temperature, N-ethyl-4-fluoro-2-[(4-methoxyphenyl)methylthio]benzamide 106a (6 g, 18.79 mmol) was dissolved in trifluoroacetic acid (60 mL). The reaction system was stirred at 70°C for 5 hours. The reaction mixture was concentrated under reduced pressure to afford the crude product. The crude product was purified by medium-pressure preparative chromatography to give the yellow solid product N-ethyl-4-fluoro-2-mercaptobenzamide 106b (3 g, 15.06 mmol, 80.15% yield).

The product was confirmed by LCMS and H-NMR.

MS-ESI calculated for $[M+H]^+$ 200.0, found 200.0.

$^1$HNMR (400MHz, $CDCl_3$): δ (ppm) 7.43 (dd, *J*=8.4, 5.6 Hz, 1H), 7.04 (dd, *J*= 10.0, 3.2 Hz, 1H), 6.85-6.80 (m, 2H), 6.00 (s, 1H), 5.08 (s, 1H), 3.52-3.44 (m, 2H), 1.26 (t, *J*=7.2 Hz, 3H).

Step 5

At room temperature, N-ethyl-4-fluoro-2-mercaptobenzamide 106b (4.5 g, 22.59 mmol) was dissolved in N,N-dimethylformamide (50 mL), and 6-iodo-1H-indazole 1d (5.5 g, 22.59 mmol), cesium carbonate (22.1 g, 67.76 mmol), tris(dibenzylideneacetone)dipalladium(0) (2.1 g, 2.26 mmol), and 4,5-bis(diphenylphosphino)-9,9-dimethylxanthene (2.6 g, 4.52 mmol) were added. The reaction system was stirred at 80°C for 16 hours under nitrogen protection. The reaction mixture was poured into water (300 mL) and extracted with ethyl acetate (100 mL x 3). The combined organic phases were washed with saturated brine (300 mL), dried over anhydrous sodium sulfate, and concentrated under reduced pressure to afford the crude product. The crude product was purified by column chromatography (petroleum ether/ethyl acetate = 10/1-1/1) to give the yellow solid product N-ethyl-4-fluoro-2-(1H-indazol-6-ylsulfanyl)benzamide 106c (5.6 g, 17.76 mmol, 78.62% yield).

The product was confirmed by LCMS and H-NMR.

MS-ESI calculated for $[M+H]^+$ 316.1, found 316.0.

$^1$HNMR (400MHz, $CDCl_3$): δ (ppm) 8.13 (s,1H), 7.80-7.77 (m, 2H), 7.60 (dd, *J*=8.8, 6.0 Hz, 1H), 7.22 (d, *J*=8.4 Hz, 1H), 6.93-6.88 (m, 1H), 6.72 (dd, *J*=9.6, 2.4 Hz, 1H), 6.26 (s, 1H), 3.55-3.48 (m, 2H), 1.25 (t, *J*=7.2 Hz, 3H).

Step 6

At room temperature, N-ethyl-4-fluoro-2-(1H-indazol-6-ylsulfanyl)benzamide 106c (6 g, 19.03 mmol) was dissolved in N,N-dimethylformamide (60 mL), and potassium carbonate (7.9 g, 57.08 mmol) and iodine (7.2 g, 28.54 mmol) were added. The reaction system was stirred at 40°C for 1 hour. The reaction mixture was poured into water (300 mL) and filtered. The filter cake was dissolved in ethyl acetate, dried over anhydrous sodium sulfate, and concentrated under reduced pressure to afford the yellow solid crude product N-ethyl-4-fluoro-2-[(3-iodo-1H-indazol-6-yl)sulfanyl]benzamide 106d (7 g, 15.86 mmol, 83.38% yield).

The product was confirmed by LCMS and H-NMR.

MS-ESI calculated for $[M+H]^+$ 442.0, found 441.9.

$^1$HNMR (400 MHz, DMSO-d6): δ (ppm) 8.51 (t, *J*=5.2 Hz, 1H), 7.74 (s, 1H), 7.58 (dd, *J*=8.8, 6.0Hz, 1H), 7.49 (d, *J*=8.4 Hz, 1H), 7.17 (d, *J*=8.4 Hz, 1H), 7.12-7.07 (m, 1H), 6.59 (dd, *J*=10.0, 2.4 Hz, 1H), 3.30-3.23 (m, 2H), 1.13 (t, *J*=*7.2* Hz, 3H).

Step 7

At room temperature, N-ethyl-4-fluoro-2-[(3-iodo-1H-indazol-6-yl)sulfanyl]benzamide 106d (7 g, 15.86 mmol) was dissolved in ethyl acetate (70 mL), and 3,4-dihydro-2H-pyran (4.0 g, 47.59 mmol) and p-toluenesulfonic acid (1.4 g, 7.93 mmol) were added. The reaction system was stirred at 70°C for 16 hours. The reaction mixture was filtered, and the filter cake was dried under vacuum to afford the yellow solid crude product N-ethyl-4-fluoro-2-[(3-iodo-1-(tetrahydro-2H-pyran-2-yl)-1H-indazol-6-yl)sulfanyl]benzamide 106e (7 g, 13.32 mmol, 83.99% yield).

The product was confirmed by LCMS and H-NMR.

MS-ESI calculated for $[M+H]^+$ 526.0, found 525.9.

$^1$HNMR (400MHz, DMSO-d6): δ (ppm) 8.51 (t, *J*=5.6 Hz, 1H), 8.04 (s, 1H), 7.61 (dd, *J*=8.4, 6.0 Hz, 1H), 7.52 (d, *J*=7.6 Hz, 1H), 7.26 (d, *J*=8.4 Hz, 1H), 7.13-7.08 (m, 1H), 6.57 (dd, *J*=10.0, 2.4 Hz, 1H), 5.92 (dd, *J*=9.6, 1.6 Hz, 1H), 3.87 (d, *J*=11.2 Hz, 1H), 3.78-3.71 (m, 1H), 3.31-3.24 (m, 2H), 2.41-2.31 (m, 1H), 2.02-1.97 (m, 2H), 1.77-1.66 (m, 1H), 1.57 (s, 2H),

1.13 (t, *J*=7.2 Hz, 3H).

Step 8

At room temperature, N-ethyl-4-fluoro-2-[(3-iodo-1-(tetrahydro-2H-pyran-2-yl)-1H-indazol-6-yl)sulfanyl]benzamide 106e (1 g, 1.90 mmol) was dissolved in 1,4-dioxane (10 mL), and 1-(3-(pyrrolidin-1-yl)propyl)-4-ethenyl-1H-pyrazole 16c (391 mg, 1.90 mmol), triethylamine (578 mg, 5.71 mmol), and [2-(2'-amino-1,1'-biphenyl-2-yl)]palladium(II) methanesulfonate (2-dicyclohexylphosphino-2',4',6'-triisopropyl-1,1'-biphenyl) (161 mg, 190.34 μmol) were added. The reaction system was stirred at 90°C for 16 hours under nitrogen protection. The reaction mixture was poured into water (30 mL) and extracted with ethyl acetate (10 mL x 3). The combined organic phases were washed with saturated brine (30 mL), dried over anhydrous sodium sulfate, and concentrated under reduced pressure to afford the crude product. The crude product was purified by column chromatography (dichloromethane/methanol = 50/1-10/1) to give the yellow solid product N-ethyl-4-fluoro-2-[(3-[(E)-2-[1-(3-(pyrrolidin-1-yl)propyl)-1H-pyrazol-4-yl]ethenyl]-1-(tetrahydro-2H-pyran-2-yl)-1H-indazol-6-yl)sulfanyl]benzamide 106f (500 mg, 829.51 μmol, 43.58% yield).
The product was confirmed by LCMS.
MS-ESI calculated for $[M+H]^+$ 603.3, found 603.1.

Step 9

At room temperature, N-ethyl-4-fluoro-2-[(3-[(E)-2-[1-(3-(pyrrolidin-1-yl)propyl)-1H-pyrazol-4-yl]ethenyl]-1-(tetrahydro-2H-pyran-2-yl)-1H-indazol-6-yl)sulfanyl]benzamide 106f (1 g, 1.66 mmol) was dissolved in dichloromethane (24 mL), and trifluoroacetic acid (8 mL) was added. The reaction system was stirred at 25°C for 16 hours. The reaction mixture was poured into water (100 mL), and the pH was adjusted to 10 with a saturated aqueous sodium carbonate solution. The mixture was extracted with dichloromethane (100 mL x 3). The combined organic phases were washed with saturated brine (100 mL), dried over anhydrous sodium sulfate, and concentrated under reduced pressure to afford the crude product. The crude product was purified by preparative high-performance liquid chromatography to give the yellow solid product N-ethyl-4-fluoro-2-({3-[(E)-2-{1-[3-(pyrrolidin-1-yl)propyl]-1H-pyrazol-4-yl}ethenyl]-1H-indazol-6-yl}sulfanyl) benzamide I-103 (124.46 mg, 239.97 μmol, 14.46% yield).
The product was confirmed by LCMS, F-NMR, H-NMR, and C-NMR.
MS-ESI calculated for $[M+H]^+$ 519.2, found 519.1.
$^1$HNMR (400MHz, $CD_3OD$): δ (ppm) 8.08 (d, *J*=8.8 Hz, 1H), 7.91 (s, 1H), 7.80 (s, 1H), 7.68 (s, 1H), 7.50 (dd, *J*=8.8, 6.0 Hz, 1H), 7.37 (d, *J*=16.8 Hz, 1H), 7.23-7.21 (m, 2H), 7.01-7.96 (m, 1H), 6.73 (dd, *J*=10.0, 2.4 Hz, 1H), 4.22 (t, *J*=6.8 Hz, 2H), 3.39-3.34 (m, 2H), 2.57 (s, 4H), 2.51-2.47 (m, 2H), 2.12-2.08 (m, 2H), 1.83-1.80 (m, 4H), 1.20 (t, *J*=7.2 Hz, 3H).
$^{13}$CNMR (100MHz, $CD_3OD$&$CDCl_3$) δ (ppm) 169.96, 166.19, 163.69, 144.89, 143.59, 141.79, 141.71, 138.48, 133.04, 132.93, 131.13, 131.05, 129.63, 127.37, 123.30, 122.97, 122.08, 121.91, 118.99, 117.43, 117.18, 114.02, 113.80, 55.17, 54.44, 51.36, 36.04, 33.16, 30.85, 30.57, 30.49, 24.35, 23.86, 15.08, 14.82.
$^{19}$FNMR (400MHz, $CD_3OD$): δ (ppm) -111.69.

I-133

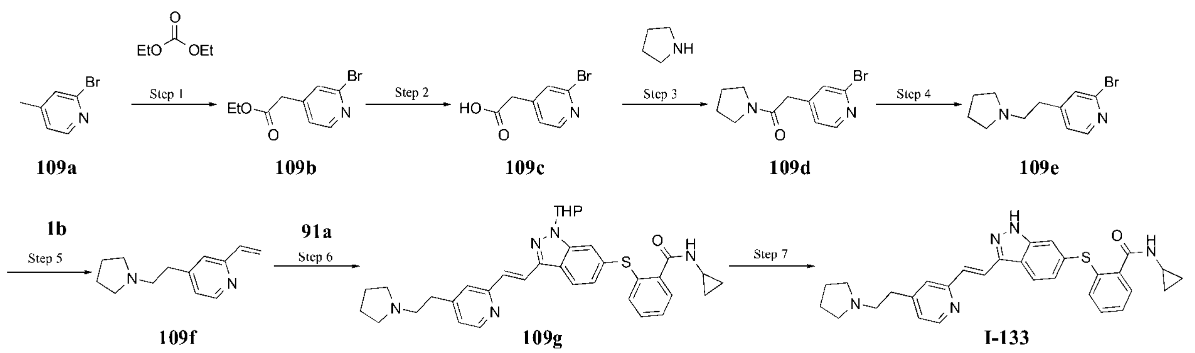


Step 1

At room temperature, 2-bromo-4-methylpyridine 109a (40 g, 232.53 mmol) was dissolved in tetrahydrofuran (1.2 L). The temperature was lowered to -70°C under nitrogen protection, and lithium diisopropylamide (2 mol/L, 174.40 mL,

348.80 mmol) was added. The reaction system was stirred at -70°C for 1 hour. Diethyl carbonate (33 g, 279.03 mmol) was slowly added at -70°C, and the reaction system was stirred at -70°C for 4 hours. The reaction mixture was poured into a saturated aqueous ammonium chloride solution (1000 mL) and extracted with ethyl acetate (500 mL x 3). The combined organic phases were washed with saturated brine (1000 mL), dried over anhydrous sodium sulfate, and concentrated under reduced pressure to afford the crude product. The crude product was purified by column chromatography (petroleum ether/ethyl acetate = 50/1-10/1) to give the yellow oily product ethyl 2-(2-bromo-4-pyridyl)acetate 109b (35 g, 143.39 mmol, 61.67% yield).

The product was confirmed by LCMS and H-NMR.

MS-ESI calculated for [M+H]+ 244.0/246.0, found 243.9/245.9.

$^1$H NMR (400MHz, $CDCl_3$): δ (ppm) 8.32 (d, *J*= 5.2 Hz, 1H), 7.45 (d, *J*= 0.8 Hz, 1H), 7.21 (dd, *J*= 5.2, 1.2 Hz, 1H), 4.19 (q, *J*= 7.2 Hz, 2H), 3.59 (s, 2H), 1.28 (t, *J*= 7.2 Hz, 3H).

Step 2

At room temperature, ethyl 2-(2-bromo-4-pyridyl)acetate 109b (35 g, 143.39 mmol) was dissolved in tetrahydrofuran (150 mL), methanol (350 mL), and water (150 mL), and lithium hydroxide (10 g, 430.18 mmol) was added. The reaction system was stirred at 25°C for 2 hours. Tetrahydrofuran was removed by rotary evaporation. The mixture was poured into water (500 mL), and the pH was adjusted to 3 with 1N dilute hydrochloric acid. The mixture was then extracted with ethyl acetate (300 mL x 3). The combined organic phases were dried over anhydrous sodium sulfate and concentrated under reduced pressure to afford the crude product 2-(2-bromo-4-pyridyl)acetic acid 109c (25 g, 115.72 mmol, 80.70% yield).

The product was confirmed by LCMS and H-NMR.

MS-ESI calculated for [M+H]+ 216.0/218.0, found 215.9/217.9.

$^1$H NMR (400MHz, $CDCl_3$): δ (ppm) 8.30 (d, *J*= 4.8 Hz, 1H), 7.47 (s, 1H), 7.23 (d, *J*= 4.8 Hz, 1H), 3.59 (s, 2H).

Step 3

At room temperature, 2-(2-bromo-4-pyridyl)acetic acid 109c (25 g, 115.72 mmol) was dissolved in N,N-dimethylformamide (250 mL), and pyrrolidine (10 g, 138.87 mmol), N,N-diisopropylethylamine (45 g, 347.17 mmol), 1-hydroxybenzotriazole (23 g, 173.58 mmol), and 1-(3-dimethylaminopropyl)-3-ethylcarbodiimide hydrochloride (33 g, 173.59 mmol) were added. The reaction system was stirred at 25°C for 16 hours. The reaction mixture was poured into water (800 mL) and extracted with ethyl acetate (300 mL x 3). The combined organic phases were washed with saturated brine (500 mL), dried over anhydrous sodium sulfate, and concentrated under reduced pressure to afford the yellow oily crude product 2-(2-bromo-4-pyridyl)-1-(pyrrolidin-1-yl)ethanone 109d (16 g, 59.45 mmol, 51.37% yield).

The product was confirmed by LCMS and H-NMR.

MS-ESI calculated for [M+H]+ 269.0/271.0, found 269.0/271.0.

$^1$H NMR (400MHz, $CDCl_3$): δ (ppm) 8.30 (d, *J*= 5.2 Hz, 1H), 7.43 (s, 1H), 7.21 (d, *J*= 5.2 Hz, 1H), 3.61 (s, 2H), 3.50 (t, *J*= 6.8 Hz, 2H), 3.45 (t, *J*= 6.8 Hz, 2H), 2.02-1.96 (m, 2H), 1.92-1.85 (m, 2H).

Step 4

At room temperature, 2-(2-bromo-4-pyridyl)-1-(pyrrolidin-1-yl)ethanone 109d (8 g, 29.72 mmol) was dissolved in tetrahydrofuran (80 mL), and borane dimethyl sulfide complex (10 mol/L, 5.94 mL, 59.44 mmol) was added. The reaction system was stirred at 70°C for 3 hours. Methanol (50 mL) was then added, and the mixture was refluxed for an additional 6 hours. The reaction mixture was evaporated to dryness, poured into water (200 mL), and extracted with ethyl acetate (50 mL x 3). The combined organic phases were washed with saturated brine (30 mL), dried over anhydrous sodium sulfate, and concentrated under reduced pressure to afford the yellow oily crude product 2-bromo-4-(2-(pyrrolidin-1-yl)ethyl) pyridine 109e (1.9 g, 7.45 mmol, 25.05% yield).

The product was confirmed by LCMS and H-NMR.

MS-ESI calculated for [M+H]+ 255.0/257.0, found 255.0/257.0.

$^1$H NMR (400MHz, $CDCl_3$): δ (ppm) 8.33 (d, *J*= 5.2 Hz, 1H), 7.42 (s, 1H), 7.22 (dd, *J*= 4.8, 1.2 Hz, 1H), 3.30-3.19 (m, 8H), 2.18-2.11 (m, 4H).

Step 5

At room temperature, 2-bromo-4-(2-(pyrrolidin-1-yl)ethyl)pyridine 109e (2 g, 7.45 mmol) was dissolved in N,N-dimethylformamide (20 mL), and potassium ethenyltrifluoroborate (1.2 g, 8.94 mmol), triethylamine (2.3 g, 22.34 mmol), and [1,1'-bis(diphenylphosphino)ferrocene]palladium(II) dichloride (200 mg, 7.45 mmol) were added. The reaction

system was stirred at 90°C for 16 hours under nitrogen protection. The reaction mixture was poured into water (30 mL) and extracted with ethyl acetate (30 mL x 3). The combined organic phases were washed with saturated brine (50 mL), dried over anhydrous sodium sulfate, and concentrated under reduced pressure to afford the crude product. The crude product was purified by preparative silica gel TLC (dichloromethane/methanol = 10/1) to afford the brown oily product 4-(2-(pyrrolidin-1-yl)ethyl)-2-ethenylpyridine 109f (0.3 g, 1.48 mmol, 19.92% yield).

The product was confirmed by LCMS and H-NMR.

MS-ESI calculated for [M+H]+ 203.1, found 203.2.

$^1$H NMR (400MHz, $CDCl_3$): δ (ppm) 8.45 (d, *J* = 5.2 Hz, 1H), 7.19 (s, 1H), 7.02 (d, *J*= 5.2 Hz, 1H), 6.80 (dd, *J* = 17.6, 10.8 Hz, 1H), 6.20 (dd, *J* = 17.6, 1.2 Hz, 1H), 5.47 (dd, *J* = 10.8,1.2 Hz, 1H), 2.87-2.82 (m, 2H), 2.78-2.74 (m, 2H), 2.63 (s, 4H), 1.85-1.82 (m, 4H).

Step 6

At room temperature, 4-(2-(pyrrolidin-1-yl)ethyl)-2-ethenylpyridine 109f (290 mg, 1.43 mmol) was dissolved in 1,4-dioxane (5 mL), and N-cyclopropyl-2-((3-iodo-1-(tetrahydro-2H-pyran-2-yl)-1H-indazol-6-yl)thio)benzamide 91a (745 mg, 1.43 mmol), triethylamine (725 mg, 7.17 mmol), tri(o-tolyl)phosphine (436 mg, 1.43 mmol), and palladium(II) chloride (127 mg, 716.78 μmol) were added. The reaction system was stirred at 90°C for 16 hours under nitrogen protection. The reaction mixture was poured into water (10 mL) and extracted with ethyl acetate (10 mL x 3). The combined organic phases were washed with saturated brine (30 mL), dried over anhydrous sodium sulfate, and concentrated under reduced pressure to afford the crude product. The crude product was purified by preparative silica gel TLC (dichloromethane/methanol = 10/1) to afford the yellow solid product N-cyclopropyl-2-((3-((E)-2-(4-(2-(pyrrolidin-1-yl)ethyl) pyridin-2-yl)ethenyl)-1-(tetrahydro-2H-pyran-2-yl)-1H-indazol-6-yl)thio)benzamide 109g (150 mg, 252.62 μmol, 17.62% yield).

The product was confirmed by LCMS.

MS-ESI calculated for [M+H]+ 594.3, found 594.2.

Step 7

At room temperature, N-cyclopropyl-2-((3-((E)-2-(4-(2-(pyrrolidin-1-yl)ethyl)pyridin-2-yl)ethenyl)-1-(tetrahydro-2H-pyran-2-yl)-1H-indazol-6-yl)thio)benzamide 109g (150 mg, 252.62 μmol) was dissolved in dichloromethane (12 mL), and trifluoroacetic acid (3 mL) was added. The reaction system was stirred at 25°C for 2 hours. The reaction mixture was poured into water (20 mL), and the pH was adjusted to 9-10 with 2 mol/L aqueous sodium hydroxide solution. The mixture was extracted with dichloromethane (20 mL x 3). The combined organic phases were washed with saturated brine (30 mL), dried over anhydrous sodium sulfate, and concentrated under reduced pressure to afford the crude product. The crude product was purified by preparative high-performance liquid chromatography to afford the yellow solid product N-cyclopropyl-2-((3-((E)-2-(4-(2-(pyrrolidin-1-yl)ethyl)pyridin-2-yl)ethenyl)-1H-indazol-6-yl)thio)benzamide I-133 (34.4 mg, 67.57 μmol, 26.75% yield).

The product was confirmed by LCMS and H-NMR.

MS-ESI calculated for [M+H]+ 510.2, found 510.3.

$^1$H NMR (400MHz, $CD_3OD$): δ (ppm) 8.43 (d, *J* = 5.2 Hz, 1H), 8.07 (d, *J* = 8.4 Hz, 1H), 7.87 (d, *J* = 16.4 Hz, 1H), 7.59 (dd, *J* = 18.0, 16.0 Hz, 3H), 7.46-7.44 (m, 1H), 7.35-7.32 (m, 2H), 7.27- 7.25 (m, 1H), 7.22-7.18 (m, 2H), 2.95-2.91 (m, 2H), 2.87-2.80 (m, 2H), 2.80-2.77 (m, 1H), 2.67 (s, 4H), 1.86-1.85 (m, 4H), 0.77 -0.72 (m, 2H), 0.55-0.51 (m, 2H).

Other compounds were prepared and characterized according to the general synthetic schemes described herein and analogous procedures to the foregoing examples, as well as the description in the examples of PCT patent application WO2024/032584A1, as shown below.

Table 1.

| No. | Structural Formula | MS-ESI [M+H]$^+$ | NMR Data |
|---|---|---|---|
| I-1 | 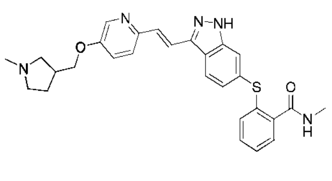 | 500.1 | $^1$H NMR (400 MHz, $CD_3OD$&$CDCl_3$) δ 8.27 (d, J = 2.8 Hz, 1H), 8.07 (d, J = 4.8 Hz, 1H), 7.65-7.77 (m, 2H), 7.43 - 7.50 (m, 4H), 7.26-7.38 (m, 2H), 7.21-7.26 (m, 2H), 4.03-4.11 (m, 2H), 2.92-2.97 (m, 1H), 2.88 (s, 3H), 2.74-2.86 (m, 3H), 2.63-2.69 (m, 1H), 2.50 (s, 3H), 2.14 - 2.23 (m, 1H), 1.72 - 1.80 (m, 1H). |

(continued)

| No. | Structural Formula | MS-ESI $[M+H]^+$ | NMR Data |
|---|---|---|---|
| I-2 | 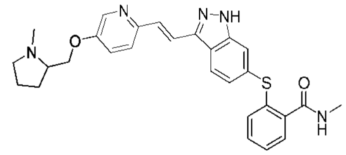 | 500.1 | $^1$H NMR (400MHz, $CDCl_3$&$CD_3OD$): δ (ppm) 8.23 (s, 1H), 7.94 (d, J = 8.0 Hz, 1H), 7.63 (s, 1H), 7.47 (dd, J = 39.8, 29.7 Hz, 4H), 7.24 (s, 2H), 4.02 (d, J = 6.4 Hz,2H), 3.08 (s, 3H), 2.85 (s, 1H), 2.70 (s, 3H), 2.28 - 2.32 (m, 1H), 2.63 (s, 1H), 1.98-2.02 (m, 1H), 1.80 (s, 1H), 0.82 (s, 1H). |
| I-3 | 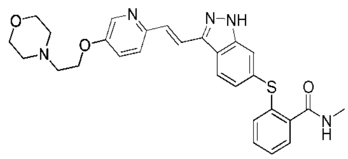 | 516.2 | $^1$H NMR (400MHz, $CDCl_3$): δ (ppm) 10.28 (s, 1H), 8.38-8.37 (m, 1H), 8.02 (d, J = 8.4 Hz, 1H), 7.78-7.74 (m, 1H), 7.68-7.66 (m, 1H), 7.57-7.52 (m, 2H), 7.44 (d, J = 8.8 Hz, 1H), 7.34-7.32 (m, 2H), 7.27-7.22 (m, 3H), 6.37-6.34 (m, 1H), 4.22 (t, J = 5.6 Hz, 2H), 3.78 (t, J = 4.4 Hz, 4H), 2.98 (d, J = 5.2 Hz, 3H), 2.87 (t, J = 5.6 Hz, 2H), 2.63 (s, 4H). |
| I-4 | 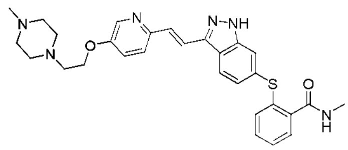 | 529.2 | $^1$H NMR (400 MHz, $CD_3OD$&$CDCl_3$) δ 8.27 (d, J=2.4 Hz, 1H), 8.05 (d, J = 11.2 Hz, 1H), 7.81 (s, 1H), 7.61-7.71 (m, 3H), 7.41-7.50 (m, 3H), 7.27-7.32 (m, 2H), 7.21 (d, J = 1.6 Hz, 2H),4.12 (s, 2H), 2.87 (d, J = 15.2 Hz, 4H), 2.50-2.73 (m, 8H), 2.33 (s, 3H). |
| I-5 | 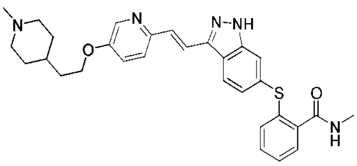 | 528.2 | $^1$H NMR (400MHz, $CDCl_3$&$CD_3OD$):δ 8.24 (d, J = 2.4 Hz, 1H), 8.06 (d, J = 8.6 Hz, 1H), 7.56-7.80 (m, 3H), 7.48-7.55 (m, 2H), 7.41-7.43 (m, 1H),7.32 (dd, J = 13.0, 6.4 Hz, 2H), 7.21 (d, J = 8.6 Hz, 2H), 4.17 (t, J = 6.2 Hz, 2H), 2.95 (d, J = 11.2 Hz, 2H), 2.88 (s, 3H), 2.34 (s, 3H), 2.16 (dd, J = 26.8, 13.4 Hz, 3H), 1.75-1.99 (m, 4H), 1.64 (s, 1H). |
| I-6 | 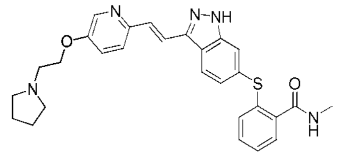 | 500.2 | $^1$H NMR (400 MHz, CD3OD) δ: 8.28 (d, 1H, J=2.8Hz), 8.06 (d, 1H, J=8.4Hz), 7.71 (d, 1H, J=16.4Hz), 7.65 (d, 1H, J=8.8Hz), 7.60 (s, 1H), 7.48-7.55 (m, 2H), 7.45 (dd, 1H, J=8.8, 2.8Hz), 7.30-7.37 (m, 2H), 7.20-7.25 (m, 2H), 4.25 (t, 2H, J=5.6Hz), 3.00 (t, 2H, J=5.6Hz), 2.88 (s, 3H), 2.72-2.74 (m, 4H), 1.85-1.89 (m, 4H). |
| I-7 | 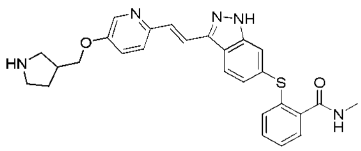 | 486.1 | $^1$H NMR (400 MHz, $CD_3OD$) δ 8.41 (s, 1H), 8.05 (m, 1H), 7.69 - 7.45 (m, 3H), 7.44 - 6.96 (m, 4H), 5.35 (s, 1H), 4.40 - 3.97 (m, 2H), 3.68 - 3.34 (m, 3H), 3.32 - 3.24 (m, 1H), 3.22 - 2.92 (m, 1H), 2.87 (d, J = 12.0 Hz, 3H), 2.42 - 2.18 (m, 1H), 2.08 - 1.84 (m, 1H). |
| I-8 | 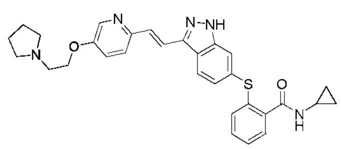 | 526.2 | $^1$H NMR (400 MHz, $CDCl_3$) δ 8.38 (d, J = 2.8 Hz, 1H), 8.01 (d, J = 8.4 Hz, 1H), 7.75 (d, J = 16.4 Hz, 1H), 7.67 (dd, J = 5.6, 3.2 Hz, 1H), 7.58 - 7.41 (m, 2H), 7.35 (dd, J = 24.0, 19.6 Hz, 2H), 7.25 (dd, J = 8.8, 2.8 Hz, 2H), 7.18 (d, J = 8.4 Hz, 1H), 6.54 (s, 1H), 4.24 (t, J = 5.6 Hz, 2H), 3.00 (t, J = 5.6 Hz, 2H), 2.89 - 2.80 (m, 1H), 2.72 (s, 4H), 1.87 (d, J = 3.2 Hz, 4H), 0.86 - 0.75 (m, 2H), 0.50 (t, J = 8.0 Hz, 2H). |

(continued)

| No. | Structural Formula | MS-ESI $[M+H]^+$ | NMR Data |
|---|---|---|---|
| I-9 | 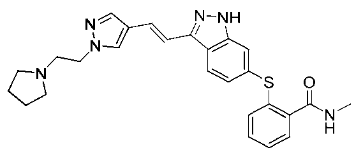 | 473.1 | $^1H$ NMR (400MHz, CD3OD): δ (ppm) 10.61-10.42 (m, 1H), 8.02-7.97 (m, 2H), 7.65 (s, 1H), 7.56 (t, J = 7.6 Hz, 1H), 7.43 (d, J = 16.1 Hz, 1H), 7.32 (d, J = 3.7 Hz, 1H), 7.24 (d, J = 8.3 Hz, 1H), 6.95 (d, J = 7.1 Hz, 1H), 6.70 (d, J = 8.3 Hz, 1H), 6.36 (s, 1H), 4.62 (t, J = 5.9 Hz, 2H), 2.98 (t, J = 6.1 Hz, 3H), 2.70 (s, 2H), 1.85 (s, 2H). |
| I-10 | 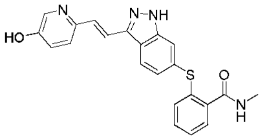 | 403.1 | $^1H$ NMR (400MHz, $CDCl_3$&$CD_3OD$): δ (ppm) 8.05-8.04 (m, 1H), 7.90 (d, J=8.4Hz, 1H), 7.51-7.42 (m, 3H), 7.39-7.35 (m, 2H), 7.21-7.18 (m, 2H), 7.14-7.07 (m, 3H), 2.82 (s, 3H) |
| I-11 | 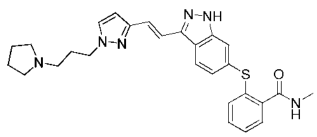 | 487.2 | $^1H$ NMR (400MHz, CD3OD): δ (ppm) δ 8.07-8.01 (m, 1H), 7.67-7.60 (m, 2H), 7.50 - 7.42 (m, 3H), 7.36-7.31(m, 2H), 7.22 (dd, J = 14.8, 8.4 Hz, 2H), 6.65 (d, J = 2.0, 1H), 4.24 (t, J = 6.8 Hz, 1H), 2.88 (s, 3H), 2.66-2.58 (m, 6H), 2.14-2.12 (m, 2H), 1.85-1.77 (m, 4H). |
| I-13 | 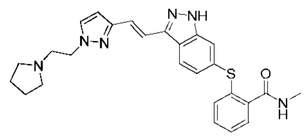 | 473.2 | $^1H$ NMR (400MHz, CD3OD): δ (ppm) δ 8.02 (d, J = 8.8 Hz, 1H), 7.75 (d, J = 2.0 Hz, 1H), 7.59 (s, 1H), 7.50 - 7.46 (m, 3H), 7.37 - 7.31 (m, 2H), 7.25-7.20 (m, 2H), 6.73 (s, 1H), 4.59 (t, J = 5.6 Hz, 2H), 3.79-3.73 (m, 4H), 3.15 (s, 2H), 2.88 (s, 3H), 2.17 (s, 2H), 2.06 (s, 2H). |
| I-14 | 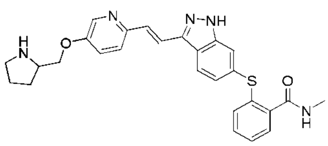 | 487.0 | $^1H$ NMR (400MHz, $CDCl_3$&$CD_3OD$): δ (ppm) 8.24-8.23 (m, 1H), 7.98 (d, J=8.8Hz, 1H), 7.65-7.61 (m, 1H), 7.55-7.52 (m, 1H), 7.50-7.48 (m, 2H), 7.44-7.39 (m, 1H), 7.29-7.22 (m, 3H), 7.19-7.15 (m, 2H), 4.05-4.02 (m, 1H), 3.94 (t, J=8.0Hz, 1H), 3.53-3.50 (m, 1H), 3.04-2.91 (m, 2H), 2.88 (s, 1H), 2.02-1.95 (m, 1H), 1.89-1.79 (m, 2H), 1.64-1.57 (m, 1H). |
| I-15 | 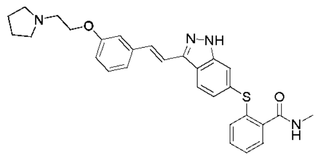 | 499.1 | $^1H$ NMR (400MHz, CD3OD): δ (ppm) 8.07 (d, J = 8.5 Hz, 1H), 7.59 (s, 1H), 7.51-7.49 (m, 3H), 7.35-7.32 (m, 5H), 7.26-7.24 (m, 2H), 6.99 (d, J = 8.0 Hz, 1H), 4.44-4.41 (t, J=9.6 Hz,2H), 3.82-3.74 (m, 2H), 3.72-3.70 (m, 2H), 3.30-3.22 (m, 2H), 2.87 (s, 3H), 2.25-2.17 (m, 2H), 2.11-2.00 (m, 2H). |
| I-16 | 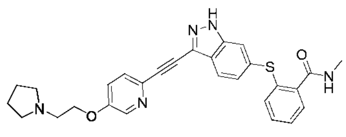 | 498.1 | $^1H$ NMR (400 MHz, $CDCl_3$) δ: 11.14 (br s, 1H), 8.39 (d, J=2.4Hz, 1H), 7.88 (d, J=8.4Hz, 1H), 7.59-7.64 (m, 3H), 7.30-7.31 (m, 2H), 7.19-7.27 (m, 3H), 6.37 (d, J=4.4Hz, 1H), 4.22 (t, J =6.0 Hz, 2H), 2.96-2.99 (m, 5H), 2.68 (s, 4H), 1.85 (s, 4H). |
| I-17 | 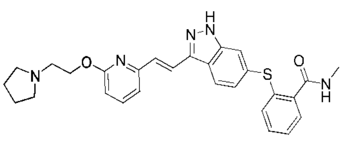 | 500.1 | $^1H$ NMR (400MHz, $CDCl_3$): δ (ppm) 10.61-10.42 (m, 1H), 8.02-7.97 (m, 2H), 7.65 (s, 1H), 7.56 (t, J = 7.6 Hz, 1H), 7.43 (d, J = 16.1 Hz, 1H), 7.32 (d, J = 3.7 Hz, 1H), 7.24 (d, J = 8.3 Hz, 1H), 6.95 (d, J = 7.1 Hz, 1H), 6.70 (d, J = 8.3 Hz, 1H), 6.36 (s, 1H), 4.62 (t, J = 5.9 Hz, 2H), 2.98 (t, J = 6.1 Hz, 3H), 2.70 (s, 2H), 1.85 (s, 2H). |

(continued)

| No. | Structural Formula | MS-ESI $[M+H]^+$ | NMR Data |
|---|---|---|---|
| I-18 | 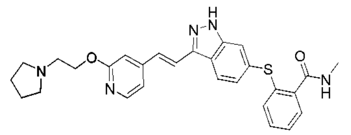 | 500.2 | $^1H$ NMR (400 MHz, $CD_3OD$&$CDCl_3$) δ 8.01 (d, J = 8.6 Hz, 1H), 7.58 (dd, J = 15.6, 8.4 Hz, 3H), 7.46-7.50 (m, 1H), 7.26-7.33 (m, 3H), 7.23 (d, J = 8.6 Hz, 1H), 7.18 (d, J = 8.0 Hz, 1H), 6.74 (d, J = 7.2 Hz, 1H), 6.62 (s, 1H), 4.13 (t, J = 6.8 Hz, 2H), 2.82 - 2.91 (m, 5H), 2.69 (s, 4H), 1.84 (s, 4H). |
| I-19 | 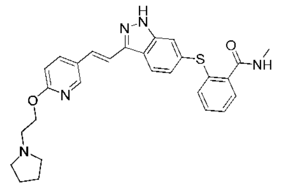 | 500.0 | $^1H$ NMR (400 MHz, $CDCl_3$) δ 11.49 (s, 1H), 7.84 (d, J = 8.5 Hz, 1H), 7.65 (d, J = 5.0 Hz, 1H), 7.43 - 7.28 (m, 6H), 7.22 - 7.08 (m, 2H), 6.94 (dd, J = 41.7, 16.5 Hz, 2H), 6.19 (d, J = 9.4 Hz, 1H), 4.13 (s, 2H), 3.07 - 2.92 (m, 3H), 2.72 (s, 2H), 1.85 (s, 2H), 1.62 (s, 4H), 1.28 (d, J = 10.3 Hz, 2H). |
| I-20 | 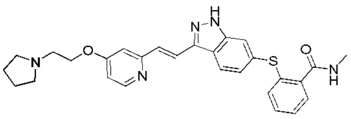 | 500.1 | $^1H$ NMR (400 MHz, CD3OD) δ: 8.38 (d, 1H, J= 6.0Hz), 8.09 (d, 1H, J=8.4Hz), 7.88 (d, 1H, J=16.4Hz), 7.62 (s, 1H), 7.49-7.56 (m, 2H), 7.31-7.36 (m, 3H), 7.23-7.26 (m, 2H), 6.94 (d, 1H, J= 4.0Hz), 4.34-4.37 (m, 2H), 3.13-3.14 (m, 2H), 2.86-2.88 (m, 7H), 1.92 (s, 4H). |
| I-21 | 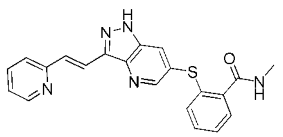 | 388.1 | $^1H$ NMR (400MHz, DMSO-d6): δ (ppm) 13.55 (s, 1H), 8.62 (d, J = 4.4 Hz, 1H), 8.55 (s, 1H), 8.46 (d, J = 4.4 Hz, 1H), 8.20-8.07 (m, 2H), 7.93 (d, J = 16.4 Hz, 1H), 7.81 (t, J = 7.6 Hz, 1H), 7.65 (d, J = 7.6 Hz, 1H), 7.53 (d, J = 8.4 Hz, 1H), 7.39-7.26 (m, 3H), 7.07 (d, J = 7.6 Hz, 1H), 2.78 (d, J = 4.4 Hz, 3H). |
| I-22 | 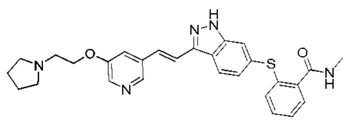 | 500.9 | $^1H$ NMR (400 MHz, $CDCl_3$) δ 8.39 (s, 1H), 8.25 (d, J = 2.6 Hz, 1H), 7.97 (d, J = 8.5 Hz, 1H), 7.70 - 7.65 (m, 1H), 7.52 (s, 1H), 7.43 (d, J = 10.4 Hz, 3H), 7.37 - 7.32 (m, 2H), 7.25 (d, J = 9.7 Hz, 2H), 6.39 (s, 1H), 4.25 (t, J = 5.8 Hz, 2H), 3.00 (dd, J = 10.3, 5.3 Hz, 5H), 2.72 (s, 4H), 1.87 (s, 4H). |
| I-23 | 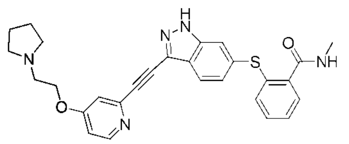 | 498.4 | $^1H$ NMR (400MHz, CD3OD): δ (ppm) 8.39 (d, J=5.6Hz, 1H), 7.89 (d, J=8.4Hz, 1H), 7.62 (s, 1H), 7.49 (d, J=7.2Hz, 1H), 7.35 (s, 3H), 7.22-7.27 (m, 2H), 7.07 (d, J=4.8Hz, 1H), 4.31 (t, J=5.2Hz, 2H), 3.02 (t, J=5.2Hz, 2H), 2.74 (s, 4H), 1.87 (s, 4H). |
| I-24 | 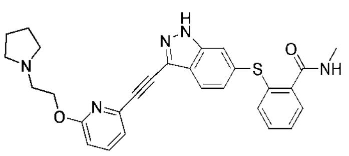 | 498.0 | $^1H$ NMR (400MHz, $CDCl_3$ + CD3OD): δ (ppm) 7.83 (d, J=8.4Hz, 1H), 7.79 (s, 1H), 7.73 (t, J=8.0Hz, 1H), 7.63 (s, 1H), 7.48-7.51 (m, 1H), 7.33-7.34 (m, 1H), 7.31-7.32 (m, 1H), 7.22-7.24 (m, 2H), 6.87 (d, J=8.4Hz, 1H), 4.59 (t, J=5.6Hz, 2H), 3.19 (t, J=5.2Hz, 2H), 2.96 (s, 4H), 2.88 (s, 3H), 1.95 (s, 4H). |
| I-25 | 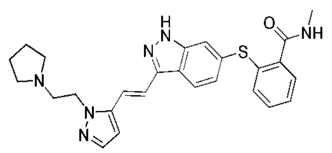 | 473.1 | $^1H$ NMR (400MHz, CD3OD): δ (ppm) δ 8.07 (d, J = 8.4 Hz, 1H), 7.61 - 7.59 (m, 2H), 7.51 - 7.49 (m, 3H), 7.37-7.34 (m, 2H), 7.25-7.21 (m, 2H), 6.81 (d, J = 2.0 Hz, 1H), 4.72 (t, J = 5.6 Hz, 2H), 3.78 (t, J = 5.6 Hz, 4H), 3.15 (s, 2H), 2.88 (s, 3H), 2.17 (s, 1H), 2.05 (s, 1H) |

(continued)

| No. | Structural Formula | MS-ESI [M+H]+ | NMR Data |
|---|---|---|---|
| I-26 | 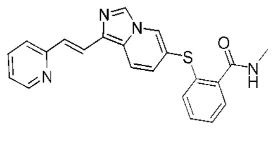 | 387.1 | $^1$H NMR (400 MHz, CD3OD+CD3Cl) δ 8.47 (d, J = 3.6 Hz, 1H), 8.20 (d, J = 16.0 Hz, 2H), 7.77 (d, J = 15.6 Hz, 1H)7.64-7.70 (m, 2H), 7.47 (d, J = 7.2 Hz, 1H), 7.34-7.40 (m, 2H), 7.20-7.30 (m, 2H), 7.15 (d, J =7.2 Hz, 1H), 6.74 (d, J = 9.6 Hz, 1H), 2.92 (s, 3H). |
| I-27 | 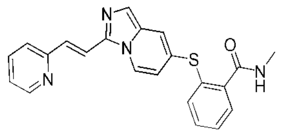 | 387.0 | $^1$H NMR (400 MHz, CD3OD) δ 8.57 (d, J = 4.4 Hz, 1H), 8.42 (d, J = 7.2 Hz, 1H), 7.96 (d, J = 15.6 Hz, 1H), 7.86 - 7.90 (m, 1H), 7.68 (d, J = 7.6 Hz, 1H), 7.63 (s, 1H), 7.51-7.56 (m, 3H), 7.92-7.44 (m, 3H), 7.32-7.35 (m, 1H), 6.67 (dd, J = 7.6, 1.6 Hz, 1H), 2.88 (s, 3H). |
| I-28 | 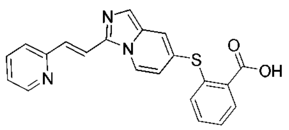 | 374.1 | $^1$H NMR (400 MHz, $CDCl_3$&CD3OD) δ 8.59-8.71 (m, 3H), 8.13 (t, J = 7.2 Hz, 1H), 8.04 (d, J = 7.6 Hz, 1H), 7.83 (d, J = 7.6 Hz, 1H), 7.76 (s, 1H), 7.66 (s, 1H), 7.51-7.55 (m, 2H), 7.34 (t, J = 7.2 Hz, 1H), 7.23 (t, J = 7.2 Hz, 1H), 7.06 (d, J = 8.0 Hz, 1H), 6.82 (d, J = 6.8 Hz, 1H). |
| I-29 | 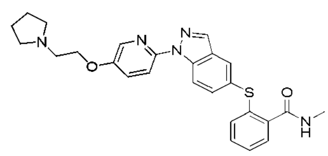 | 474.2 | $^1$H NMR (400 MHz, CD3OD) δ: 8.73 (d, 1H, J=8.8Hz), 8.23 (d, 1H, J=2.8Hz), 8.16 (d, 1H, J= 0.4Hz), 7.93-7.98 (m, 2H), 7.58-7.60 (m, 1H), 7.55 (dd, 1H, J=8.8, 1.6Hz), 7.46 (dd, 1H, J =8.8, 2.8Hz), 7.21-7.23 (m, 2H), 7.01-7.04 (m, 1H), 6.30 (s, 1H), 4.23 (t, 2H, J=6.0Hz), 3.04 (d, 3H, J=4.8Hz), 2.98 (t, 2H, J=6.0Hz), 2.66-2.68 (m, 4H), 1.84-1.88 (m, 4H). |
| I-30 | 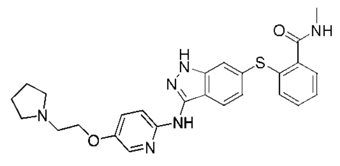 | 489.1 | $^1$H NMR (400MHz, DMSO-d6): δ 12.18 (s,1H), 9.46 (s,1H), 8.37-8.38 (m,1H), 8.07 (d, 1H, J=8.4Hz), 7.91-7.95 (m, 2H), 7.49 (d, 1H, J=7.6Hz), 7.38-7.42 (m, 2H), 7.24-7.32 (m, 2H), 6.96-7.02 (m, 2H), 4.07-4.09 (m, 2H), 2.77-2.79 (m, 6H), 2.51-2.52 (m, 3H), 1.68 (s, 4H). |
| I-31 | 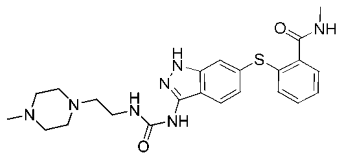 | 468.1 | $^1$H NMR (400MHz, $CDCl_3$&$CD_3OD$): δ (ppm) 7.74 (s, 1H), 7.46 (d, J = 19.2 Hz, 3H), 7.28 - 7.24 (m, 1H), 7.17 (s, 1H), 7.04 (d, J = 8.5 Hz, 1H), 3.47 (t, J = 6.2 Hz, 2H), 2.89 (s, 3H), 2.83 - 2.35 (m, 10H), 2.31 (s, 3H). |
| I-32 | 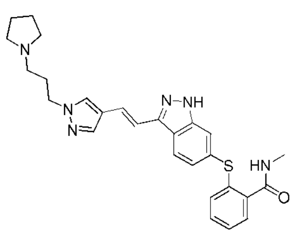 | 487.1 | $^1$H NMR (400 MHz, $CDCl_3$) δ (ppm) 7.91 (d, J = 8.0 Hz, 1H), 7.74 (s, 1H), 7.66-7.62 (m, 1H), 7.58 (s, 1H), 7.47 (s, 1H), 7.34-7.29 (m, 3H), 7.25-7.21 (m, 2H), 7.20-7.17 (m, 1H), 7.11 (d, J = 16.8 Hz, 1H), 6.33 (s, 1H), 4.22 (t, J = 6.8 Hz, 2H), 2.96 (d, J = 4.8 Hz, 3H), 2.59 (d, J = 19.2 Hz, 5H), 2.18-2.11 (m, 2H), 1.84 (s, 5H). |
| I-33 | 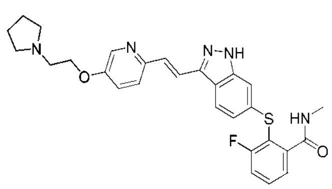 | 518.1 | $^1$H NMR (400MHz, $CDCl_3$&$CD_3OD$): δ (ppm) 8.26 (d, J = 2.8 Hz, 1H), 8.01 (d, J = 8.4 Hz, 1H), 7.67 (d, J = 16.8 Hz, 1H), 7.61 (d, J = 8.8 Hz, 1H), 7.54 - 7.48 (m, 2H), 7.40 (dd, J = 8.8, 2.8 Hz, 1H), 7.29-7.23 (m, 2H), 7.17 (dd, J = 8.4, 1.2 Hz, 1H), 7.11-7.06 (m, 1H), 4.23 (t, J = 5.6 Hz, 2H), 2.98 (t, J = 5.2 Hz, 2H), 2.87 (s, 3H), 2.71 (s, 4H), 1.86 (t, J = 3.2 Hz, 4H). |

(continued)

| No. | Structural Formula | MS-ESI $[M+H]^+$ | NMR Data |
|---|---|---|---|
| I-34 | 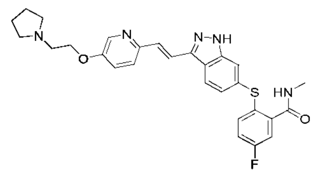 | 518.0 | $^1H$ NMR (400MHz, $CDCl_3$&$CD_3OD$): δ (ppm) 8.28 (d, J = 2.4 Hz, 1H), 8.13 (d, J = 8.4 Hz, 1H), 7.74 - 7.70 (m, 2H), 7.68-7.63 (m, 1H), 7.57 - 7.50 (m, 2H), 7.44 (dd, J = 8.8, 2.8 Hz, 1H), 7.28 (d, J = 9.6 Hz, 1H), 6.97 - 6.93 (m, 1H), 6.70 (dd, J = 10.0, 2.4 Hz, 1H), 4.26 (t, J = 5.2 Hz, 2H), 3.03 (s, 2H), 2.91 (s, 3H), 2.76 (s, 4H), 1.89 (s, 4H). |
| I-35 | 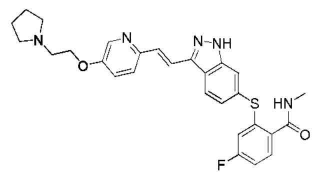 | 518.1 | $^1H$ NMR (400MHz, $CDCl_3$&$CD_3OD$): δ (ppm) δ 8.29 (d, J = 2.8 Hz, 1H), 8.13 (d, J= 8.4 Hz, 1H), 7.75 - 7.70 (m, 2H), 7.68-7.63 (m, 1H), 7.57-7.54 (m, 1H), 7.53 - 7.50 (m, 1H), 7.44 (dd, J = 8.8, 2.8 Hz, 1H), 7.28 (d, J = 10.0 Hz, 1H), 6.98-6.93 (m, 1H), 6.71 (dd, J = 9.6, 2.4 Hz, 1H), 4.27 (t, J = 5.6 Hz, 2H), 3.05 (t, J = 5.2 Hz, 2H), 2.91 (s, 3H), 2.78 (s, 4H), 1.89 (s, 4H). |
| I-36 | 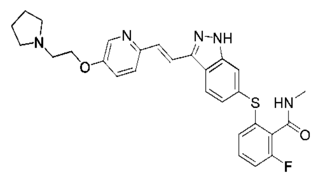 | 518.1 | $^1H$ NMR (400 MHz, CD3OD) δ 8.29 (d, J = 2.8 Hz, 1H), 8.09 (d, J = 8.4 Hz, 1H), 7.74 - 7.64 (m, 3H), 7.56-7.45 (m, 2H), 7.37-7.31 (m, 1H), 7.26 (d, J = 8.4 Hz, 1H), 7.12-7.05 (m, 2H), 4.27 (t, J = 5.2 Hz, 2H), 3.03 (t, J = 6.0 Hz, 2H), 2.89 (s, 3H), 2.77 (s, 4H), 1.89 (s, 4H). |
| I-37 | 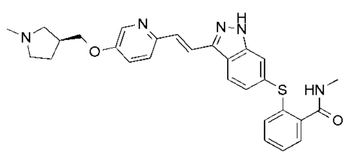 | 500.1 | $^1H$ NMR (400 MHz, $CD_3OD$) δ 8.27 (d, J = 2.8 Hz, 1H), 8.04 (d, J = 8.4 Hz, 1H), 7.78 (s, 1H), 7.69 (d, J = 16.4 Hz, 1H), 7.65 - 7.60 (m, 2H), 7.49 (s, 1H), 7.42 (dd, J = 8.8, 2.8 Hz, 1H), 7.29 (dd, J = 13.2, 6.8 Hz, 2H), 7.23 - 7.18 (m, 2H), 4.11 (d, J = 5.2 Hz, 2H), 3.13 (dd, J = 9.2, 4.8 Hz, 1H), 2.88 (s, 3H), 2.82 (s, 1H), 2.54 (s, 3H), 2.45 - 2.38 (m, 1H), 2.16 - 2.08 (m, 1H), 1.90 - 1.82 (m, 2H), 1.77 (dd, J = 13.2, 6.0 Hz, 1H). |
| I-38 | 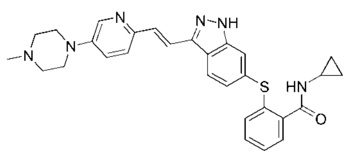 | 511.0 | $^1H$ NMR (400MHz, CD3OD): δ (ppm) 8.22 (d, J = 2.8 Hz, 1H), 8.02 (d, J = 8.8 Hz, 1H), 7.64 - 7.54 (m, 3H), 7.49-7.43 (m, 2H), 7.40 - 7.29 (m, 3H), 7.25 - 7.23 (m, 1H), 7.17 (d, J = 8.4 Hz, 1H), 3.33 (s, 4H), 2.79-2.75 (m, 1H), 2.65-2.62 (m, 4H), 2.36 (s, 3H), 0.77-0.72 (m, 2H), 0.55 - 0.51 (m, 2H). |
| I-39 | 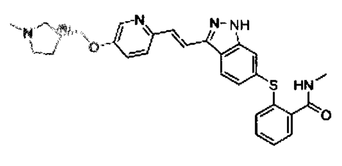 | 500.1 | $^1H$ NMR (400 MHz, $CD_3OD$&$CDCl_3$) δ 8.27 (s, 1H), 8.04 (d, J = 8.4 Hz, 1H), 7.76 (s, 1H), 7.71-7.67 (m, 1H), 7.64-7.61 (m, 2H), 7.49-7.47 (m, 1H), 7.43-7.41 (m, 1H), 7.33-7.27 (m, 2H), 7.21 (t, J = 8.0 Hz, 2H), 4.11 (d, J = 5.2 Hz, 2H), 3.15 - 3.13 (m, 1H), 2.89 (s, 3H), 2.82 (s, 1H), 2.54 (s, 3H), 2.43-2.39(m, 1H), 2.16-2.11 (m, 1H), 1.88-1.85 (m, 2H), 1.80-1.75 (m, 2H). |
| I-40 | 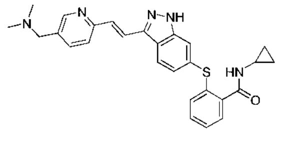 | 470.1 | $^1H$ NMR (400MHz, CD3OD): δ 8.49 (s, 1H), 8.05 (d, J = 8.64 Hz, 1H), 7.86 (d, J = 16.8 Hz, 1H), 7.80 (dd, J = 8.0 Hz, 1.6 Hz, 1H), 7.68 (d, J = 8.0 Hz, 1H), 7.56 (d, J = 16.6 Hz, 2H), 7.49 - 7.44 (m, 1H), 7.37 - 7.27 (m, 2H), 7.24-7.2 (m, 2H), 3.57 (s, 2H), 2.63-2.8 (m,1H), 2.3 (s, 6H), 0.76 (q, J = 7.0 Hz, 2H), 0.60 - 0.51 (m, 2H). |

(continued)

| No. | Structural Formula | MS-ESI $[M+H]^+$ | NMR Data |
|---|---|---|---|
| I-41 | 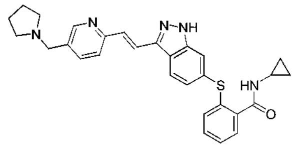 | 496.1 | $^1H$ NMR (400MHz, CD3OD): δ 8.53 (d, J = 1.6 Hz, 1H), 8.06 (d, J = 8.5 Hz, 1H), 7.85 (dd, J = 16.7 Hz, 9.3 Hz, 2H), 7.69 (d, J = 8.1 Hz, 1H), 7.60 - 7.53 (m, 2H), 7.45 (dd, J = 7.1 Hz, 1.9 Hz, 1H), 7.34-7.32 (m, 2H), 7.28 - 7.24 (m, 1H), 7.20 (dd, J = 8.5 Hz, 1.4 Hz, 1H), 3.75 (s, 2H), 2.78-2.64 (m, 1H), 2.64 (s, 4H), 1.86 (s, 4H), 0.78 - 0.71 (m, 2H), 0.56 - 0.50 (m, 2H). |
| I-42 | 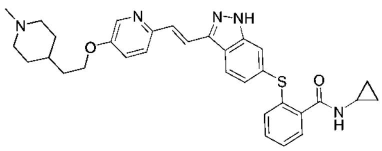 | 554.1 | $^1H$ NMR (400MHz, $CD_3OD$): δ (ppm) 8.30 (s, 2H), 8.23 (d, *J* = 2.8 Hz, 1H), 8.03 (d, *J* = 8.4 Hz, 1H), 7.66 (dd, *J* = 23.2, 12.6 Hz, 2H), 7.58-7.47 (m, 2H), 7.44-7.42(m, 2H), 7.38-7.29 (m, 2H), 7.28-7.15 (m, 2H), 4.18 (t, *J* = 6.0 Hz, 2H), 3.49 (d, *J* = 11.4 Hz, 2H), 3.02 (s, 2H), 2.85 (s, 3H), 2.81-2.75 (m, 1H), 2.07 (d, *J* = 14.6 Hz, 2H), 1.91 (s, 1H), 1.88-1.81 (m, 2H), 1.56 (s, 2H), 0.74 (dt, *J* = 6.8, 3.4 Hz, 2H), 0.61-0.48 (m, 2H). |
| I-43 | 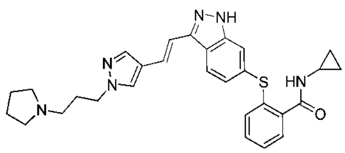 | 513.1 | $^1H$ NMR (400MHz, $CD_3OD$): δ (ppm) 8.54 (s, 1H), 7.98 (d, *J* = 8.6 Hz, 1H), 7.91 (s, 1H), 7.82 (s, 1H), 7.51 (s, 1H), 7.47-7.42 (m, 1H), 7.37-7.30 (m, 3H), 7.26-7.22 (m, 1H), 7.15 (s, 2H), 4.26 (t, *J* = 6.4 Hz, 2H), 3.07 (s, 4H), 2.99-2.92 (m, 2H), 2.82-2.73 (m, 1H), 2.27-2.18 (m, 2H), 1.98 (dd, *J* = 8.4, 5.4 Hz, 4H), 0.75 (td, *J* = 7.2, 5.2 Hz, 2H), 0.58-0.48 (m, 2H). |
| I-44 | 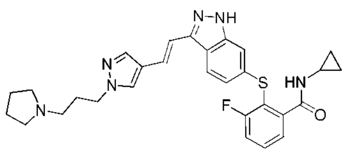 | 531.1 | $^1H$ NMR (400MHz,$CD_3OD$): δ (ppm) 7.92-7.89 (m, 2H), 7.78 (s, 1H), 7.54-7.49 (m, 1H), 7.33-7.26 (m, 4H), 7.16-7.05 (m, 2H), 4.11 (t, *J* = 6.8 Hz, 2H), 2.78-2.73 (m, 1H), 2.75 (s, 4H), 2.49 (t, *J* = 7.6 Hz, 2H), 2.13-2.05 (m, 2H), 1.82-1.80 (m, 4H), 0.75-0.70 (m, 2H), 0.52-0.48 (m, 2H). |
| I-46 | 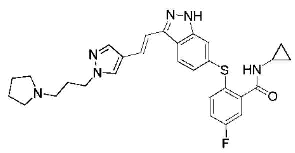 | 531.0 | $^1H$ NMR (400 MHz $CD_3OD$): δ (ppm) 7.98 (d, J = 8.4 Hz, 1H), 7.90 (s, 1H), 7.79 (s, 1H), 7.45 (s, 1H), 7.37-7.32 (m, 2H), 7.24 (dd, J = 8.4, 2.8 Hz, 1H), 7.18-7.13 (m, 2H), 7.11 (dd, J = 8.4, 1.2 Hz, 1H), 4.23 (t, J = 6.8 Hz, 2H), 2.78-2.75 (m, 1H), 2.61 (s, 4H), 2.53 (t, J = 7.6 Hz, 2H), 2.14-2.07 (m, 2H), 1.84-1.81 (m, 4H), 0.76-0.71 (m, 2H), 0.53-0.49 (m, 2H). |
| I-51 | 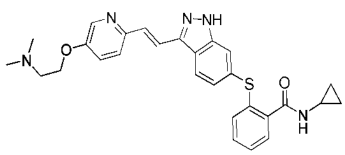 | 500.2 | $^1H$ NMR (400MHz, $CDCl_3$): δ (ppm) 8.36 (d, *J* = 2.8 Hz, 1H), 7.99 (d, *J* = 8.6 Hz, 1H), 7.75-7.63 (m, 2H), 7.73 (d, *J* = 16.0 Hz, 1H), 7.68-7.65 (m, 1H), 7.50-7.46 (m, 1H), 7.43-7.41 (m, 2H), 7.35-7.31 (m, 3H), 7.17 (d, *J* = 8.0 Hz, 1H), 6.51 (s, 1H), 4.25 (s, 2H), 2.92 (s, 2H), 2.85-2.78 (m, 1H), 2.48 (s, 6H), 0.80-0.78 (m, 2H), 0.49-0.45 (m, 2H). |
| I-52 | 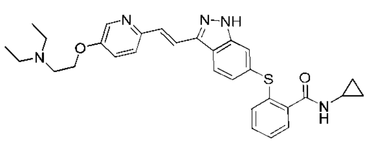 | 528.2 | $^1H$ NMR (400MHz, $CD_3OD$): δ (ppm) 8.28 (d, *J* = 2.8 Hz, 1H), 8.04 (d, *J* = 8.4 Hz, 1H), 7.68 (dd, *J* = 24.6, 12.6 Hz, 2H), 7.57-7.42 (m, 4H), 7.34-7.31 (m, 2H), 7.25 (dd, *J* = 7.4, 1.8 Hz, 1H), 7.18 (dd, *J* = 8.4, 1.4 Hz, 1H), 4.29 (t, *J* = 5.4 Hz, 2H), 3.18 (t, *J* = 5.2 Hz, 2H), 2.92 (q, *J* = 7.2 Hz, 4H), 2.78 (d, *J* = 3.8 Hz, 1H), 1.20 (t, *J* = 7.2 Hz, 6H), 0.75 (dd, *J* = 7.2, 1.8 Hz, 2H), 0.54 (dd, *J* = 3.8, 2.2 Hz, 2H). |

(continued)

| No. | Structural Formula | MS-ESI $[M+H]^+$ | NMR Data |
|---|---|---|---|
| I-53 | 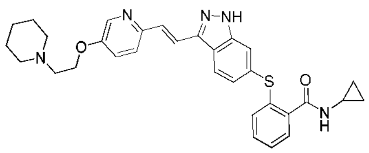 | 540.2 | $^{1}$H NMR (400MHz, $CD_3OD$): δ (ppm) 8.28 (d, $J$ = 2.8 Hz, 1H), 8.05 (d, $J$ = 8.6 Hz, 1H), 7.71 (d, $J$ = 16.8 Hz, 1H), 7.65 (d, $J$ = 8.6 Hz, 1H), 7.55 (d, $J$ = 3.4 Hz, 1H), 7.44 (dd, $J$ = 4.6, 1.8 Hz, 1H), 7.36-7.30 (m, 1H), 7.27-7.24 (m, 1H), 7.18 (dd, $J$ = 8.6, 1.4 Hz, 1H), 4.29 (t, $J$ = 5.4 Hz, 2H), 2.95 (s, 2H), 2.81-2.66 (m, 5H), 1.74-1.64 (m, 2H), 1.53 (s, 2H), 0.77-0.69 (m, 2H), 0.56-0.49 (m, 2H). |
| I-55 | 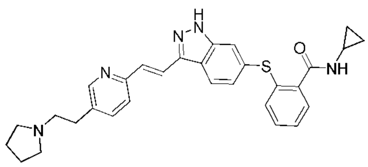 | 512.2 | $^{1}$H NMR (400MHz, $CD_3OD$): δ (ppm) 8.53 (s, 1H), 8.49 (s, 1H), 8.03 (d, $J$ = 8.4 Hz, 1H), 7.81 (dd,$J$ = 26.2, 12.4 Hz, 2H), 7.64 (d, $J$ = 8.0 Hz, 1H), 7.57-7.42 (m, 3H), 7.39-7.28 (m, 2H), 7.24 (d, $J$ = 7.2 Hz, 1H), 7.18 (d, $J$ = 8.4 Hz, 1H), 3.48-3.32 (m, 6H), 3.15-3.04 (m, 2H), 2.84 -2.73 (m, 1H), 2.09 (s, 4H), 0.82-0.66 (m, 2H), 0.61-0.47 (m, 2H). |
| I-59 | 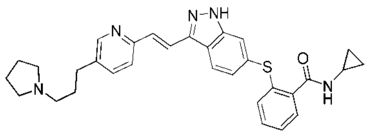 | 540.2 | $^{1}$H NMR (400MHz, $CD_3OD$): δ (ppm) 8.42 (d, $J$ = 1.6 Hz, 1H), 8.05 (d, $J$ = 8.8 Hz, 1H), 7.82 (d, $J$ = 16.8 Hz, 1H), 7.73 (dd, $J$ = 8.4, 2.4 Hz, 1H), 7.65 (d, $J$ = 8.0 Hz, 1H), 7.57-7.53 (m, 2H), 7.45 (dd, $J$ = 6.8, 1.6 Hz, 1H), 7.34-7.30 (m, 2H), 7.27-7.25 (m, 1H), 7.19 (dd, $J$ = 8.8, 1.6 Hz, 1H), 2.80-2.68 (m, 8H), 2.03-1.88 (m, 7H), 0.90-0.88 (m, 2H), 0.55-0.51 (m, 2H). |
| I-60 | 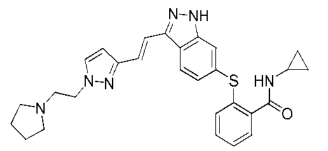 | 499.1 | $^{1}$H NMR (400 MHz, $CD_3OD$) δ (ppm) 7.99 (d, $J$ = 8.6 Hz, 1H), 7.63 (d, $J$ = 2.4 Hz, 1H), 7.56 (s, 1H), 7.40 (d, $J$ = 5.4 Hz, 3H), 7.30 (d, $J$ = 7.4, 3.6 Hz, 2H), 7.24-7.19 (m, 1H), 7.17 (dd, $J$ = 8.6, 1.4 Hz, 1H), 6.61 (d, $J$ = 2.4 Hz, 1H), 4.31 (s, 2H), 3.02 (d, $J$ = 6.9 Hz, 2H), 2.85-2.76 (m, 1H), 2.60 (s, 4H), 1.85-1.78 (m, 4H), 0.80-0.72 (m, 2H), 0.60-0.53 (m, 2H). |
| I-61 | 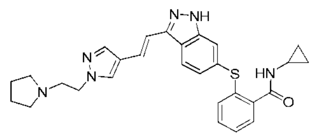 | 499.1 | $^{1}$H NMR (400MHz, $CD_3OD$): δ (ppm) 7.99 (d, $J$ = 8.4 Hz, 1H), 7.93 (s, 1H), 7.81 (s, 1H), 7.52 (s, 1H), 7.45-7.43 (m, 1H), 7.37-7.29 (m, 3H), 7.25-7.23 (m, 1H), 7.19-7.13 (m, 2H), 4.33 (t, $J$ = 6.8 Hz, 2H), 3.04 (t, $J$ = 6.8 Hz, 2H), 2.79-2.75 (m, 1H), 2.65 (s, 4H), 1.83-1.82 (m, 4H), 0.76-0.72 (m, 2H), 0.56-0.52 (m, 2H). |
| I-67 | 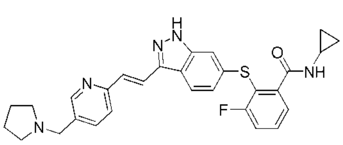 | 514.2 | $^{1}$H NMR (400MHz,$CD_3OD$): δ (ppm) 8.51 (d, $J$ = 1.6 Hz, 1H), 7.98 (d, $J$ = 8.8 Hz, 1H), 7.88-7.79 (m, 2H), 7.67 (d, $J$ = 8.0 Hz, 1H), 7.58-7.47 (m, 2H), 7.39 (s, 1H), 7.30-7.26 (m, 2H), 7.13-7.10 (m, 1H), 3.70 (s, 2H), 2.78-2.74 (m, 1H), 2.59 (s, 4H), 1.84 (s, 4H), 0.78-0.68 (m, 2H), 0.52-0.48 (m, 2H). |
| I-68 | 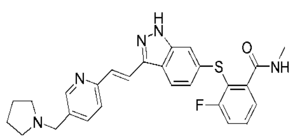 | 488.3 | $^{1}$H NMR (400MHz, $CD_3OD$): δ (ppm) 8.49 (d, J = 1.2 Hz, 1H), 7.97 (d, J= 8.8 Hz, 1H), 7.84-7.79 (m, 2H), 7.64 (d, J= 8.0 Hz, 1H), 7.54-7.50 (m, 2H), 7.39 (s, 1H), 7.32-7.26 (m, 2H), 7.12 (d, J=8.4 Hz, 1H), 3.69 (s, 2H), 2.83 (s, 3H), 2.59 (s, 4H), 1.83 (s, 4H). |

(continued)

| No. | Structural Formula | MS-ESI $[M+H]^+$ | NMR Data |
|---|---|---|---|
| I-69 | 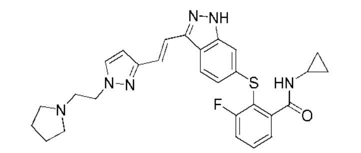 | 517.1 | $^1H$ NMR (400MHz, $CD_3OD$): δ (ppm) 7.90 (d, *J* = 8.8 Hz, 1H), 7.63 (d, *J* = 2.4 Hz, 1H), 7.53-7.48 (m, 1H), 7.42-7.32 (m, 3H), 7.29-7.24 (m, 2H), 7.08 (dd, *J* = 8.4, 1.6 Hz, 1H), 6.60 (d, *J* = 2.4 Hz, 1H), 4.29 (t, *J* = 6.8 Hz, 2H), 2.99 (t, *J* = 6.8 Hz, 2H), 2.78-2.74 (m, 1H), 2.58-2.56 (m, 4H), 1.81-1.78 (m, 4H), 0.75-0.70 (m, 2H), 0.52-0.48 (m, 2H). |
| I-73 | 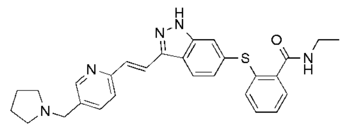 | 484.2 | $^1H$ NMR (400MHz, $CD_3OD$): δ (ppm) 8.66 (d, *J* = 1.6 Hz, 1H), 8.48 (s, 1H), 8.05 (d, *J* = 8.6 Hz, 1H), 7.99-7.88 (m, 2H), 7.72 (d, *J* = 8.2 Hz, 1H), 7.61-7.52 (m, 2H), 7.51-7.44 (m, 1H), 7.39-7.28 (m, 2H), 7.28-7.17 (m, 2H), 4.32 (s, 2H), 3.34 (dd, *J* = 14.2, 6.8 Hz, 2H), 3.25 (s, 4H), 2.06 (s, 4H), 1.16 (t, *J* = 7.4 Hz, 3H). |
| I-74 | 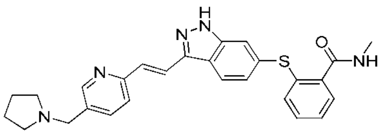 | 470.1 | $^1H$ NMR (400 MHz, $CD_3OD$): δ (ppm) 8.53 (s, 1H), 8.07 (d, *J* = 8.6 Hz, 1H), 7.85 (dd, *J* = 18.2, 9.4 Hz, 2H), 7.69 (d, *J* = 8.2 Hz, 1H), 7.57 (d, *J* = 16.8 Hz, 2H), 7.49-7.45 (m, 1H), 7.34-7.31 (m, 2H), 7.23 (dd, *J* = 9.0, 4.6 Hz, 1H), 3.74 (s, 2H), 2.86 (s, 2H), 2.63 (s, 4H), 1.85 (s, 4H). |
| I-75 | 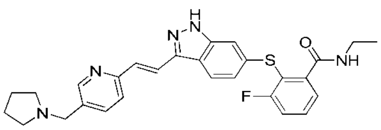 | 502.2 | $^1H$ NMR (400 MHz, $CD_3OD$): δ (ppm) 8.63 (s, 1H), 7.98-7.83 (m, 3H), 7.70 (d, J=8.0, 1H), 7.56-7.51 (m, 2H), 7.38-7.27 (m, 3H), 7.13 (d, J=8.4, 1H), 4.26 (s, 2H), 3.35-3.29 (m, 2H), 3.19 (s, 4H), 2.03 (s, 4H), 1.13 (t, J=7.2, 3H). |
| I-76 | 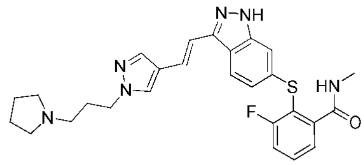 | 505.2 | $^1H$ NMR (400MHz, $CD_3OD$): δ (ppm) 7.90-7.89 (m, 2H), 7.82 (s, 1H), 7.55-7.50 (m, 1H), 7.34-7.26 (m, 4H), 7.17-7.06 (m, 2H), 4.27 (t, J=6.4 Hz, 2H), 3.21 (s, 4H), 3.07 (t, J=4.6 Hz, 2H), 2.83 (s, 3H), 2.29-2.22 (m, 2H), 2.04 (s, 4H). |
| I-77 | 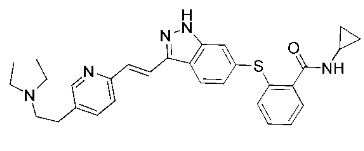 | 512.2 | $^1H$ NMR (400MHz, $CD_3OD$): δ (ppm) 8.52 (s, 1H), 8.34 (s, 2H), 8.05 (d, *J* = 8.4 Hz, 1H), 7.84 (dd, *J*= 19.6, 12.4 Hz, 2H), 7.67 (d, *J* = 8.2 Hz, 1H), 7.60-7.45 (m, 3H), 7.39-7.17 (m, 4H), 3.41 (dd, *J* = 10.4, 6.6 Hz, 2H), 3.35 (d, *J* = 7.4 Hz, 2H), 3.32-3.25 (m, 2H), 3.12 (dd, *J* = 10.4, 6.4 Hz, 2H), 2.85-2.74 (m, 1H), 1.38 (t, *J* = 7.2 Hz, 6H), 0.83-0.69 (m, 2H), 0.61-0.49 (m, 2H). |
| I-78 | 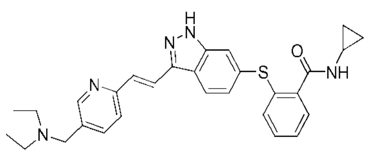 | 498.2 | $^1H$ NMR (400MHz, $CD_3OD$): δ (ppm) 8.54 (s, 1H), 8.07 (d, *J* = 8.4 Hz, 1H), 7.90-7.84 (m, 2H), 7.70 (d, *J* = 7.6 Hz, 1H), 7.59-7.55 (m, 2H), 7.45 (dd, *J* = 6.8, 1.6 Hz, 1H), 7.37-7.32 (m, 2H), 7.27 -7.25 (m, 1H), 7.20 (d, *J* = 8.4 Hz, 1H), 3.79 (s, 2H), 2.80-2.75 (m, 1H), 2.70 (s, 4H), 1.15 (t, *J* = 7.2 Hz, 6H), 0.77-0.72 (m, 2H), 0.55-0.51 (m, 2H). |
| I-79 | 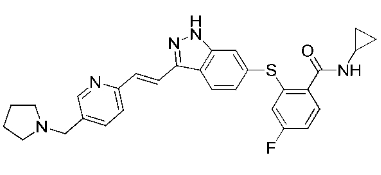 | 514.2 | $^1H$ NMR (400 MHz, $CD_3OD$) δ (ppm) 8.68 (s, 1H), 8.44 (s, 1H), 8.13 (d, J = 8.56 Hz, 1H), 7.99-7.95 (m, 2H), 7.75-7.71 (m, 2H), 7.59 (d, J = 16.4 Hz, 1H), 7.49 (dd, J = 8.6, 5.8 Hz, 1H), 7.26 (d, J = 8.4 Hz, 1H), 7.01-6.96 (m, 1H), 6.75 (dd, J = 9.8, 2.4 Hz, 1H), 4.39 (s, 2H), 3.31 (s, 4H), 2.92-2.72 (m, 1H), 2.09 (s, 4H), 0.8 1-0.76 (m, 2H), 0.61-0.57 (m, 2H). |

(continued)

| No. | Structural Formula | MS-ESI [M+H]+ | NMR Data |
|---|---|---|---|
| I-80 | 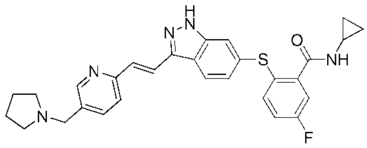 | 514.3 | $^1$H NMR (400MHz, $CD_3OD$): δ (ppm) 8.51 (d, *J* = 1.6 Hz, 1H), 8.04 (d, *J* = 8.6 Hz, 1H), 7.83 (dd, *J* = 15.0, 9.4 Hz, 2H), 7.67 (d, *J* = 8.0 Hz, 1H), 7.60-7.45 (m, 2H), 7.37 (dd, *J* = 8.8, 5.2 Hz, 1H), 7.24 (dd, *J* = 8.6, 2.8 Hz, 1H), 7.20-7.04 (m, 2H), 3.71 (s, 2H), 2.79-2.74 (m, 1H), 2.60 (s, 4H), 1.84 (s, 4H), 0.73 (dd, *J* = 7.0, 1.8 Hz, 2H), 0.51 (dd, *J* = 3.8, 2.0 Hz, 2H). |
| I-81 | 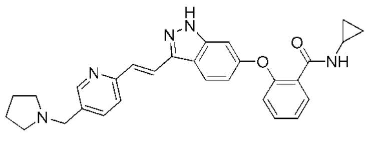 | 480.3 | $^1$H NMR (400MHz, $CD_3OD$): δ (ppm) 8.52 (s, 1H), 8.10 (d, J = 9.2 Hz, 1H), 7.88-7.84 (m, 2H), 7.76 (d, J = 7.6 Hz, 1H), 7.69 (d, J = 7.6 Hz, 1H), 7.59-7.49 (m, 2H), 7.30 (t, J = 7.6 Hz, 1H), 7.07 (d, J = 8.4 Hz, 1H), 7.01 (d, J = 8.8 Hz, 1H), 6.93 (s, 1H), 3.71 (s, 2H), 2.73-2.70 (m, 1H), 2.60 (s, 4H), 1.84 (s, 4H), 0.71-0.66 (m, 2H), 0.39-0.35 (m, 2H). |
| I-82 | 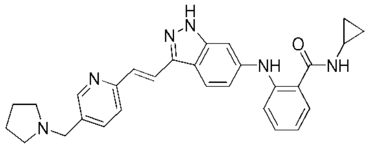 | 479.1 | $^1$H NMR (400MHz, $CD_3OD$): δ (ppm) 8.68 (d, *J* = 1.6 Hz, 1H), 8.03-7.89 (m, 3H), 7.74 (d, *J* = 8.2 Hz, 1H), 7.61-7.52 (m, 2H), 7.45 (d, *J* = 8.2 Hz, 1H), 7.40-7.33 (m, 1H), 7.21 (d, *J* = 1.6 Hz, 1H), 7.05 (dd, *J* = 8.8, 1.6 Hz, 1H), 6.92 (t, *J* = 7.6 Hz, 1H), 4.41 (s, 2H), 3.33 (dd, *J* = 11.0, 4.2 Hz, 4H), 2.84-2.75 (m, 1H), 2.10 (t, *J* = 6.6 Hz, 4H), 0.80-0.72 (m, 2H), 0.59-0.51 (m, 2H). |
| I-83 | 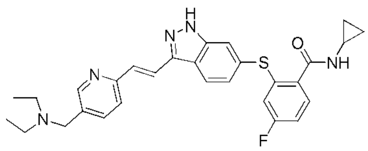 | 516.3 | $^1$H NMR (400MHz, $CD_3OD$): δ (ppm) 8.68 (s, 1H), 8.43 (s, 1H), 8.15 (d, *J*= 8.6 Hz, 1H), 7.99 (t, *J* = 10.2 Hz, 2H), 7.80-7.69 (m, 2H), 7.61 (d, *J* = 16.4 Hz, 1H), 7.50 (dd, *J* = 8.6, 5.8 Hz, 1H), 7.27 (dd, *J* = 8.6, 1.2 Hz, 1H), 6.99 (d, *J* = 2.4 Hz, 1H), 6.75 (dd, *J* = 9.8, 2.4 Hz, 1H), 4.35 (s, 2H), 3.18 (q, *J* = 7.2 Hz, 4H), 2.81 (s, 1H), 1.35 (t, *J* = 7.2 Hz, 6H), 0.83-0.73 (m, 2H), 0.59 (dd, *J* = 3.8, 2.2 Hz, 2H). |
| I-84 | 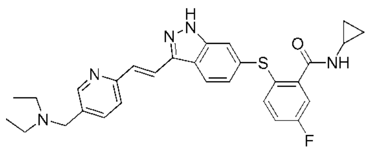 | 516.0 | $^1$H NMR (400MHz, $CD_3OD$): δ (ppm) 8.63 (s, 1H), 8.51 (s, 1H), 8.04 (d, *J* = 8.6 Hz, 1H), 7.97-7.90 (m, 2H), 7.72 (d, *J* = 8.0 Hz, 1H), 7.60-7.46 (m, 2H), 7.38 (dd, *J* = 8.8, 5.2 Hz, 1H), 7.25 (dd, *J* = 8.6, 2.8 Hz, 1H), 7.18-7.14 (m, 2H), 4.18 (s, 2H), 3.04 (q, *J* = 7.2 Hz, 4H), 2.85-2.70 (m, 1H), 1.29 (t, *J* = 7.2 Hz, 6H), 0.74-0.71 (m, 2H), 0.58-0.47 (m, 2H). |
| I-85 | 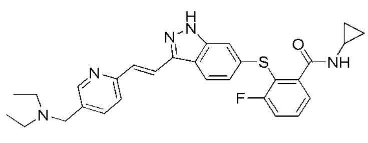 | 516.3 | $^1$H NMR (400MHz, $CD_3OD$): δ (ppm) 8.44 (s, 1H), 7.95 (d, J = 8.8 Hz, 1H), 7.81-7.72 (m, 2H), 7.59 (d, J = 8.0 Hz, 1H), 7.52-7.47 (m, 2H), 7.38 (s, 1H), 7.29-7.24 (m, 2H), 7.10 (d, J = 8.8 Hz, 1H), 3.58 (s, 2H), 2.79-2.75 (m, 1H), 2.52 (q, J = 7.2 Hz, 4H), 1.05 (t, J = 7.2 Hz, 6H), 0.74-0.70 (m, 2H), 0.53-0.49 (m, 2H).<br>$^{19}$F NMR (400MHz, $CD_3OD$): δ (ppm) -105.58. |
| I-86 | 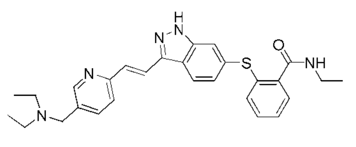 | | $^1$H NMR (400MHz, $CD_3OD$): δ (ppm) 8.50 (d, J = 1.8 Hz, 1H), 8.06 (d, J = 8.4 Hz, 1H), 7.90 -7.77 (m, 2H), 7.67 (d, J = 8.2 Hz, 1H), 7.62-7.46 (m, 3H), 7.39-7.30 (m, 2H), 7.28-7.17 (m, 2H), 3.65 (s, 2H), 3.40-3.34 (m, 2H), 2.61-2.55 (m, 4H), 1.18 (t, J = 7.4 Hz, 3H), 1.10 (t, J = 7.2 Hz, 6H). |

(continued)

| No. | Structural Formula | MS-ESI $[M+H]^+$ | NMR Data |
|---|---|---|---|
| I-87 | 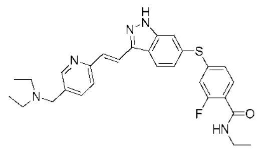 | 504.3 | $^1H$ NMR (400MHz, $CDCl_3$): δ (ppm) 8.58 (s, 1H), 8.02 (d, J = 8.4 Hz, 1H), 7.95-7.88 (m, 2H), 7.71 (d, J = 8.0 Hz, 1H), 7.65-7.60 (m, 2H), 7.44 (d, J = 8.0 Hz, 1H), 7.23 (d, J = 8.4 Hz, 1H), 6.96 (d, J = 8.4 Hz, 1H), 6.80 (d, J = 12.4 Hz, 1H), 6.70-6.67 (m, 1H), 3.62 (s, 2H), 3.52-3.45 (m, 2H), 2.57 (q, J = 7.2 Hz, 4H), 1.22 (t, J = 7.2 Hz, 3H), 1.07 (t, J = 7.2 Hz, 6H). |
| I-88 | 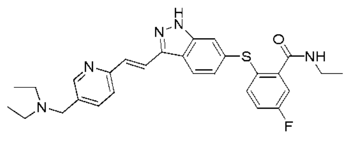 | 504.1 | $^1H$ NMR (400MHz, CD3OD): δ (ppm) 8.50 (s, 1H), 8.04 (d, J = 8.4 Hz, 1H), 7.83 (t, J = 11.7 Hz, 2H), 7.66 (d, J = 8.2 Hz, 1H), 7.59 - 7.45 (m, 2H), 7.37 (dd, J = 8.4, 5.2 Hz, 1H), 7.29 - 7.09 (m, 3H), 3.66 (s, 2H), 3.34 (d, J = 7.2 Hz, 2H), 2.58 (q, J = 7.2 Hz, 4H), 1.15 - 1.08 (m, 9H). |
| I-90 | 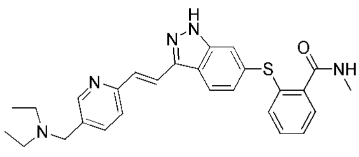 | 472.0 | $^1H$ NMR (400MHz, $CD_3OD$): δ (ppm) 8.52 (d, *J* = 1.6 Hz, 1H), 8.09 (d, *J* = 8.6 Hz, 1H), 7.85 (dd, *J* = 18.0, 9.4 Hz, 2H), 7.70 (d, *J* = 8.2 Hz, 1H), 7.63-7.55 (m, 2H), 7.52-7.46 (m, 1H), 7.38-7.30 (m, 2H), 7.27-7.21 (m, 2H), 3.69 (s, 2H), 2.88 (s, 3H), 2.60 (d, *J* = 7.2 Hz, 4H), 1.12 (t, *J* = 7.2 Hz, 6H). |
| I-91 | 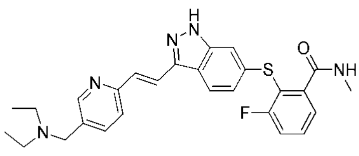 | 490.2 | $^1H$NMR (400 MHz, $CD_3OD$) δ (ppm) 8.56 (d, *J* = 1.2 Hz, 1H), 8.54 (s, 1H), 7.97 (d, *J* = 8.6 Hz, 1H), 7.91-7.82 (m, 2H), 7.67 (d, *J* = 8.2 Hz, 1H), 7.56-7.48 (m, 2H), 7.39-7.25 (m, 3H), 7.12 (dd, *J* = 8.6, 1.4 Hz, 1H), 4.00 (s, 2H), 2.89 (q, *J* = 7.2 Hz, 4H), 2.83 (s, 3H), 1.22 (t, *J* = 7.2 Hz, 6H). |
| I-92 | 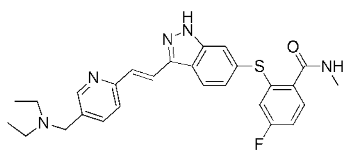 | 490.3 | $^1H$ NMR (400MHz, $CD_3OD$): δ (ppm) 8.55 (s, 1H), 8.16 (d, *J* = 8.4 Hz, 1H), 7.98-7.83 (m, 2H), 7.80-7.66 (m, 2H), 7.61 (d, *J* = 16.4 Hz, 1H), 7.52 (dd, *J* = 8.6, 5.8 Hz, 1H), 7.29 (dd, J= 8.6, 1.4 Hz, 1H), 7.01-6.96 (m, 1H), 6.73 (dd, *J* = 9.8, 2.4 Hz, 1H), 3.83 (s, 2H), 2.88 (s, 3H), 2.72 (d, *J* = 6.8 Hz, 4H), 1.26-1.01 (m, 6H). |
| I-93 | 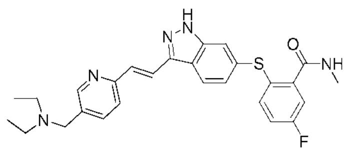 | 490.3 | $^1H$ NMR (400 MHz, $CD_3OD$): δ (ppm) 8.63 (s, 1H), 8.05 (d, *J* = 8.4 Hz, 1H), 7.98-7.88 (m, 2H), 7.73 (d, *J* = 8.2 Hz, 1H), 7.60-7.51 (m, 2H), 7.35 (dd, *J* = 8.8, 5.2 Hz, 1H), 7.26 (dd, *J* = 8.8, 2.8 Hz, 1H), 7.21-7.11 (m, 2H), 4.18 (s, 2H), 3.03 (q, *J* = 7.2 Hz, 4H), 2.84 (s, 3H), 1.29 (t, *J* = 7.2 Hz, 6H). |
| I-94 | 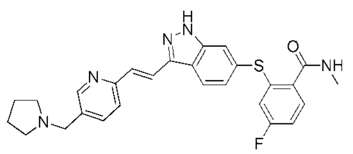 | 488.1 | $^1H$ NMR (400MHz, $CD_3OD$): δ (ppm) 8.69 (s, 1H), 8.45 (s, 1H), 8.14 (d, *J* = 8.8 Hz, 1H), 8.04-7.94 (m, 2H), 7.74 (d, *J* = 9.6 Hz, 2H), 7.61-7.51 (m, 2H), 7.27 (dd, *J* = 8.6, 1.2 Hz, 1H), 6.98 (d, *J* = 2.6 Hz, 1H), 6.72 (dd, *J* = 9.8, 2.6 Hz, 1H), 4.41 (s, 2H), 3.43-3.32 (m, 4H), 2.89 (s, 3H), 2.10 (s, 4H). |
| I-95 | 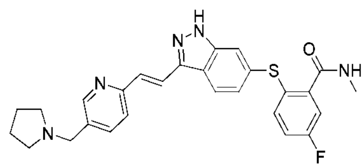 | 488.1 | $^1H$ NMR (400 MHz, $CD_3OD$): δ (ppm) 8.67 (s, 1H), 8.03 (d, *J* = 8.6 Hz, 1H), 7.90-7.97 (m, 2H), 7.70 (d, *J* = 8.0 Hz, 1H), 7.54 (d, *J* = 17.2 Hz, 2H), 7.34 (dd, *J* = 8.8, 5.2 Hz, 1H), 7.27 (dd, *J* = 8.8, 2.8 Hz, 1H), 7.20-7.07 (m, 2H), 4.36 (s, 2H), 3.32-3.23 (m, 4H), 2.86 (s, 3H), 2.08 (s, 4H). |

(continued)

| No. | Structural Formula | MS-ESI $[M+H]^+$ | NMR Data |
|---|---|---|---|
| I-97 | 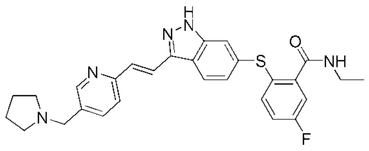 | 502.0 | $^1H$ NMR (400MHz, $CD_3OD$): δ (ppm) 8.51 (d, *J* = 1.6 Hz, 1H), 8.04 (d, *J* =8.4 Hz, 1H), 7.87-7.80 (m, 2H), 7.66 (d, *J* =8.0 Hz, 1H), 7.57-7.50 (m, 2H), 7.38-7.35 (m, 1H), 7.25 (dd, *J* =8.8, 2.8 Hz, 1H), 7.18-7.13 (m, 2H), 3.69 (s, 2H), 3.33-3.29 (m, 2H), 2.58 (s, 4H), 1.85-1.81 (m, 4H), 1.14 (t, *J* = 7.2Hz, 3H). |
| I-98 | 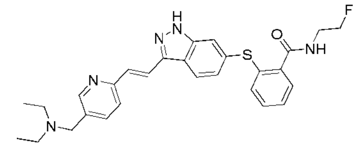 | 504.3 | $^1H$ NMR (400 MHz, $CD_3OD$) δ (ppm) 8.50 (d, J = 1.8 Hz, 1H), 8.07 (d, J = 8.4 Hz, 1H), 7.86 (d, J = 16.6 Hz, 2H), 7.69 (s, 1H), 7.58 (dd, J = 11.4, 8.6 Hz, 2H), 7.52-7.49 (m, 1H), 7.34 (d, J = 1.0 Hz, 2H), 7.27-7.19 (m, 2H), 4.57 (s, 1H), 4.45 (s, 1H), 3.69-3.54 (m, 4H), 2.57 (q, J = 7.2 Hz, 4H), 1.10 (m, J = 7.2 Hz, 6H). |
| I-99 | 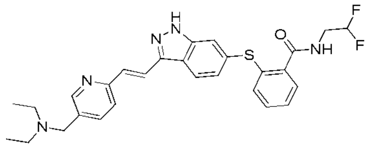 | 522.1 | $^1H$ NMR (400MHz, $CD_3OD$): δ (ppm) 8.50 (s, 1H), 8.07 (d, J=8.4 Hz, 1H), 7.88-7.61 (m, 2H), 7.68 (d, J=8.0 Hz, 1H), 7.60-7.50 (m, 3H), 7.36-7.32 (m, 2H), 7.25-7.20 (m, 2H), 6.09-5.81 (m, 1H), 3.69-3.64 (m, 4H), 2.60-2.55 (m, 4H), 1.13-1.07 (m, 6H). |
| I-100 | 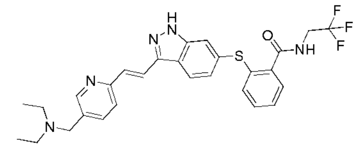 | 540.0 | $^1H$ NMR (400MHz, $CD_3OD$): δ (ppm) 8.74 (s, 1H), 8.13-8.04 (m, 3H), 7.91 (d, *J* = 8.0 Hz , 1H), 7.62 (d, *J* = 16.0 Hz, 2H), 7.54-7.49 (m, 1H), 7.40-7.32 (m, 2H), 7.27-7.22 (m, 1H), 4.47 (s, 2H), 4.09-4.02 (m, 2H), 3.30-3.21 (m, 4H), 1.39 (t, *J* = 7.3 Hz, 6H). |
| I-101 | 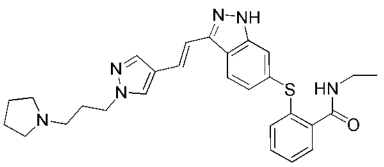 | 501.2 | $^1H$ NMR (400MHz, $CD_3OD$): δ (ppm) 7.99 (d, J=8.4 Hz, 1H), 7.89 (s, 1H), 7.78 (s, 1H), 7.53 (s, 1H), 7.46 (d, J=7.2 Hz, 1H), 7.36-7.32 (m, 3H), 7.24-7.14 (m, 3H), 4.21 (t, J=6.8 Hz, 2H), 3.36-3.33 (m, 2H), 2.56 (s, 4H), 2.48 (t, J=7.6 Hz, 2H), 2.13-2.06 (m, 2H), 1.81 (s, 4H), 1.16 (t, J=7.2 Hz, 3H). |
| I-102 | 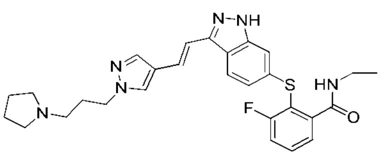 | 519.1 | $^1H$ NMR (400MHz, $CD_3OD$): δ (ppm) 7.92-7.89 (m, 2H), 7.78 (s, 1H), 7.55-7.50 (m, 1H), 7.34-7.25 (m, 4H), 7.13 (d, *J* = 16.8 Hz, 1H), 7.07 (dd, *J* = 8.4, 1.6 Hz, 1H), 4.20 (t, *J* = 6.8 Hz, 2H), 3.33-3.31 (m, 2H), 2.58-2.55 (m, 4H), 2.50-2.46 (m, 2H), 2.12-2.05 (m, 2H), 1.83-1.79 (m, 4H), 1.12 (t, *J* = 7.2 Hz, 3H). |
| I-103 | 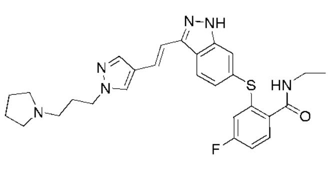 | 519.1 | $^1HNMR$ (400MHz, $CD_3OD$): δ (ppm) 8.08 (d, *J*=8.8 Hz, 1H), 7.91 (s, 1H), 7.80 (s, 1H), 7.68 (s, 1H), 7.50 (dd, *J*=8.8, 6.0 Hz, 1H), 7.37 (d, *J*=16.8 Hz, 1H), 7.23-7.21 (m, 2H), 7.01-7.96 (m, 1H), 6.73 (dd, *J*=10.0, 2.4 Hz, 1H), 4.22 (t, *J*=6.8 Hz, 2H), 3.39-3.34 (m, 2H), 2.57 (s, 4H), 2.51-2.47 (m, 2H), 2.12-2.08 (m, 2H), 1.83-1.80 (m, 4H), 1.20 (t, *J*=7.2 Hz, 3H). |
| I-104 | 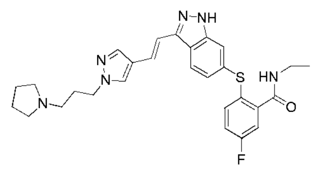 | 519.1 | $^1H$ NMR (400MHz, $CD_3OD$): δ (ppm) 7.95 (d, J=8.8 Hz, 1H), 7.91 (s, 1H), 7.84 (s, 1H), 7.46 (s, 1H), 7.36-7.24 (m, 3H), 7.18-7.10 (m, 3H), 4.29 (t, J=6.4, 2H), 3.66 (s, 2H), 3.35-3.10 (m, 2H), 3.23 -3.19 (m, 2H), 3.06-3.03 (m, 2H), 2.33-2.26 (m, 2H), 2.13 (s, 2H), 2.02-1.99 (m, 2H), 1.14 (t, J=7.2, 3H). |

(continued)

| No. | Structural Formula | MS-ESI [M+H]+ | NMR Data |
|---|---|---|---|
| I-106 | 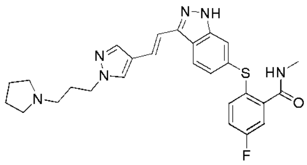 | 505.1 | $^1$H NMR (400 MHz, $CD_3OD$) δ (ppm) 8.54 (s, 1H), 7.96 (d, J = 8.6 Hz, 1H), 7.91 (s, 1H), 7.83 (s, 1H), 7.48 (d, J = 0.8 Hz, 1H), 7.37-7.29 (m, 2H), 7.26 (dd, J = 8.8, 2.8 Hz, 1H), 7.19 -7.10 (m, 3H), 4.28 (t, J = 6.6 Hz, 2H), 3.25 (s, 4H), 3.14-3.07 (m, 2H), 2.84 (s, 3H), 2.31-2.22 (m, 2H), 2.03 (dd, J = 8.4, 5.4 Hz, 4H). |
| I-107 | 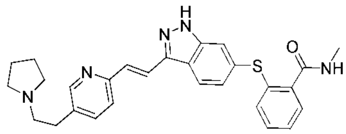 | 484.1 | $^1$H NMR (400MHz, $CD_3OD$): δ (ppm) 8.45 (d, *J* = 1.6 Hz, 1H), 8.06 (d, *J* = 8.4 Hz, 1H), 7.83 (d, *J* = 16.8 Hz, 1H), 7.76-7.33 (m, 1H), 7.65 (d, *J* = 8.0 Hz, 1H), 7.59-7.53 (m, 2H), 7.48-7.46 (m, 1H), 7.34-7.31 (m, 2H), 7.24-7.20 (m, 2H), 2.91-2.86 (m, 7H), 2.76 (s, 4H), 1.88 (s, 4H). |
| 1-108 | 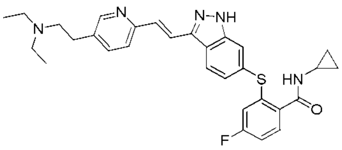 | 530.2 | $^1$H NMR (400 MHz, $CD_3OD$): δ (ppm) 8.43 (s, 1H), 8.13 (d, J = 8.4 Hz, 1H), 7.84 (d, J = 16.8 Hz, 1H), 7.73-7.71 (m, 2H), 7.64 (d, J = 8.4 Hz, 1H), 7.57 (d, J = 16.4 Hz, 1H), 7.51-7.47 (m, 1H), 7.26 (d, J = 8.8 Hz, 1H), 7.01-6.96 (m, 1H), 6.75 (dd, J = 9.6, 2.0 Hz, 1H), 2.85-2.75 (m, 5H), 2.68 (q, J = 7.2 Hz, 4H), 1.10 (t, J = 6.8 Hz, 6H), 0.80-0.76 (m, 2H), 0.63-0.57 (m, 2H). |
| I-109 | 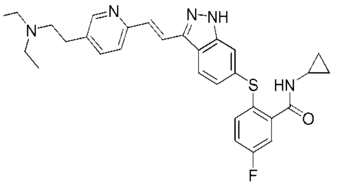 | 530.1 | $^1$H NMR (400MHz,$CD_3OD$): δ (ppm) 8.42 (d, *J* = 1.6 Hz, 1H), 8.04 (d, *J* = 8.4 Hz, 1H), 7.81 (d, *J* = 16.4 Hz, 1H), 7.73 (dd, *J* = 8.4, 2.0 Hz, 1H), 7.64 (d, *J* = 8.0 Hz, 1H), 7.56-7.49 (m, 2H), 7.38 (dd, *J* = 8.8, 5.2 Hz, 1H), 7.24 (dd, *J* = 8.8, 2.8 Hz, 1H), 7.18-7.13 (m, 2H), 2.85-2.74 (m, 5H), 2.69 (q, *J* = 7.2 Hz, 4H), 1.10 (t, *J* = 7.2 Hz, 6H), 0.76-0.71 (m, 2H), 0.53-0.49 (m, 2H). |
| I-110 | 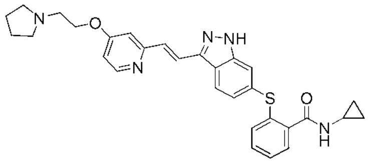 | 526.1 | $^1$H NMR (400MHz, $CD_3OD$): δ (ppm) 8.35 (d, J=5.6 Hz, 1H), 8.06 (d, J= 8.4 Hz, 1H), 7.85 (d, J= 16.8 Hz, 1H), 7.56-7.44 (m, 3H), 7.37-7.30 (m, 2H), 7.20 (m, 2H), 7.20-7.18 (dd, J= 8.8, 1.6 Hz, 1H), 6.91 (dd, J= 5.6, 2.4 Hz, 1H), 4.29 (t, J= 5.6 Hz, 2H), 2.97 (t, J=5.6 Hz, 2H), 2.81-2.75 (m, 1H), 2.69 (s, 4H), 1.85 (s, 4H), 0.77-0.72 (m, 2H), 0.55-0.51 (m, 2H). |
| I-111 | 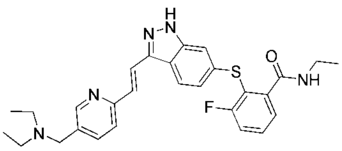 | 504.3 | $^1$H NMR (400MHz, $CD_3OD$): δ (ppm) 8.49 (s, 1H), 7.98 (d, J = 8.0 Hz, 1H), 7.84-7.80 (m, 2H), 7.65 (d, J = 8.0 Hz, 1H), 7.54-7.50 (m, 2H), 7.39 (s, 1H), 7.32-7.26 (m, 2H), 7.12 (d, J = 8.4 Hz, 1H), 3.65 (s, 2H), 3.31 (s, 2H), 2.57 (d, J = 6.8 Hz, 4H), 1.14-1.07 (m, 9H). |
| I-112 | 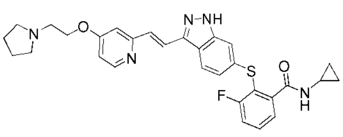 | 544.3 | $^1$H NMR (400MHz, $CD_3OD$): δ (ppm) 8.34 (d, J=6.0 Hz, 1H), 7.95 (d, J= 8.4 Hz, 1H), 7.81-7.76 (m, 1H), 7.52-7.40 (m, 3H), 7.30-7.20 (m, 3H), 7.12 (dd, J=8.4, 1.2 Hz, 1H), 6.87 (dd, J=6.0, 2.4 Hz, 1H), 4.27 (t, J=5.6 Hz, 2H), 2.98 (t, J=5.6 Hz, 2H), 2.79-2.75 (m, 1H), 2.70 (s, 4H), 1.86 (s, 4H), 0.76-0.71 (m, 2H), 0.54-0.50 (m, 2H). |

(continued)

| No. | Structural Formula | MS-ESI [M+H]+ | NMR Data |
|---|---|---|---|
| I-113 | 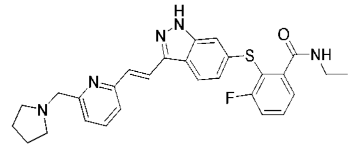 | 502.1 | $^1$H NMR (400MHz, $CD_3OD$): δ (ppm) 7.89 (d, *J* = 8.4 Hz, 1H), 7.78 (d, *J* = 16.4 Hz, 1H), 7.66 (t, *J* = 7.6 Hz, 1H), 7.46-7.40 (m, 3H), 7.30 (s, 1H), 7.23-7.16 (m, 3H), 7.03 (d, *J* = 8.4 Hz, 1H), 3.71 (s, 2H), 3.25-3.20 (m, 2H), 2.56 (s, 4H), 1.73 (s, 4H), 1.04 (t, *J* = 7.6 Hz, 3H). |
| I-114 | 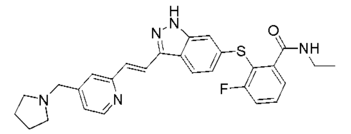 | 502.1 | $^1$H NMR (400MHz, $CD_3OD$): δ (ppm) 8.48 (d, *J* = 5.2 Hz, 1H), 7.98 (d, *J* = 8.4 Hz, 1H), 7.84 (d, *J*= 16.8 Hz, 1H), 7.68 (s, 1H), 7.55-7.51 (m, 2H), 7.39 (s, 1H), 7.32-7.26 (m, 3H), 7.13 (d, *J* = 8.4 Hz, 1H), 3.72 (s, 2H), 3.31 (s, 2H), 2.61 (s, 4H), 1.85 (s, 4H), 1.13 (t, *J* = 7.2 Hz, 3H). |
| I-115 | 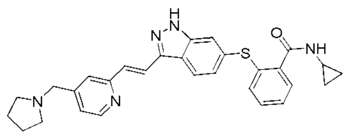 | 496.1 | $^1$H NMR (400MHz, $CDCl_3$): δ (ppm) 8.56 (d, *J* = 4.8 Hz, 1H), 8.00 (d, *J* = 8.4 Hz, 1H), 7.92 (d, *J* = 16.4 Hz, 1H), 7.65-7.62 (m, 1H), 7.57 (d, *J* = 16.4 Hz, 1H), 7.51 (s, 2H), 7.31-7.29 (m, 2H), 7.25-7.22 (m, 1H), 7.18-7.16 (m, 2H), 6.53 (s, 1H), 3.67 (s, 2H), 2.85-2.82 (m, 1H), 2.57 (s, 4H), 1.83 (s, 4H), 0.83-0.78 (m, 2H), 0.51-0.47 (m, 2H). |
| I-116 | 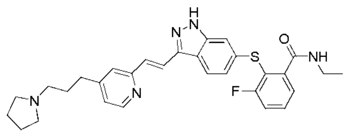 | 530.1 | $^1$H NMR (400MHz,$CD_3OD$): δ (ppm) 8.41 (d, *J* = 5.2 Hz, 1H), 7.98 (d, *J* = 8.8 Hz, 1H), 7.83 (d, *J* = 16.8 Hz, 1H), 7.57-7.49 (m, 3H), 7.39 (s, 1H), 7.32-7.26 (m, 2H), 7.18 (d, *J* = 5.2 Hz, 1H), 7.13 (dd, *J* = 8.8, 1.2 Hz, 1H), 3.34-3.31 (m, 2H), 2.73 (t, *J* = 7.6 Hz, 2H), 2.60-2.54 (m, 6H), 1.97-1.89 (m, 2H), 1.84-1.80 (m, 4H), 1.13 (t, *J* = 7.2 Hz, 3H). |
| I-117 | 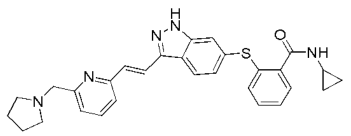 | 496.1 | $^1$H NMR (400MHz, $CD_3OD$): δ (ppm) 8.08 (d, *J* = 8.4 Hz, 1H), 7.93 (d, *J* = 16.8 Hz, 1H), 7.79 (t, *J* =7.6Hz, 1H), 7.60-7.56 (m, 3H), 7.46-7.44 (m, 1H), 7.37-7.30 (m, 3H), 7.27-7.25 (m, 1H), 7.19 (d, J = 8.4 Hz, 1H), 3.85 (s, 2H), 2.80-2.75 (m, 1H), 2.70 (s, 4H), 1.85 (s, 4H), 0.77-0.72 (m, 2H), 0.55-0.51 (m, 2H). |
| I-118 | 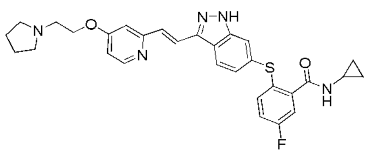 | 544.2 | $^1$H NMR (400MHz, $CD_3OD$): δ (ppm) 8.34 (d, *J* = 5.6 Hz, 1H), 8.03 (d, *J* = 8.4 Hz, 1H), 7.83 (d, J= 16.4 Hz, 1H), 7.53-7.45 (m, 2H), 7.37 (dd, *J* = 8.8, 5.6 Hz, 1H), 7.27-7.21 (m, 2H), 7.18-7.13 (m, 2H), 6.89 (dd, *J* = 6.0, 2.4 Hz, 1H), 4.28 (t, *J* = 5.4 Hz, 1H), 2.96 (t, *J* = 5.6 Hz, 1H), 2.79-2.73 (m, 1H), 2.72-2.65(m, 4H), 1.87-1.80(m, 4H), 0.76-0.70(m, 2H), 0.54-0.48 (m, 2H). |
| I-119 | 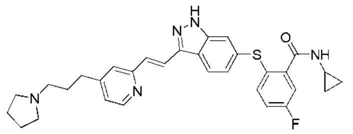 | 542.1 | $^1$H NMR (400MHz, $CD_3OD$): δ (ppm) 8.42 (d, *J* = 5.2 Hz, 1H), 8.05 (d, *J* = 8.8 Hz, 1H), 7.85 (d, *J* = 16.4 Hz, 1H), 7.58-7.49 (m, 3H), 7.38 (dd, *J* = 8.8, 5.2 Hz, 1H), 7.26-7.14 (m, 4H), 2.79-2.72 (m, 3H), 2.58-2.53 (m, 6H), 1.97-1.89 (m, 2H), 1.84-1.80 (m, 4H), 0.76-0.71 (m, 2H), 0.53-0.49 (m, 2H). |

(continued)

| No. | Structural Formula | MS-ESI $[M+H]^+$ | NMR Data |
|---|---|---|---|
| I-120 | 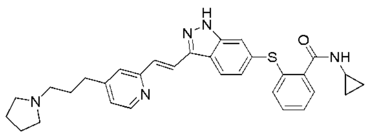 | 524.2 | $^1H$ NMR (400MHz, $CD_3OD$): δ (ppm) 8.41 (d, *J* = 4.8 Hz, 1H), 8.05 (d, *J* = 8.4 Hz, 1H), 7.85 (d, *J* = 16.4 Hz, 1H), 7.56-7.51 (m, 3H), 7.46-7.44 (m, 1H), 7.35-7.29 (m, 2H), 7.25-7.23 (m, 1H), 7.19-7.16 (m, 1H), 2.80-2.75 (m, 1H), 2.72 (t, *J* = 7.6 Hz, 2H), 2.59-2.53 (m, 6H), 1.96-1.88 (m, 2H), 1.81 (s, 4H), 0.77-0.72 (m, 2H), 0.56-0.52 (m, 2H). |
| I-121 | 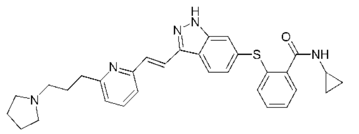 | 524.0 | $^1H$ NMR (400MHz, $CD_3OD$): δ (ppm) 8.08 (d, *J* = 8.8 Hz, 1H), 7.86 (d, *J* = 16.4 Hz, 1H), 7.73 (t, *J* = 7.6 Hz, 1H), 7.59-7.51 (m, 3H), 7.46-7.44 (m, 1H), 7.37-7.30 (m, 2H), 7.26 (d, *J* = 7.2 Hz, 1H), 7.21-7.17 (m, 2H), 2.86 (t, *J* = 7.6 Hz, 2H), 2.64 (s, 6H), 2.07-1.99 (m, 2H), 1.83 (s, 4H), 0.77-0.72 (m, 2H), 0.55-0.51 (m, 2H). |
| I-122 | 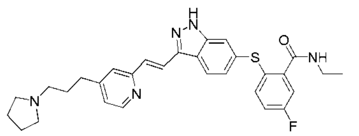 | 530.3 | $^1H$ NMR (400MHz, $CD_3OD$): δ (ppm) 8.40 (d, *J* = 4.8 Hz, 1H), 8.03 (d, *J* = 8.8 Hz, 1H), 7.84 (d, *J* = 16.4 Hz, 1H), 7.55-7.50 (m, 3H), 7.35 (dd, *J* = 8.8, 5.6 Hz, 1H), 7.25 (dd, *J* = 8.8, 3.2 Hz, 1H), 7.17-7.12 (m, 3H), 3.35-3.29 (m, 2H), 2.71 (t, *J* = 7.6 Hz, 2H), 2.57-2.50 (m, 6H), 1.95-1.87 (m, 2H), 1.84-1.76 (m, 4H), 1.14 (t, *J* = 7.2 Hz, 3H). |
| I-123 | 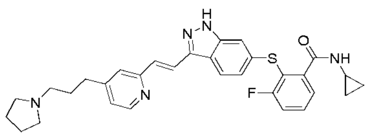 | 542.1 | $^1H$NMR(400MHz, $CD_3OD$):δ (ppm) 8.42 (d, *J* = 4.8 Hz, 1H), 7.98 (d, *J* = 8.4 Hz, 1H), 7.83 (d, *J* = 16.4 Hz, 1H), 7.58 (s, 1H), 7.55-7.50 (m, 2H), 7.39 (s, 1H), 7.31-7.27 (m, 2H), 7.19 (d, *J* = 5.2 Hz, 1H), 7.12 (dd, *J* = 8.8, 1.6 Hz, 1H), 2.79-2.63 (m, 9H), 2.00-1.92 (m, 2H), 1.87-1.84 (m, 4H), 0.75-0.70 (m, 2H), 0.52-0.48 (m, 2H). |
| I-124 | 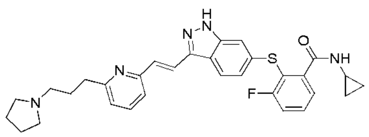 | 542.3 | $^1H$ NMR (400MHz, $CD_3OD$): δ (ppm) 8.00 (d, *J* = 8.4 Hz, 1H), 7.83 (d, *J* = 16.8 Hz, 1H), 7.72 (t, *J* = 8.0 Hz, 1H), 7.56-7.50 (m, 3H), 7.39 (s, 1H), 7.30-7.26 (m, 2H), 7.17 (d, *J* = 7.6 Hz, 1H), 7.12 (dd, *J* = 8.4, 1.2 Hz, 1H), 2.85 (t, *J* = 7.6 Hz, 2H), 2.79-2.74 (m, 1H), 2.63-2.57 (m, 6H), 2.05-1.98 (m, 2H), 1.84-1.80 (m, 4H), 0.75-0.70 (m, 2H), 0.52-0.48 (m, 2H). |
| 1-128 | 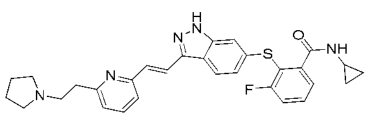 | 528.1 | $^1H$ NMR (400MHz,$CD_3OD$): δ (ppm) 8.01 (d, *J* = 8.4 Hz, 1H), 7.85 (d, *J* = 16.8 Hz, 1H), 7.73 (t, *J* = 8.0 Hz, 1H), 7.56-7.50 (m, 3H), 7.39 (s, 1H), 7.32-7.27 (m, 2H), 7.20 (d, *J* = 7.6 Hz, 1H), 7.13 (dd, *J* = 8.8, 1.6 Hz, 1H), 3.34-3.31 (m, 2H), 3.08-3.05 (m, 2H), 3.01-2.96 (m, 2H), 2.77-2.66 (m, 4H), 1.90-1.82 (m, 4H), 1.13 (t, *J* = 7.2 Hz, 3H). |
| I-129 | 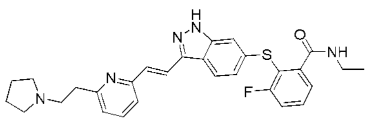 | 516.1 | $^1H$ NMR (400MHz,$CD_3OD$): δ (ppm) 8.01 (d, *J* = 8.4 Hz, 1H), 7.85 (d, *J* = 16.8 Hz, 1H), 7.73 (t, *J* = 8.0 Hz, 1H), 7.56-7.50 (m, 3H), 7.39 (s, 1H), 7.32-7.29 (m, 2H), 7.20 (d, *J* = 7.6 Hz, 1H), 7.13 (dd, *J* = 8.8, 1.6 Hz, 1H), 3.30-3.28 (m, 2H), 3.09-3.05 (m, 2H), 3.01-2.96 (m, 2H), 2.72 (s, 4H), 1.86 (t, *J* = 3.6 Hz, 4H), 1.13 (t, *J* = 7.2 Hz, 3H). |

(continued)

| No. | Structural Formula | MS-ESI [M+H]+ | NMR Data |
|---|---|---|---|
| I-132 | 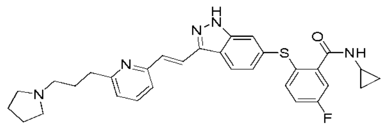 | 542.2 | $^1$H NMR (400MHz, $CD_3OD$): δ (ppm) 8.05 (d, *J* = 8.8 Hz, 1H), 7.85 (d, *J* = 16.4 Hz, 1H), 7.72 (t, *J* = 7.6 Hz, 1H), 7.57-7.49 (m, 3H), 7.37 (dd, *J* = 8.8, 5.4 Hz, 1H), 7.24 (dd, *J* = 8.8, 2.8 Hz, 1H), 7.18-7.13 (m, 3H), 2.85 (t, *J* = 7.6 Hz, 2H), 2.79-2.74 (m, 1H), 2.72-2.64 (m, 6H), 2.07-2.00 (m, 2H), 1.88-1.80 (m, 4H), 0.76-0.71 (m, 2H), 0.53-0.50 (m, 2H). |
| I-133 | 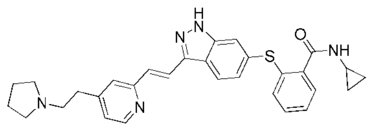 | 510.3 | $^1$H NMR (400MHz, $CD_3OD$): δ (ppm) 8.43 (d, *J* = 5.2 Hz, 1H), 8.07 (d, *J* = 8.4 Hz, 1H), 7.87 (d, *J* = 16.4 Hz, 1H), 7.59 (dd, *J* = 18.0, 16.0 Hz, 3H), 7.46-7.44 (m, 1H), 7.35-7.32 (m, 2H), 7.27- 7.25 (m, 1H), 7.22-7.18 (m, 2H), 2.95-2.91 (m, 2H), 2.87-2.80 (m, 2H), 2.80-2.77 (m, 1H), 2.67 (s, 4H), 1.86-1.85 (m, 4H), 0.77 - 0.72 (m, 2H), 0.55-0.51 (m, 2H). |
| I-134 | 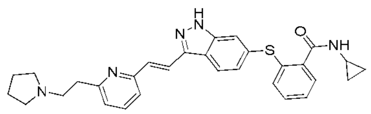 | 510.3 | $^1$H NMR (400MHz, $CD_3OD$): 8.09 (d, *J* = 8.8 Hz, 1H), 7.87 (d, *J* = 16.8 Hz, 1H), 7.73 (t, *J* = 8.0 Hz, 1H), 7.61-7.41 (m, 4H), 7.39-7.15 (m, 5H), 3.08-3.04 (m, 2H) , 2.96-2.93 (m, 2H) , 2.82-2.74 (m, 1H), 2.68 (s, 4H), 1.93-1.78 (m, 4H), 0.81-0.66 (m, 2H), 0.58-0.49 (m, 2H). |
| 1-135 | 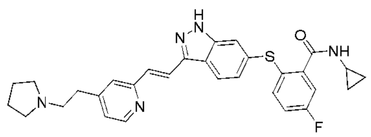 | 528.3 | $^1$H NMR (400MHz, $CD_3OD$): δ (ppm) 8.47 (d, *J* = 4.8 Hz, 1H), 8.04 (d, *J* = 8.8 Hz, 1H), 7.88 (d, *J* = 16.4 Hz, 1H), 7.63 (s, 1H), 7.57-7.49 (m, 1H), 7.40 (dd, *J* = 8.8, 5.2 Hz, 1H), 7.26-7.23 (m, 1H), 7.19-7.14 (m, 1H), 3.30 (s, 1H), 3.16-3.14 (m, 1H), 3.04-3.02 (m, 2H), 2.79-2.74 (m, 1H), 2.01-1.94 (m, 2H), 0.76- 0.71 (m, 1H), 0.53 -0.49 (m, 2H). |
| I-136 | 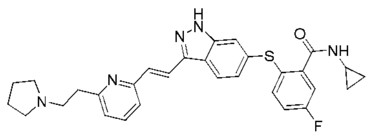 | 528.2 | $^1$H NMR (400MHz, $CD_3OD$): δ (ppm) 8.07 (d, *J* = 8.4 Hz, 1H), 7.90 (d, *J* = 16.4Hz, 1H), 7.78 (t, *J* = 8.0 Hz, 1H), 7.58-7.52 (m, 2H), 7.48 (s, 1H), 7.40 (dd, *J* = 8.8, 5.25 Hz, 1H), 7.27-7.23(m, 2H), 7.20-7.14 (m, 2H), 3.50 (t, *J* = 7.6 Hz, 2H), 3.25-3.20 (m, 6H), 2.80-2.73 (m, 1H), 2.07-2.03 (m, 4H), 0.76-0.72 (m, 2H), 0.54-0.50 (m, 2H). |
| I-137 | 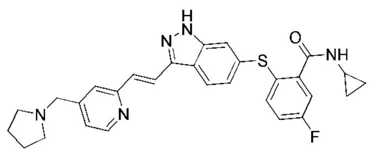 | 514.1 | $^1$H NMR (400MHz, $CD_3OD$): δ (ppm) 8.48 (d, *J* = 5.2 Hz, 1H), 8.05 (d, *J* = 8.8 Hz, 1H), 7.86 (d, *J* = 16.8 Hz, 1H), 7.68 (s, 1H), 7.57-7.49 (m, 2H), 7.39-7.36 (m, 1H), 7.30 (d, *J* = 4.8 Hz, 1H), 7.25 (dd, *J* = 8.8, 2.8 Hz, 1H), 7.18-7.13 (m, 2H), 3.72 (s, 2H), 2.78-2.74 (m, 1H), 2.60-2.58 (m, 4H), 1.86-1.83 (m, 4H), 0.76-0.71 (m, 2H), 0.53-0.49 (m, 2H). |
| I-138 | 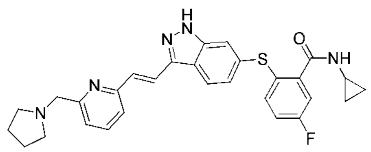 | 514.1 | $^1$H NMR (400MHz, $CD_3OD$): δ (ppm) 8.06 (d, *J* = 8.4 Hz, 1H), 7.91 (d, *J* = 16.4 Hz, 1H), 7.78 (t, *J* = 8.4 Hz, 1H), 7.59-7.54 (m, 2H), 7.49 (d, *J* = 0.8 Hz, 1H), 7.39-7.33 (m, 2H), 7.24 (dd, *J* = 8.8, 3.2 Hz, 1H), 7.18-7.13 (m, 2H), 3.83 (s, 2H), 2.79-2.74 (m, 1H), 2.69-2.65 (m, 4H), 1.86-1.82 (m, 4H), 0.76-0.69 (m, 2H), 0.53-0.49 (m, 2H). |

(continued)

| No. | Structural Formula | MS-ESI $[M+H]^+$ | NMR Data |
|---|---|---|---|
| I-139 | 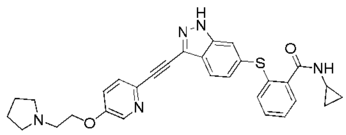 | 524.2 | $^1H$ NMR (400MHz, $CD_3OD$): δ (ppm) 8.32 (d, *J* = 3.2 Hz, 1H), 7.84 (d, *J* = 8.4 Hz, 1H), 7.70 (d, *J* = 8.4 Hz, 1H), 7.57 (s, 1H), 7.51 (dd, *J* = 8.8, 3.2 Hz, 1H), 7.47-7.44 (m, 1H), 7.37-7.33 (m, 2H), 7.29-7.27 (m, 1H), 7.19 (dd, *J* = 8.4, 1.2 Hz, 1H), 4.31 (t, *J* = 5.6 Hz, 2H), 3.15-3.13 (m, 2H), 2.87 (s, 4H), 2.79-2.74 (m, 1H), 1.92 (s, 4H), 0.77-0.72 (m, 2H), 0.54-0.50 (m, 2H). |

Example 2

Assay for Inhibitory Activity of Compounds Against VEGFR and EGFR Kinases

This assay directly monitors the inhibitory activity of compounds on kinase phosphorylation by measuring non-phosphorylated and phosphorylated substrates in the kinase reaction using Mobility Shift Assays/IMAP™ analysis.

Table 2. Reagents and Instruments

| Reagent | Supplier | Catalog No. |
|---|---|---|
| FLTI (VEGFR1) | Invitrogen | PR6731B |
| KDR (VEGFR2) | Carna | 08-191 |
| FLT4 (VEGFR3) | Invitrogen | PV4129 |
| EGFR | Carna | 08-115 |
| ATP | Sigma | 2383-5G |
| MgCl2 | Sigma | M2670-500g |
| DTT | Sigma | D0632-10G |
| EGTA | Sigma | E3889-25G |
| DMSO | Sigma | D2650 |
| Staurosporine | MCE | HY-15141 |
| Plate Reader | Caliper LifeSciences | CaliperEZ Reader II |

Assay Protocol and Steps:

Kinase and DTT were added to 1X kinase buffer to form a 2.5X kinase solution; 10 μL of the 2.5X kinase solution was transferred to a 384-well reaction plate, and 1X kinase buffer was added to the negative control wells. The plate was incubated at room temperature for 10 minutes; FAM-labeled peptide, $MgCl_2$, ATP, and other reagents were added to 1X kinase buffer to form a 2.5X substrate solution; 10 μL of the above 2.5X substrate solution was transferred to the 384-well reaction plate; the plate was incubated at 28°C for varying times, and then 25 μL of stop solution was added to the 384-well reaction plate to terminate the reaction. Conversion rate data were read on a CaliperEZ Reader II. The conversion rate was converted into inhibition rate data.

$$\%Inhibition = \frac{Max - Conversion}{Max - Min} \times 100\%$$

"Min" represents the reading from control wells where no enzyme was added for the reaction; "Max" represents the reading from control wells where DMSO was added. $IC_{50}$ values were fitted using XLFit excel add-in version 5.4.0.8.

$$Y = Bottom + \frac{Top - Bottom}{1 + \frac{IC_{50}}{X}^{(LogIC_{50} - X)Hill\ Slope}}$$

X is the log value of compound concentration, Y is the inhibition rate (% inhibition).

The $IC_{50}$ results of the compounds of the present invention for VEGFR2 are shown in the table below:

Table 3. $IC_{50}$ Results of Compounds for VEGFR2

| No. | IC50 (nM) | No. | $IC_{50}$ (nM) | No. | IC50 (nM) | No. | $IC_{50}$ (nM) |
|---|---|---|---|---|---|---|---|
| I-1 | A | I-137 | B | I-38 | A | I-81 | C |
| I-10 | B | I-138 | B | I-39 | A | I-82 | A |
| I-100 | B | I-14 | B | I-4 | A | I-83 | A |
| I-101 | A | I-15 | B | I-40 | A | I-84 | B |
| I-102 | C | I-16 | B | I-41 | A | I-85 | B |
| I-104 | A | I-17 | B | I-42 | A | I-86 | A |
| I-106 | B | I-18 | B | I-43 | A | I-87 | D |
| I-107 | B | I-19 | B | I-44 | B | I-88 | B |
| I-108 | A | I-2 | A | I-46 | A | I-9 | B |
| I-109 | A | I-20 | C | I-5 | A | I-90 | B |
| I-11 | B | I-21 | C | I-55 | A | I-91 | B |
| I-110 | B | I-22 | C | I-59 | A | I-92 | B |
| I-111 | B | I-23 | C | I-6 | B | I-93 | B |
| I-112 | C | I-24 | C | I-60 | B | I-94 | B |
| I-113 | D | I-25 | D | I-61 | B | I-95 | B |
| I-114 | D | I-26 | D | I-67 | B | I-97 | B |
| I-115 | B | I-27 | D | I-68 | B | I-98 | B |
| I-116 | C | I-28 | D | I-69 | C | I-99 | B |
| I-117 | B | I-29 | D | I-7 | B | I-136 | B |
| I-118 | B | I-3 | A | I-73 | B | I-132 | B |
| I-119 | A | I-30 | D | I-74 | B | I-103 | A |
| I-120 | A | I-31 | D | I-75 | B | I-133 | B |
| I-121 | A | I-32 | B | I-76 | B | I-129 | C |
| I-122 | B | I-33 | B | I-77 | A | I-128 | C |
| I-123 | C | I-34 | B | I-78 | A | I-139 | C |
| I-124 | C | I-35 | B | I-79 | A | I-135 | C |
| I-13 | B | I-36 | B | I-8 | B | | |
| I-134 | B | I-37 | A | I-80 | A | | |

Wherein, $IC_{50}$: A represents 1 nM or less, B represents greater than 1 nM to 10 nM inclusive, C represents greater than 10 nM to 100 nM inclusive, D represents greater than 100 nM.

The $IC_{50}$ results of the compounds of the present invention for EGFR are shown in the table below:

Table 4. $IC_{50}$ Results of Compounds for EGFR

| No. | IC50 (nM) | No. | $IC_{50}$ (nM) | No. | IC50 (nM) | No. | $IC_{50}$ (nM) |
|---|---|---|---|---|---|---|---|
| I-1 | C | I-137 | D | I-38 | B | I-81 | C |
| I-10 | C | I-138 | D | I-39 | C | I-82 | C |
| I-100 | C | I-14 | C | I-4 | C | I-83 | C |
| I-101 | C | I-15 | C | I-40 | C | I-84 | C |
| I-102 | C | I-16 | C | I-41 | C | I-85 | C |
| I-104 | D | I-17 | C | I-42 | D | I-86 | C |
| I-106 | C | I-18 | C | I-43 | C | I-87 | D |
| I-107 | C | I-19 | D | I-44 | C | I-88 | C |
| I-108 | C | I-2 | C | I-46 | C | I-9 | C |
| I-109 | C | I-20 | C | I-5 | C | I-90 | C |
| I-11 | C | I-21 | C | I-55 | C | I-91 | C |
| I-110 | C | I-22 | D | I-59 | C | I-92 | C |
| I-111 | C | I-23 | C | I-6 | C | I-93 | C |
| I-112 | C | I-24 | C | I-60 | B | I-94 | C |
| I-113 | C | I-25 | C | I-61 | C | I-95 | C |
| I-114 | D | I-26 | D | I-67 | C | I-97 | C |
| I-115 | C | I-27 | C | I-68 | C | I-98 | C |
| I-116 | D | I-28 | A | I-69 | C | I-99 | C |
| I-117 | D | I-29 | D | I-7 | C | I-136 | D |
| I-118 | D | I-3 | C | I-73 | C | I-132 | C |
| I-119 | D | I-30 | C | I-74 | C | I-103 | C |
| I-120 | D | I-31 | D | I-75 | C | I-133 | D |
| I-121 | C | I-32 | C | I-76 | B | I-129 | D |
| I-122 | D | I-33 | C | I-77 | C | I-128 | D |
| I-123 | D | I-34 | C | I-78 | C | I-139 | D |
| I-124 | C | I-35 | C | I-79 | C | I-135 | D |
| I-13 | C | I-36 | C | I-8 | C | | |
| I-134 | D | I-37 | C | I-80 | C | | |

Wherein, $IC_{50}$: A represents 600 nM or less, B represents greater than 600 nM to 6,000 nM inclusive, C represents greater than 6,000 nM to 60,000 nM inclusive, D represents greater than 60,000 nM.

The $IC_{50}$ results of the compounds of the present invention for VEGFR1 are shown in the table below:

Table 5. $IC_{50}$ Results of Compounds for VEGFR1

| No. | IC50 (nM) | No. | $IC_{50}$ (nM) | No. | IC50 (nM) | No. | IC50 (nM) |
|---|---|---|---|---|---|---|---|
| I-1 | A | I-15 | B | I-26 | D | I-37 | A |
| I-10 | B | I-16 | B | I-27 | D | I-38 | A |
| I-11 | B | I-17 | B | I-28 | D | I-39 | A |
| I-115 | B | I-18 | B | I-29 | D | I-4 | A |
| I-116 | C | I-19 | B | I-3 | A | I-40 | A |
| I-117 | B | I-2 | A | I-30 | D | I-44 | B |

(continued)

| No. | IC50 (nM) | No. | $IC_{50}$ (nM) | No. | IC50 (nM) | No. | IC50 (nM) |
|---|---|---|---|---|---|---|---|
| I-118 | C | I-20 | C | I-31 | D | I-5 | A |
| I-119 | B | I-21 | C | I-32 | B | I-6 | A |
| I-120 | B | I-22 | C | I-33 | B | I-7 | A |
| I-121 | B | I-23 | C | I-34 | B | I-8 | B |
| I-13 | B | I-24 | C | I-35 | B | I-9 | B |
| I-14 | B | I-25 | C | I-36 | D | I-97 | B |

Wherein, $IC_{50}$: A represents 1 nM or less, B represents greater than 1 nM to 10 nM inclusive, C represents greater than 10 nM to 100 nM inclusive, D represents greater than 100 nM.

The $IC_{50}$ results of the compounds of the present invention for VEGFR3 are shown in the table below:

Table 6. $IC_{50}$ Results of Compounds for VEGFR3

| No. | IC50 (nM) | No. | IC50 (nM) | No. | IC50 (nM) | No. | IC50 (nM) |
|---|---|---|---|---|---|---|---|
| I-1 | A | I-15 | B | I-26 | D | I-37 | B |
| I-10 | A | I-16 | B | I-27 | D | I-38 | A |
| I-11 | B | I-17 | B | I-28 | D | I-39 | B |
| I-115 | C | I-18 | B | I-29 | D | I-4 | A |
| I-116 | C | I-19 | B | I-3 | A | I-40 | B |
| I-117 | B | I-2 | B | I-30 | D | I-44 | C |
| I-118 | C | I-20 | C | I-31 | D | I-5 | A |
| I-119 | C | I-21 | C | I-32 | B | I-6 | B |
| I-120 | B | I-22 | C | I-33 | B | I-7 | B |
| I-121 | A | I-23 | D | I-34 | B | I-8 | B |
| I-13 | A | I-24 | C | I-35 | B | I-9 | B |
| I-14 | B | I-25 | C | I-36 | B | I-97 | B |

Wherein, $IC_{50}$: A represents 1 nM or less, B represents greater than 1 nM to 10 nM inclusive, C represents greater than 10 nM to 100 nM inclusive, D represents greater than 100 nM.

The results show that the compounds provided by the present invention can have kinase inhibitory activity, for example, the $IC_{50}$ (nM) for VEGFR1/2/3 is 100 or less, or the $IC_{50}$ (nM) for VEGFR1/2/3 is 10 or less, or the $IC_{50}$ (nM) for VEGFR1/2/3 is 1 or less. Furthermore, the compounds provided by the present invention exhibit high selectivity for inhibiting EGFR.

Example 3

Assay for Compound Inhibition of VEGFR2 Phosphorylation in HUVEC Cells

VEGF is a potent growth factor that promotes angiogenesis and vascular permeability. Vascular endothelial growth factor receptor 2 (VEGFR2) is the primary receptor for VEGF-induced endothelial cell signaling. Upon ligand binding, VEGFR2 undergoes autophosphorylation and is activated. The primary function from VEGFR2 signaling is to induce blood vessel formation during development and after tissue injury, and to bypass blocked vessels. Pathologically, VEGF and VEGFR2 are involved in tumor angiogenesis and vascular leakage. They are considered major players in many different cancers and ocular diseases accompanied by pathological neovascularization. This assay uses ELISA to measure the compound's ability to inhibit VEGF-induced VEGFR2 autophosphorylation (pVEGFR2) in HUVEC cells.

Table 7. Reagents and Instruments

| Reagent or Instrument | Supplier | Catalog Number or Model |
|---|---|---|
| Endothelial Cell Growth Base Media | R&D | 390598 |
| Endothelial Cell Growth Supplement (50X) | R&D | 390599 |
| Foetal bovine serum | Invitrogen | 10099141C |
| DPBS | Corning | 21-031-CV |
| Trypsin-EDTA (0.05%), phenol red | Invitrogen | 25300062 |
| Human VEGF-165 Recombinant Protein | CST | 48143 |
| Phosphatase Inhibitor Cocktail 3 | Sigma | P0044 |
| Phosphatase Inhibitor Cocktail 2 | Sigma | P5726 |
| PathScan® Phospho-VEGFR-2 (Tyr1175) Sandwich ELISA Antibody Pair | CST | 7824S |
| Protease Inhibitor | Roche | 4693159001 |
| 10X Cell Lysis Buffer | CST | 9803S |
| Tween® 20 | Sigma | P1379 |
| Bovine Serum Albumin (BSA) | Sigma | B2064-100G |
| TMB Substrate | CST | 7004P4 |
| STOP Solution | CST | 7002P4 |
| Microplate Reader | Molecular Devices | SpectraMax® 340 PC 384 |

Experimental Steps:

Before cell seeding, cells reached 60%-80% confluency in cell culture flasks. The cell suspension was diluted with pre-warmed complete medium to $4.5\times10^6$ cells/mL. 80 microliters of cell suspension was added to all wells of a 96-well cell plate (Corning-3599); incubated overnight at 37°C and 5% $CO_2$. Compound powder was dissolved in DMSO to 10 mM and stored in a nitrogen cabinet. Compound dilution master plate: In a 96-well V-bottom plate (Axygen- WIPP02280), reference compound Ponatinib or test compound concentrations were added, starting from 5 mM (5 microliters of 10 mM compound solution plus 5 microliters DMSO) with 3-fold serial dilutions for a total of 8 concentration points. For the 100% inhibition control group, the reference compound Ponatinib concentration in the V-bottom plate was 2.5 mM (3 microliters of 10 mM compound solution plus 9 microliters DMSO). 20 microliters of diluted compound was transferred from the intermediate plate to the cell plate. The cell plate was then incubated at 37°C and 5% $CO_2$ for 1 hour. One hour after compound treatment, 25 microliters of 250 ng/mL human VEGF was added to the 96-well plate, resulting in a final concentration of 50 ng/mL human VEGF; after incubation at 37°C and 5% $CO_2$ for 5 minutes, the plate was centrifuged at 3000 rpm for 10 minutes, the medium was removed, and cells were washed once with 300 microliters/well of pre-cooled DPBS. Then, it was centrifuged at 4000 rpm for 10 minutes, the DPBS was removed, and 110 microliters/well of pre-cooled 1X cell lysis buffer was added to the 96-well plate. The plate was shaken at 4°C for 40 minutes. The plate was then frozen in a -80°C freezer. The next day, the cell plate was taken out from -80°C, thawed, centrifuged at 3500 rpm for 10 minutes, and 100 microliters/well of supernatant was taken and added to the ELISA detection plate. The plate was sealed and incubated at 37°C for 2 hours. Wells were washed 4 times with 1X Wash Buffer. 100 microliters of recombinant detection antibody was added to each well. The plate was sealed, incubated at 37°C for 1 hour, and then the washing step was repeated. 100 microliters of secondary antibody was added to each well. The plate was sealed, incubated at 37°C for 30 minutes, and then the washing step was repeated. 100 microliters of TMB (protected from light) was added to each well. The plate was sealed, incubated at 37°C for 30 minutes, and then 100 microliters of stop solution was added to each well. It was gently shaken for 30 seconds, and the absorbance was measured within 30 minutes using a SpectraMax® 340 PC 384 Absorbance Microplate Reader, reading the optical density value at 450 nm. Data analysis was performed using XL Fit software:

$$\text{Inhibition Rate(\%Inhibition)} = \frac{\text{Average of Negative Control Group} - \text{Sample Value}}{\text{Average of Negative Control Group} - \text{Average of Positive Control Group}}$$

"Human VEGF-stimulated cells with DMSO" served as the negative control group (0% inhibition rate), and "Human VEGF-stimulated cells with 5 μM Ponatinib" served as the positive control group (100% inhibition rate). All other values are expressed as a percentage of these controls (inhibition rate).

Curve fitting was performed using the 4 Parameter Logistic Model or Sigmoidal Dose-Response Model:

$$Y = Bottom + \frac{Top - Bottom}{1 + 10^{(LogIC_{50} - X) \times Hill\ Slope}}$$

X is the log value of compound concentration, Y is the inhibition rate (%inhibition), Top is the reading from the negative control wells; Bottom is the reading from the positive control wells.

The $IC_{50}$ results of the compounds of the present invention for pVEGFR2 are shown in the table below:

Table 8. $IC_{50}$ Results of Compounds for pVEGFR2

| No. | IC50 (nM) | No. | IC50 (nM) | No. | IC50 (nM) | No. | IC50 (nM) |
|---|---|---|---|---|---|---|---|
| I-100 | A | I-34 | A | I-6 | B | I-86 | A |
| I-104 | A | I-40 | A | I-61 | A | I-88 | A |
| I-107 | A | I-41 | A | I-67 | A | I-9 | A |
| I-109 | B | I-42 | A | I-77 | A | I-90 | A |
| I-110 | B | I-43 | A | I-78 | A | I-97 | A |
| I-113 | C | I-44 | B | I-80 | A | | |
| I-20 | B | I-55 | A | I-83 | A | | |
| I-3 | A | I-59 | A | I-84 | A | | |

Wherein, IC50: A represents 1 nM or less, B represents greater than 1 nM to 5 nM inclusive, C represents greater than 5 nM.

Example 4

Solubility Test of Compounds

Table 9. Reagents and Instruments

| Reagent or Instrument | Supplier | Catalog Number or Model |
|---|---|---|
| Trisodium citrate dihydrate | Sinopharm Chemical Reagent Co., Ltd. | 10019418 |
| Citric acid monohydrate | Sinopharm Chemical Reagent Co., Ltd. | 10007118 |
| Methanol | ASTOON | AT-3292 |
| Acetonitrile | ASTOON | AT-3022 |
| Verapamil hydrochloride | Aladdin | V111249 |
| Glibenclamide | Aladdin | G127198 |
| Water | Self-made deionized water | - |

(continued)

| Reagent or Instrument | Supplier | Catalog Number or Model |
|---|---|---|
| Positive control compound I-12, with the structural formula 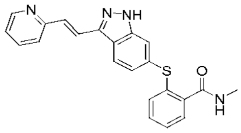 | Aladdin | |

Experimental Protocol and Steps:

Thermodynamic solubility: Accurately weigh approximately 2 mg of compound in triplicate, add appropriate volumes of buffer solutions with pH 2.0, 6.5, and 7.4 respectively, to obtain solutions with a concentration of 2 mg/mL. After sonication for 10 minutes, they were fixed on a shaker and shaken at room temperature for 8 hours. After shaking, they were sonicated for 10 minutes and centrifuged at 13000 rpm for 15 minutes. 0.1 mL of supernatant was aspirated into a new tube, rinsed with 5 mL of buffer by shaking, and the liquid was discarded. 0.5 mL of supernatant was transferred again to a new tube, centrifuged at 13000 rpm for 15 minutes, and the supernatant was taken (diluted with water if necessary) for LC-MS injection analysis. Sample concentrations were quantified using a 3-5 point calibration curve.
The solubility of the compounds of the present invention is shown in the table below:

Table 10. Solubility of Compounds

| No. | Solubility (ug/mL) | No. | Solubility (ug/mL) | No. | Solubility (ug/mL) | No. | Solubility (ug/mL) |
|---|---|---|---|---|---|---|---|
| I-1 | 14.2 | I-21 | 0.9 | I-43 | 71.6 | I-83 | 79.6 |
| I-100 | 209.0 | I-22 | 57.4 | I-44 | 563.5 | I-84 | 599.0 |
| I-104 | 2178.0 | I-23 | 859.0 | I-5 | 177.0 | I-86 | 1125.5 |
| I-106 | 1580.0 | I-25 | 216.0 | I-6 | 226.0 | I-88 | 546.0 |
| I-109 | 1948.0 | I-26 | 142.0 | I-67 | 118.0 | I-9 | 363.0 |
| I-11 | 866.0 | I-3 | 0.5 | I-7 | 294.0 | I-90 | 31.9 |
| I-12 | 2.0 | I-31 | 542.0 | I-76 | 1774.0 | I-91 | 1648.0 |
| I-14 | 46.8 | I-32 | 327.0 | I-77 | 1940.0 | I-94 | 31.8 |
| I-15 | 78.8 | I-33 | 57.7 | I-78 | 27.6 | I-97 | 318.7 |
| I-16 | 27.2 | I-34 | 19.3 | I-79 | 80.4 | I-98 | 456.0 |
| I-17 | 278.0 | I-35 | 26.6 | I-8 | 1870.0 | I-99 | 341.0 |
| I-2 | 28.8 | I-36 | 16.2 | I-80 | 199.0 | | |
| I-20 | 128.0 | I-4 | 413.0 | I-82 | 294.0 | | |

The results show that the compounds provided by the present invention can have the advantage of improved solubility, for example, a thermodynamic solubility of 10 μg/mL or above, or a thermodynamic solubility of 100 μg/mL or above, or a thermodynamic solubility of 1000 μg/mL or above. The thermodynamic solubility of the positive compound Axitinib was less than 2 μg/mL, consistent with literature reports.

Example 5

Detection of Compound Binding to Melanin
Pigmented ocular tissues contain melanin within intracellular melanosomes. Drugs bind to melanin to varying degrees, from no binding to extensive binding. Therefore, melanin binding is an important characteristic affecting ocular drug delivery and biodistribution. The ability of compounds to bind to melanin must be considered in ocular drug development.

Reagents:

Melanin derived from cuttlefish (Sepia officinalis) melanin was purchased from Sigma Aldrich (St Louis, MO).

Experimental Steps:

First, prepare a 1 mg/mL melanin suspension in PBS (pH 7.4). As a matrix washing step, suspended particles were centrifuged at 3000 rpm for 5 minutes. The resulting pellet was resuspended in fresh PBS buffer, heated to 37°C with continuous shaking, and incubated for 1 hour. Accurately pipette 2 microliters of test compound stock solution (0.1 mM) into 98 microliters of melanin suspension (1 mg/mL), resulting in a final incubation concentration of 2 μM. Similarly, accurately pipette 2 microliters of stock solution (0.1 mM) into 98 microliters of PBS buffer (containing 0.1% BSA) as a blank control sample. Samples were incubated at 37°C for 2 hours on a shaker (80-90 rpm). Then centrifuged at room temperature. 50 microliters of supernatant was aspirated and added to 50 microliters of precipitation agent (containing internal standard). All samples above were subjected to protein precipitation, centrifuged, the supernatant was taken, diluted with water (if necessary), and then injected for analysis. Simultaneously, high-binding compounds Sunitinib and chloroquine, and the low-binding compound celecoxib were measured as positive controls. An LC-MS/MS analytical method for determining compound concentration was established. The ratio of analyte/internal standard peak areas (Aanalyte/AIS) measured in the samples, calibrated by the dilution factor, was used as the surrogate concentration for data processing. The binding rate of the compound to melanin ($F_b$) is calculated by the formula:

$$Fb = 100 - \frac{C_{test}}{C_{control}} \times 100\%$$

$C_{test}$ is the drug concentration of the compound in the melanin incubation sample or the peak area ratio of the test compound to the internal standard. $C_{control}$ is the drug concentration of the compound in the blank control sample or the peak area ratio of the test compound to the internal standard. Percentage of free drug ($F_u$%) = 100 - $F_b$ The binding rates of the compounds of the present invention to melanin are shown in the table below:

Table 11. Binding Rate of Compounds to Melanin

| No. | Fu % | No. | Fu % | No. | Fu % | No. | Fu % |
|---|---|---|---|---|---|---|---|
| I-1 | C | I-20 | B | I-40 | B | I-79 | C |
| I-100 | C | I-21 | B | I-41 | C | I-8 | B |
| I-104 | C | I-22 | B | I-42 | B | I-80 | B |
| I-106 | C | I-23 | B | I-43 | B | I-82 | B |
| I-107 | B | I-25 | C | I-44 | B | I-83 | C |
| I-109 | C | I-26 | B | I-5 | B | I-84 | C |
| I-11 | B | I-27 | B | I-51 | B | I-86 | B |
| I-115 | B | I-28 | C | I-52 | C | I-88 | C |
| I-116 | B | I-3 | B | I-53 | C | I-9 | B |
| I-117 | B | I-31 | B | I-55 | B | I-90 | B |
| I-118 | A | I-32 | B | I-59 | B | I-91 | B |
| I-13 | B | I-33 | C | I-6 | B | I-93 | B |
| I-14 | B | I-34 | C | I-61 | B | I-94 | B |
| I-15 | B | 1-35 | B | I-67 | C | I-97 | B |
| I-16 | A | I-36 | C | I-7 | C | I-98 | C |
| I-17 | B | I-37 | B | I-73 | B | I-99 | C |
| I-18 | B | I-38 | B | I-76 | C | | |
| I-19 | B | 1-39 | B | I-77 | B | | |
| I-2 | B | I-4 | C | I-78 | B | | |

Wherein, Fu %: A represents less than or equal to 1%, B represents >1% to ≤5%, C represents greater than 5%. The test results provide the percentage of free drug (not binding to melanin, capable of interacting with receptors in ocular tissues) for the compounds of the present invention.

Example 6

Detection of Plasma and Ocular Tissue Distribution of Compounds after Single Ocular Administration in Dutch Belted Rabbits

Age-related macular degeneration and diabetic retinopathy are associated with neovascularization in the posterior segment of the eye. A major problem with topical eye drops for treating posterior segment disorders is the inefficient delivery of the agent to the posterior segment sites due to the corneal and conjunctival barriers. Therefore, it is important to measure the distribution of compounds from ocular formulations in several major ocular tissues. Increasing the concentration of the compound in the posterior segment may significantly improve efficacy to enhance clinical outcomes for patients with age-related macular degeneration and diabetic retinopathy. This experiment used Dutch Belted rabbits. After ocular administration of an eye drop formulation containing the compound, plasma and major ocular tissues were collected to determine the concentration of the analyte, studying the drug distribution in rabbit ocular tissues and its pharmacokinetic characteristics.

Experimental Protocol:

Male Dutch Belted rabbits used in this experiment were purchased from Pizhou Dongfang Breeding Co., Ltd., with animal weights ranging from 1.5 to 2.5 kg. All procedures performed on experimental animals in this experiment complied with the relevant SOP requirements of Suzhou Sunstone New Drug Development Co., Ltd. and were approved by the Institutional Animal Care and Use Committee (IACUC) of Suzhou Sunstone New Drug Development Co., Ltd. Formulation Preparation: Weigh approximately 1 mg of the test substance into a glass vial. After necessary calculation/conversion, sequentially add the eye drop formulation vehicle (Tris-HCl buffer (0.05 mol/L): Tween 80: 0.5% CMC-Na = 49: 1:50). Vortex and sonicate after adding Tris-HCl buffer and Tween 80, then add 0.5% CMC-Na, vortex, and sonicate or cut if necessary, to prepare a 1 mg/mL dosing formulation. All prepared dosing formulations were freshly prepared on the day of administration, sampled, and the actual concentration of the dosing formulation was determined.

On the day of the experiment, animals were administered 1 mg/mL (0.05 mg/eye, 50 μL/eye). There were 2 animals per group. Animals were euthanized at 0.5, 4, or 8 hours after administration, respectively, by an appropriate method, and blood samples and ocular tissues (i.e., cornea, retina, choroid, and sclera) were collected. The collected 1 mL of whole blood was placed in an EDTA-$K_2$ anticoagulant tube, inverted several times to mix thoroughly, and centrifuged (1500-1600 g) for 10 minutes within 30 minutes to separate plasma. The obtained plasma samples were stored at -80°C for bioanalysis. After collection, ocular tissues were washed with pre-cooled saline, dried, and stored at -80°C or under equivalent conditions for subsequent tissue homogenization and sample analysis. For ocular tissues requiring homogenization, the entire or a portion of the tissue was selected, accurately weighed, and homogenized at a 1:10 ratio with 20% methanol-water. The tissue homogenate was stored at -80°C for sample analysis. The concentrations of each analyte in rabbit ocular tissues and plasma obtained in this experiment were determined using an LC-MS/MS analytical method. The matrices were Dutch rabbit plasma (EDTA-$K_2$ anticoagulated) and rabbit ocular tissue (1:10, 20% methanol-water). Left and right eyes were tested separately. BLOQ (Below the Limit of Quantification) is the lowest analyte concentration that can be quantified with acceptable precision and accuracy. Compounds were diluted 1:10 with homogenate before analysis, and if the concentration after dilution was less than 1 ng/mL, it was marked as BLOQ.

Experimental Results:

Test results showed that compounds of the present invention with pVEGFR $IC_{50}$ <=5 nM (as shown in Table 8) were able to maintain sufficient drug concentrations in the posterior segment of the eye (choroid, 27.2 ng/g to 137 ng/g; retina, 14.5 ng/g to 71.9 ng/g) to bind relevant receptors targeting posterior segment ocular tissues, increasing posterior segment ocular bioavailability. Furthermore, they degraded rapidly in plasma (2.4 ng/mL to BLOQ), resulting in low systemic exposure of the compounds. In contrast, Axitinib showed low exposure in the posterior segment of the eye (choroid, 17.2 ng/g to 24 ng/g; retina, below BLOQ).

In the following ophthalmic formulation preparation examples, "solution" refers to a clear, transparent liquid without visible insoluble matter. "Near-clear solution" refers to a liquid that appears close to a solution upon visual observation but may contain a small amount of insoluble particles or have a slightly opaque color. "Suspension" refers to a liquid that is visibly turbid or opaque. "Gel" refers to a completely or partially gel-like solid.

Example 7: Ophthalmic Formulations Based on Polyethylene Glycol Series Vehicles

Preparation Method:

According to the formulation composition shown in the tables below, weigh the compound powder, add it to polyethylene glycol 400, and sonicate until dissolved or nearly dissolved (heat to 50°C if necessary). Add this to a Tris-HCl buffer solution containing hydroxypropyl-β-cyclodextrin, poloxamer 188, and gellan gum (or other viscosity modifier), vortex to mix, and prepare into an eye drop formulation.

Formulation examples are as follows:

Table 12 Formulation Composition and State

| Component | Percentage %* |
|---|---|
| Compound I-32 | 1mg/ml |
| Polyethylene glycol 400 | 5 |
| Hydroxypropyl-β-cyclodextrin | 10 |
| Tris-HCl buffer (pH 8.3) | 10mM |
| Hydroxypropyl methylcellulose (400 mPa.s) | 1 |
| Poloxamer 188 | 1 |
| Deionized water | q.s. to 100 |
| State Description | pH ~8, solution |
| * Liquid ratio is v/v, solid ratio is w/v (same below). | |

Hydroxypropyl methylcellulose can significantly increase viscosity. Although the exact viscosity was not measured, controlling its concentration at 1% w/v is more conducive to aspiration and compliance. Adding a buffer system helps to approach physiological pH. Adding poloxamer 188 can improve thermodynamic stability and reduce the rate of compound crystallization from the liquid.

Table 13 Formulation Composition and State

| Component | Percentage % |
|---|---|
| Compound I-84 | 1mg/ml |
| Polyethylene glycol 400 | 5 |
| Hydroxypropyl-β-cyclodextrin | 10 |
| Tris-HCl buffer (pH 8.3) | 50mM |
| Carbomer | 0.48 |
| Poloxamer 188 | 1 |
| Deionized water | q.s. to 100 |
| State Description | pH ~5, homogeneous suspension |

Carbomer can be used to increase the viscosity of eye drops. Its thermosensitive properties can cause the eye drops to gel on the ocular surface, increasing residence time.

Table 14 Formulation Composition and State

| Component | Percentage % |
|---|---|
| Compound I-84 | 5mg/ml |

(continued)

| Component | Percentage % |
|---|---|
| Polyethylene glycol 400 | 10 |
| Hydroxypropyl-β-cyclodextrin | 10 |
| Gellan Gum® Gelrite | 0.3 |
| Poloxamer 188 | 1 |
| 0.5 M Tris solution | 2 |
| 0.1 M Citric acid (for pH adjustment) | 1 |
| Deionized water | q.s. to 100 |
| State Description | pH ~7.5, near-clear solution |

Gellan gum, due to its ion-sensitive gelling properties, can increase ocular surface residence time of eye drops. Because the formulation contains gellan gum, its viscosity changes at different concentrations and pH, resulting in a significantly higher overall viscosity for the eye drop formulation.

Table 15 Formulation Composition and State (Compound Concentration: 10 mg/mL)

| Component | Percentage % | | | |
|---|---|---|---|---|
| | Compound 1-86 | Compound I-97 | Compound I-77 | Compound I-84 |
| Polyethylene glycol 400 | 10 | 10 | 10 | 10 |
| Hydroxypropyl-β-cyclodextrin | 10 | 10 | 10 | 10 |
| Gellan Gum® Gelrite | 0.3 | 0.3 | 0.3 | 0.3 |
| Poloxamer 188 | 1 | 1 | 1 | 1 |
| 0.5 M Tris solution | - | 1 | 8 | 5 |
| 0.1 M Citric acid | 6 | 3 | 2 | 5 |
| Deionized water | q.s. to 100 | q.s. to 100 | q.s. to 100 | q.s. to 100 |
| State Description | pH ~5.5, near-clear solution | pH ~5.5, gel | pH ~5.5, gel | pH ~5.5, gel |
| Note: In the above table, to investigate the effect of pH, the composition containing 1-86 does not contain a Tris solution (to allow for easier pH adjustment). | | | | |

Due to the pH sensitivity of gellan gum, salt-form compounds tend to form gels. Ideally, the pH should not be lower than 4 before adding gellan gum.

Table 16 Formulation Composition and State (Compound Concentration: 2 mg/mL)

| Component | Percentage % | | | | | |
|---|---|---|---|---|---|---|
| | Compound I-86 | Compound I-104 | Compound I-97 | Compound I-84 | Compound I-67 | Compound I-75 |
| Polyethylene Glycol 400 | 5 | 5 | 5 | 5 | 5 | 5 |
| Poloxamer 188 | 1 | 1 | 1 | 1 | 1 | 1 |
| Hydroxypropyl-β-cyclodextrin | 6 | 6 | 6 | 6 | 6 | 6 |
| Gellan Gum® Gelrite | 0.15 | 0.15 | 0.15 | 0.15 | 0.15 | 0.15 |
| Tris(hydroxymethyl ) aminomethane-Hydrochloric Acid Buffer (pH 6.8) | 25mM | 25mM | 25mM | 25mM | 25mM | 25mM |

(continued)

| Component | Percentage % | | | | | |
|---|---|---|---|---|---|---|
| | Compound I-86 | Compound I-104 | Compound I-97 | Compound I-84 | Compound I-67 | Compound I-75 |
| Deionized Water | q.s. to 100 | q.s. to 100 | q.s. to 100 | q.s. to 100 | q.s. to 100 | q.s. to 100 |
| State Description | pH ~7, solution | pH ~7, solution | pH ~7, solution | pH ~7, solution | pH ~7, solution | pH ~7, solution |
| Osmolality (mOsm/kg) | ~300 | ~300 | ~300 | ~300 | ND | ND |
| ND: Not detected. | | | | | | |

Gellan Gum® Gelrite exhibits relatively high viscosity and is prone to gelation when adjusting pH or when compound concentration increases; therefore, the 0.3% Gelrite was reduced to 0.15%.

Overall Description:

Most compounds show good solubility in Polyethylene Glycol 400, exceeding 10 mg/mL, with some compounds reaching 100 mg/mL. Therefore, using Polyethylene Glycol 400 or similar co-solvents to first dissolve the compound is highly effective in eliminating the limitations of drug crystal form on the dissolution process. However, the osmolality of Polyethylene Glycol 400 is relatively high, so its content needs to be controlled, preferably within 10%, more preferably within 5%.

Example 8: Ophthalmic Formulation Based on In Situ Salt Formation Technology

During the research, it was found that the compounds of the present invention are pH-sensitive; lowering the pH significantly enhances the solubilizing effect on the compounds. Therefore, by preparing ophthalmic formulation compositions based on in situ salt formation technology, the solubility of the compounds can be further improved.

Preparation Method:

According to the formulation composition shown in the table below, weigh the compound powder, add 50% of the total volume (v/v) of a 30% (w/v) aqueous solution of hydroxypropyl-β-cyclodextrin (containing surfactant), and disperse by sonication; next, add an appropriate volume of pH regulator and sonicate until clear; then, add an equal volume of buffer containing solubilizer, viscosity modifier, etc., for dilution, and immediately vortex to mix; finally, add an appropriate volume of alkali regulator to adjust the solution pH to approximately 7, to prepare an eye drop formulation.

Formulation examples are as follows:

Table 17 Formulation Composition and State (Compound I-97 Concentration: 5 mg/mL)

| Component | Percentage % | | | | | |
|---|---|---|---|---|---|---|
| Hydroxypropyl-β-cyclodextrin | 15 | 15 | 15 | 15 | 15 | 15 |
| Poloxamer 188 | 1 | 1 | 1 | 1 | 1 | 1 |
| Gellan Gum® Gelrite | 0.15 | 0.15 | 0.15 | 0.15 | 0.15 | 0.15 |
| Tris(hydroxymethyl)ami nomethane-Hydrochloric Acid Buffer (pH 6.8) | 25mM | 25mM | 25mM | 25mM | 25mM | 25mM |
| 1M Methanesulfonic Acid | 1 | -- | -- | -- | -- | -- |
| 1M Lactic Acid | -- | 1 | -- | -- | -- | -- |
| 1M Tartaric Acid | -- | -- | 1 | -- | -- | -- |
| 1M Acetic Acid | -- | -- | -- | 1 | -- | -- |

(continued)

| Component | Percentage % | | | | | |
|---|---|---|---|---|---|---|
| 0.05M Fumaric Acid | -- | -- | -- | -- | 10 | -- |
| 1M Maleic Acid | -- | -- | -- | -- | -- | 1 |
| 0.5M Tris(hydroxymethyl)aminomethane | 0.5 | 0.5 | 1 | 0.5 | 0.5 | 1.5 |
| Deionized Water | q.s. to 100 | q.s. to 100 | q.s. to 100 | q.s. to 100 | q.s. to 100 | q.s. to 100 |
| State Description | pH ~7, near-clear solution | pH ~7, near-clear solution | pH ~7, near-clear solution | pH ~7, near-clear solution | pH ~7, suspension | pH ~7, near-clear solution |

Using the in situ salt formation method, the compound is first dispersed in a solution containing Hydroxypropyl-β-cyclodextrin, then different acids are added to adjust the pH to 3-5, allowing the compound to fully or nearly fully dissolve, followed by adding the remaining components and adjusting the pH to approximately 7. Different acids exhibit varying degrees of solubilizing effect. Based on the visual dissolution effect after acid addition, methanesulfonic acid, acetic acid, and lactic acid were preliminarily selected as pH regulators. The preliminarily selected acids were used in subsequent tests with higher compound concentrations.

Table 18 Formulation Composition and State

| Component | Percentage % | | | | | |
|---|---|---|---|---|---|---|
| Compound I-97 | 10mg/ml | 10 mg/ml | 10 mg/ml | 15 mg/ml | 20 mg/ml | 20 mg/ml |
| Hydroxypropyl-β-cyclodextrin | 15 | 15 | 15 | 15 | 15 | 15 |
| Polyoxyethylene castor oil EL | -- | -- | -- | -- | -- | 1 |
| Poloxamer 188 | 1 | 1 | 1 | 1 | 1 | 1 |
| Gellan Gum® Gelrite | 0.15 | 0.15 | 0.15 | 0.15 | 0.15 | 0.15 |
| Tris(hydroxymethyl) a minomethane-Hydrochloric Acid Buffer (pH 6.8) | 25mM | 25mM | 25mM | 25mM | 25mM | 25mM |
| 1M Methanesulfonic Acid | 2 | -- | -- | 3 | 4 | 4 |
| 1M Lactic Acid | -- | 2 | -- | -- | -- | -- |
| 1M Acetic Acid | -- | -- | 2 | -- | -- | -- |
| 0.5M Tris(hydroxymethyl)a minomethane | 1 | 1 | 1 | 1 | 0.5 | 1 |
| Deionized Water | q.s. to 100 | q.s. to 100 | q.s. to 100 | q.s. to 100 | q.s. to 100 | q.s. to 100 |
| State Description | pH ~7, near-clear solution, with opalescence | pH ~7, near-clear solution, with opalescence | pH ~7, near-clear solution, with opalescence | pH ~7, near-clear solution, with opalescence | pH ~7, near-clear solution, with opalescence | pH ~7, solution |

The addition of the surfactant polyoxyethylene castor oil EL can improve the solubility of the compound.

Table 19 Formulation Composition and State

| Component | Percentage % | | | | | |
|---|---|---|---|---|---|---|
| Compound 1-67 | 5 mg/ml | 5 mg/ml | 5 mg/ml | 10 mg/ml | 10 mg/ml | 20 mg/ml |
| Hydroxypropyl-β-cyclodextrin | 15 | 15 | 15 | 15 | 15 | 15 |
| Polyoxyethylene castor oil EL | -- | -- | 1 | -- | -- | 1 |
| Poloxamer 188 | 1 | 1 | 1 | 1 | 1 | 1 |
| Gellan Gum® Gelrite | 0.15 | 0.15 | 0.15 | 0.15 | 0.15 | 0.15 |
| Tris(hydroxymethyl)a minomethane-Hydrochloric Acid Buffer (pH 6.8) | 25mM | 25mM | 25mM | 25mM | 25mM | 25mM |
| 1M Methanesulfonic Acid | 1 | -- | 1 | -- | -- | 4 |
| 1M Benzenesulfonic Acid | -- | 1 | -- | -- | -- | -- |
| 1M Lactic Acid | -- | -- | -- | 2 | -- | -- |
| 1M Acetic Acid | -- | -- | -- | -- | 2 | -- |
| 0.5M Tris(hydroxymethyl)a minomethane | 0.5 | -- | 0.5 | 1 | 1 | 1.5 |
| Deionized Water | q.s. to 100 | q.s. to 100 | q.s. to 100 | q.s. to 100 | q.s. to 100 | q.s. to 100 |
| State Description | pH ~7, near-clear solution | pH ~7, solution | pH ~7, solution | pH ~7, solution | pH ~7, solution | pH ~7, near-clear solution |
| Note: In the above table, the composition shown in column 2 does not contain alkali because the system pH was approximately 7 after equal-volume dilution with the buffer containing solubilizer, viscosity modifier, etc., so no additional alkali was added. | | | | | | |

Polyoxyethylene castor oil EL can increase compound solubility. Different acids have varying solubilizing capacities for the compounds, requiring different acid concentrations to achieve the same solubility effect.

Table 20 Formulation Composition and State

| Component | Percentage % | |
|---|---|---|
| Compound I-110 | 10 mg/ml | 20 mg/ml |
| Hydroxypropyl-β-cyclodextrin | 15 | 15 |
| Polyoxyethylene castor oil EL | 1 | 1 |
| Poloxamer 188 | 1 | 1 |
| Gellan Gum® Gelrite | 0.15 | 0.15 |
| Tris(hydroxymethyl)aminomethane-Hydrochloric Acid Buffer (pH 6.8) | 25mM | 25mM |
| 1M Methanesulfonic Acid | 2 | 4 |
| 0.5M Tris(hydroxymethyl)aminomethane | 2 | 2 |
| Deionized Water | q.s. to 100 | q.s. to 100 |
| State Description | pH ~7, near-clear solution | pH ~7, near-clear solution |

Table 21 Formulation Composition and State

| Component | Percentage % | |
|---|---|---|
| Compound 1-44 | 10 mg/ml | 20 mg/ml |
| Hydroxypropyl-β-cyclodextrin | 15 | 15 |
| Polyoxyethylene castor oil EL | 1 | 1 |
| Poloxamer 188 | 1 | 1 |
| Gellan Gum® Gelrite | 0.15 | 0.15 |
| Tris(hydroxymethyl)aminomethane-Hydrochloric Acid Buffer (pH 6.8) | 25mM | 25mM |
| 1M Methanesulfonic Acid | 1 | 4 |
| 0.5M Tris(hydroxymethyl)aminomethane | -- | 1 |
| Deionized Water | q.s. to 100 | q.s. to 100 |
| State Description | pH ~7, near-clear solution | pH ~7, near-clear solution |

Overall Description: The compounds of the present invention can form solutions or near-clear solutions with a certain viscosity at 10 mg/mL; some compounds can form solutions or near-clear solutions with higher viscosity at 20 mg/mL, while ensuring good stability over a period of time, with a final pH range of 5-8.

Example 9: Detection of Plasma and Ocular Tissue Distribution after Single Ocular Administration of Formulations in Dutch Belted Rabbits

The experimental procedure refers to Example 6. Animals were administered eye drops at 5 mg/mL (0.25 mg/eye, 50 μL/eye) or 1 mg/mL (0.05 mg/eye, 50 μL/eye), respectively. The administered formulations and results are shown in the table below.

Table 22 Experimental Results

| Compound | Compound I-32 | | |
|---|---|---|---|
| Formulation Number & Concentration | Suspension A | Suspension A | Solution B |
| Concentration (mg/mL) | 5 | 1 | 1 |
| Formulation pH | 8.4 | 8.4 | 8.3 |
| Plasma Concentration 0.5h (ng/mL) | 5.6 | 2.4 | BLOQ |
| Cornea AUC (ng*h/g) | 5175 | 3380 | 590 |
| Conjunctiva AUC (ng*h/g) | 17380.5 | 6157 | 2142 |
| Retina AUC (ng*h/g) | **613** | **330** | **784** |
| Choroid AUC (ng*h/g) | 1594.5 | 526 | 381 |
| Sclera AUC (ng*h/g) | 2439.5 | 2175 | 998 |
| Iris AUC (ng*h/g) | 549.5 | 162 | NA |

Except for compound I-32, the other components of Suspension A and Solution B are respectively:

Suspension A: Tris(hydroxymethyl)aminomethane-Hydrochloric Acid Buffer (0.05M): Tween 80: 0.5% (w/v) Sodium Carboxymethylcellulose aqueous solution = 49:1:50 (v/v/v);

Solution B: Polyethylene Glycol 400: 50mM Tris(hydroxymethyl)aminomethane-Hydrochloric Acid Buffer (containing 10% Hydroxypropyl-β-cyclodextrin, 1% Poloxamer 188, 0.3% Gellan Gum Gelrite, w/v) = 5:95 (v/v). The above results show that the formulation of the present invention (Solution B, an example of Formulation I of the present invention) can significantly increase drug concentration in the retina, reduce corneal concentration, and lower the risk of toxic side effects.

Example 10: Effect of Gellan Gum on Formulation Viscosity and Viscosity Change after Adding Simulated Tear Fluid

This example investigates the effect of gellan gum on formulation viscosity and simulates the scenario of eye drop instillation by adding simulated tear fluid to the formulation at a volume ratio of 3.6:1. The experimental results are shown in the table below.

Table 23 Effect of Gellan Gum on Formulation Viscosity

| Formulation | Compound | Concentration (mg/mL) | Gellan Gum Ratio | Test Temperature (°C) | Formulation Viscosity (mPa.s) | Viscosity after Adding STF (mPa.s) |
|---|---|---|---|---|---|---|
| 1 | I-97 | 5 | 0.15% w/v | 25 | 19.01 | 24.19 |
| | | | | 37 | 7.97 | 21.95 |
| 2 | I-44 | 5 | 0.15% w/v | 25 | 23.27 | 27.26 |
| | | | | 37 | 9.53 | 24.06 |

The ophthalmic drop formulation in this example was prepared using the preparation method from Example 8 (i.e., one type of Formulation II of the present invention). Except for compound , the other components in the formulation are: 30% Hydroxypropyl-β-cyclodextrin solution (containing 2% polyoxyethylene castor oil EL): 50 mM Tris(hydroxymethyl)aminomethane-Hydrochloric Acid Buffer (containing 2% Poloxamer 188, 0.3% Gellan Gum Gelrite, w/v) = 50:50 v/v, adjusted to pH using Methanesulfonic Acid and Tris(hydroxymethyl)aminomethane. The above results show that at room temperature (25°C), the ophthalmic drop formulation of the present invention has a suitable viscosity, and its viscosity increases after adding simulated tear fluid (STF), with the simultaneous observation of gel formation, demonstrating the in-situ gelling effect of gellan gum. Under conditions close to ocular temperature (37°C), although the viscosity of the formulation itself decreases, in the simulated eye drop scenario, the viscosity increases significantly and remains at a reasonable level, which is beneficial for the retention of the compound in the eye.

The foregoing detailed description has been presented for purposes of illustration and explanation, and is not intended to limit the scope of the appended claims. Many variations of the embodiments of the invention presently exemplified will be apparent to one of ordinary skill in the art and remain within the scope of the appended claims and their equivalents.

## Claims

**1.** An ophthalmic formulation, comprising:

a. a compound or a pharmaceutically acceptable salt thereof;
b. a co-solvent;
c. a pharmaceutically acceptable excipient;

wherein said pharmaceutically acceptable excipient comprises one or more of a solubilizer, a stabilizer, a viscosity modifier, and a buffer;
said compound has the following structure:

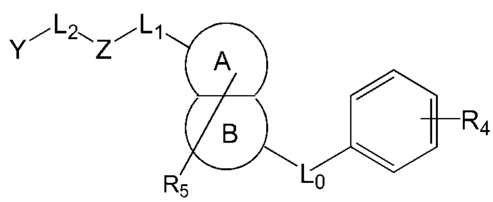

(II)

wherein,
Ring A is a 5- to 7-membered nitrogen-containing heterocycle;
Ring B is a 5- to 7-membered aromatic ring or heterocycle;

Z is selected from: -C(O)NH-, -NHC(O)-, or

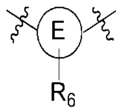

;

Ring E is an aromatic ring or heteroaromatic ring;

$L_0$ is selected from: O, S, $N(R_{La})$, $C_1$-$C_6$ alkylene, C(O); $R_{La}$ is selected from: H, $C_1$-$C_6$ alkyl;

$L_1$ is selected from: a single bond, $C_1$-$C_6$ alkylene, $C_2$-$C_6$ alkenylene, $C_2$-$C_6$ alkynylene, -($C_0$-$C_6$ alkylene)-$Q_1$-($C_0$-$C_6$ alkylene)-, $Q_1$ is selected from: -O-, -S-, -C(O)-, -C(O)O-, -OC(O)-, -C(O)N($R_{Lb}$)-, -N($R_{Lb}$)C(O)-, - N($R_{Lb}$)C(O)O-, -N($R_{Lb}$)C(O)N($R_{Lb}$)-, -N($R_{Lb}$)-, -S(O)$_2$-, -S(O)$_2$N($R_{Lb}$)-, -N($R_{Lb}$)S(O)$_2$-, -S(O)-, -S(O)N($R_{Lb}$)-, - N($R_{Lb}$)S(O)-; H atoms in said alkylene may optionally be substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lb}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$L_2$ is selected from: a single bond, $C_1$-$C_6$ alkylene, -($C_0$-$C_6$ alkylene)-$Q_2$-($C_0$-$C_6$ alkylene)-, $Q_2$ is selected from: - O-, -S-, -C(O)-, -C(O)O-, -OC(O)-, -C(O)N($R_{Lc}$)-, -N($R_{Lc}$)C(O)-, -N($R_{Lc}$)C(O)O-, -N($R_{Lc}$)C(O)N($R_{La}$)-, -N($R_{Lc}$)-, -S(O)$_2$-, -S(O)$_2$N($R_{Lc}$)-, -N($R_{Lc}$)S(O)$_2$-, -S(O)-, -S(O)N($R_{Lc}$)-, -N($R_{Lc}$)S(O)-; H atoms in said alkylene may optionally be substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lc}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

Y is selected from: H, -$NR_7R_8$, nitrogen-containing heterocyclyl, wherein said nitrogen-containing heterocyclyl may optionally be substituted by one or more $R_9$ groups, and $R_9$ is selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), - ($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_7$ and $R_8$ are each independently selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, 4- to 10-membered heterocyclyl, 4- to 10-membered heterocyclylalkyl; wherein said $C_1$-$C_{10}$ alkyl, $C_2$-$C_{10}$ alkenyl, $C_2$-$C_{10}$ alkynyl, $C_3$-$C_{10}$ cycloalkyl, 4- to 10-membered heterocyclyl may optionally be substituted by one or more groups selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$R_4$ is one or more independent substituents on the benzene ring, each $R_4$ being independently selected from: H, halogen, $C_1$-$C_{10}$ alkyl, cyano, $C_1$-$C_{10}$ haloalkyl, $C_1$-$C_{10}$ haloalkoxy, $C_1$-$C_{10}$ cyanoalkyl, -$OR_{401}$, -C(O)$R_{401}$, - C(O)O$R_{401}$, -$NR_{402}$C(O)O$R_{401}$, -OC(O)$R_{401}$, -$NR_{402}SO_2R_{401}$, -$SO_2NR_{401}R_{402}$, -$NR_{402}$C(O)$R_{401}$, -C(O)$NR_{401}R_{402}$, - $NR_{401}R_{402}$, -($C_0$-$C_6$ alkylene)-$NR_{401}R_{402}$, -$SR_{401}$, -S(O)$R_{401}$, -S(O)$_2R_{401}$, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); wherein said $C_0$-$C_6$ alkylene, $C_1$-$C_{10}$ alkyl, $C_2$-$C_{10}$ alkenyl, $C_2$-$C_{10}$ alkynyl, $C_3$-$C_{10}$ cycloalkyl, $C_6$-$C_{10}$ aryl, 4- to 10-membered heterocyclyl may optionally be substituted by one or more groups selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl),

-$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_{401}$ and $R_{402}$ are each independently selected from: H, $C_1$-$C_{10}$ alkyl, $C_3$-$C_{10}$ cycloalkyl, $C_3$-$C_{10}$ cycloalkylalkyl, $C_6$-$C_{10}$ aryl, $C_6$-$C_{10}$ aralkyl, 4- to 10-membered heterocyclyl, 4- to 10-membered heterocyclylalkyl;

$R_5$ is one or more independent substituents on the

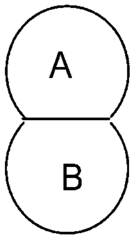

ring, each $R_5$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_6$ is one or more independent substituents on Ring E, each $R_6$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl).

**2.** The ophthalmic formulation according to claim 1, wherein the concentration of said compound is 0.01% w/v to 2.5% w/v, preferably 0.1% w/v to 1.5% w/v.

**3.** The ophthalmic formulation according to claim 1 or 2, wherein said co-solvent is selected from: polyethylene glycol (particularly liquid polyethylene glycol, such as polyethylene glycol 200, 300, 400), propylene glycol, glycerol; preferably polyethylene glycol 400; and/or, the concentration of said co-solvent is 0.1% v/v to 10% v/v, preferably 0.5% v/v to 5% v/v.

**4.** The ophthalmic formulation according to any one of claims 1-3, wherein said solubilizer is cyclodextrin, preferably hydroxypropyl-β-cyclodextrin, sulfobutyl ether-β-cyclodextrin, hydroxypropyl-γ-cyclodextrin; and/or, the concentration of said solubilizer is 1% w/v to 30% w/v.

**5.** The ophthalmic formulation according to any one of claims 1-4, wherein said stabilizer is selected from:
poloxamer, polyethenyl alcohol, polyethenylpyrrolidone, or a combination thereof, preferably poloxamer 188; and/or, the concentration of said stabilizer is 0.1% w/v to 10% w/v.

**6.** The ophthalmic formulation according to any one of claims 1-5, wherein said viscosity modifier is selected from: a cellulose derivative (e.g., methylcellulose, hydroxypropyl cellulose, hydroxypropyl methylcellulose), a biocompatible polysaccharide (e.g., gellan gum, xanthan gum, chitosan and derivatives thereof, sodium alginate, sodium hyaluronate), carbomer, or a combination thereof;

preferably, said viscosity modifier is gellan gum at a concentration ranging from 0.01% v/v to 1% v/v, preferably 0.1% v/v to 0.5% v/v; or,
said viscosity modifier is carbomer at a concentration ranging from 0.01% v/v to 1% v/v, preferably 0.1% v/v to 0.6% v/v; or,

said viscosity modifier is hydroxypropyl methylcellulose at a concentration ranging from 0.01% v/v to 1% v/v.

**7.** The ophthalmic formulation according to any one of claims 1-6, wherein said buffer is selected from: borate, phosphate, bicarbonate, carbonate, citrate, tetraborate, hydrogen phosphate, tromethamine, 2-morpholinoethanol, tris(hydroxymethyl)aminomethane, L-carnosine;
preferably, said buffer is tris(hydroxymethyl)aminomethane-hydrochloric acid buffer.

**8.** The ophthalmic formulation according to any one of claims 1-7, wherein said pharmaceutically acceptable excipient further comprises a surfactant;

preferably, said surfactant is selected from: polyoxyethylene castor oil, tyloxapol, polysorbate, polyoxyl hydrogenated castor oil, or a combination thereof, preferably polyoxyethylene castor oil EL;
preferably, the concentration of said surfactant is 0.1% w/v to 10% w/v.

**9.** The ophthalmic formulation according to any one of claims 1-8, wherein said pharmaceutically acceptable excipient further comprises one or more of a pH adjuster, an osmotic pressure adjuster, a preservative, and a solvent.

**10.** The ophthalmic formulation according to any one of claims 1-8, wherein said ophthalmic formulation comprises:

a. said compound or a pharmaceutically acceptable salt thereof;
b. polyethylene glycol 400;
c. hydroxypropyl-β-cyclodextrin, poloxamer 188, gellan gum, tris(hydroxymethyl)aminomethane-hydrochloric acid buffer, deionized water; or,

said ophthalmic formulation comprises:

a. said compound or a pharmaceutically acceptable salt thereof;
b. polyethylene glycol 400;
c. hydroxypropyl-β-cyclodextrin, poloxamer 188, carbomer, tris(hydroxymethyl)aminomethane-hydrochloric acid buffer, deionized water; or,

said ophthalmic formulation comprises:

a. said compound or a pharmaceutically acceptable salt thereof;
b. polyethylene glycol 400;
c. hydroxypropyl-β-cyclodextrin, poloxamer 188, hydroxypropyl methylcellulose,

tris(hydroxymethyl)aminomethane-hydrochloric acid buffer, deionized water.

**11.** The ophthalmic formulation according to any one of claims 1-10, wherein said compound is selected from compounds I-1 to I-11, I-13 to I-139.

**12.** The ophthalmic formulation according to any one of claims 1-11, wherein said ophthalmic formulation is a homogeneous formulation, such as a solution, emulsion, suspension, gel, particularly a solution;

preferably, the pH of said ophthalmic formulation is 5 to 9, preferably 6 to 8;
preferably, the osmotic pressure of said ophthalmic formulation is 200 mOsm/kg to 500 mOsm/kg, preferably 250 mOsm/kg to 450 mOsm/kg.

**13.** A method for preparing the ophthalmic formulation according to any one of claims 1-12, comprising the following steps:

(1) adding said compound or a pharmaceutically acceptable salt thereof to a co-solvent, and dispersing;
(2) adding the mixture obtained in step (1) to said pharmaceutically acceptable excipient, and mixing;
preferably, step (2) comprises: adding the mixture obtained in step (1) to a buffer containing a solubilizer, a stabilizer, a surfactant, and a viscosity modifier, and mixing.

**14.** An ophthalmic formulation, the raw materials used for its preparation comprise:

a. a compound;
b. a pharmaceutically acceptable excipient;
c. an acidic pH adjuster;
d. a basic pH adjuster;

wherein, said pharmaceutically acceptable excipient comprises one or more of a solubilizer, a stabilizer, a surfactant, a viscosity modifier, and a buffer;
said compound has the following structure:

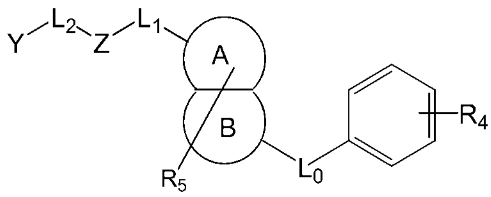

(II)

wherein,
Ring A is a 5- to 7-membered nitrogen-containing heterocycle;
Ring B is a 5- to 7-membered aromatic ring or heterocycle;
Z is selected from: -C(O)NH-, -NHC(O)-, or

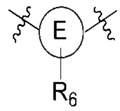
;

Ring E is an aromatic ring or heteroaromatic ring;
$L_0$ is selected from: O, S, $N(R_{La})$, $C_1$-$C_6$ alkylene, C(O); $R_{La}$ is selected from: H, $C_1$-$C_6$ alkyl;
$L_1$ is selected from: a single bond, $C_1$-$C_6$ alkylene, $C_2$-$C_6$ alkenylene, $C_2$-$C_6$ alkynylene, -($C_0$-$C_6$ alkylene)-$Q_1$-($C_0$-$C_6$ alkylene)-, $Q_1$ is selected from: -O-, -S-, -C(O)-, -C(O)O-, -OC(O)-, -C(O)N($R_{Lb}$)-, -N($R_{Lb}$)C(O)-, - N($R_{Lb}$)C(O)O-, -N($R_{Lb}$)C(O)N($R_{Lb}$)-, -N($R_{Lb}$)-, -S(O)$_2$-, -S(O)$_2$N($R_{Lb}$)-, -N($R_{Lb}$)S(O)$_2$-, -S(O)-, -S(O)N($R_{Lb}$)-, - N($R_{Lb}$)S(O)-; H atoms in said alkylene may optionally be substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lb}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;
$L_2$ is selected from: a single bond, $C_1$-$C_6$ alkylene, -($C_0$-$C_6$ alkylene)-$Q_2$-($C_0$-$C_6$ alkylene)-, $Q_2$ is selected from: - O-, -S-, -C(O)-, -C(O)O-, -OC(O)-, -C(O)N($R_{Lc}$)-, -N($R_{Lc}$)C(O)-, -N($R_{Lc}$)C(O)O-, -N($R_{Lc}$)C(O)N($R_{La}$)-, -N($R_{Lc}$)-, -S(O)$_2$-, -S(O)$_2$N($R_{Lc}$)-, -N($R_{Lc}$)S(O)$_2$-, -S(O)-, -S(O)N($R_{Lc}$)-, -N($R_{Lc}$)S(O)-; H atoms in said alkylene may optionally be substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lc}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;
Y is selected from: H, -$NR_7R_8$, nitrogen-containing heterocyclyl, wherein said nitrogen-containing heterocyclyl may optionally be substituted by one or more $R_9$ groups, and $R_9$ is selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), - ($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);
$R_7$ and $R_8$ are each independently selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, 4- to 10-membered heterocyclyl, 4- to 10-membered heterocyclylalkyl;

wherein said $C_1$-$C_{10}$ alkyl, $C_2$-$C_{10}$ alkenyl, $C_2$-$C_{10}$ alkynyl, $C_3$-$C_{10}$ cycloalkyl, and 4- to 10-membered heterocyclyl may optionally be substituted by one or more groups selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$R_4$ is one or more independent substituents on the benzene ring, each $R_4$ being independently selected from: H, halogen, $C_1$-$C_{10}$ alkyl, cyano, $C_1$-$C_{10}$ haloalkyl, $C_1$-$C_{10}$ haloalkoxy, $C_1$-$C_{10}$ cyanoalkyl, -$OR_{401}$, -C(O)$R_{401}$, - C(O)O$R_{401}$, -$NR_{402}$C(O)O$R_{401}$, -OC(O)$R_{401}$, -$NR_{402}SO_2R_{401}$, -$SO_2NR_{401}R_{402}$, -$NR_{402}$C(O)$R_{401}$, -C(O)$NR_{401}R_{402}$, - $NR_{401}R_{402}$, -($C_0$-$C_6$ alkylene)-$NR_{401}R_{402}$, -$SR_{401}$, -S(O)$R_{401}$, -S(O)$_2R_{401}$, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); wherein said $C_0$-$C_6$ alkylene, $C_1$-$C_{10}$ alkyl, $C_2$-$C_{10}$ alkenyl, $C_2$-$C_{10}$ alkynyl, $C_3$-$C_{10}$ cycloalkyl, $C_6$-$C_{10}$ aryl, and 4- to 10-membered heterocyclyl may optionally be substituted by one or more groups selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_{401}$ and $R_{402}$ are each independently selected from: H, $C_1$-$C_{10}$ alkyl, $C_3$-$C_{10}$ cycloalkyl, $C_3$-$C_{10}$ cycloalkylalkyl, $C_6$-$C_{10}$ aryl, $C_6$-$C_{10}$ aralkyl, 4- to 10-membered heterocyclyl, 4- to 10-membered heterocyclylalkyl;

$R_5$ is one or more independent substituents on the

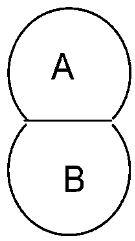

ring, each $R_5$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_6$ is one or more independent substituents on Ring E, each $R_6$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl).

**15.** The ophthalmic formulation according to claim 14, wherein the concentration of said compound is 0.01% w/v to 2.5% w/v.

**16.** The ophthalmic formulation according to claim 14 or 15, wherein said solubilizer is a cyclodextrin, preferably selected

from hydroxypropyl-β-cyclodextrin, sulfobutylether-β-cyclodextrin, hydroxypropyl-γ-cyclodextrin; and/or,
the concentration of said solubilizer is 1% w/v to 30% w/v.

**17.** The ophthalmic formulation according to any one of claims 14-16, wherein said stabilizer is selected from:

poloxamer, polyethenyl alcohol, polyethenylpyrrolidone, or a combination thereof, preferably poloxamer 188; and/or,
the concentration of said stabilizer is 0.1% w/v to 10% w/v.

**18.** The ophthalmic formulation according to any one of claims 14-17, wherein said viscosity modifier is selected from: a cellulose derivative (e.g., methylcellulose, hydroxypropyl cellulose, hydroxypropyl methylcellulose), a biocompatible polysaccharide (e.g., gellan gum, xanthan gum, chitosan and derivatives thereof, sodium alginate, sodium hyaluronate), carbomer, or a combination thereof;

preferably, said viscosity modifier is gellan gum at a concentration ranging from 0.01% v/v to 1% v/v, preferably 0.1% v/v to 0.5% v/v; or,
said viscosity modifier is carbomer at a concentration ranging from 0.01% v/v to 1% v/v, preferably 0.1% v/v to 0.6% v/v; or,
said viscosity modifier is hydroxypropyl methylcellulose at a concentration ranging from 0.01% v/v to 1% v/v.

**19.** The ophthalmic formulation according to any one of claims 14-18, wherein said buffer is selected from:

borate, phosphate, bicarbonate, carbonate, citrate, tetraborate, hydrogen phosphate, tromethamine, 2-(2-hydroxyethyl)morpholine, tris(hydroxymethyl)aminomethane, L-carnosine;
preferably, said buffer is tris(hydroxymethyl)aminomethane-hydrochloric acid buffer.

**20.** The ophthalmic formulation according to any one of claims 14-19, wherein said acidic pH adjuster is selected from: aspartic acid, glutamic acid, malonic acid, salicylic acid, maleic acid, fumaric acid, succinic acid, benzoic acid, propionic acid, acetic acid, decanoic acid, stearic acid, oleic acid, hexanoic acid, octanoic acid, tartaric acid, citric acid, malic acid, gluconic acid, glycolic acid, lactic acid, hydrobromic acid, hydrochloric acid, sulfuric acid, nitric acid, phosphoric acid, methanesulfonic acid, benzenesulfonic acid, p-toluenesulfonic acid, ethanesulfonic acid, hydroxyethanesulfonic acid, preferably selected from: methanesulfonic acid, lactic acid, acetic acid, particularly methanesulfonic acid; and/or,
said alkaline pH adjuster is selected from: sodium hydroxide, tris(hydroxymethyl)aminomethane, preferably tris(hydroxymethyl)aminomethane.

**21.** The ophthalmic formulation according to any one of claims 14-20, wherein said surfactant is selected from:

polyoxyethylene castor oil, tyloxapol, polysorbate, polyoxyl hydrogenated castor oil, or a combination thereof, preferably polyoxyethylene castor oil EL;
preferably, the concentration of said surfactant is 0.1% w/v to 10% w/v.

**22.** The ophthalmic formulation according to any one of claims 14-21, wherein said pharmaceutically acceptable excipient further comprises one or more of an osmotic pressure adjuster, a preservative, and a solvent.

**23.** The ophthalmic formulation according to any one of claims 14-22, wherein the raw materials used for preparation of said ophthalmic formulation comprise:

a. said compound;
b. hydroxypropyl-β-cyclodextrin, poloxamer 188, gellan gum, tris(hydroxymethyl)aminomethane-hydrochloric acid buffer, deionized water;
c. methanesulfonic acid;
d. tris(hydroxymethyl)aminomethane.

**24.** The ophthalmic formulation according to any one of claims 14-23, wherein said compound is selected from compounds I-1 to I-11, I-13 to I-139.

**25.** The ophthalmic formulation according to any one of claims 14-24, wherein said ophthalmic formulation is a

homogeneous formulation, such as a solution, emulsion, suspension, gel, particularly a solution;

preferably, the pH of said ophthalmic formulation is 5 to 9, preferably 6 to 8;
preferably, the osmotic pressure of said ophthalmic formulation is 200 mOsm/kg to 500 mOsm/kg, preferably 250 mOsm/kg to 450 mOsm/kg.

**26.** A method for preparing the ophthalmic formulation according to any one of claims 14-25, comprising the following steps:

(1) adding said compound to a solution containing a portion of pharmaceutically acceptable excipients, and dispersing;
(2) adding an acidic pH adjuster, and mixing;
(3) adding the remaining pharmaceutically acceptable excipients, and mixing;
(4) adding an alkaline pH adjuster;

said portion of pharmaceutically acceptable excipients in step (1) is a solubilizer and a surfactant;
preferably, the solution containing a portion of pharmaceutically acceptable excipients in step (1) accounts for 50% of the total volume of said formulation;
Preferably, the amount of said acidic pH adjuster added in step (2) is to adjust the pH to 3-5;
preferably, step (3) is adding a buffer containing a stabilizer and a viscosity modifier, and mixing;
preferably, the amount of said alkaline pH adjuster added in step (4) is to adjust the pH to 5 to 9, particularly 6 to 8.

**27.** An ophthalmic formulation, comprising:

a. a compound or a pharmaceutically acceptable salt thereof;
b. a pharmaceutically acceptable excipient;

preferably, said pharmaceutically acceptable excipient comprises one or more of a solubilizer, a stabilizer, a surfactant, a viscosity modifier, and a buffer;
said compound has the following structure:

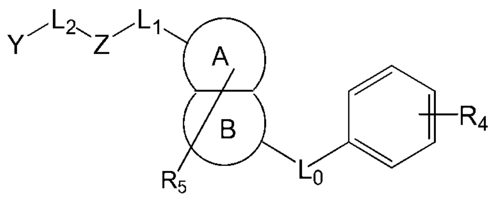

(II)

wherein,
Ring A is a 5- to 7-membered nitrogen-containing heterocycle;
Ring B is a 5- to 7-membered aromatic ring or heterocycle;
Z is selected from: -C(O)NH-, -NHC(O)-, or

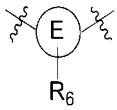

;

Ring E is an aromatic ring or heteroaromatic ring;
Lo is selected from: O, S, N($R_{La}$), $C_1$-$C_6$ alkylene, C(O); $R_{La}$ is selected from: H, $C_1$-$C_6$ alkyl;
$L_1$ is selected from: a single bond, $C_1$-$C_6$ alkylene, $C_2$-$C_6$ alkenylene, $C_2$-$C_6$ alkynylene, -($C_0$-$C_6$ alkylene)-$Q_1$-($C_0$-$C_6$ alkylene)-, $Q_1$ is selected from: -O-, -S-, -C(O)-, -C(O)O-, -OC(O)-, -C(O)N($R_{Lb}$)-, -N($R_{Lb}$)C(O)-, - N($R_{Lb}$)C(O)O-, -N($R_{Lb}$)C(O)N($R_{Lb}$)-, -N($R_{Lb}$)-, -S(O)$_2$-, -S(O)$_2$N($R_{Lb}$)-, -N($R_{Lb}$)S(O)$_2$-, -S(O)-, -S(O)N($R_{Lb}$)-, - N($R_{Lb}$)S(O)-; H atoms in said alkylene may optionally be substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$

alkoxy, $C_1$-$C_{10}$ haloalkoxy, amino, $C_1$-$C_{10}$ alkylamino; $R_{Lb}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$L_2$ is selected from: a single bond, $C_1$-$C_6$ alkylene, -($C_0$-$C_6$ alkylene)-$Q_2$-($C_0$-$C_6$ alkylene)-, $Q_2$ is selected from: - O-, -S-, -C(O)-, -C(O)O-, -OC(O)-, -C(O)N($R_{Lc}$)-, -N($R_{Lc}$)C(O)-, -N($R_{Lc}$)C(O)O-, -N($R_{Lc}$)C(O)N($R_{La}$)-, -N($R_{Lc}$)-, -S(O)$_2$-, -S(O)$_2$N($R_{Lc}$)-, -N($R_{Lc}$)S(O)$_2$-, -S(O)-, -S(O)N($R_{Lc}$)-, -N($R_{Lc}$)S(O)-; H atoms in said alkylene may optionally be substituted by the following groups: H, $C_1$-$C_{10}$ alkyl, $C_2$-$C_{10}$ alkenyl, $C_2$-$C_{10}$ alkynyl, halogen, cyano, nitro, azido, $C_1$-$C_{10}$ haloalkyl, hydroxy, $C_1$-$C_{10}$ alkoxy, $C_1$-$C_{10}$ haloalkoxy, amino, $C_1$-$C_{10}$ alkylamino; $R_{Lc}$ is selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

Y is selected from: H, -$NR_7R_8$, nitrogen-containing heterocyclyl, wherein said nitrogen-containing heterocyclyl may optionally be substituted by one or more $R_9$ groups, and $R_9$ is selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), - ($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl);

$R_7$ and $R_8$ are each independently selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, 4- to 10-membered heterocyclyl, 4- to 10-membered heterocyclylalkyl; wherein said $C_1$-$C_{10}$ alkyl, $C_2$-$C_{10}$ alkenyl, $C_2$-$C_{10}$ alkynyl, $C_3$-$C_{10}$ cycloalkyl, and 4- to 10-membered heterocyclyl may optionally be substituted by one or more groups selected from: H, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $C_6$-$C_{10}$ aryl, $C_7$-$C_{12}$ aralkyl, 4- to 10-membered heterocyclyl, 4- to 10-membered heterocyclylalkyl;

$R_4$ is one or more independent substituents on the benzene ring, each $R_4$ being independently selected from: H, halogen, $C_1$-$C_{10}$ alkyl, cyano, $C_1$-$C_{10}$ haloalkyl, $C_1$-$C_{10}$ haloalkoxy, $C_1$-$C_{10}$ cyanoalkyl, -$OR_{401}$, -C(O)$R_{401}$, - C(O)O$R_{401}$, -$NR_{402}$C(O)O$R_{401}$, -OC(O)$R_{401}$, -$NR_{402}SO_2R_{401}$, -$SO_2NR_{401}R_{402}$, -$NR_{402}$C(O)$R_{401}$, -C(O) $NR_{401}R_{402}$, - $NR_{401}R_{402}$, -($C_0$-$C_6$ alkylene)-$NR_{401}R_{402}$, -$SR_{401}$, -S(O)$R_{401}$, -S(O)$_2R_{401}$, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); wherein said $C_0$-$C_6$ alkylene, $C_1$-$C_{10}$ alkyl, $C_2$-$C_{10}$ alkenyl, $C_2$-$C_{10}$ alkynyl, $C_3$-$C_{10}$ cycloalkyl, $C_6$-$C_{10}$ aryl, and 4- to 10-membered heterocyclyl may optionally be substituted by one or more groups selected from: halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_{401}$ and $R_{402}$ are each independently selected from: H, $C_1$-$C_{10}$ alkyl, $C_3$-$C_{10}$ cycloalkyl, $C_3$-$C_{10}$ cycloalkylalkyl, $C_6$-$C_{10}$ aryl, $C_6$-$C_{10}$ aralkyl, 4- to 10-membered heterocyclyl, 4- to 10-membered heterocyclylalkyl;

$R_5$ is one or more independent substituents on the

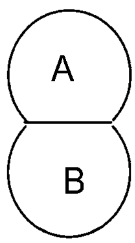

ring, each $R_5$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), - $SO_2$-($C_0$-$C_6$ alkylene)-(4- to 10-membered heterocyclyl), -S-($C_0$-$C_6$ alkylene)-(4- to 10-membered heterocyclyl), - O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl); $R_6$ is one or more independent substituents on Ring E, each $R_6$ is independently selected from: H, halogen, cyano, amino, hydroxy, $C_1$-$C_{10}$ alkyl, $C_1$-$C_{10}$ haloalkyl, $C_1$-$C_{10}$ alkoxy, $C_1$-$C_{10}$ haloalkoxy, $C_1$-$C_{10}$ alkylamino, $C_1$-$C_{10}$ cyanoalkyl, $C_1$-$C_{10}$ hydroxy-substituted alkyl, $C_1$-$C_{10}$ alkoxy-substituted alkyl, $C_1$-$C_{10}$ alkylamino-substituted alkyl, -($C_0$-$C_6$ alkylene)-($C_6$-$C_{10}$ aryl), -$SO_2$-($C_0$-$C_6$ alkylene)-($C_6$-$C_{10}$ aryl), -S-($C_0$-$C_6$ alkylene)-($C_6$-$C_{10}$ aryl), -O-($C_0$-$C_6$ alkylene)-($C_6$-$C_{10}$ aryl), -($C_0$-$C_6$ alkylene)-(4- to 10-membered heterocyclyl), -$SO_2$-($C_0$-$C_6$ alkylene)-(4- to 10-membered heterocyclyl), -S-($C_0$-$C_6$ alkylene)-(4- to 10-membered heterocyclyl), -O-($C_0$-$C_6$ alkylene)-(4- to 10-membered heterocyclyl), -($C_0$-$C_6$ alkylene)-($C_3$-$C_{10}$ cycloalkyl), -$SO_2$-($C_0$-$C_6$ alkylene)-($C_3$-$C_{10}$ cycloalkyl), -S-($C_0$-$C_6$ alkylene)-($C_3$-$C_{10}$ cycloalkyl), -O-($C_0$-$C_6$ alkylene)-($C_3$-$C_{10}$ cycloalkyl).

**28.** Use of the ophthalmic formulation according to any one of claims 1-12, 14-25, 27 or the ophthalmic formulation prepared by the method of claim 13 or 26 in the manufacture of a medicament for preventing and/or treating an ocular disease;

preferably, said ocular disease is selected from: diabetic retinopathy (including non-proliferative diabetic retinopathy, proliferative diabetic retinopathy, and diabetic macular edema); age-related macular degeneration (AMD) (including neovascular (wet/exudative) AMD, dry AMD, and geographic atrophy); pathological choroidal neovascularization (CNV) arising from any pathological mechanism (i.e., high myopia, trauma, sickle cell (anemia) disease; ocular histoplasmosis, angioid streaks, traumatic choroidal rupture, optic nerve drusen, and certain retinal dystrophies); pathological retinal neovascularization arising from any pathological mechanism (i.e., sickle cell retinopathy, Eales disease, ocular ischemic syndrome, internal carotid artery-cavernous sinus fistula, familial exudative vitreoretinopathy, hyperviscosity syndrome, idiopathic obliterative arteriolitis, birdshot retinochoroidopathy, retinal vasculitis, sarcoidosis, or toxoplasmosis); uveitis; retinal vein occlusion (central or branch); ocular trauma; surgically induced edema; surgically induced neovascularization; cystoid macular edema; ocular ischemia; retinopathy of prematurity; Coats' disease; sickle cell retinopathy and/or neovascular glaucoma;
more preferably, said ocular disease is diabetic retinopathy (including non-proliferative and proliferative types and diabetic macular edema) or retinal vein occlusion (central or branch).

## INTERNATIONAL SEARCH REPORT

International application No. **PCT/CN2024/124727**

**A. CLASSIFICATION OF SUBJECT MATTER**

A61K31/416(2006.01)i；C07D413/14(2006.01)i；C07D401/06(2006.01)i；C07D401/12(2006.01)i；C07D401/14(2006.01)i；C07D403/12(2006.01)i；C07D403/14(2006.01)i；C07D487/04(2006.01)i；C07D487/06(2006.01)i；A61K9/08(2006.01)i；A61K47/34(2017.01)i；A61K47/40(2006.01)i；A61K47/00(2006.01)i；A61P27/02(2006.01)i；A61P27/06(2006.01)i；A61P35/00(2006.01)i

According to International Patent Classification (IPC) or to both national classification and IPC

**B. FIELDS SEARCHED**

Minimum documentation searched (classification system followed by classification symbols)

IPC: A61K, C07D, A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, CNKI, ISI-WEB OF SCIENCE, PUBMED, REGISTRY, CAPLUS: 吲唑, 咪唑并吡啶, 吡唑并吡啶, 式II的structural formula search, 阿昔替尼, 眼, 滴眼剂, 聚乙二醇, 环糊精, 糖尿病性视网膜病变, Indazole, imidazo pyridine, pyrazolo pyridine, Formula II search, Axitinib, eye, eye drops, polyethylene glycol, cyclodextrin, diabetic retinopathy等

**C. DOCUMENTS CONSIDERED TO BE RELEVANT**

| Category* | Citation of document, with indication, where appropriate, of the relevant passages | Relevant to claim No. |
|---|---|---|
| PX | WO 2024032584 A1 (BEYOND THERAPEUTICS CO., LTD.) 15 February 2024 (2024-02-15)<br>description, page 11, line 6-page 32, line 4 and pages 182-185, table 8, and embodiments 4 and 6 | 1-28 |
| X | CN 1374950 A (AGOURON PHARMACETUICALS, INC.) 16 October 2002 (2002-10-16)<br>description, page 6, paragraph 3, page 8, paragraph 2-page 12, page 22, paragraph 2, page 23, line 33-page 24, line 5, page 323, paragraph 1-page 326, table 7, and page 327, embodiment 3 | 1-10, 12-23, 25-28 |
| X | US 2023263907 A1 (OCULIS SA) 24 August 2023 (2023-08-24)<br>description, paragraphs 0009-0014, 0068-0070, 0087, and 0098-0122, page 16, tables 14 and 15, and embodiments 13 and 14 | 1-10, 12-23, 25-28 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2024** | **15 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No. **PCT/CN2024/124727**

**C. DOCUMENTS CONSIDERED TO BE RELEVANT**

| Category* | Citation of document, with indication, where appropriate, of the relevant passages | Relevant to claim No. |
|---|---|---|
| X | CN 116251186 A (CHENGDU RUIMU BIOMEDICAL TECHNOLOGY CO., LTD.) 13 June 2023 (2023-06-13) claims 1-33, description, paragraphs 0098-0181, and in particular, embodiments 4, 5 and 9 in table 1 | 1-10, 12-23, 25-28 |
| X | CN 106458920 A (SENJU PHARMACEUTICAL CO., LTD.) 22 February 2017 (2017-02-22) description, paragraphs 0026-0047, 0081-0084, 0091-0100, 0105, 0660-0662, 0764-0766, 0807-0809, 0904-0906, and 0918-0975 | 1-28 |

Form PCT/ISA/210 (second sheet) (July 2022)

**INTERNATIONAL SEARCH REPORT**
**Information on patent family members**

International application No.
**PCT/CN2024/124727**

| Patent document cited in search report | Publication date (day/month/year) | Patent family member(s) | Publication date (day/month/year) |
|---|---|---|---|
| WO 2024032584 A1 | 15 February 2024 | None | |
| CN 1374950 A | 16 October 2002 | CY 2013009 I1 | 13 April 2016 |
| | | JP 2006348043 A | 28 December 2006 |
| | | JP 3969669 B2 | 05 September 2007 |
| | | BE 2013C015I2 | 21 May 2019 |
| | | DK 1614683 T3 | 25 March 2008 |
| | | TNSN 00146 A1 | 10 November 2005 |
| | | EP 1614683 A1 | 11 January 2006 |
| | | EP 1614683 B1 | 21 November 2007 |
| | | DE 60036879 D1 | 06 December 2007 |
| | | DE 60036879 T2 | 14 February 2008 |
| | | CZ 20014634 A3 | 11 September 2002 |
| | | CZ 301667 B6 | 19 May 2010 |
| | | WO 0102369 A2 | 11 January 2001 |
| | | WO 0102369 A3 | 25 April 2002 |
| | | DE 60037211 D1 | 03 January 2008 |
| | | DE 60037211 T2 | 11 December 2008 |
| | | NO 2013004 I1 | 18 March 2013 |
| | | NO 2013004 I2 | 02 June 2014 |
| | | LU 92154 I2 | 22 April 2013 |
| | | CY 1107148 T1 | 25 January 2012 |
| | | HK 1085470 A1 | 25 August 2006 |
| | | EE 200100717 A | 17 February 2003 |
| | | EE 05585 B1 | 15 October 2012 |
| | | UY 26231 A1 | 31 January 2001 |
| | | PL 355757 A1 | 17 May 2004 |
| | | PL 212108 B1 | 31 August 2012 |
| | | NZ 516676 A | 26 September 2003 |
| | | BG 106380 A | 30 September 2002 |
| | | BG 66070 B1 | 31 January 2011 |
| | | SK 19252001 A3 | 06 November 2002 |
| | | SK 286936 B6 | 06 July 2009 |
| | | IL 146710 A0 | 25 July 2002 |
| | | NO 20060596 L | 01 March 2002 |
| | | KR 20020027379 A | 13 April 2002 |
| | | KR 100529639 B1 | 16 January 2006 |
| | | AP 200202392 A0 | 31 March 2002 |
| | | AP 2002002392 A0 | 01 November 2005 |
| | | MEP 45108 A | 10 May 2011 |
| | | MY 139999 A | 30 November 2009 |
| | | UA 66933 C2 | 15 June 2004 |
| | | IS 6207 A | 19 December 2001 |
| | | IS 2791 B | 15 June 2012 |
| | | CR 6517 A | 21 September 2006 |
| | | NO 20015797 D0 | 28 November 2001 |
| | | NO 20015797 L | 01 March 2002 |
| | | NO 322507 B1 | 16 October 2006 |
| | | HRP 20020109 A2 | 31 December 2003 |
| | | HRP 20020109 B1 | 31 July 2008 |
| | | AR 035554 A1 | 16 June 2004 |

Form PCT/ISA/210 (patent family annex) (July 2022)

**INTERNATIONAL SEARCH REPORT**
**Information on patent family members**

International application No.
**PCT/CN2024/124727**

| Patent document cited in search report | | | Publication date (day/month/year) | Patent family member(s) | | | Publication date (day/month/year) |
|---|---|---|---|---|---|---|---|
| | | | | HK | 1048813 | A1 | 17 April 2003 |
| | | | | PT | 1614683 | E | 24 January 2008 |
| | | | | DZ | 3191 | A1 | 11 January 2001 |
| | | | | ATE | 376543 | T1 | 15 November 2007 |
| | | | | SI | 1614683 | T1 | 29 February 2008 |
| | | | | CA | 2383630 | A1 | 11 January 2001 |
| | | | | CA | 2383630 | C | 18 November 2008 |
| | | | | FR | 1G0010 | I1 | 22 March 2013 |
| | | | | FR | 1G0010 | I2 | 16 August 2013 |
| | | | | AU | 5785200 | A | 22 January 2001 |
| | | | | AU | 777701 | B2 | 28 October 2004 |
| | | | | MA | 26803 | A1 | 20 December 2004 |
| | | | | PT | 1218348 | E | 14 December 2007 |
| | | | | SI | 1218348 | T1 | 29 February 2008 |
| | | | | GT | 200000107 | A | 21 December 2001 |
| | | | | ES | 2293906 | T3 | 01 April 2008 |
| | | | | MXPA | 01012795 | A | 02 September 2002 |
| | | | | EA | 200200120 | A1 | 29 August 2002 |
| | | | | EA | 004460 | B1 | 29 April 2004 |
| | | | | HUP | 0202490 | A2 | 28 November 2002 |
| | | | | HUP | 0202490 | A3 | 28 January 2003 |
| | | | | HU | 228502 | B1 | 28 March 2013 |
| | | | | EP | 1218348 | A2 | 03 July 2002 |
| | | | | EP | 1218348 | B1 | 24 October 2007 |
| | | | | CY | 1107147 | T1 | 24 October 2012 |
| | | | | HK | 1065037 | A1 | 08 February 2005 |
| | | | | SV | 2002000121 | A | 02 December 2002 |
| | | | | MY | 137622 | A | 27 February 2009 |
| | | | | OA | 11980 | A | 18 April 2006 |
| | | | | CO | 5190686 | A1 | 29 August 2002 |
| | | | | ES | 2296014 | T3 | 16 April 2008 |
| | | | | AR | 065937 | A2 | 15 July 2009 |
| | | | | YU | 92901 | A | 03 September 2004 |
| | | | | RS | 50339 | B | 10 November 2009 |
| | | | | GEP | 20063885 | B | 10 August 2006 |
| | | | | JO | 2319 | B | 12 September 2005 |
| | | | | JP | 2003503481 | A | 28 January 2003 |
| | | | | JP | 3878849 | B2 | 07 February 2007 |
| | | | | PE | 20010306 | A1 | 29 March 2001 |
| | | | | CR | 10194 | A | 30 September 2008 |
| | | | | BR | 0012352 | A | 14 May 2002 |
| | | | | BRPI | 0012352 | B1 | 16 August 2016 |
| | | | | BRPI | 0012352 | B8 | 25 May 2021 |
| | | | | DK | 1218348 | T3 | 25 February 2008 |
| | | | | ZA | 200110061 | B | 06 February 2003 |
| | | | | PA | 8498001 | A1 | 26 August 2002 |
| | | | | JO | 2319 | B1 | 12 September 2005 |
| | | | | AP | 200202392 | D0 | 31 March 2002 |
| US | 2023263907 | A1 | 24 August 2023 | WO | 2022003037 | A1 | 06 January 2022 |
| | | | | TW | 202216119 | A | 01 May 2022 |

Form PCT/ISA/210 (patent family annex) (July 2022)

**INTERNATIONAL SEARCH REPORT**
**Information on patent family members**

International application No.
**PCT/CN2024/124727**

| Patent document cited in search report | Publication date (day/month/year) | Patent family member(s) | Publication date (day/month/year) |
|---|---|---|---|
| | | EP 4171507 A1 | 03 May 2023 |
| CN 116251186 A | 13 June 2023 | EP 4424302 A1 | 04 September 2024 |
| | | WO 2023103835 A1 | 15 June 2023 |
| CN 106458920 A | 22 February 2017 | ES 2647262 T3 | 20 December 2017 |
| | | US 2017096400 A1 | 06 April 2017 |
| | | US 9617222 B1 | 11 April 2017 |
| | | JPWO 2015152117 A1 | 13 April 2017 |
| | | JP 6407257 B2 | 17 October 2018 |
| | | WO 2015152117 A1 | 08 October 2015 |
| | | CA 2942888 A1 | 08 October 2015 |
| | | CA 2942888 C | 07 September 2021 |
| | | RU 2016142578 A | 07 May 2018 |
| | | RU 2016142578 A3 | 20 September 2018 |
| | | EP 3127900 A1 | 08 February 2017 |
| | | EP 3127900 A4 | 22 February 2017 |
| | | EP 3127900 B1 | 18 October 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2024032584 A1


### Non-patent literature cited in the description


- **P. JANSOOK et al.** *Eur J Pharm Biopharm*, 2010, vol. 76, 208-214
- **T. J..ARVINEN et al.** *J Ocul Pharmacol Ther*, 1995, vol. 11, 95-106
- **P. JARHO et al.** *J Pharm Pharmacol*, 1996, vol. 48, 264-270
- **P. SUHONEN et al.** *Pharmaceutical Research*, 1995, vol. 12, 529-533
- **P. JANSOOK**. *Eur J Pharm Biopharm*, 2010, vol. 76, 208-214
- **T. J"ARVINEN**. *J Ocul Pharmacol Ther*, 1995, vol. 11, 95-106
- **P. JARHO**. *J Pharm Pharmacol*, 1996, vol. 48, 264-270
- **P. SUHONEN**. *Pharmaceutical Research*, 1995, vol. 12, 529-533